# EUROPEAN PATENT SPECIFICATION

(11) **EP 1 399 406 B1**
(45) Date of publication and mention of the grant of the patent: **10.08.2005**
(21) Application number: 01272663.4
(22) Date of filing: 24.12.2001
(51) Int. Cl.: C07C 49/747, C07C 251/44, C07C 323/17, C07C 43/315, C07C 39/42, C07C 39/17, C07D 339/06, C07D 317/72, C07D 339/08, C07D 307/94, C07D 311/96, A61K 31/122, A61K 31/045, A61K 31/557, A61K 31/385, A61K 31/39, A61P 19/00

(54) **NOVEL ESTROGEN RECEPTOR LIGANDS AND METHODS I**
ESTROGENREZEPTORLIGANDEN UND VERFAHREN
NOUVEAUX LIGANDS DE RECEPTEURS D'OESTROGENES ET PROCEDES I

(30) Priority: 04.01.2001 GB 0100163; 04.01.2001 GB 0100164; 15.03.2001 GB 0106434
(43) Date of publication of application: 24.03.2004
(73) Proprietor: KARO BIO AB, 141 57 Huddinge (SE); Merck & Co., Inc., Rahway, NJ 07065-0907 (US)
(72) Inventor: KOEHLER, Konrad, S-141 50 Huddinge (SE); HENSSEN, Cecilia, S-141 33 Huddinge (SE); NILSSON, Marita, S-141 37 Huddinge (SE); GILLNER, Mikael, S-116 31 Stockholm (SE); LIU, Ye, S-146 36 Tullinge (SE); SANIN, Andrei, S-143 41 Varby (SE); WILKENING, Robert, R., Maplewood, NJ 07040 (US); RATCLIFFE, Ronald, W., Matawan, NJ 07747 (US); WENSBO, David, S-14771 Grodinge (SE)
(74) Representative: Bannerman, David Gardner
(86) International application number: PCT/EP2001/015230
(87) International publication number: WO 2002/053522

(56) References cited:
- EP-A- 0 606 661
- EP-A- 0 873 992
- WO-A-01/82923
- WO-A-99/66915
- JOVANOVIC-SANTA, SUZANA ET AL: "Synthesis and biological activity of new 16,17-secoestrone derivatives" COLLECTION OF CZECHOSLOVAK CHEMICAL COMMUNICATIONS (2000), 65(1), 77-82 , January 2000 (2000-01), XP002212976
- MEYERS C.Y. ET AL: 'Activities of a non-classical estrogen, Z-bis-dehydrodoisynolic acid with ER(alpha) and ER(beta)' JOURNAL OF STEROID BIOCHEMISTRY AND MOLECULAR BIOLOGY vol. 82, 2002, pages 33 - 44

## Description

### Field of Invention

This invention relates to novel compounds which are estrogen receptor ligands and are preferably selective for either the estrogen receptor α or β isoforms, to methods of preparing such compounds and to methods for using such compounds such as for estrogen hormone replacement therapy and for diseases modulated by the estrogen receptor such as osteoporosis, elevated blood triglyercide levels, atherosclerosis, endometriosis, cognitive disorders, urinary incontinence, autoimmune disease, and cancer of the lung; colon, breast, uterus and prostate.

### Background of Invention

The estrogen receptor (ER) is a ligand activated mammalian transcription factor involved in the up and down regulation of gene expression. The natural hormone for the estrogen receptor is β-17-estradiol (E2) and closely related metabolites.. Binding of estradiol to the estrogen receptor causes a dimerization of the receptor and the dimer in turn binds to estrogen response elements (ERE's) on DNA. The ER/DNA complex recruits other transcription factors responsible for the transcription of DNA downstream from the ERE into mRNA which is eventually is translated into protein. Alternatively the interaction of ER with DNA may be indirect through the intermediacy of other transcription factors, most notably fos and jun. Since the expression of a large number of genes is regulated by the estrogen receptor and since the estrogen receptor is expressed in many cell types, modulation of the estrogen receptor through binding of either natural hormones or synthetic ER ligands can have profound effects on the physiology and pathophysiology of the organism.

Estrogens are critical for sexual development in females. In addition, estrogens play an important role in maintaining bone density, regulation of blood lipid levels, and appear to have neuroprotective effects. Consequently decreased estrogen production in post-menopausal women is associated with a number of diseases such as osteoporosis, atherosclerosis, and cognitive disorders. Conversely certain types of proliferative diseases such as breast and uterine cancer and endometriosis are stimulated by estrogens and therefore antiestrogens (*i*.*e*., estrogen antagonists) have utility in the prevention and treatment of these types of disorders.

In addition to women suffering from breast cancer, men afflicted with prostatic cancer can also benefit from anti-estrogen compounds. Prostatic cancer is often endocrine sensitive and androgen stimulation fosters tumor growth, while androgen suppression retards tumor growth. The administration of estrogen is helpful in the treatment and control of prostatic cancer because estrogen administration lowers the level of gonadotropin and consequently androgen levels.

The use of natural and synthetic estrogens in hormone replacement therapy has been shown to markedly decrease the risk of osteoporosis. In addition, there is evidence that hormone replacement therapy has cardiovascular and neuroprotective benefits. However hormone replacement therapy is also associated with an increase risk of breast and uterine cancer. It is known that certain types of synthetic ER ligands display a mixed agonist/antagonist profile of activity showing agonist activity in some tissues and antagonist activity in other tissues. Such ligands are referred to as selective estrogen receptor modulators (SERMS). For example tamoxifen and raloxifene are known to be agonists in bone (and therefore prevent osteoporosis) while displaying antagonistic properties in breast (and therefore lowers the risk of breast cancer). However neither tamoxifen nor raloxifene is ideal for hormone replacement therapy as neither of these SERMS are as efficacious as estradiol in preventing bone loss. Furthermore the use of tamoxifen is still associated with an increased risk of uterine cancer and both tamoxifen and raloxifene are known to aggravate hot flashes.

Historically it has been believed there was only one estrogen receptor. However recently a second subtype (ER-β) has been discovered. While both the "classical" ER-α and the more recently discovered ER-β are widely distributed in different tissues, they nevertheless display markedly different cell type and tissue distributions. Therefore synthetic ligands which are either ER-α or ER-β selective may preserve the beneficial effects of estrogen while reducing the risk of undesirable side effects.

What is needed in the art are compounds that can produce the same positive responses as estrogen replacement therapy without the negative side effects. Also needed are estrogen-like compounds that exert selective effects on different tissues of the body.

The compounds of the instant invention are ligands for estrogen receptors and as such may be useful for treatment or prevention of a variety of conditions related to estrogen functioning including bone loss, bone fractures, osteoporosis, cartilage degeneration, endometriosis, uterine fibroid disease, hot flashes, increased levels of LDL cholesterol, cardiovascular disease, impairment of cognitive function, cerebral degenerative disorders, restinosis, gynecomastia, vascular smooth muscle cell proliferation, obesity, incontinence, autoimmune disease and cancer of the lung, colon, breast, uterus, and prostate.

### Description of Invention

In accordance with the present invention, compounds are provided which are estrogen receptor ligands and have the general formula **I, II,** or **III** 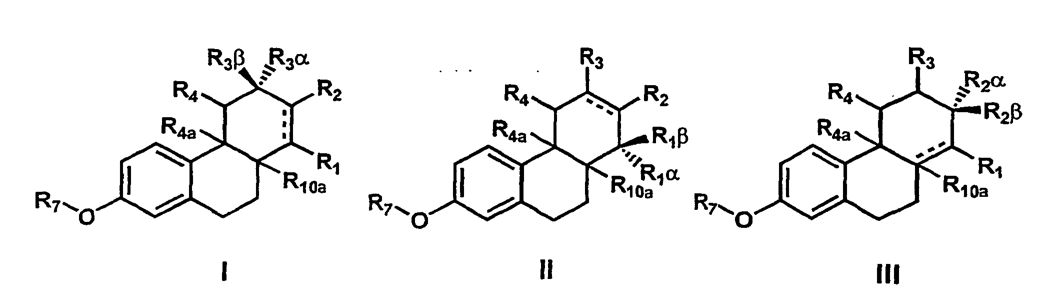 wherein the bond between the C1 and C2 carbon atoms (of compounds of general formula **I**) or the bond between C2 and C3 (of compounds of general formula **II**) or the bond between C1 and C10 (of compounds of general formula **III**) is either a single or double bond;
R₁ (of compounds of general formula **I** or **III**) is an R^{A} group other than a phenyl group;
R^{A} is selected from the group hydrogen, alkyl, alkenyl, alkynyl, cycloalkyl, cycloalkylalkyl, cycloalkenyl, aryl or arylalkyl;
R₁α and R₁β (of compounds of general formula **II**) are the same or are different and each is an R^{A} group;
R₂ (of compounds of general formula **I** or **II**) is a hydroxyl or R^{A} group or is a hydroxyalkyl or aminoalkyl group of 1 to 2 carbon atoms;
R₃α and R₃β (of compounds of general formula **I**) may together be a single oxygen or sulfur atom; or R₃α and R₃β may together be a single nitrogen atom which in turn is bonded to a group selected from R^{A} or OR^{A}; or R₃α and R₃β may together be a single carbon atom (*i.e.,* an exo methylene carbon atom) which in turn is bonded to two R^{A} groups which may be the same or are different; or R₃α and R₃β may be the same or are different and each may be selected from R^{A}, OR^{A}, SR^{A}, or N(R^{A})₂ wherein the individual R^{A} groups may be the same or are different and may be taken together with any attached and intervening atoms to form a 3-8 membered ring;
R₂α and R₂β (of compounds of general formula **III**) may together be a single oxygen or sulfur atom; or R₂α and R₂β may together be a single nitrogen atom which in turn is bonded to a group selected from R^{A} or OR^{A}; or R₂α and R₂β may together be a single carbon atom which in turn is bonded to two R^{A} groups which may be the same or are different; or R₂α and R₂β may be the same or are different and each may be selected from hydroxyalkyl, aminoalkyl, R^{A}, OR^{A}, SR^{A}, or N(R^{A})₂ wherein the individual R^{A} groups may be the same or are different and may optionally be taken together with any attached and intervening atoms to form a 3-8 membered ring;
R₃ (of compounds of general formula **II** or **III**) is an R^{A} group;
R₄ is an R^{A} group;
R₄ₐ is a hydrogen atom or a methyl or ethyl group in compounds of formula **I** or **II,** or a methyl or ethyl group in compounds of formula **III**.;
R₇ is a hydrogen atom or a linear or branched alkyl or cycloalkyl group or acyl group of 1 to 4 carbon atoms;
R₁₀ₐ is an R^{A} group;
with the proviso that not all of R₁, R₂, R₃ and R₄ are hydrogen in compounds of formula **I** or **II**, and that R₄ is not hydrogen in compounds of formula **III**.
and pharmaceutically acceptable salts and stereoisomers thereof.

### Detailed Description of Invention

The present invention relates to compounds useful as estrogen receptor modulators and have the general formula **I**, **II** or **III** as described above.

One embodiment of the present invention relates to compounds according to the general formula **I**, **II** or **III** wherein the substituents R₄ₐ and R₁₀ₐ have a trans relative stereochemistry.

Another embodiment of the present invention relates to compounds according to the general formula **I** or **III**, wherein the substituent R₁ is R^{B} and R^{B} is selected from hydrogen, *n*-propyl, 2-propenyl, 2-propynyl, *n*-butyl, 2-butenyl, 3-butenyl, 2-butynyl, 3-butynyl, *n*-pentyl, 3-methylbutyl, 3-methyl-1-butenyl, 3-methyl-2-butenyl, 3-methylpentyl, 3-ethylpentyl, cyclopropylethyl, cyclopentylethyl, cyclohexylethyl, cycloheptylethyl, cyclopropylpropyl, cyclopentylpropyl, benzyl, and phenethyl.

Another embodiment of the present invention relates to compounds according to the general formula **II**, wherein R₁α is R^{B} (where R^{B} is defined as above) and R₁β is a hydrogen atom or methyl group.

Another embodiment of the present invention relates to compounds according to the general formula **I, II** or **III** wherein R₁₀ₐ is R^{B}.

Another embodiment of the present invention relates to compounds according to the general formula **I** or **II**, wherein R₂ is a hydrogen atom or a methyl, ethyl, or hydroxymethyl group.

Another embodiment of the present invention relates to compounds according to the general formula **III**, wherein R₂α and R₂β may be the same or are different and each may be selected from hydroxyalkyl, R^{A}, OR^{A}, or SR^{A} where the individual R^{A} groups (where R^{A} is defined as above) may be the same or are different and may optionally be taken together with any attached and intervening atoms to form a 3-8 membered ring.

Another embodiment of the present invention relates to compounds with the general formula **I**, wherein R₃α and R₃β may be the same or are different and each may be selected from R^{A}, OR^{A}, or SR^{A} where the individual R^{A} groups (where R^{A} is defined as above) may be the same or are different and may optionally be taken together with any attached and intervening atoms to form a 3-8 membered ring.

Another embodiment of the present invention relates to compounds with the general formula **I, II** or **III** wherein R₄ is a hydrogen atom or methyl or ethyl group.

Another embodiment of the present invention relates to compounds with the general formula **I, II** or **III** wherein R₇ is hydrogen atom or an acyl group of 1 to 4 carbon atoms.

Another embodiment of the present invention relates to compounds with the general formula **I** or **III** wherein R₁ is selected from the group hydrogen, methyl, or ethyl and R₁₀ₐ is R^{B}.

Another embodiment of the present invention relates to compounds with the general formula **I** or **III** wherein R₁ is R^{B} and R₁₀ₐ is selected from hydrogen, methyl, or ethyl.

Another embodiment of the present invention relates to compounds with the general formula **III** wherein R₂α and R₂β may be the same or are different and each may be selected from hydroxyalkyl, R^{A}, OR^{A}, or SR^{A} where the individual R^{A} groups (where R^{A} is defined as above) may be the same or are different and may optionally be taken together with any attached and intervening atoms to form a 3-8 membered ring.

Another embodiment of the present invention relates to compounds with the general formula **II**, wherein R₁α is R^{B} and R₁₀ₐ is selected from hydrogen, methyl, or ethyl.

Another embodiment of the present invention relates to compounds with the general formula **II**, wherein R₁α is selected from hydrogen, methyl, or ethyl and R₁₀ₐ is R^{B}.

Compounds of the invention include, but are not limited to, the following:
*(rac)-(4aS,10aS)*-7-hydroxy-1,4,4a,9,10,10a-hexahydro-*2H*-phenanthren-3-one (**E1**);
*(rac)-(4aS,10aS)*-3,3-ethanediyldimercapto-7-hydroxy-1,2,3,4,4a,9,10,10a-octahydro-phena nthrene (**E2**);
*(rac)-(4aR,10aS)*-7-hydroxy-10a-methyl-4a,9,10,10a-tetrahydro-*4H*-phenanthren-3-one (**E3a**);
*(rac)-(4aS,10aS)*-7-hydroxy-10a-methyl-4a,9,10,10a-tetrahydro-*4H*-phenanthren-3-one (**E3b**);
*(rac)-(4aS,10aS)*-10a-butyl-7-hydroxy-4a,9,10,10a-tetrahydro-*4H*-phenanthren-3-one (**E4**);
*(rac)-(1S,4aS,10aS)*-1-butyl-7-hydroxy-1,4,4a,9,10,10a-hexahydro-*2H*-phenanthren-3-one (**E5a**);
*(rac)-(1R,4aS,10aS)*-1-butyl-7-hydroxy-1,4,4a,9,10,10a-hexahydro-*2H*-phenanthren-3-one (**E5b**);
*(rac)-(1S,4aS,10aS)*-1-butyl-3,3-ethanediyldimercapto-7-hydroxy-1,2,3,4,4a,9,10,10a-octahydro-phenanthrene **(E6a);**
*(rac)-(1R,4aS,10aS)*-1-butyl-3,3-ethanediyldimercapto-7-hydroxy-1,2,3,4,4a,9,10,10a-octahydro-phenanthrene **(E6b);**
*(rac)-(1S,4aS,10aS)*-1-butyl-7-hydroxy-10a-methyl-1,4,4a,9,10,10a-hexahydro-*2H*-phenant hren-3-one (**E7**);
*(rac)-(1S,4aS, 10aS)*-1-butyl-3,3-ethanediyldimercapto-7-hydroxy-10a-methyl-1,2,3,4,4a,9, 10,10a-octahydro-phenanthrene **(E8);**
*(rac)-(1S,4aS,10aS)*-1-butyl-7-hydroxy-10a-methyl-1,2,3,4,4a,9,10,10a-octahydro-phenanthrene **(E9);**
*(rac)-(4aS,10aR)*-10a-ethyl-7-hydroay-3-methyl-4a,9,10,10a-tetrahydro-*4H*-phenanthren-1-one (**E10a**);
*(rac)-(4aR,10aR)*-10a-ethyl-7-hydroxy-3-methyl-4a,9,10,10a-tetrahydro-*4H*-phenanthren-1-one (**E10b**);
*(rac)-(1R,2S,4aS,10aS)*-1-butyl-7-hydroxy-2-methyl-1,4,4a,9,10,10a-hexahydro-*2H*-phenanthren-3-one (**E11a**);
*(rac)-(1R,2R,4aS,10aS)*-1-butyl-7-hydroxy-2-methyl-1,4,4a,9,10,10a-hexahydro-*2H*-phenanthren-3-one(**E11b**);
*(rac)-(1s,4R,4aR,10aS)*-1-butyl-7-hydroxy-4-methyl-1,4,4a,9,10,10a-hexahydro-*2H*-phenanthren-3-one (**E11c**);
*(rac)-(1R,2R,4aS,10aS)*-1-butyl-3,3-ethanediyldimercapto-7-hydroxy-2-methyl-1,2,3,4,4a,9, 10,10a-octahydro-phenanthrene (**E12a**);
*(rac)-(1R,2S,4aS,10aS)*-1-butyl-3,3-ethanediyldimercapto-7-hydroxy-2-methyl-1,2,3,4,4a,9, 10,10a-octahydro-phenanthrene (**E12b**);
*(rac)-(1S,4aS, 10aS)*-7-hydroxy-10a-methyl-1-(3-methyl-butyl)-1,4,4a,9,10,10a-hexahydro-*2H*-phenanthren-3-one **(E13);**
*(rac)-(1S,4aS,10aS)*-3*,*3-ethanediyldimercapto-7-hydroxy-1-(3-methyl-butyl)-10a-methyl-1, 2,3,4,4a,9,10,10a-octahydro-phenanthrene (**E14**);
*(rac)-(1S,4aS,10aS)*-7-hydroxy-10a-methyl-1-phenethyl-1,4,4a,9,10,10a-hexahydro-*2H*-phenanthren-3-one (**E15**);
*(rac)-(1S,2S,4aS,10aS)*-7-hydroxy-2,10a-dimethyl-1-(3-methyl-butyl)-1,4,4a,9,10,10a-hexahydro-*2H*-phenanthren-3-one (**E16a**);
*(rac)-(1S,2R, 4aS,10aS)*-7-hydroxy-2,10a-dimethyl-1-(3-methyl-butyl)-1,4,4a,9,10,10a-hexahydro-*2H*-phenanthren-3-one (**E16b**);
*(rac)-(4bS,8S,8aS)*-6,6-dimethoxy-8a-methyl-8-(3-methyl-butyl)-4b,5,6,7,8,8a,9,10-octahydro-phenanthren-2-ol (**E17a**);
*(rac)-(1S,4aS,10aS)*-7-hydroxy-10a-methyl-1-(3-methyl-butyl)-1,4,4a,9,10,10a-hexahydro-*2H*-phenanthren-3-one (**E17b**);
*(rac)-(1S,4aS,10aS)*-3,3-ethanediyldioxy-7-hydroxy-1-(3-methyl-butyl)-10a-methyl-1,2,3,4, 4a,9,10, 10a-octahydro-phenanthrene (**E18**);
*(rac)-(4bS,8R,8aS)*-8a-methyl-8-(3-methyl-butyl)-4b,5,6,7,8,8a,9,10-octahydro-phenanthren-2-ol (**E19a**);
*(rac)-(4bS,6R,8S,8aS)*-6-ethylsulfanyl-8a-methyl-8-(3-methyl-butyl)-4b,5,6,7,8,8a,9,10-octahydro-phenanthren-2-ol (**E19b**);
*(rac)-(1S,4aS,10aS)*-3,3-(propane-1,3-diyldimercapto)-7-hydroxy-1-(3-methyl-butyl)-10a-methyl-1,2,3,4,4a,9,10,10a-octahydro-phenanthrene (**E20**);
*(rac)-(4aS,10aS)*-7-hydroxy-10a-methyl-1,4,4a,9,10,10a-hexahydro-2H-phenanthren-3-one (**E21**);
*(rac)-(4aS,10aS)*-3,3-ethanediyldimercapto-7-hydroay-10a-methyl-1,2,3,4,4a,9,10,10a-octahydro-phenanthrene (**E22**);
*(rac)-(4aS,10aS)*-10a-ethyl-7-hydroxy-1,4,4a,9,10,10a-hexahydro-*2H*-phenanthren-3-one (**E23**);
*(rac)-(4aS,10aS)*-3,3-ethanediyldimercapto-10a-ethyl-7-hydroxy-1,2,3,4,4a,9,10,10a-octahydro-phenanthrene (**E24**);
*(rac)-(1S,4aS,10aS)*-1-(3-methyl-butyl)-10a-ethyl-7-hydroxy-1,4,4a,9,10,10a-hexahydro-*2H*-phenanthren-3-one (**E26**);
*(1S,4aS,10aS)*-7-hydroxy-10a-methyl-1-(3-methyl-butyl)-1,4,4a,9,10,10a-hexahydro-*2H*-phenanthren-3-one (**E27**);
*(1R,4aRS,10aR)*-7-benzyloxy-10a-methyl-1-(3-methyl-butyl)-1,4,4a,9,10,10a-hexahydro-*2H*-phenanthren-3-one (**E28**);
*(rac)-(1S,4aS, 10aS)*-1-(3-methyl-butyl)-3,3-ethanediyldimercapto-7-hydroxy-1,4,4a,9,10,10a-octahydrophenanthrene **(E29);**
*(rac)-(4aR,10aR)*-7-hydroxy-4a,10a-dimethyl-3,4,4a,9,10,10a-hexahydro-*1H*-phenanthren-2-one **(E30);**
*(rac)-(1S,4aS,10aS)*-7-hydroxy-10a-methyl-4-phenyl-1,4,4a,9,10,10a-hexahydro-*2H*-phenanthren-3-one (**E31**); *(rac)-(4R,4aS, 10aS)*-3,3-ethanediyldimercapto-7-hydroxy-10a-methyl-4-phenyl-1,4,4a,9,10, 10a-octahydro-phenanthrene (**E32**);
*(rac)-(1S,4aS, 10aS)*-3,3-(ethane-1,2-diyldimercapto)-7-hydroxy-1-(2-phenylethyl)-10a-methyl-1,2,3,4,4a,9,10,10a-octahydro-phenanthrene (**E33**);
*(rac)-(1S*,*4aS,10aS)*-3,3-ethanediyldioxy-7-hydroxy-10a-methyl-1, 2,3,4,4a,9,10,10a-octahydro-phenanthrene (**E34a**);
*(rac)-(1S,4aS,10aR)*-3,3-ethanediyldioxy-7-hydroxy-10a-methyl-1, 2,3,4,4a,9,10,10a-octahydro-phenanthrene (**E34b**);
*(rac)-(3S,4aS,10aS)*-7-hydroxy-3-pentyl-10a-methyl-1, 2,3,4,4a,9,1,10a-octahydro-phenanthrene (**E35a**);
*(rac)-(3R,4aS,10aS)*-7-hydroxy-3-pentyl-10a-methyl-1, 2,3,4,4a,9,1,10a-octahydro-phenanthrene **(E35b);**
*(rac)-(1S,2R,4aS,10aS)*-2*,*10a-dimethyl-3,3-(ethane-1,2-diyldimercapto)-7-hydroxy-1-(3-methyl-butyl-1,2,3,4,4a,9,10,10a-octahydro-phenanthrene (**E36**);
*(rac)-(1S,4aS,10aS)*-7-Hydroxy-1-(3'-methyl-butyl)-1,4,4a,9,10,10a-hexahydro-*2H*-phenanthren-3-one (**E37**);
*(rac)-(4aS,10aS)*-3,3-(propane-1,3-diyldimercapto)-7-hydroxy-1,2,3,4,4a,9,10,10a-octahydro-phenanthrene **(E38);**
*(rac)-(1S,4S,4aS,10aS)*-7-Hydroxy-1-butyl-4,10a-dimethyl-1,4,4a,9,10,10a-hexahydro-*2H*-phenanthren-3-one **(E39);**
*(rac)-(4S,4aS,10aS)*-3,3-ethanediyldimercapto-7-hydroxy-4, 10a-dimethyl-1,2,3,4,4a,9,10,10a-octahydro-phenanthrene **(E40);**
*(rac)-(4S,4aS,10aS)*-7-Hydroxy-4-benzyl-10a-methyl-1,4,4a,9,10,10a-hexahydro-*2H*-phenanthren-3-one (**E41a**);
*(rac)-(4aS,10aS)*-7-hydroxy-4,4-dibenzyl-10a-methyl-1,4,4a,9,10,10a-hexahydro-*2H*-phenanthren-3-one (**E41b**);
*(rac)-(4aS,10aS)*-7-hydroxy-10a-methyl-3,3-methylene-1,2,3,4,4a,9,10,10a-octahydrophenanthrene **(E42);**
*(rac)-(4aS,10aS)*-3,3-ethanediyl-7-hydroxy-10a-methyl-1,2,3,4,4a,9,10,10a-octahydrophenanthrene **(E43);**
*(rac)-(3R,4aS,10aS)*-1',2',3',4'-tetrachloro-7-hydroxy-10a-methyl-1,2,3,4,4a,9,10,10a-octahydrospiro[phenanthrene-3,6'-cyclohexane]-1',3'-diene **(E44);**
*(rac)-(3S,4aS,10aS)*-3,7-dihydroxy-10a-methyl-3-[1-(phenylthio)cyclopropyl]-1,2,3,4,4a,9, 10,10a-octahydrophenanthrene **(E45);**
*(rac)-(3S,4aS,10aS)*-7-hydroxy-10a-methyl-1,2,3,4,4a,9,10,10a-octahydrospiro[phenanthrene-3,2'-cyclobutane]-1'-one **(E46a);**
*(rac)-(3R,4aS,10aS)*-7-hydroxy-10a-methyl-1,2,3,4,4a,9,10,10a-octahydrospiro[phenanthrene-3,2'-cyclobutane]-1'-one **(E46b);**
*(rac)-(3S,4aS,10aS)*-1',1'-ethanediyldimercapto-7-hydroxy-10a-methyl-1,2,3,4,4a,9,10,10a-octahydrospiro[phenanthrene-3,2'-cyclobutane] **(E47);**
*(rac)-(4aS,10aS)*-7-hydroxy-10a-methyl-1,2,3,4,4a,9,10,10a-octahydrospiro[phenanthrene-3,1'-cyclobutane] (**E48**);
*(rac)-(4aS,10aS)*-3-(1-cyclopenten-1-yl)-7-hydroxy-10a-methyl-1,4,4a,9,10,10a-hexahydro-phenanthrene **(E49);**
*(rac)-(3S,4aS,10aS)*-7-hydroxy- 10a-methyl-1,2,3,4,4a,9,10,10a,2', 3',4',5'-dodecahydrospiro[phenanthrene-3,2'-furan] (**E50a**);
*(rac)-(3R,4aS,10aS)*-7-hydroxy-10a-methyl-1,2,3,4,4a,9,10,10a,2', 3',4',5'-dodecahydrospiro[phenanthrene-3,2'-furan] (**E50b**);
*(rac)-(3S,4aS,10aS)*-7-hydroxy-1 0a-methyl-1,2,3,4,4a,9,10,10a,3', 4',5',6'-dodecahydrospiro[phenanthrene-3,2'-*2H*-pyran] (**E51a**);
*(rac)-(3R,4aS,10aS)*-7-hydroxy-10a-methyl-1,2,3,4,4a,9,10,10a,3', 4',5',6'-dodecahydrospiro[phenanthrene-3,2'-*2H*-pyran] (**E51b**);
*(rac)-(1S,3R,4aS,10aS)*-7-hydroxy-10a-methyl-1-(3-methylbutyl)-1,2,3,4,4+9,10,10a,2', 3',4',5'-dodecahydrospiro[phenanthrene-3,2'-furan] (**E52**);
*(rac)-(1S,4aS,10aS)*-7-hydroxy-10a-methyl-1-(3-methylbutyl)-1,2,3,4,4a,9,10,10a-octahydrospiro[phenanthrene-3,1'-cyclobutane] (**E53**);
*(rac)-(1S,4aS,10aS)*-7-hydrohy-3-(3-hydroxypropyl)-10a-methyl-1-(3-methylbutyl)-1,4,4a, 9,10,10a-hexahydrophenanthrene (**E54a**);
*(rac)-(1S,3S,4aS,10aS)*-7-hydroxy-10a-methyl-1-(3-methylbutyl)-1,2,3,4,4a,9,10,10a,2', 3',4',5'-dodecahydrospiro[phenanthrene-3,2'-furan] (**E54b**);
*(rac)-(4aR,10aR)*-10a-Butyl-7-hydroay-4a-methyl-3,4,4a,9,10,10a-hexahydro-*1H*-phenanthren-2-one (**E55**);
*(rac)*-7-Hydroxy-4a-methyl-4,4a,9,10-tetrahydro-*3H*-phenanthren-2-one (**E56**);
*(rac)*-4b-methyl-4b,5,6,7,9,10-hexahydro-phenanthren-2-ol (**E57**);
*(rac)*-7-Hydroxy-1,4a-dimethyl-4,4a,9,10-tetrahydro-*3H*-phenanthren-2-one (**E58**);
*(rac)*-4a,8-dimethyl-4b,5,6,7,9,10-hexahydro-phenanthren-2-ol (**E59**);
*(rac)*-4b,8-dimethyl-4b,5,6,7,9,10-hexahydro-phenanthren-2-ol (**E60a, E60b, E60c, E60d**);
*(rac)*-*(3S,4aR)*-7-hydroxy-3,4a-dimethyl-4,4a,9,10-tetrahydro-*3H*-phenanthren-2-one (**E61**);
*(rac)-(4S,4aR)*-7-Hydroxy-4a-methyl-4-propyl-4,4a,9,10-tetrahydro-*3H*-phenanthren-2-one (**E62**);
*(rac)-(4R, 4aR)*-7-Hydroxy-4,4a-dimethyl-4,4a,9,10-tetrahydro-*3H*-phenanthren-2-one (**E63a**);
*(rac)-(4S,4aR)*-7-Hydroxy-4,4a-dimethyl-4,4a,9,10-tetrahydro-*3H*-phenanthren-2-one (**E63b**);
*(rac)*-1-Ethyl-7-hydroxy-4a-methyl-4,4a,9,10-tetrahydro-*3H*-phenanthren-2-one (**E64**);
*(rac)*-8-Ethyl-4b-methyl-4b,5,6,7,9,10-hexahydro-phenanthren-2-ol (**E65**);
*(rac)-(4aR,10aR,1S)*-1-Ethyl-7-hydroxy-4a-methyl-1,4,4a,9,10,10a-hexahydro-*3H*-phenanthren-2-one (**E66**);
*(rac)*-1-Butyl-7-hydroxy-4a-methyl-4,4a,9,10-tetrahydro-*3H*-phenanthren-2-one (**E67**);
*(rac)*-8-Butyl-4b-methyl-4b,5,6,7,9,10-hexahydro-phenanthren-2-ol (**E68**);
*(rac)-(8R,4bR,8aR)*-8-Butyl-4b-methyl-4b,5,6,7,8,8a,9,10-octahydro-phenanthren-2-ol (**E69a**);
*(rac)-(8S,4bR,8aR)*-8-Butyl-4b-methyl-4b,5,6,7,8,8a,9,10-octahydro-phenanthren-2-ol (**E69b**);
*(rac)*-4a-Butyl-7-hydroxy-1-methyl-4,4a,9,10-tetrahydro-*3H*-phenanthren-2-one (**E70**);
*(rac)*-4a-Butyl-7-hydroxy-4,4a,9,10-tetrahydro-*3H*-phenanthren-2-one (**E71**);
*(rac)-(4aR,10aR*-*)*4a-Butyl-7-hydroxy-3,4,4a,9,10,10a-hexahydro-*1H*-phenanthren-2-one (**E72a**);
*(rac)-(4aR,10aS)*-4a-Butyl-7-hydroxy-3,4,4a,9,10,10a-hexahydro-*1H*-phenanthren-2-one (**E72b**);
*(rac)-(4aR, 10aS)*-7-hydroxy-4a-methyl-3,4,4a,9,10,10a-hexahydro-*1H*-phenanthren-2-one (**E73**);
*(rac)-(4aR,10aS)*-2,2-ethanediyldimercapto-7-hydroxy-4a-methyl-1,2,3,4,4a,9,10,10a-octahydrophenanthrene (**E74**);
*(rac)-(4aR,10aS)*-7-hydroxy-4a-methyl-3,4,4a,9,10,10a-hexahydro-*1H*-phenanthren-2-one oxime **(E75);**
and pharmaceutically acceptable salts and stereoisomers thereof.

Another embodiment of the invention is a method of eliciting an estrogen receptor modulating effect in a mammal in need thereof, comprising administering to the mammal a therapeutically-effective amount of any of the compounds or any of the pharmaceutical compositions described above.

A class of this embodiment is the method wherein the estrogen receptor modulating effect is an agonizing effect.

A first subclass of this class of embodiment is the method wherein the estrogen receptor is an ERα receptor.

A second subclass of this class of embodiment is the method wherein the estrogen receptor is an ERβ receptor.

A third subclass of this class of embodiment is the method wherein the estrogen receptor modulating effect is a mixed ERα and ERβ agonizing effect.

A second class of this embodiment is the method wherein the estrogen receptor modulating effect is an antagonizing effect.

A first subclass of this class of embodiment is the method wherein the estrogen receptor is an ERα receptor.

A second subclass of this class of embodiment is the method wherein the estrogen receptor is an ERβ receptor.

A third subclass of this class of embodiment is the method wherein the estrogen receptor modulating effect is a mixed ERα and ERβ antagonizing effect.

Another embodiment of the invention is a method of treating or preventing hot flashes in a mammal in need thereof by administering to the mammal a therapeutically effective amount of any of the compounds or pharmaceutical compositions described above.

Exemplifying the invention is a pharmaceutical composition comprising any of the compounds described above and a pharmaceutically acceptable carrier. Also exemplifying the invention is a pharmaceutical composition made by combining any two or more of the compounds described above and a pharmaceutically acceptable carrier. An illustration of the invention is a process for making a pharmaceutical composition comprising /combining any of the compounds described above and a pharmaceutically acceptable carrier.

As one specific embodiment of this invention, 140 mg of
*(rac)-(1R,2R,4aS,10aS)*-1-butyl-3,3-ethanediyldimercapto-7-hydroxy-2-methyl-1,2,3,4,4a,9,10,10a-octahydro-phenanthrene is formulated with sufficient finely divided lactose to provide a total amount of 580 to 590 mg to fill a size 0, hard-gelatine capsule.

As one specific embodiment of this invention, 200 mg of *(rac)*-4b,8-dimethyl-4b,5,6,7,9,10-hexahydro-phenanthren-2-ol is formulated with sufficient finely divided lactose to provide a total amount of 580 to 590 mg to fill a size 0, hard-gelatine capsule.

Further exemplifying the invention is the use of any of the compounds described above in the preparation of a medicament for the treatment and/or prevention of osteoporosis in a mammal in need thereof. Still further exemplifying the invention is the use of any of the compounds described above in the preparation of a medicament for the treatment and/or prevention of bone loss, bone resorption, bone fractures, osteoporosis, cartilage degeneration, endometriosis, uterine fibroid disease, hot flashes, increased levels of LDL cholesterol, cardiovascular disease, impairment of cognitive functioning, cerebral degenerative disorders, restinosis, gynecomastia, vascular smooth muscle cell proliferation, obesity, incontinence, autoimmune disease, lung cancer, colon cancer, breast cancer, uterine cancer, prostate cancer, and/or disorders related to estrogen functioning.

The present invention is also directed to combinations of any of the compounds or any of the pharmaceutical compositions described above with one or more agents useful in the prevention or treatment of osteoporosis. For example, the compounds of the instant invention may be effectively administered in combination with effective amounts of other agents such as an organic bisphosphonate or a cathepsin K inhibitor. Nonlimiting examples of said organic bisphosphonates include adendronate, clodronate, etidronate, ibandronate, incadronate, minodronate, neridronate, risedronate, piridronate, pamidronate, tiludronate, zoledronate, pharmaceutically acceptable salts or esters therof, and mixtures thereof. Preferred organic biphosphonate include alendronate and pharmaceutically acceptable salts and mixtures thereof. Most preferred is alendronate monosodium trhihydrate.

The precise dosage of the bisphonate will vary with the dosing schedule, the oral potency of the particular bisphosphonate chosen, the age, size, sex and condition of the mammal or human, the nature and severity of the disorder to be treated, and other relevant medical and physical factors. Thus, a precise pharmaceutically effective amount cannot be specified in advance and can be readily determined by the caregiver or clinician. An appropriate amount can be determined by routine experimentation from animal models and human clinical studies. Generally, an appropriate amount of bisphosphonate is chosen to obtain a bone resorption inhibiting effect, i.e. a bone resorption inhibiting amount of the bisphonsphonate is administered. For humans, an effective oral dose of bisphosphonate is typically from about 1.5 to about 6000 µg/kg of body weight and preferably about 10 to about 2000 µg/kg of body weight.

For human oral compositions comprising alendronate, pharmaceutically acceptable salts thereof, or pharmaceutically acceptable derivatives thereof, a unit dosage typically comprises from about 8.75 mg to about 140 mg of the alendronate compound, on an alendronic acid active weight basis, i.e. on the basis of the corresponding acid.

The compounds of the present invention can be used in combination with other agents useful for treating estrogen-mediated conditions. The individual components of such combinations can be administer separately at different times during the course of therapy or concurrently in divided or single combination forms. The instant invention is therefore to be understood as embracing all such regimes of simultaneous or alternating treatment and the term "administering" is to be interpreted accordingly. It will be understood that the scope of combinations of the compounds of this invention with other agents useful for treating estrogen-mediated conditions includes in principle any combination with any pharmaceutical composition useful for treating disorders related to estrogen functioning.

The compounds of the present invention can be administered in such oral dosage forms as tablets, capsules (each of which includes sustained release or timed release formulations), pills, powder, granules, elixirs, tinctures, suspensions, syrups and emulsions. Likewise, they may also be administered in intravenous (bolus or infusion), intraperitoneal, topical (*e.g.*, ocular eyedrop), subcutaneous, intramuscular, or transdermal (*e.g.*, patch) form, all using forms well known to those of ordinary skill in the pharmaceutical arts.

The dosage regimen utilizing the compounds of the present invention is selected in accordance with a variety of factors including type, species, age, weight, sex, and medical condition of the patient; the severity of the condition to be treated; the route of administration; the renal and hepatic function of the patient; and the particular compound or salt thereof employed. An ordinarily skilled physician, veterinarian or clinician can readily determine and prescribe the effective amount of the drug required to prevent, counter or arrest the progress of the condition.

Oral dosages of the present invention, when used for the indicated effects, will range between about 0.01 mg per kg of body weight per day (mg/kg/day) to about 100 mg/kg/day, preferably 0.01 mg per kg of body weight per day (mg/kg/day) to 10 mg/kg/day, and most preferably 0.1 to 5.0 mg/kg/day. For oral administration, the compositions are preferably provided in the form of tablets containing 0.01, 0.05, 0.1, 0.5, 1.0, 2.5, 5.0, 10.0, 15.0, 25.0, 50.0, 100, and 500 milligrams of the active ingredient for the symptomatic adjustment of the dosage to the patient to be treated. A medicament typically contains from about 0.01 mg to about 500 mg of the active ingredient, preferably from about 1 mg to about 100 mg of active ingredient. Intravenously, the most preferred doses will range from about 0.1 to about 10 mg/kg/minute during a constant rate infusion. Advantageously, compounds of the present invention may be administered in a single daily dose, or the total daily dosage may be administered in divided doses of two, three or four times daily. Furthermore, preferred compounds for the present invention can be administered in intranasal form via topical use of suitable intranasal vehicles, or via transdermal routes, using those forms of transdermal skin patches will known to those of ordinary skill in the art. To be administered in the form of a transdermal delivery system, the dosage administration will, of course, be continuous rather than intermittent throughout the dosage regimen.

In the methods of the present invention, the compounds herein described in detail can form the active ingredient, and are typically administered in admixture with suitable pharmaceutical diluents, exipients or carriers (collectively referred to herein as "carrier" materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water, and the like. Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms includes sodium oleate, sodium stearate, magnesium stearate, sodium benzoate, sodium acetate, sodium chloride and the like. Disintegrators include without limitation starch, methylcellulose, agar, bentonite, xanthan gum and the like.

The compounds of the present invention can also be administered in the form of liposome delivery systems, such as small unilamellar vesicles, large unilamellar vesicles and multilamellar vesicles. Liposomes can be formed form a variety of phospholipids, such as 1,2-dipalmitoylphosphatidylcholine, phosphatidyl ethanolamine (cephalin), or phosphatidylcholine (lecithin).

The following definitions apply to the terms as used throughout this specification, unless otherwise limited in specific instances.

The term "estrogen receptor ligand" as used herein is intended to cover any moiety which binds to a estrogen receptor. The ligand may act as an agonist, an antagonist, a partial agonist or a partial antagonist. The ligand may be either ERα or ERβ selective or display mixed ERα and ERβ activity.

The term "aliphatic hydrocarbon(s)" as used herein refers to acyclic straight or branched chain groups which include alkyl, alkenyl or alkynyl groups.

The term "aromatic hydrocarbon(s)" as used herein refers to groups including aryl groups as defined herein.

Unless otherwise indicated, the term "lower alkyl", "alkyl", or "alk" as employed herein alone or as part of another group includes both straight and branched chain hydrocarbons, containing 1 to 12 carbon atoms (in the case of alkyl) in the normal chain and preferably 1 to 6 carbons, such as methyl, ethyl, propyl, isopropyl, butyl, t-butyl, or isobutyl, pentyl, hexyl, isohexyl, heptyl, 4,4-dimethylpentyl, octyl, 2,2,4-trimethylpentyl, nonyl, decyl, undecyl, dodecyl.

The term "cycloalkyl" as employed herein alone or as part of another group refers to 3- to 7-membered fully saturated mono cyclic ring system and include cyclopropyl, cyclobutyl, cyclopentyl, cyclohexyl, and cycloheptyl.

The term "cycloalkylalkyl" as employed herein alone or as part of another group refers to an cycloalkyl group containing 3 to 7 carbon atoms attached through available carbon atoms to a straight or branched chain alkyl radical containing 1 to 6 carbon atoms and include but are not limited to cyclopropylmethyl (-CH₂C₃H₅), cyclobutylethyl (-CH₂CH₂C₄H₇), and cyclopentylpropyl (-CH₂CH₂CH₂C₅H₉).

The term "aryl" as employed herein alone or as part of another group refers to monocyclic and bicyclic aromatic groups containing 6 to 10 carbons in the ring portion and include but are not limited to phenyl, 1-naphthyl, and 2-naphthyl and may be optionally substituted through available carbon atoms with 1, 2, or 3 groups selected from hydrogen, halo, alkyl, haloalkyl, alkoxy, haloalkoxy, alkenyl, amino, trifluoromethyl, trifluoromethoxy, alkynyl, hydroxy, nitro, cyano, or carboxy.

The term "arylalkyl" as employed herein alone or as part of another group refers to an aryl group containing 6 to 10 carbon atoms attached through available carbon atoms to a straight or branched chain alkyl radical containing 1 to 6 carbon atoms and include but are not limited to benzyl (-CH₂Ph), phenethyl (-CH₂CH₂Ph), phenpropyl (-CH₂CH₂CH₂Ph), and 1-napthylmethylene (-CH₂C₁₀H₇).

Unless otherwise indicated, the term "lower alkenyl" or "alkenyl" as used herein by itself or as part of another group refers to straight or branched chain radicals of 2 to 12 carbons, preferably 2 to 6 carbons, in the normal chain, which include one to six double bonds in the normal chain, such as vinyl, 2-propenyl, 3-butenyl, 2-butenyl, 4-pentenyl, 3-pentenyl, 2-hexenyl, 3-hexenyl, 2-heptenyl, 3-heptenyl, 4-heptenyl, 3-octenyl, 3-nonenyl, 4-decenyl, 3-undecenyl, 4-dodecenyl, and the like.

Unless otherwise indicated, the term "lower alkynyl" or "alkynyl" as used herein by itself or as part of another group refers to straight or branched chain radicals of 2 to 12 carbons, preferably 2 to 6 carbons, in the normal chain, which include one triple bond in the normal chain, such as 2-propynyl, 3-butynyl, 2-butynyl, 4-pentynyl, 3-pentynyl, 2-hexynyl, 3-hexynyl, 2-heptynyl, 3-heptynyl, 4-heptynyl, 3-octynyl, 3-nonynyl, 4-decynyl, 3-undecynyl, 4-dodecynyl and the like.

The term "halogen" or "halo" as used herein alone or as part of another group refers to chlorine, bromine, fluorine, and iodine as well as CF₃.

The term "acyl" as employed herein alone or as part of another group refers to a carbonyl group (C=O) which in turn is bonded a linear or branched alkyl group of from 1 to 4 carbon atoms and includes but is not limited to acetyl [-(C=O)CH₃], propionyl [-(C=O)CH₂CH₃], and butyryl [-(C=O)CH₂CH₂CH₃].

The term "hydroxyalkyl" as employed herein alone or as part of another group refers to linear chain alkyl radical containing 1 to 2 carbon atoms attached through available carbon atoms to a hydroxyl group and include hydroxymethyl (-CH₂OH), 1-hydroxyethyl [-CH₂(OH)CH₃] and 2-hydroxyethyl (-CH₂CH₂OH).

The term "aminoalkyl" as employed herein alone or as part of another group refers to linear chain alkyl radical containing 1 to 2 carbon atoms attached through available carbon atoms to a primary, secondary, or tertiary amino group and include aminomethyl (-CH₂NR₂), 1-aminoethyl [-CH₂(NR₂)CH₃] and 2-aminoethyl (-CH₂CH₂NR₂) where R is a hydrogen atom or methyl or ethyl group.

The compounds of formula **I, II** or **III** can be present as salts, in particular pharmaceutically acceptable salts. If the compounds of formula **I, II** or **III** have, for example, at least one basic center, they can form acid addition salts. These are formed, for example, with strong inorganic acids, such as mineral acids, for example sulfuric acid, phosphoric acid or a hydrohalic acid, with strong organic carboxylic acids, such as alkanecarboxylic acids of 1 to 4 carbon atoms which are unsubstituted or substituted, for example, by halogen, for example acetic acid, such as saturated or unsaturated dicarboxylic acids, for example oxalic, malonic, succinic, maleic, fumaric, phthalic or terephthalic acid, such as hydroxycarboxylic acids, for example ascorbic, glycolic, lactic, malic, tartaric or citric acid, such as amino acids, (for example aspartic or glutamic acid or lysine or arginine), or benzoic acid, or with organic sulfonic acids, such as (C₁-C₄)-alkyl- or aryl-sulfonic acids which are unsubstituted or substituted, for example by halogen, for example methane- or p-toluene-sulfonic acid. Corresponding acid addition salts can also be formed having, if desired, an additionally present basic center. The compounds of formula **I, II** or **III** having at least one acid group (for example COOH) can also form salts with bases. Suitable salts with bases are, for example, metal salts, such as alkali metal or alkaline earth metal salts, for example sodium, potassium or magnesium salts, or salts with ammonia or an organic amine, such as morpholine, thiomorpholine, piperidine, pyrrolidine, a mono-, di- or tri-lower alkylamine, for example ethyl-, tert-butyl-, diethyl-, diisopropyl-, triethyl-, tributyl- or dimethyl-propylamine, or a mono-, di- or trihydroxy lower alkylamine, for example mono-, di- or triethanolamine. Corresponding internal salts may furthermore be formed. Salts which are unsuitable for pharmaceutical uses but which can be employed, for example, for the isolation or purification of free compounds I or their pharmaceutically acceptable salts are also included.

Preferred salts of the compounds of formula **I, II** or **III** which include a basic group include monohydrochloride, hydrogensulfate, methanesulfonate, phosphate or nitrate.

Preferred salts of the compounds of formula **I, II** or **III** which include an acid group include sodium, potassium and magnesium salts and pharmaceutically acceptable organic amines.

The compounds in the invention contain at least one chiral center and therefore exist as optical isomers. The invention therefore comprises the optically inactive racemic (*rac*) mixtures (a one to one mixture of enantiomers), optically enriched scalemic mixtures as well as the optically pure individual enantiomers. The compounds in the invention also may contain more than one chiral center and therefore may exist as diastereomers. The invention therefore comprises individual diastereomers as well as mixtures of diastereomers in cases where the compound contains more than one stereo center. The compounds in the invention also may contain acyclic alkenes or oximes and therefore exist as either the E (entgegen) or Z (zusammen) isomers. The invention therefore comprises individual E or Z isomers as well as mixtures of E and Z isomers in cases where the compound contains an acylic alkene or oxime funtional group. Also included within the scope of the invention are polymorphs, hydrates, and solvates of the compounds of the instant invention.

The present invention includes within its scope prodrugs of the compounds of this invention. In general, such prodrugs will be functional derivatives of the compounds of this invention which are readily convertible *in vivo* into the required compound. Thus, in the methods of treatment of the present invention, the term "administering" shall encompass the treatment of the various conditions described with the compound specifically disclosed or with a compound which may not be specifically disclosed, but which converts to the specified compound *in vivo* after administration to the patient. Conventional procedures for the selection and preparation of suitable prodrug derivatives are described, for example in "Design of Prodrugs" ed. H. Bundgaard, Elsevier, 1985, which is incorporated by reference herein in its entirety. Metabolites of the compounds includes active species produced upon introduction of compounds of this invention into the biological milieu.

The present invention also relates to pharmaceutical compositions comprising the compounds of the present invention and a pharmaceutically acceptable carrier.

The present invention also relates to methods for making the pharmaceutical compositions of the present invention.

The present invention also relates to methods for treating or preventing disorders elated to estrogen functioning, bone loss, bone fractures, osteoporosis, cartilage degeneration, endometriosis, uterine fibroid disease, autoimmune disease, lung, colon, breast, uterus, or prostate cancer, hot flashes, cardiovascular disease, impairment of cognitive function, cerebral degenerative disorders, restenosis, gynecomastia, vascular smooth muscle cell proliferation, obesity and incontinence in a mammal in need thereof by administering the compounds and pharmaceutical compositions of the present invention.

The present invention also relates to methods for reducing bone loss, lowering LDL cholesterol levels and eliciting a vasodilatory effect, in a mammal in need thereof by administering the compounds and pharmaceutical compositions of the present invention.

The novel compounds of the present invention can be prepared according to the procedure of the following Schemes and examples, using appropriate materials and are further exemplified by the following specific examples. The compounds illustrated in the examples are not, however, to be construed as forming the only genus that is considered as the invention. The following examples further illustrate details for the preparation of the compounds of the present invention. Those skilled in the art will readily understand that known variation of the conditions and processes of the following preparative procedures can be used to prepare these compounds. The compounds of the present invention of formulae **I** and **II** are prepared according to the general methods outlined in **Schemes 1-4**, and according to the related methods described. Those of formula **III** are prepared according to general methods outlined in **Schemes 5-6** and according to the related methods described. All temperatures are degrees in Celsius unless otherwise noted. The following abbreviations, reagents, expressions or equipment, which are amongst those used in the descriptions below, are explained as follows: 20-25°C (room temperature, r.t.), molar equivalent (eq.), dimethyl formamide, (DMF) dichloromethane (DCM), ethyl acetate (EtOAc), tetrahydrofuran (THF), lithium diisopropylamide (LDA), methyl *t*-butyl ether (MTBE), rotating glass sheet coated with a silica gel-gypsum mixture used for chromatographic purification (chromatotron), preparative liquid chromatography with a C8 stationary phase and ammonium acetate acetonitrile-water buffer as mobile phase (PBPLC), electrospray mass spectroscopy (ES/MS).

A general route for the construction of the tetrahydro-phenanthrone nucleus of formulae **I** and **II** is shown in **Scheme 1.** This methodology is based on the chemistry described by Dyker, *et al., J. Org. Chem*. **1998**, *63*, 6043-6047. In step 1, aryl iodide **1** is coupled with an homoallylic alcohol **2a** or **2b** under the influence of a palladium catalyst, to give **3a** or **3b** respectively. Compound **3a** or **3b** is then cyclized under acidic or basic conditions in step 2, to provide the tetrahydro-phenanthrone derivative **4** or **5** respectively. Representative protocols for step **1** (**method A**) and step 2 (**method B** or **method C**) in **Scheme 1** are as follows:

### Method A

To a mixture of the aryl iodide **1** (**Scheme 1**, 1.0 eq.), the homoallylic alcohol "J" (**2** in **Scheme 1,** "K" eq.), ethyl diisopropylamine ("L" eq.), and LiCl (1.0 eq.) in DMF ("M" mL/mmol of aryl iodide) is added Pd(OAc)₂ (0.05 eq.). This mixture is then stirred in a sealed tube under a nitrogen atmosphere at 80°C for "N" h before being added to DCM.

This DCM solution is then filtered through a short plug of silica gel that is further eluted with EtOAc before concentration of the combined filtrates *in vacuo* (approximately 10 mmHg/60 °C) to provide a crude material.

### Method B

The crude material from **method A** is taken up in DCM ("N" mL) and treated with conc. HCl (aq., "O" mL) at "P" °C, and for "Q" h. The mixture is then first washed with saturated aqueous Sodium bicarbonate and then with water. Back extraction of the combined aqueous phases is done with EtOAc. The combined organic phases are then dried over anhydrous sodium sulfate and concentrated *in vacuo* to yield the crude product.

### Method C

The crude material from **method A** is dissolved in EtOAc ("N" mL) to which 1/3 of this volume saturated HCI/EtOAc is added followed by stirring at r.t. for "Q" h. The crude product is obtained by concentrating this solution *in vacuo.*

The homoallylic alcohols **2a** and **2b** which are used as starting materials in **Scheme 1** could, when not commercially available, be prepared by known methods such as the allylation of aldehydes (see Wada, *et al., Tetrahedron Lett.* **1997**, *38,* 8045-8048 and references cited therein). A general method Employing Mg / BiCl₃ as reagents for this purpose, is outlined in **Scheme 2.** When either of the allylic bromides **6a** or **6b** is reacted with formaldehyde under these conditions, the primary homoallylic alcohol **2a** is formed. The secondary homoallylic alcohol **2b** is formed in an analogous way by reaction with aldehyde 7. A representative protocol for the reaction shown in **Scheme 2 (method D)** is as follows:

### Method D

While stirring at room temperature, the allylic bromide **6a** or **6b** (**Scheme 2**, 1.0 eq.) is added to a suspension of bismuth trichloride (1.1 eq.) and magnesium turnings (2.4 eq.) in a THF-H₂O (4:1) mixture. After additional stirring for 20 min., the aldehyde formaldehyde 7 (**Scheme 2**, "J" eq.) is added in one portion. The resulting mixture is then stirred for "K" h before quenching with 1.0M HCl. The organic materials are extracted with diethyl ether, the combined organic extracts washed with water and saturated aqueous NaCl, dried over anhydrous sodium sulfate, and concentrated *in vacuo* to furnish the crude product.

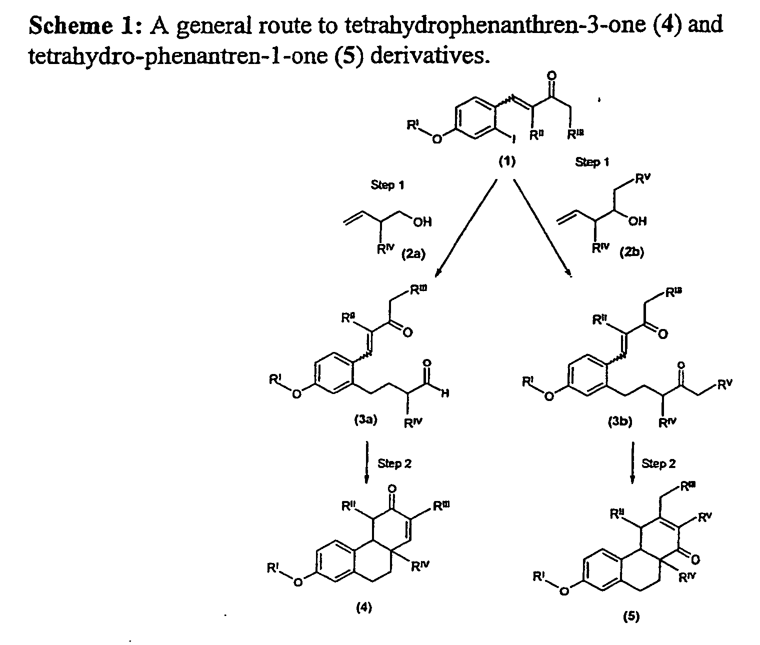

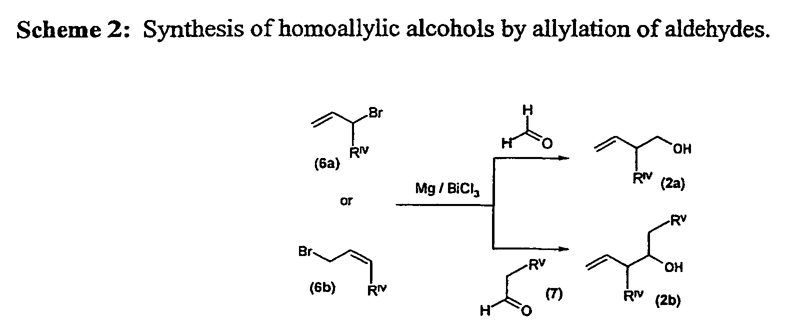

The substituted benzylideneacetones **1** used as starting material in **Scheme 1**, can be prepared from the corresponding aromatic aldehyde **8** in a conventional way by aldol condensation with ketones **9** as shown in **Scheme 3**. When ketone **9** is unsymmetric (*i*.*e*., when R^{II} and R^{III} are different), the reacting site can be controlled by varying the reaction conditions according to known methods (see Irie, *et al., Bull. Chem. Soc. Jpn.* **1980**, *53*, 1366-1371 and Iranpoor, *et al., Tetrahedron* **1998**, *55*, 9475-9480 and references cited therein). When R¹ in **8** is hydrogen, a non-hydrogen protecting group can be introduced on 1 after the aldol condensation.

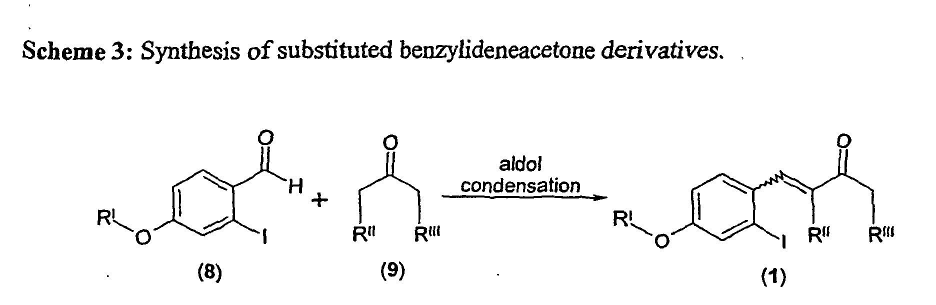

The 4a,9,10,10a-tetrahydro-*4H*-phenanthren-3-ones 4 (**Scheme 1**), can be further modified by employment of known methods for the reactions of enone and carbonyl functionalities as shown in **Scheme 4.** For example, in step 1 of **Scheme 4,** a non-hydrogen R^{VI}-substituent in (10) can be introduced by treatment of **4** with an organocopper reagent carrying the R^{VI}-substituent (see Lipshuts B.H. "*Synthetic Procedures Involving Organocopper Reagents*" in "*Organometallics in Synthesis*", Schlosser M. (ed.), John Wiley & Sons **1994**). A representative protocol (**method E**) for the introduction of a non-hydxogen R^{VI}-substituent (step 1 of **Scheme 4**) is as follows:

### Method E

To a cool (dry ice / acetone bath) mixture of the copper(I)-reagent "J" ("K" eq.) in (additional) "L" mL of the solvent "M" is added while stirring, a solution of the organometallic reagent "N" ("O" eq.). The mixture is then allowed to come to 0 °C for 15 min., before again cooling (dry-ice / acetone bath). A solution of the enone **4** (**Scheme 4,** 1.0 eq.) is then added dropwise. Stirring is continued at "P" °C for "Q" h, before quenching with saturated aqueous NH₄Cl. The organic materials are then taken up in EtOAc. This solution is dried over anhydrous sodium sulfate and filtered through a short plug of silica gel before concentration *in vacuo* to yield the crude product.
Another example is the introduction, in step 1 of **Scheme 4**, of hydrogen as R^{VI}-substituent in (**10**) by hydrogenation of (**4**). A representative protocol (**method F**) for hydrogenation/hydrogenolysis is as follows:

### Method F

A flask, containing a mixture, in the solvent "J" ("K" mL), of the substrate (1.0 eq.) to be hydrogenated/hydrogenolyzed, and a catalyst "L" ("M" mg), is evacuated and filled with hydrogen three times before stirring at room temperature and atmospheric pressure for "N" h. Workup is done by filtering the mixture through a short plug of celite®, followed by concentration of the filtrate *in vacuo* to obtain the crude product.

In step 2 of **Scheme 4,** the carbonyl group of ketone **10** can be altered using traditional carbonyl chemistry to generate derivatives **11**, where the R^{VII}- and R^{VIII}-substituents has been introduced. For example, the thioketal can be prepared from **10** by treatment with a thiol, or dithiol, under acidic conditions. A representative protocol (**method G**) for the preparation of thioketals (**11**, R^{VII}, R^{VIII}= S-alkyl) from **10** is as follows:

### Method G

To a cool (dry ice / acetone bath) solution of the ketone **10** (**Scheme 4**, 1.0 eq.) in dry DCM ("J" mL) is added the thiol or dithiol "K" ("L" eq.), followed by BF₃OEt₂ (1.5 eq.), while stirring. The mixture is then allowed to slowly come to room temperature, and stirred there for another "M" h before quenching with saturated aqueous Sodium bicarbonate. The organic materials are then taken up in EtOAc. This solution is dried over anhydrous sodium sulfate before passage through a short plug of silica gel and concentration *in vacuo* to give the crude product.

Another example is the preparation of ketals of the general formula **11** (R^{VII}, R^{VIII}= O-alkyl) from **10.** A representative protocol (**method H**) is as follows:

### Method H

A mixture of the ketone **10** (**Scheme 4**, 1.0 eq.), the alcohol or diol "J" ("K" mL), Amberlyst 15® ("L" mg), and DCM ("M" mL) is stirred at "N" °C for "O" h before filtration through celite®. The celite®-bed is then washed with DCM, and the combined filtrates washed with water, dried over anhydrous sodium sulfate and filtered through a short plug of silica gel. The crude product is obtained by concentrating this filtrate *in vacuo*.

The R^{II}- and R^{III}-substituents of **10 (Scheme 4)** can be separately changed from hydrogen to non-hydrogen by alkylation of the corresponding enolates of **10**. Thus, a non-hydrogen R^{III}-substituent can be introduced by alkylation of **10** (enolate A), while a non-hydrogen R^{II}-substituent can be introduced by alkylation of **10** (enolate B). Enolate **10** (enolate A) can for example be prepared from (4) (R^{III}= H) by 1,4-addition of a negatively charged nucleophile to the enone moiety. Other general methods for the generation of enolates involves treatment of the corresponding ketone with a strong base such as LDA. When the R^{VII}- and R^{VIII}-substituents of **11** are attached via a sulfur atom (i.e., R^{VII}, R^{VIII}= S-alkyl) as in thioketals, one or both of R^{VII} and R^{VIII} can be exchanged to hydrogen by treatment with a desulfurizing agent, such as Raney-Ni. Two representative protocols (**method I** and **method J**) for the desulfurization of **11** (**Scheme 4**) are as follows:

### Method I

To a stirred solution of the thioketal **11** (**Scheme 4**, R^{VII}, R^{VIII}= S-alkyl, 1.0 eq.) in abs. ethanol ("J" mL) is added Raney-Ni ("K" mg). The weight of the Raney-Ni is measured as the weight of the wet material that is obtained on a flat spatula after picking from a sedimented suspension in abs. ethanol. This mixture is then rapidly stirred at "L" °C for "M" h, before filtration through celite® and successive washing with abs. ethanol and EtOAc. The crude product is obtained by concentrating the combined filtrate and washings *in vacuo.*

### Method J

To a stirred mixture of the thioketal **11** (**Scheme 4**, R^{VII}, R^{VIII}= S-alkyl, 1.0 eq.) and Al-Ni-alloy (Aldrich no. 22,165-1, "J" mg).in abs. EtOH ("K" mL), is slowly added 3.0M NaOH (aq., "L" drops) at room temperature. Another portion of abs. EtOH ("M" mL) is added after "N" h of stirring, before filtration through celite®. The filtrate is then partitioned between aqueous ammonium acetate and MTBE. The organic phase is dried over anhydrous sodium sulfate and filtered through a short plug of silica gel, before concentration *in vacuo,* to provide the crude product.

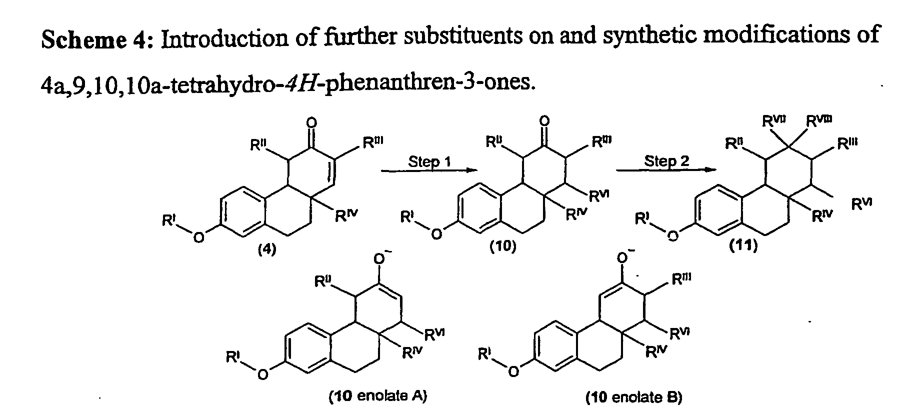

Deprotection (*i*.*e*., to change the R^{I}-substituent from a non-hydrogen to hydrogen) can be done by several different known methods depending on the nature of the R^{I}-substituent.
For example, when R^{I}= CH₂Ph deprotection can be done by hydrogenolysis (for a representative protocol see **method F**), and when R^{I}= CH₃ deprotection can be done by treatment with BBr₃ (for a representative protocol see **method K**).

### Method K

To a cool (dry-ice / acetone bath) and stirred solution of the aryl methyl ether substrate (1.0 eq.) in dry DCM ("J" mL) is added BBr₃ (1.0M sol in DCM, "K" eq.). The mixture is then allowed to come to "L" °C, and kept at that temperature for "M" h before quenching with saturated aqueous sodium bicarbonate. The organic materials are then taken up in EtOAc. This solution is dried over anhydrous sodium sulfate before passage through a short plug of silica gel and concentration *in vacuo* to furnish the crude product.

A general route for the construction of the tetrahydro-phenanthrone nucleus of formulae **III** is shown in **Scheme 5.** This methodology is based on the chemistry described by Fétizon and Delobelle, *C.R. Hebd. Seances Acad Sci*, **1957**, 245; 850-852 and by Howell and Taylor, *J. Chem. Soc,* **1958,** 1248-1253. In step 1, tetralone **12** is formed by oxidation of enone **11** by mCPBA. Robinson-type annulation of tetralone **12** with enone **13** results in phenantrone **14.** Representative protocols for step 1 (**method L**) and step 2 (**method M**) in **Scheme 5** are as follows:

### Method L

To a cooled solution (~5°C) of 1.0 eq. the olefin in DCM was added in portions while stirring mCPBA (50 weight % dispersion in water, 1.2 eq.) at such a rate that the temperature never exceeded 10 °C. The mixture was then stirred at 5-10 °C for 12 h before filtration through celite® and washing of the filtrate with saturated aqueous sodium bicarbonate, water, and saturated aqueous NaCl. To the solution thus obtained was added PPTS (0.05 eq.) before heating at reflux for 2.5 h. Concentration *in vacuo* then followed to yield the crude product.

### Method M

To a stirred solution of the tetralone (1.0 eq.) in toluene or benzene, was first added the enone coupling partner (1.2 eq.), followed by the pTsOH catalyst (0.05 eq.). The mixture was, after being heated at 85 °C for 20 h, then filtered through a short plug of silica gel. Elution of this plug with enough of MTBE to totally deliver all of the desired material, followed by concentration of the eluate *in vacuo,* furnished the crude product.

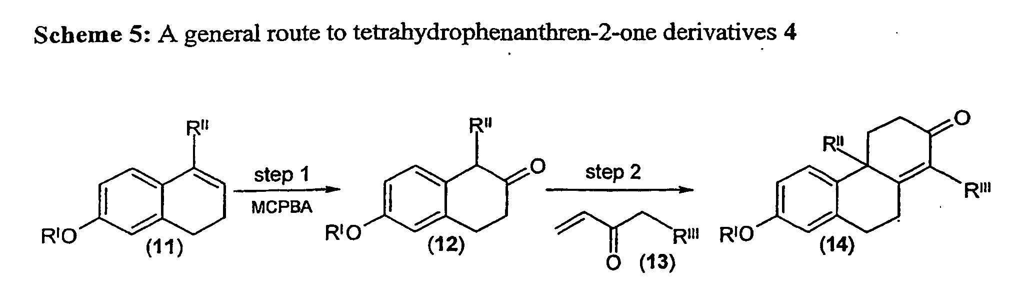

Deprotection (i.e., to change the R^{I}-substituent from a non-hydrogen to hydrogen) can be done by several different known methods depending on the nature of the R^{I}-substituent. For example when R^{I}= CH₃, deprotection can be done by treatment with BBr₃ (for a representive protocol see, **method O**).

### Method O

To a cool (dry-ice / acetone bath) and stirred solution of the aryl methyl ether (1.0 eq.) in dry DCM was added BBr₃ (1.0 M sol in DCM). The mixture was then allowed to come to -20°C, and kept at that temperature for the time indicated before quenching with saturated aqueous Sodium bicarbonate. The organic materials were then taken up in EtOAc. This solution was dried (anhydrous sodium sulfate) before passage through a short plug of silica gel and concentration *in vacuo* to furnish the crude product.

The 3,4,4a,9,10,10a-hexahydro-*1H*-phenanthren-2-ones **14** (**Scheme 5**) can by further modified by employment of known methods for the reactions of enone and carbonyl functionalities as shown in **Scheme 6.** For example, a non-hydrogen R^{IV}-substituent in (**20**) can be introduced by treatment of **14** with and organocopper reagent carrying the R^{IV}-substituent (see Lipshuts B.H. "*Synthetic Procedures Involving Organocopper Reagents"* in "*Organomettalics in Synthesis",* Schlosser M. (ed.), John Wiley & Sons **1994**), or by treatment of **4** with an organotitaniumate complex (see Flemming, S. *et*.*al*., *Tet. Lett* **1994**, *35 (33),* 6075-6078 and Kabbara, S. *et*.*al*., *Liebigs Ann.* **1995,** 401-406. A protocol for the introduction of a non-hydrogen R^{IV}-substituent (**method P** and **Q**, step 1 of **Scheme 6**) is as follows:

### Method P

To a cool (dry ice / acetone bath) suspension of CuCN in diethylether was added while stirring, a solution of the allcyl lithium. The mixture was then allowed to come to 0 °C, before cooling again (dry-ice / acetone bath). A solution of 1.0 eq. of the enone was then added dropwise. Stirring was continued at that temperature, and for the time indicated, before quenching with saturated aqueous NH₄Cl. The organic materials were thereafter taken up in EtOAc. This solution was dried (anhydrous sodium sulfate) and filtered through a short plug of silica gel before concentration *in vacuo,* to yield the crude product.

### Method Q

To a cool (-30 °C) suspension of titanium (IV) isopropoxide in anhydrous THF was added while stirring, a solution of the alkyl magnesium chloride. The mixture was then allowed to come to 0 °C, before again cooling (dry-ice / acetone bath). A solution of Nickel(II) acetylacetonate (0.05 eq.) and the enone (1.0 eq.) in anhydrous THF was then added dropwise. Stirring was continued at -15 °C for 30 min., and the mixture was then stirred at r.t overnight. The mixture was diluted with diethyl ether and quenched with water. After stirring for 1 hour, the precipitate was filtered of and the residue was concentrated *in vacuo* to yield the crude product.

In step step 2 of **Scheme 6,** the carbonyl functionality of **14** can be reduced using traditional carbonyl chemistry. A representative protocol (**method R**) for the reduction of carbonyl groups is as follows:

### Method R

To a cool (-40 °C) solution of the enone (1.0 eq.) in acetonitrile was added while stirring sodium borohydride (5.0 eq.), followed by trifluoro acetic acid (1/4 of the volume of acetonitrile). The remaining mixture was thereafter allowed to come to room temperature in approximately 1 h, and then stirred at that temperature for another 2 h before quenching with saturated aqueous Sodium bicarbonate. The organic material was taken up in EtOAc, dried (anhydrous sodium sulfate), and filtered through a short plug of silica gel before concentration *in vacuo* to give the crude product.

Another example is the introduction of hydrogen as R^{IV}-substituent in (10) by hydrogenation of **4** or **6** (step 3 and 4 of **Scheme 6**). A representative protocol (**methods** for hydrogenation is as follows:

### Method S

A flask containing a solution of the substrate (1.0 eq.) and a palladium catalyst, was evacuated and filled with hydrogen three times before stirring the mixture at room temperature and atmospheric pressure for the time indicated. Workup was done by filtering the mixture through a short plug of celite®, followed by concentration of the filtrate *in vacuo* to obtain the crude product

In step 5 and 6 of **Scheme 6,** the carbonyl group of ketone **7** can be altered using traditional carbonyl chemistry to generate derivatives **19** and **20.** For example, thioketal **19** can be prepared from **17** by treatment with a dithiol under acidic conditions, *N*-oxime **20** (R^{VII}= N) can be prepared from **17** by treatment with hydroxylamine and thioketone **10** (R^{VII}= S) can be prepared from **17** by treatment with Lawesson's reagent. Representative protocols (**methods T and U**) for the preparation of thioketals ((**19**), R^{V}, R^{VI} = S-alkyl) and *N*-oximes from **17** are as follows:

### Method T

To a cool (dry ice/ acetone bath) solution of the ketone **17** (**Scheme 6**, 1.0 eq.) in dry DCM is added the dithiol (2.0 eq.) followed by BF₃-OEt₂ (1.5 eq.) while stirring. The mixture is then allowed to slowly come to room temperature, and stirred there for the time indicated before quenching with saturated aqueous Sodium bicarbonate. The organic phases are then taken up in EtOAc. This solution is dried over anhydrous sodium sulfate before passage through a short plug of silica gel and concentration *in vacuo* to give the crude product.

### Method U

A mixture of the ketone, NH2OH·HCl (10eq.) and sodium acetate (10 eq.) is stirred in methanol under N₂ atmosphere at r.t. during 3 days. Water is added and the organic materials are then taken up in EtOAc. This solution is dried over anhydrous sodium sulfate before passage through a short plug of silica gel and concentration *in vacuo* to give the crude product.

### Method V

5-Bromo-1-pentene in anhydrous THF is added to magnesium in anhydrous THF, at such speed that the mixture gently refluxed (an I₂ crystal is added to start the reaction). After addition, the reaction mixture is heated at 70 °C for 30 minutes and allowed to cool to room temperature.

To a stirred solution of the above described Grignard reagent (4.0 eq.) is added ketone **20, Scheme 4** (1eq.) at room temperature and the reaction mixture is stirred for 20 minutes before quenching with saturated aqueous NH₄Cl. The organic materials are then taken up in EtOAc. This solution is dried over anhydrous sodium sulphate and filtered through a short plug of silica gel to yield the crude product.

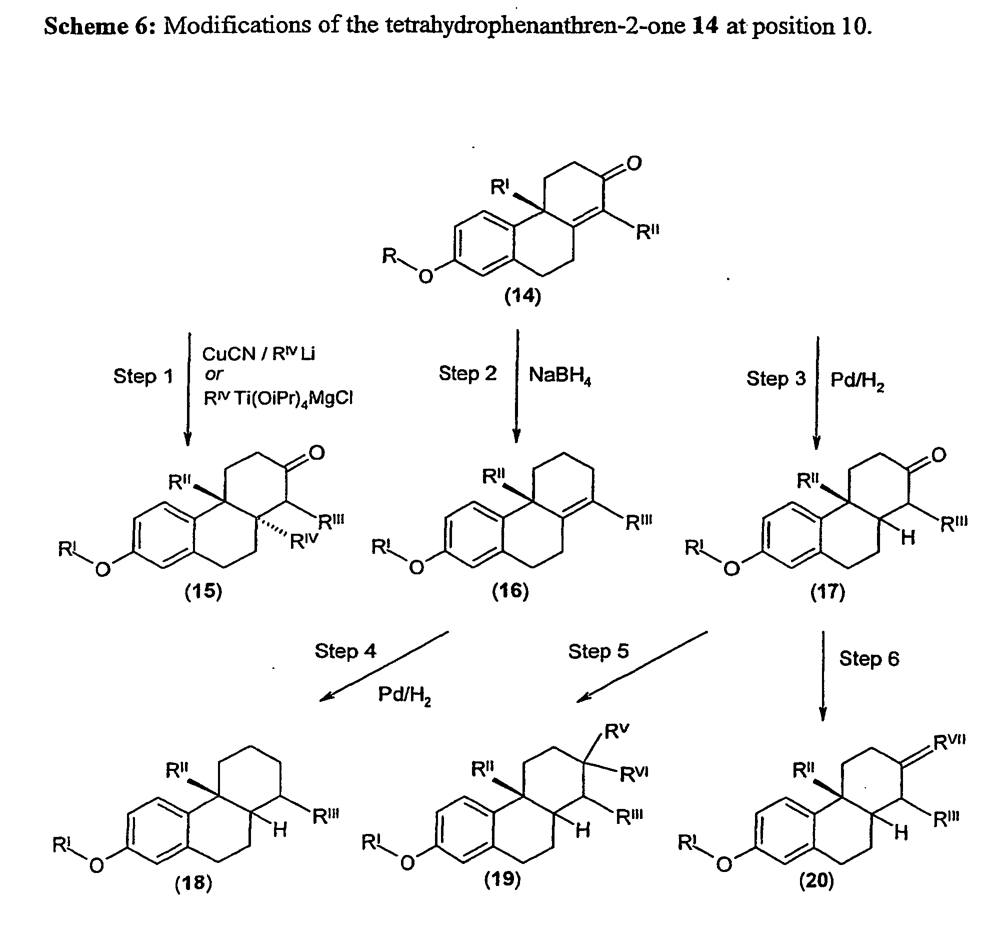

The following examples represent preferred, but non-limiting embodiments of the invention. Examples 1-54 relate to compounds of formulae I and II, and Examples 55-75 relate to compounds of formula III.

### Example 1: Synthesis of (rac)-(4aS,10aS)-7-hydroxy-1,4,4a,9,10,10a-hexahydro-2H-phenanthren-3-one (E1).

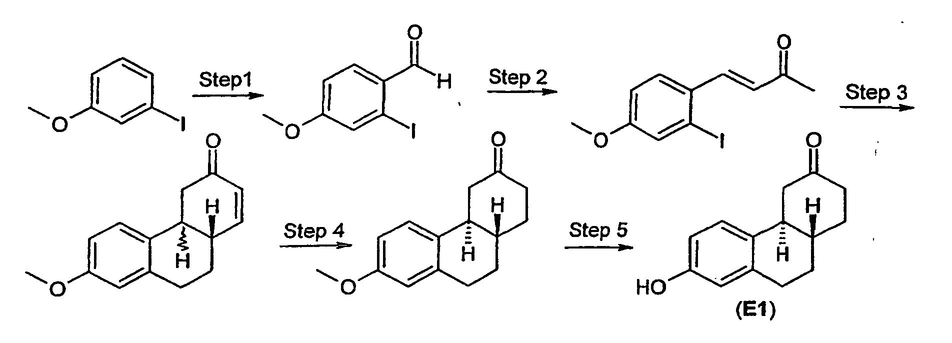

**Step 1:** 2-Iodo-4-methoxy-benzaldehyde. To a stirred mixture of *m*-iodo-anisole (47.2 g, 202 mmol) and DMF (20 mL) was added POCl₃ (1.1 eq.) dropwise. The mixture was then heated at 100 °C for 12 h before sequential addition of further portions of DMF (12 mL) and POCl₃ (0. 5eq.), and then again stirred for another 12 h at 100 °C. The dark mixture was then poured onto 500 mL of 2.0M NaOH before extraction with diethyl ether and DCM. The combined extracts were dried over anhydrous sodium sulfate before concentration *in vacuo,* to yield the crude product as a dark syrup, that was purified using silica gel flash chromatography twice with EtOAc/*n*-heptane (8:2, v:v) as eluent. 2-Iodo-4-methoxy-benzaldehyde was obtained as white needles by gently concentrating the solution obtained after the chromatography. Another portion of 2-iodo-4-methoxy-benzaldehyde was obtained from the mother liquid. ¹H NMR (270 MHz, CDCl₃) δ 9.90 (s, 1H), 7.85 (d, 1H), 7.40 (d, 1H), 6.95 (ddd, 1H), 3.85 (s, 3H); ¹³C NMR (CDCl₃) δ 195.05, 165.5, 131.75, 128.75, 125.60, 114.95, 102.30, 55.70.

**Step 2:** 4-(2-Iodo-4-methoxy-phenyl)-but-3-en-2-one. To a stirred suspension of 2-iodo-4-methoxy-benzaldehyde (558 mg, 2.13 mmol) in a water-acetone mixture (6.0 mL, 3:2), was added aqueous NaOH (16.0M, 1.2 eq.) before heating at reflux for 10 min. The yellow precipitate formed upon cooling to r.t. was collected, washed with water, and dried over P₂O₅ to give 4-(2-iodo-4-methoxy-phenyl)-but-3-en-2-one. ¹H NMR (270 MHz, CDCl₃) δ 7.70 (d, 1H), 7.50 (d, 1H), 7.40 (d, 1H), 6.90 (DD, 1H), 6.45 (d, 1H), 3.80 (s, 3H), 2.40 (s, 3H).

**Step 3:** *(rac)-(4aR,10aS)*-7-Methoxy-4a,9,10,10a-tetrahydro-*4H*-phenanthren-3-one and *(rac)-(4aS,10aS)*-7-Methoxy-4a,9,10,10a-tetrahydro-*4H*-phenanthren-3-one. The aryl iodide 4-(2-iodo-4-methoxy-phenyl)-but-3-en-2-one (362 mg) was treated according to **method A** ("J" = but-3-en-1-ol, "K" = 2.5, "L" = 8.0, "M" = 4, "N" = 3.5). The material obtained was then treated according to **method B** ("N" = 3 0, "O" = 0.3, "P" = 25, "Q" = 20). The crude product obtained was purified by silica gel flash chromatography using EtOAc:*n*-heptane (2.5:97.5, 5:95, 1:9, stepwise gradient) as eluent, followed by fractionation with PHPLC. The first eluted fraction contained
*(rac)-(4aR,10aS)*-7-methoxy-4a,9,10,10a-tetrahydro-*4H*-phenanthren-3-one (yellowish solid), and the second
*(rac)-(4aS,10aS)*-7-methoxy-4a,9,10,10a-tetrahydro-*4H*-phenanthren-3-one (white solid).
*(rac)-(4aR,10aS)*-7-Methoay-4a,9,10,10a-tetrahydro-*4H*-phenanthren-3-one: ES/MS m/z: 229.3 (pos., M+H); ¹H NMR (270 MHz, CDCl₃) δ 6.95-7.05 (m, 2H), 6.75 (dd, 1H), 6.65 (d, 1H), 6.05 (dd, 1H), 3.80 (s, 3H), 3.35-3.45 (m, 1H), 2.85-2.95 (m, 2H), 2.55-2.75 (m, 3H), 1.70-2.00 (m, 2H).
*(rac)-(4aS,10aS)*-7-Methoxy-4a,9,10,10a-tetrahydro-*4H*-phenanthren-3-one: ES/MS m/z: 229.3 (pos., M+H); ¹H NMR (270 MHz, CDCl₃) δ 7.10 (d, 1H), 6.95 (dd, 1H), 6.75 (dd, 1H), 6.70 (d, 1H), 6.10 (dd, 1H), 3.75 (s, 3H), 3.20 (dd, 1H), 2.90-3.10 (m, 3H), 2.25-2.50 (m, 2H), 2.10-2.20 (m, 1H), 1.65-1.80 (m, 1H).

**Step 4:** *(rac)-(4aS,10aS)*-7-Methoxy-1,4,4a,9,10,10a-hexahydro-*2H*-phenanthren-3-one. The substrate (*rac*)-(*4aS,10aS*)-7-methoxy-4a,9,10,10a-tetrahydro-*4H*-phenanthren-3-one (30 mg) was treated according to **method F** ("J" = methanol, "K" = 5.0, "L" = 5% Pd/C, "M" = 10, "N" = 24) to obtain
*(rac)-(4aS,10aS)*-7-methoxy-1,4,4a,9,10,10a-hexahydro-*2H*-phenanthren-3-one. ES/MS m/z: 231.1 (pos., M+H).

**Step 5:** *(rac)*-*(4aS*,*10aS)*-7-Hydroxy-1,4,4a,9,10,10a-hexahydro-*2H*-phenanthren-3-one. The substrate
(*rac*)-(*4aS, 10aS*)*-*7-methoxy-1,4,4a,9,10,10a-hexahydro-*2H*-phenanthren-3-one (28 mg) was treated according to **method K** ("J" =1.0, "K" = 3.0, "L" = - 20, "M" = 4.0). The crude product was purified on chromatotron using EtOAc:*n*-heptane (3:7, v:v) as eluent to yield *(rac)*-*(4aS*,*10aS)*-7-hydroxy-1,4,4a,9,10,10a-hexahydro-*2H*-phenanthren-3-one (white solid). ES/MS m/z: 217.0 (pos., M+H), 215.2 (neg., M-H); ¹H NMR (270 MHz, CDCl₃) δ 7.00 (d, 1H), 6.65 (dd, 1H), 6.60 (s, 1H), 4.70 (s, 1H), 3.05-3.15 (m, 1H), 2.75-3.00 (m, 2H), 2.60-2.70 (m, 1H), 2.35-2.55 (m, 2H), 2.25 (t, 1H), 2.05-2.15 (m, 1H), 1.95-2.05 (m, 1H), 1.40-1.80 (m, 3H).

### Example 2: Synthesis of

### (rac)-(4aS,10aS)-3,3-ethanediyldimercapto-7-hydroxy-1,2,3,4,4a,9,10,10a-octahydro-phenanthrene (E2).

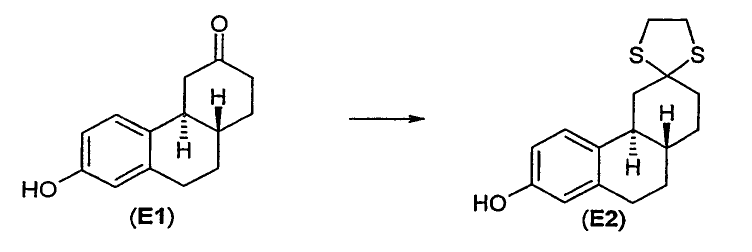

The ketone (*rac*)-(*4aS*,*10aS*)-7-hydroxy-1,4,4a,9,10,10a-hexahydro-*2H*-phenanthren-3-one (9.3 mg) was treated according to **method G** ("J" = 2.5, "K" = 1,2-ethanedithiol, "L" = 2.0, "M" = 12). The crude product was purified on chromatotron, using EtOAc:*n*-heptane (2:8, v:v) as eluent to yield

*(rac)*-*(4aS,10aS)*-3,3-ethanediyldimercapto-7-hydroxy-1,2,3,4,4a,9,10,10a-octahydro-phena nthrene (white solid). ES/MS m/z: 292.9 (pos., M+H), 290.8 (neg., M-H); ¹H NMR (270 MHz, CDCl₃) δ 7.10 (d, 1H), 6.60 (dd, 1H), 6.55 (d, 1H), 4.50 (s, 1H), 3.25-3.40 (m, 4H), 2.70-2.90 (m, 3H), 2.45-2.55 (m, 1H), 2.20 (dq, 1H), 2.00 (dt, 1H), 1.75-1.90 (m, 3H), 1.40-1.60 (m, 2H), 1.20-1.35 (m, 1H).

### Example 3: Synthesis of (rac)-(4aR,10aS)-7-hydroxy-10a-methyl-4a,9,10,10a-tetrahydro-4H-phenanthren-3-one (E3a); and (rac)-(4aS,10aS)-7-hydroxy-10a-methyl-4a,9,10,10a-tetrahydro-4H-phenanthren-3-one (E3b).

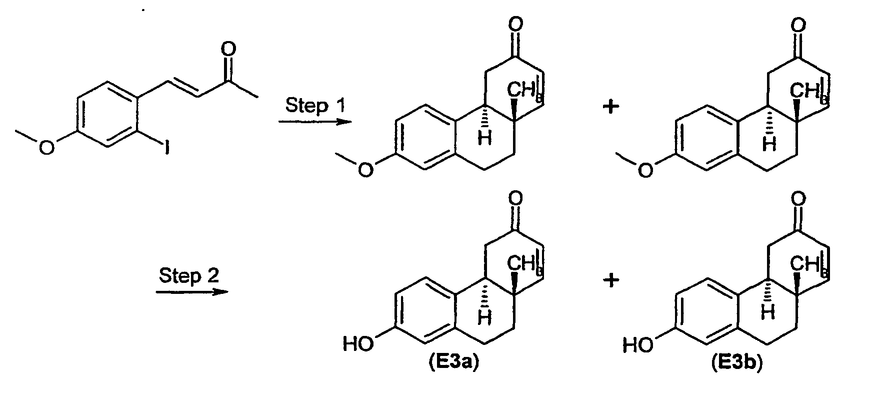

**Step 1:**
*(rac)-(4aR,10aS)*-7-Methoxy-10a-methyl-4a,9,10,10a-tetrahydro-*4H*-phenanthren-3-one and
*(rac)-(4aS,10aS)*-7-Methoxy-10a-methyl-4a,9,10,10a-tetrahydro-*4H*-phenanthren-3-one.
The aryl iodide 4-(2-iodo-4-methoxy-phenyl)-but-3-en-2-one (325 mg) was treated according to **method A** ("J" = 2-methyl-but-3-en-1-ol, "K" = 2.5, "L" = 8.0, "M" = 10, "N" = 3.0). The material obtained was then treated according to **method B** ("N" = 25, "O" = 0.5, "P" = 40, "Q" = 4). The crude product obtained was fractionated on chromatotron using EtOAc:*n*-heptane (2:8, 9:1, slow stepwise gradient) as eluent. The first eluted fraction contained
*(rac)-(4aS,10aS)*-7-methoxy-10a-methyl-4a,9,10,10a-tetrahydro-*4H*-phenanthren-3-one (solid), the second a mixture of
*(rac)-(4aR, 10aS)*-7-methoxy-10a-methyl-4a,9,10,10a-tetrahydro-*4H*-phenanthren-3-one *and*
*(rac)*-*(4aS*,*10aS)*-7-methoxy-10a-methyl-4a,9,10,10a-tetrahydro-*4H*-phenanthren-3-one, and the third
*(rac)-(4aR*,*10aS)*-7-methoxy-10a-methyl-4a,9,10,10a-tetrahydro-*4H*-phenanthren-3-one.
*(rac)-(4aS*,*10aS)*-7-Methoxy-10a-methyl-4a,9,10,10a-tetrahydro-*4H*-phenanthren-3-one: ES/MS m/z: 243.4 (pos., M+H); ¹H NMR (270 MHz, CDCl₃) δ 7.00 (d, 1H), 6.90 (d, 1H), 6.75 (dd, 1H), 6.70 (d, 1H), 5.95 (d, 1H), 3.80 (s, 3H), 3.20 (dd, 1H), 3.05 (dd, 1H), 2.90-3.00 (m, 2H), 2.40-2.55 (m, 1H), 1.80-1.90 (m, 2H), 0.95 (s, 3H).

**Step 2:**
*(rac)-(4aR,10aS)*-7-Hydroxy-10a-methyl-4a,9,10,10a-tetrahydro-*4H*-phenanthren-3-one and
*(rac)-(4aS,10aS)*-7-Hydroxy-10a-methyl-4a,9,10,10a-tetrahydro-*4H*-phenanthren-3-one.
As substrate, a mixture of
(*rac*)-(*4aR,10aS*)-7-methoxy-10a-methyl-4a,9,10,10a-tetrahydro-*4H*-phenanthren-3-one and
(*rac*)-(*4aS,10aS*)-7-methoxy-10a-methyl-4a,9,10,10a-tetrahydro-*4H*-phenanthren-3-one (43 mg, 3:2) was used according to **method K** ("J" = 3.0, "K" = 4.0, "L" = -20, "M" = 12).
The crude product thus obtained was purified on chromatotron, using EtOAc:*n*-heptane (3:7) as eluent, followed by fractionation on PHPLC. The first eluted fraction contained *(rac)-(4aR,10aS)*-7-hydroxy-10a-methyl-4a,9,10,10a-tetrahydro-*4H*-phenanthren-3-one, and the second a mixture of
*(rac)-(4aR,10aS)*-7-hydroxy-10a-methyl-4a,9,10,10a-tetrahydro-*4H*-phenanthren-3-one and
*(rac)-(4aS,10aS)*-7-hydroxy-10a-methyl-4a,9,10,10a-tetrahydro-*4H*-phenanthren-3-one. *(rac)-(4aR,10aS)*-7-Hydroxy-10a-methyl-4a,9,10,10a-tetrahydro-*4H*-phenanthren-3-one: ES/MS m/z: 229.3 (pos., M+H), 227.2 (neg., M-H); ¹H NMR (270 MHz, CDCl₃) δ 6.95 (d, 1H), 6.70 (d, 1H), 6.65 (dd, 1H), 6.60 (d, 1H), 5.95 (d, 1H), 5.00-5.30 (br. s, 1H), 3.00 (dd, 1H), 2.80-2.85 (m, 2H), 2.65 (dd, 1H), 2.50 (dd, 1H), 1.80-1.95 (m, 1H), 1.60-1.70 (m, 1H), 1.15 (s, 3H); ¹³C NMR (CDCl₃) δ
*(rac)-(4aS,10aS)*-7-Hydroxy-10a-methyl-4a,9,10,10a-tetrahydro-*4H*-phenanthren-3-one: ¹H NMR (270 MHz, CDCl₃) δ 6.95 (d, 1H), 6.90 (d, 1H), 6.60-6.70 (m, 2H), 6.00 (d, 1H), 3.20 (dd, 1H), 3.05 (dd, 1H), 2.90-3.00 (m, 2H), 2.45 (dd, 1H), 1.80-1.90 (m, 2H), 0.95 (s, 3H); ¹³C NMR (CDCl₃) δ 199.70, 161.05, 154.00, 136.95, 129.25, 127.35, 125.80, 115.55, 113.10, 42.50, 37.50, 35.45, 34.55, 25.90, 17.00.

### Example 4: Synthesis of (rac)-(4aS,10aS)-10a-butyl-7-hydroxy-4a,9,10,10a-tetrahydro-4H-phenanthren-3-one (E4).

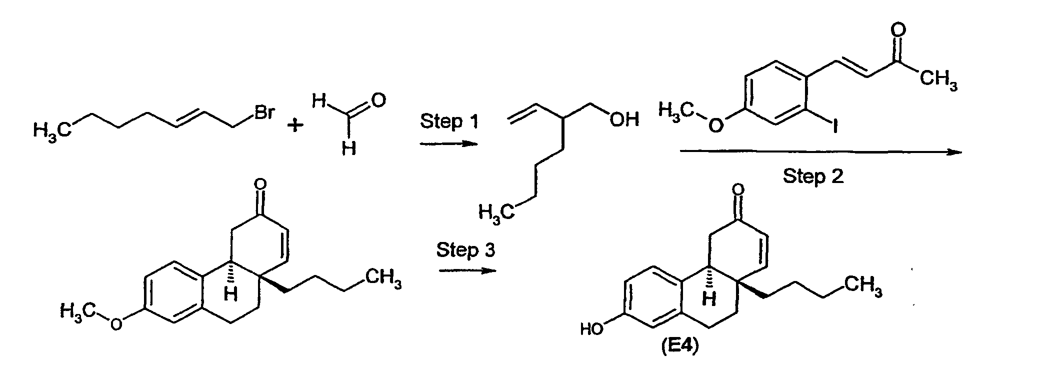

**Step 1**: *(rac)*-2-Butyl-but-3-en-1-ol. Using the allylic bromide 1-bromo-2-heptene (9.5 g) and the aldehyde formaldehyde (37% solution in water) as starting materials according to **method D** ("J" = 3.0, "K" = 18), a crude product was obtained. This was purified by distillation (79-81 °C/65 mmHg) to yield *(rac)*-2-butyl-but-3-en-1-ol as a colorless oil. ¹H NMR (270 MHz, CDCl₃) δ 5.40-5.80 (m, 1H), 4.90-5.20 (m, 2H), 3.85-4.05 (m, 1H - OH), 3.55 (dd, 1H), 3.40 (dd, 1H), 1.90-2.25 (m, 1H), 1.05-1.45 (m, 6H), 0.80 (t, 3H).

**Step 2:**
*(rac)-(4aS,10aS)*-10a-Butyl-7-methoxy-4a,9,10,10a-tetrahydro-*4H*-phenanthren-3-one.
The aryl iodide 4-(2-iodo-4-methoxy-phenyl)-but-3-en-2-one (107 mg) was treated according to **method A** ("J" = (rac)-2-butyl-but-3-en-1-ol, "K" = 2.5, "L" = 8.0, "M" = 28, "N" = 72). The material obtained was then treated according to **method B** ("N" = 5.0, "O" = 0.5, "P" = 40, "Q" = 2). The crude *(rac)-(4aS,10aS)*-10a-Butyl-7-methoxy-4a,9,10,10a-tetrahydro-*4H*-phenanthren-3-one obtained was used directly in step 3.

**Step 3:**
*(rac)-(4aS,10aS)*-10a-Butyl-7-hydroxy-4a,9,10,10a-tetrahydro-*4H*-phenanthren-3-one. The substrate *(rac)-(4aS,10aS)*-10a-butyl-7-methoxy-4a,9,10,10a-tetrahydro-*4H*-phenanthren-3-one (10 mg) was treated according to **method K** ("J" = 1.0, "K" = 4.0, "L" = -20, "M" = 12). The crude product was purified on chromatotron, using EtOAc:*n*-heptane (2:8) as eluent to yield *(rac)-(4aS,10aS)*-10a-Butyl-7-hydroxy-4a,9, 10,10a-tetrahydro-*4H*-phenanthren-3-one. ES/MS m/z: 271.3 (pos., M+H), 268.9 (neg., M-H); ¹H NMR (270 MHz, CDCl₃) δ 7.00 (dd, 2H), 6.70 (dd, 1H), 6.6 (s, 1H), 6.05 (d, 1H), 3.25 (dd, 1H), 3.05 (dd, 1H), 2.85-2.90 (m, 2H), 2.55 (dd, 1H), 2.10-2.15 (m, 1H), 1.70-1.80 (m, 1H), 1.15-1.50 (m, 6H), 0.85 (t, 3H).

### Example 5: Synthesis of (rac)-(1S,4aS,10aS)-1-butyt-7-hydroxy-1,4,4a,9,10,10a-hexahydro-2H-phenanthren-3-one (E5a); and (rac)-(1R,4aS,10aS)-1-butyl-7-hydroxy-1,4,4a,9,10,10a-hexahydro-2H-phenanthren-3-one (E5b).

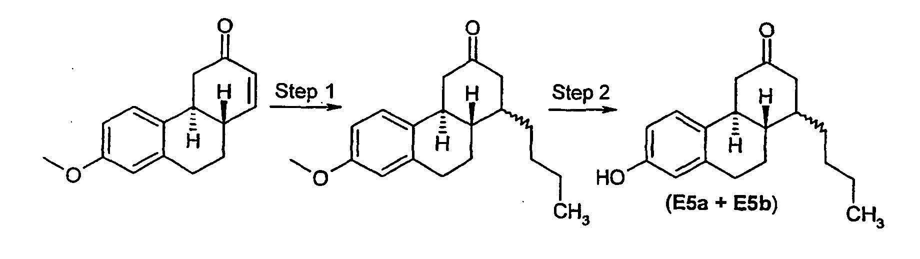

**Step 1:**
*(rac)-(1S,4aS,10aS)-*1-Butyl-7-methoxy-1,4,4a,9,10,10a-hexahydro-*2H*-phenanthren-3-one and
*(rac)-(1R,4aS,10aS)-*1-Butyl-7-methoxy-1,4,4a,9,10,10a-hexahydro-*2H*-phenanthren-3-one. The enone (*rac*)*-*(*4aS,*10aS)-7-methoxy-4a,9,10,10a-tetrahydro-*4H*-phenanthren-3-one (39 mg) from **Example 1** dissolved in diethyl ether/THF (3.0 mL, 2:1), was treated according to **method E** ("J" = CuCN, "K" = 2.2, "L" = 2.0, "M" = diethyl ether, "N" = *n*-butyl lithium [1.6M solution in hexanes], "O" = 4.2, "P" = -78, "Q" = 0.5). The crude product consisted of a mixture of *(rac)-(1S,4aS,10aS)*-1-butyl-7-methoxy-1, 4,4a,9,10,10a -hexahydro-*2H*-phenanthren-3-one and *(rac)-(1R,4aS,10aS)*-1-butyl-7-methoxy-1, 4,4a,9,10,10a-hexahydro-*2H*-phenanthren-3-one that was used in the next step (step 2).

**Step 2:**
*(rac)-(1S,4aS,10aS)*-1-Butyl-7-hydroxy-1,4,4a,9,10,10a-hexahydro-*2H*-phenanthren-3-one and *(rac)-(1R,4aS,10aS)-*1-Butyl-7-hydroxy-1,4,4a,9,10,10a-hexahydro-*2H*-phenanthren-3-one. As substrate, a mixture (47 mg) of (*rac*)*-*(*1S,4aS,10aS*)-1-butyl-7-methoxy-1, 4,4a,9,10,10a-hexahydro-*2H*-phenanthren-3-one and (*rac*)*-*(*1R,4aS,10aS*)-1-butyl-7-methoxy-1,4,4a,9,10,10a-hexahydro-*2H*-phenanthren-3-one from step 1 was treated according to **method K** ("J" = 1.0, "K" = 3.0, "L" = -20, "M" = 4.0). The crude product obtained was purified on chromatotron, using EtOAc:*n*-heptane (3:7, v:v) as eluent to yield a mixture of *(rac)-(1S,4aS,10aS)*-1-butyl-7-hydroxy-1,4,4a,9,10,10a-hexahydro-*2H*-phenanthren-3-one and *(rac)-(1R,4aS,10aS)*-1-butyl-7-hydroxy-1,4,4a,9,10,10a-hexahydro-*2H*-phenanthren-3-one as a glass. ES/MS m/z (mixture): 273.1 (pos., M+H), 271.0 (neg., M-H); ¹H NMR (distinguishable signals of the mixture, 270 MHz, CDCl₃) δ 7.05 (d, H-C5, minor isomer), 7.00 (d, H-C5, major isomer), 4.57 (s, -OH, minor isomer), 4.55 (s, -OH, major isomer).

### Example 6: Synthesis of (rac)-(1S,4aS,10aS)-1-butyl-3,3-ethanediyldimercapto-7-hydroxy-1,2,3,4,4a,9,10,10a-octahydro-phenanthrene; (E6a) and (rac)-(1r,4aS,10aS)-1-butyl-3,3-ethanediyldimercapto-7-hydroxy-1,2,3,4,4a,9,10,10a-octahydro-phenanthrene (E6b).

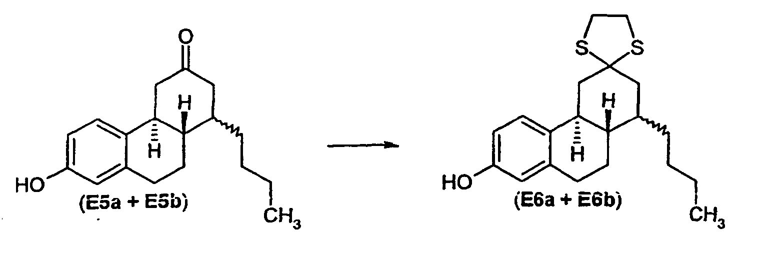

A mixture of the ketones
(*rac*)*-*(*1S,4aS,10aS*)-1-butyl-7-hydroxy-1,4,4a,9,10,10a-hexahydro-*2H*-phenanthren-3-one and
(*rac*)-(*1R,4aS,10aS*)-1-butyl-7-hydroxy-1,4,4a,9,10,10a-hexahydro-*2H*-phenanthren-3-one (2:1 or 1:2, 10.5 mg) was treated according to **method G** ("J" = 2.5, "K" = 1,2-ethanedithiol, "L" = 2.0, "M" =12). The crude product was purified on chromatotron, using EtOAc:*n*-heptane (2:8) as eluent to yield a mixture of *(rac)-(1S,4aS,10aS)*-1-butyl-3, 3-ethanediyldimercapto-7-hydroxy-1,2,3,4,4a,9,10,10a-octahydro-phenanthrene and *(rac)-(1R,4aS,10aS)*-1-butyl-3,3-ethanediyldimercapto-7-hydroxy-1,2,3,4,4a, 9,10,10a-octahydrophenanthrene as a colorless glass. ES/MS m/z (mixture): 349.3 (pos., M+H), 347.2 (neg., M-H); ¹H NMR (distinguishable signals of the mixture, 270 MHz, CDCl₃) δ 7.110 (d, H-C5, minor isomer), 7.120 (d, H-C5, major isomer), 4.505 (s, -OH, minor isomer), 4.495 (s, -OH, major isomer), 0.875 (t, CH₃, minor isomer), 0.905 (t, CH₃, major isomer).

### Example 7: Synthesis of (rac)-(1S,4aS,10aS)-1-butyl-7-hydroxy-10a-methyl-1,4,4a,9,10,10a-hexahydro-2H-phenanthren-3-one (E7).

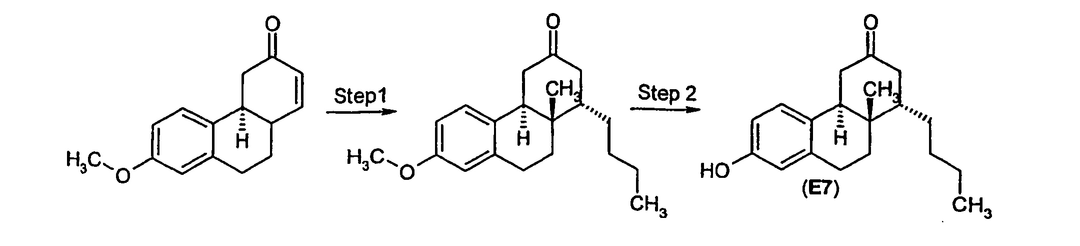

**Step 1:**
*(rac)-(1S,4aS,10aS)*-1-Butyl-7-methoxy-10a-methyl-1,4,4a,9,10,10a-hexahydro-*2H*-phenanthren-3-one. The enone (*rac*)-(*4aS,10aS*)-7-methoxy-10a-methyl-4a, 9,10,10a-tetrahydro-*4H*-phenanthren-3-one (39 mg) from **Example 3** dissolved in diethyl ether (1.5 mL) was treated according to **method E** ("J" = CuCN, "K" = 2.0, "L" = 2.5, "M" = diethyl ether, "N" = *n*-butyl lithium [1.6M solution in hexanes], "O" = 4.0, "P" = -78, "Q" = 0.5) to furnish crude *(rac)-(1S,4aS,10aS)*-1-butyl-7-methoxy-10a-methyl-1, 4,4a,9,10,10a -hexahydro-*2H*-phenanthren-3-one that was used in the next step.

**Step 2:**
*(rac)-(1S,4aS,10aS)*-1-Butyl-7-hydroxy-10a-methyl-1,4,4a,9,10,10a-hexahydro-*2H*-phenanthren-3-one. As substrate (*rac*)-(*1S,4aS,10aS*)-1-butyl-7-methoxy-10a-methyl-1,4,4a, 9,10,10a-hexahydro-*2H*-phenanthren-3-one (49 mg) from the previous step was treated according to **method K** ("J" = 2.0, "K" = 3.5, "L" = -20, "M" = 5.0). The crude product thus obtained was purified on chromatotron, using EtOAc:*n*-heptane (2:8, 4:6, v:v; stepwise gradient) as eluent to yield *(rac)-(1S,4aS,10aS)*-1-butyl-7-hydroxy-10a-methyl-1,4,4a,9,10,10a-hexahydro-*2H*-phenanthren-3-one. ES/MS m/z: 287.2 (pos., M+H), 285.1 (neg., M-H); ¹H NMR (270 MHz, CDCl₃) δ 6.90 (d, 1H), 6.60-6.65 (m, 2H), 5.00 (br. s, 1H), 3.00 (dd, 1H), 2.70-2.95 (m, 4H), 2.45 (dt, 1H), 2.30 (t, 1H), 1.90-2.05 (m, 1H), 1.70-1.80 (m, 1H), 1.55-1.65 (m, 1H), 1.40-1.50 (m, 2H), 1:10-1.35 (m, 4H), 1.05 (s, 3H), 0.90 (t, 3H); ¹³C NMR (CDCl₃) δ 14.00, 17.95, 22.70, 26.70, 27.95, 30.15, 32.55, 35.05, 38.60, 41.75, 41.85, 46.35, 112.85, 115.05, 126.25, 130.45, 137.25, 154.05, 212.40.

### Example 8: Synthesis of (rac)-(1S,4aS,10aS)-1-butyl-3,3-ethanediyldimercapto-7-hydroxy-10a-methyl-1,2,3,4,4a,9,10,10a -octahydro-phenanthrene (E8).

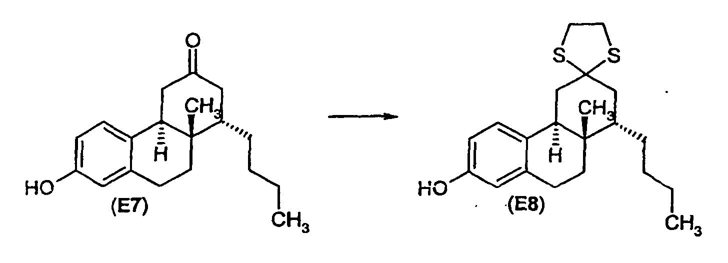

The ketone
(*rac*)*-*(*1S,4aS,10aS*)-1-butyl-7-hydroay-10a-methyl-1,4,4a,9,10,10a-hexahydro-*2H*-phenanthren-3-one (11 mg) was treated according to **method G** ("J" = 2.0, "K" = 1,2-ethanedithiol, "L" = 2.0, "M" = 12). The crude product was purified purified on chromatotron using EtOAc:*n*-heptane (2:8, v:v) as eluent to yield
*(rac)-(1S,4aS,10aS)*-1-butyl-3,3-ethanediyldimercapto-7-hydroxy-10a-methyl-1,2,3,4,4a,9, 10,10a-octahydro-phenanthrene. ES/MS m/z: 363.4 (pos., M+H), 361.3 (neg., M-H); ¹H NMR (270 MHz, CDCl₃) δ 7.05 (d, 1H), 6.50-6.65 (m, 2H), 4.65 (s, 1H), 3.25-3.35 (m, 4H), 1.80-2.95 (m, 9H), 1.10-1.65 (m, 7H), 0.85 (t, 3H), 0.80 (s, 3H).

### Example 9: Synthesis of (rac)-(1S,4aS,10aS)-1-butyl-7-hydroxy-10a-methyl-1,2,3,4,4a,9,10,10a-octahydro-phenanthrene(E9).

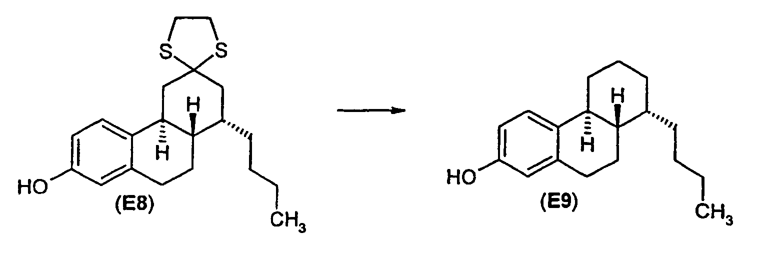

The thioketal (*rac*)*-*(*1S,4aS,10aS*)-1-butyl-3,3-ethanediyldimercapto-7-hydroxy-10a-methyl-1,2,3,4,4a,9,10,10a-octahydro-phenanthrene (6.2 mg) was treated according to **method J** ("J" = 100, "K" = 3.0, "L" = 5, "M" = 10, "N" = 96) to provide *(rac)-(1S,4aS,10aS)*-1-butyl-7-hydroxy-10a-methyl-1,2,3,4,4a,9,10,10a-octahydro-phenanthrene. ES/MS m/z: 273.4 (pos., M+H), 271.3 (neg., M-H); ¹H NMR (270 MHz, CDCl₃) δ 7.05 (d, 1H), 6.60 (d, 1H), 6.55 (d, 1H), 3.0-4.0 (br. s, 1H), 2.65-2.95 (m, 2H), 2.60 (dd, 1H), 2.10 (dd, 1H), 1.70-1.95 (m, 2H), 1.10-1.70 (m, 12M, 0.90 (t, 3H), 0.85 (s, 3H).

### Example 10: Synthesis of (rac)-(4aS,10aR)-10a-ethyl-7-hydroxy-3-methyl-4a, 9,10,10a-tetrahydro-4H-phenanthren-1-one (E10a); and (rac)-(4aR,10aR)-10a-ethyl-7-hydroxy-3-methyl-4a,9,10,10a-tetrahydro-4H-phenanthren-1-one (E10b).

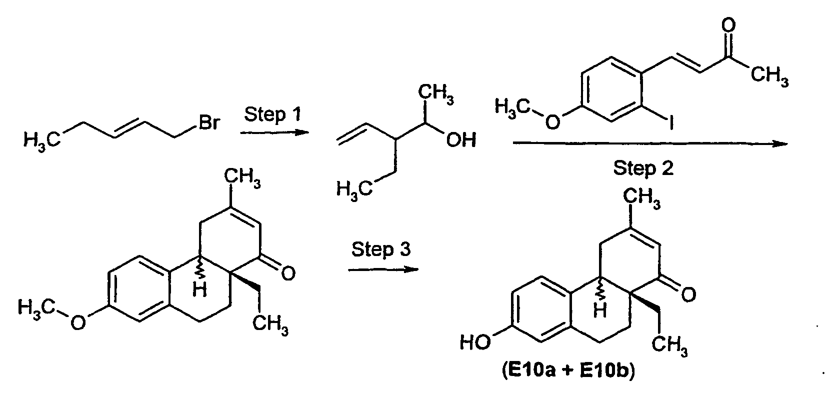

**Step 1:** *(rac)-(2R,3R)-(rac)-(2S,3R)*-3-Ethyl-pent-4-en-2-ol. The allylic bromide 1-bromo-2-pentene (2.49 g) and the aldehyde acetaldehyde was used as starting materials according to **method D** ("J" = 3.0, "K" = 18). The crude product was purified by silica gel flash chromatography using EtOAc:*n*-heptane (3:7) as eluent, to yield *(rac)-(2R,3R)-(rac)-(2S,3R)*-3-ethyl-pent-4-en-2-ol as an oil. ¹H NMR (270 MHz, DMSO-d6) δ 5.45-5.65 (m, 1H), 4.95-5.20 (m, 2H), 3.50-3.75 (m, 1H), 1.30-2.00 (m, 3H), 1.10 (d, 3H), 0.85 (t, 3H).

**Step 2:**
*(rac)-(4aS,10aR)*-10a-Ethyl-7-methoxy-3-methyl-4a,9,10,10a-tetrahydro-*4H*-phenanthren-1-one and *(rac)-(4aR,10aR)*-10a-ethyl-7-methoxy-3-methyl-4a,9,10, 10a-tetrahydro-*4H*-phenanthren-1-one. The aryl iodide 4-(2-iodo-4-methoxy-phenyl)-but-3-en-2-one (51 mg) was treated according to **method A** ("J" = *(rac)-(2R,3R)*-(*rac*)*-*(*2S,3R*)-3-ethyl-pent-4-en-2-ol, "K" = 2.5, "L" = 8.0, "M" = 44, "N" = 48). The residue obtained was dissolved in DCM (9.0mL) and stirred with 2.0 M aqueous KOH (1.0 mL) at reflux for 12 h before washing of this solution with water. Back extraction of the aqueous phase was done with EtOAc followed by drying over anhydrous sodium sulfate of the combined organic phases, and concentration of these *in vacuo* to yield a mixture of *(rac) -(4aS,10aR)-*10a-ethyl-7-methoxy-3-methyl-4a,9,10,10a-tetrahydro-*4H*-phenanthren-1-one and *(rac)-(4aR,10aR)*-10a-ethyl-7-methoxy-3-methyl-4a, 9,10,10a-tetrahydro-*4H*-phenanthren-1-one that was used in the next step.
*(rac)-(4aS,10aR)*-10a-Ethyl-7-methoxy-3-methyl-4a,9,10,10a-tetrahydro-*4H*-phenanthren-1-one: ES/MS m/z: 271.3 (pos., M+H); ¹H NMR (270 MHz, CDCl₃) δ 7.1 (d, 1H), 6.55-6.75 (m, 2H), 5.75-5.90 (m, 1H), 3.75 (s, 3H), 3.1-3.3 (m, 1H), 2.7-2.9 (m, 3H), 2.3-2.5 (m, 2H), 1.8-2.1 (m, 3H), 1.0-1.7 (m, 3H), 0.6-0.9 (m, 3H). *(rac)-(4aR,10aR)*-10a-Ethyl-7-methoxy-3-methyl-4a,9,10,10a-tetrahydro-*4H*-phenanthren-1-one: ES/MS m/z: 271.3 (pos., M+H); ¹H NMR (270 MHz, CDCl₃) δ 7.20 (d, 1H), 6.55-6.75 (m, 2H), 5.75-5.90 (m, 1H), 3.75 (s, 3H), 3.1-3.3 (m, 1H), 2.7-2.9 (m, 3H), 2.3-2.5 (m, 2H), 1.8-2.1 (m, 3H), 1.0-1.7 (m, 3H), 0.6-0.9 (m, 3H).

**Step 3:**
*(rac)-(4aS,10aR)*-10a-Ethyl-7-hydroxy-3-methyl-4a,9,10,10a-tetrahydro-*4H*-phenanthren-1-one and *(rac)-(4aR,10aR)*-10a-Ethyl-7-hydroxy-3-methyl-4a,9,10,10a-tetrahydro-*4H*-phenanthren-1-one. As substrate, a mixture of *(rac)-(4aS,10aR)*-10a-ethyl-7-methoxy-3-methyl-4a,9,10,10a-tetrahydro-*4H*-phenanthren-1-one and (*rac*)-(*4aR,10aR*)-10a-ethyl-7-methoxy-3-methyl-4a,9,10,10a-tetrahydro-*4H*-phenanmren-1-one (26 mg) was used according to **method K** ("J" = 2.5, "K" = 4.0, "L" = -20, "M" = 12). The crude product thus obtained was purified on a chromatotron using EtOAc-*n*-heptane (3:7) as eluent to yield a mixture of *(rac)-(4aS,10aR)*-10a-ethyl-7-hydroxy-3- methyl-4a, 9,10,10a-tetrahydro-*4H*-phenanthren-1-one and *(rac)-(4aR,10aR)*-10a-ethyl-7-hydroxy-3-methyl-4a,9,10,10a-tetrahydro-*4H*-phenanthren-1-one. *(rac)-(4aS,10aR)*-10a-Ethyl-7-hydroxy- 3-methyl-4a,9,10,10a-tetrahydro-*4H*-phenanthren-1-one: ES/MS m/z: 257.2 (pos, M+H), 255.1 (neg, M-H); ¹H NMR (500 MHz, CDCl₃) δ 7.00 (d, 1H), 6.70 (d, 1H), 6.60 (s, 1H), 5.90 (s, 1H), 4.95 (s, 1H, OH), 3.15-3.25 (m, 1H), 2.70-2.85 (m, 3H), 2.40-2.45 (m, 2H), 1.95 (s, 3H), 1.50-1.60 (m, 1H), 1.35-1-45 (m, 2H), 0.80 (s, 3H). *(rac)-(4aR,10aR)*-10a-Ethyl-7-hydroxy-3-methyl-4a,9,10,10a-tetrahydro-*4H*-phenanthren-1-one: ES/MS m/z: 257.2 (pos., M+H), 255.1 (neg., M-H); ¹H NMR (500 MHz, CDCl₃) δ 7.10 (d, 1H), 6.70 (d, 1H), 6.60 (s, 1H), 5.85 (s, 1H), 5.00 (s, H-OH), 3.15-3.25 (m, 1H), 2.70-2.85 (m, 3H), 2.40-2.45 (m, 2H), 2.0 (s, 3H),1.50-1.60 (m, 1H), 1.35-1-45 (m, 2H), 0.75 (s, 3H).

### Example 11: Synthesis of (rac)-(1R,2S,4aS,10aS)-1-butyl-7-hydroxy-2-methyl-1, 4,4a,9,10,10a-hexahydro-2H-phenanthren-3-one (E11a); (rac)-(1R,2R,4aS,10aS)-1-butyl-7-hydroxy-2-methyl-1,4,4a,9,10,10a-hexabydro-2H-phenanthren-3-one (E11b); and (rac)-(1S,4R,4aR,10aS)-1-butyl-7-hydroxy-4-methyl-1,4,4a,9,10, 10a-hexahydro-2H-phenanthren-3-one (E11c).

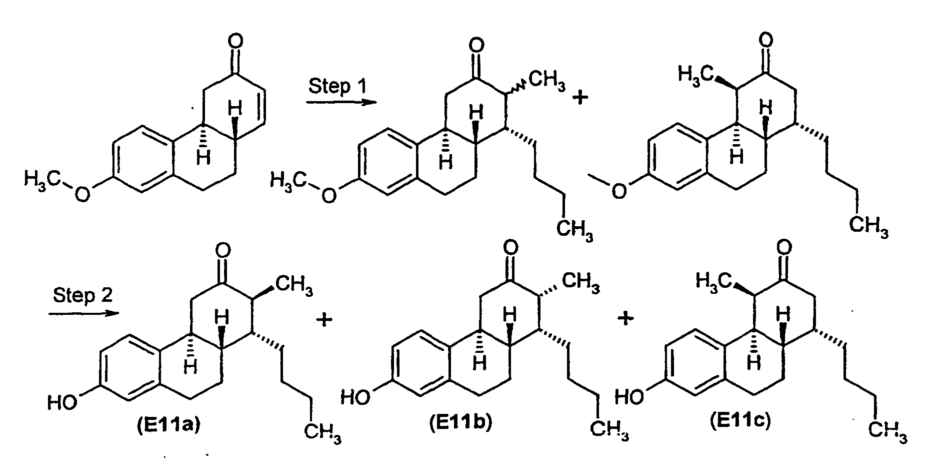

**Step 1:**
*(rac)-(1R,2S,4aS,10aS)*-1-Butyl-7-methoxy-2-methyl-1,4,4a,9,10,10a-hexahydro-*2H*-phenanthren-3-one and *(rac)-(1R,2R,4aB,10aS)*-1*-*Butyl-7-methoxy-2-methyl-1,4,4a,9,10, 10a-hexahydro-*2H*-phenanthren-3-one and *(rac)-(1S,4R,4aR,10aS)*-1-Butyl-7-methoxy-4-methyl-1,4,4a,9,10,10a-hexahydro-*2H*-phenanthren-3-one. The enone *(rac)-(4aS,10aS)*-7-methoxy-4a,9,10,10a-tetrahydro-*4H*-phenanthren-3-one (31 mg) from **Example 1** dissolved in diethyl ether / THF (3.0 mL, 2:1), was treated according to **method E** ("J" = CuCN, "K" = 2.2, "L" = 2.0, "M" = diethyl ether, "N" = *n*-butyl lithium [1.6M solution in hexanes], "O" = 4.2, "P" = -78, "Q" = 0.5) with the following exception. Methyl iodide (65 eq.) was added to the reaction mixture, and stirring was continued for another 40 h at room temperature before quenching. The crude product was purified on chromatotron, using EtOAc:*n*-heptane (1:9, v:v) as eluent, to yield *(rac)-(1R,2S,4aS,10aS)*-1-butyl-7-methoxy-2-methyl-1,4,4a,9,10,10a-hexahydro-*2H*-phenanthren-3-one, *(rac)-(1R,2R,4aS, 10aS)*-1-butyl-7-methoxy-2-methyl-1,4,4a,9,10,10a-hexahydro*-2H*-phenanthren-3-one and *(rac)-(1S,4R,4aR*,*10aS)-*1-butyl-7-methoxy-4-methyl-1,4,4a,9,10, 10a-hexahydro-*2H*-phenanthren-3-one as a mixture (colorless glass) that was used in the next step (step 2).

**Step 2:**
*(rac)-(1R,2S,4aS,10aS)*-1-Butyl-7-hydroxy-2-methyl-1,4,4a,9,10,10-a-hexahydro-*2H*-phenanthren-3-one and *(rac)-(1R,2R,4aS,10aS)*-1-Butyl-7-hydroxy-2-methyl-1,4,4a,9,10, 10a-hexahydro-*2H*-phenanthren-3-one and *(rac)-(1S,4R,4aR,10aS)*-1-Butyl-7-hydroxy-4-methyl-1,4,4a,9,10,10a-hexahydro-*2H*-phenanthren-3-one. As substrate, a mixture (22.5 mg) of (*rac*)*-*(*1R,2S,4aS,10aS*)*-*1-butyl-7-methoxy-2-methyl-1,4,4a,9,10,10a-hexahydro-*2H*-phenanthren-3-one, (*rac*)*-*(*1R,2R,4aS,10aS*)-1-butyl-7-methoxy-2-methyl-1,4,4a,9,10, 10a-hexahydro-*2H*-phenanthren-3-one and (*rac*)-(*1S,4R,4aR,10aS*)-1-butyl-7-methoxy-4-methyl-1,4,4a,9,10,10a-hexahydro-*2H*-phenanthren-3-one from the previous step (step 1) was treated according to **method K** ("J" = 2.0, "K" = 3.0, "L" = -20, "M" = 20). The crude product thus obtained was purified on chromatotron, using EtOAc:*n*-heptane (3:7) as eluent. This material was then further fractionated using PHPLC. *(rac)-(1S,4R,4aR,10aS)*-1-butyl-7-hydroxy-4-methyl-1,4,4a,9,10,10a-hexahydro-*2H*-phenanthren-3-one (3.5 mg) was obtained in the first eluted fraction, a mixture of *(rac)-(1R,2S,4aS,10aS)*-1-butyl-7-hydroxy-2-methyl-1,4,4a,9,10,10a-hexahydro-*2H*-phenanthren-3-one and *(rac)-(1R,2R,4aS,10aS)*-1-butyl-7-hydroxy-2-methyl-1,4,4a,9,10,10a-hexahydro-*2H*-phenanthren-3-one in the second, and *(rac)-(1R,2R,4aS,10aS)*-1-butyl-7-hydroxy-2-methyl-1,4,4a,9,10,10a-hexahydro-*2H*-phenanthren-3-one in the third. *(rac)-(1R,2R,4aS,10aS)*-1-Butyl-7-hydroxy-2-methyl-1,4,4a,9,10,10a-hexahydro-*2H*-phenanthren-3-one: ES/MS m/z: 287.2 (pos., M+H), 285.1 (neg., M-H); ¹H NMR (270 MHz, CDCl₃) δ 7.00 (d, 1H), 6.65 (dd, 1H), 6.60 (d, 1H), 4.65 (br. s, 1H), 2.80-3.05 (m, 4H), 2.70 (t, 1H), 2.20 (t, 1H), 1.95-2.10 (m, 2H), 1.65-1.90 (m, 2H), 1.00-1.40 (m, 6H), 1.05 (d, 3H, CH₃-C2), 0.85 (t, 3H). *(rac)-(1R,2S,4aS,10aS)-*1-Butyl-7-hydroxy-2-methyl-1,4,4a,9,10,10a-hexahydro-*2H*-phenanthren-3-one: ¹H NMR (270 MHz, CDCl₃, selected signal) δ 1.25 (d, 3H, CH₃-C2). *(rac)-(1S,4R,4aR,10aS)*-1-Butyl-7-hydroxy-4-methyl-1,4,4a,9,10,10a-hexahydro-*2H*-phenanthren-3-one: ES/MS m/z: 287.2 (pos., M+H), 285.1 (neg., M-H); ¹H NMR (270 MHz, CDCl₃, selected signal) δ 0.95 (d, 3H, CH₃-C4).

### Example 12: Synthesis of (rac)-(1R,2R,4aS,10aS)-1-butyl-3,3-ethanediyldimercapto-7-hydroxy-2-methyl-1,2,3,4,4a,9,10,10a-octahydro-phenanthrene (E12a); and (rac)-(1R,2S,4aS,10aS)-1-butyl-3,3-ethanediyldimercapto-7-hydroxy-2-methyl-1,2,3,4,4a, 9,10,10a-octahydro-phenanthrene (E12b).

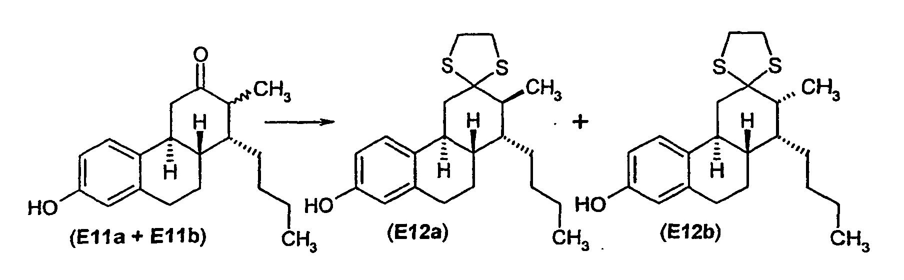

A mixture (6.0 mg, 1:1) of the ketones *(rac)-(1R,2S,4aS,10aS)*-1-butyl-7-hydroxy-2-methyl-1,4,4a,9,10,10a-hexahydro-*2H*-phenanthren-3-one and (*rac*)*-*(*1R,2R,4aS,10aS*)-1-butyl-7-hydroxy-2-methyl-1,4,4a,9,10,10a-hexahydro-*2H*-phenanthren-3-one was treated according to **method G** ("J" = 2.5, "K" = 1,2-ethanedithiol, "L" = 2.0, "M" = 12). The crude product was fractionated using PHPLC. *(rac)-(1R,2S,4aS,10aS)-1-*Butyl-3,3-ethanediyldimercapto-7-hydroxy-2-methyl-1,2,3,4,4a,9,10,10a-octahydro-phenanthrene was obtained from the first eluted fraction and *(rac)-(1R,2R,4aS,10aS)*-1-butyl-3,3-ethanediyldimercapto-7-hydroxy-2-methyl-1,2,3,4,4a,9,10,10a-octahydro-phenanthrene from the second. *(rac)-(1R,2S,4aS,10aS)*-1-Butyl-3,3-ethanediyldimercapto-7-hydroxy-2-methyl-1,2,3,4,4a,9,10,10a-octahydro-phenanthrene: ES/MS m/z: 361.3 (neg., M-H); ¹H NMR (270 MHz, CDCl₃) δ 7.10 (d, 1H), 6.60 (dd, 1H), 6.55 (d, 1H), 3.5-4.5 (br. s, 1H), 3.35-3.45 (m, 2H), 3.15-3.25 (m, 2H), 2.70-2.90 (m, 3H), 2.45-2.55 (m, 2H), 1.90-2.05 (m, 1H), 1.70-1.90 (m, 2H), 1.50-1.70 (m, 4H), 1.10-1.50 (m, 4H), 1.30 (d, 3H), 0.90 (t, 3H). *(rac)-(1R,2R,4aS,10aS)*-1-Butyl-3,3-ethanediyldimercapto-7-hydroxy-2-methyl-1,2,3, 4,4a,9,10,10a-octahydro-phenanthrene: ES/MS m/z: 361.3 (neg., M-H); ¹H NMR (270 MHz, CDCl₃) δ 7.15 (d, 1H), 6.60 (dd, 1H), 6.50 (d, 1H), 3.5-4.0 (br. s, 1H), 3.10-3.40 (m, 4H), 2.70-2.90 (m, 3H), 2.15-2.25 (m, 1H), 1.80 (t, 1H), 1.50-1.75 (m, 5H), 1.05-1.40 (m, 6H), 1.25 (d, 3H), 0.85 (t, 3H).

### Example 13: Synthesis of (rac)-(1S,4aS,10aS)-7-hydroxy-10a-methyl-1-(3-methyl-butyl)-1,4,4a,9,10,10a-hexahydro-2H-phenanthren-3-one (E13).

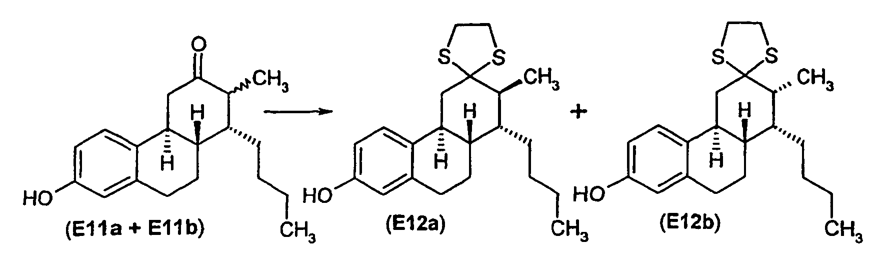

**Step 1:** 2-Iodo-4-(4-methoxy-benzyloxy)-benzaldehyde. A mixture of 4-hydroxy-2-iodo-benzaldehyde (26.45 g, 106.6 mmol), p-methoxybenzyl chloride (1.3 eq.), ethyl diisopropylamine (1.4 eq.), and sodium iodide (0.1 eq.) in DCM (500 mL) was refluxed for 69 h. The reaction mixture was then washed with 1.0M HCl (aq., 3 x 100 mL), water (200 mL), saturated Sodium bicarbonate (100 mL), and saturated NH₄Cl (100 mL) before drying over anhydrous magnesium sulfate and concentration *in vacuo.* The crude product thus obtained, was purified by silica gel flash chromatography, using EtOAc:n-heptane (1:9, 2:8, 3:7, stepwise gradient) as eluent, to yield pure 2-iodo-4-(4-methoxy-benzyloxy)-benzaldehyde in the first eluted fraction as a yellowish solid, together with a mixture of unconsumed 4-hydroxy-2-iodo-benzaldehyde and pure 2-iodo-4-(4-methoxy-benzyloxy)-benzaldehyde in the second. ¹H NMR (270 MHz, CDCl₃) δ 9.90 (s, 1H), 7.85 (d, 1H), 7.50 (d, 1H), 7.35 (d, 2H), 7.00 (dd, 1H), 6.90 (d, 2H), 5.05 (s, 2H), 3.80 (s, 3H).

**Step 2:** 4-[2-Iodo-4-(4-methoxy-benzyloxy)-phenyl]-but-3-en-2-one. To a stirred suspension of 2-iodo-4-(4-methoxy-benzyloxy)-benzaldehyde (14.7 g, 39.9 mmol) in a water:acetone mixture (150 mL, 1:2), was added aqueous NaOH (16.0M, 1.2 eq.) before heating at reflux for 10 min. The yellow precipitate formed after having added another portion of water (100 mL) and allowed to cool to room temperature was collected, washed with water, and dried over P₂O₅ to give 4-[2-iodo-4-(4-methoxy-benzyloxy)-phenyl] -but-3-en-2-one. ¹H NMR (270 MHz, CDCl₃) δ 7.70 (d, 1H), 7.50-7.55 (m, 2H), 7.30 (d, 2H), 6.95 (dd, 1H), 6.90 (d, 2H), 6.45 (d, 1H), 5.00 (s, 2H), 3.80 (s, 3H), 2.40 (s, 3H).

**Step 3:** *(rac)-(4aR,10aS)*-7-Hydroxy-10a-methyl-4a,9,10,10a-tetrahydro-*4H*-phenanthren-3-one and *(rac)-(4aS,10aS)*-7-Hydroxy-10a-methyl-4a,9,10,10a-tetrahydro-*4H*-phenanthren-3-one. The aryl iodide 4-[2-iodo-4-(4-methoxy-benzyloxy)- phenyl]-but-3-en-2-one (2.02 g) was treated according to **method A** ("J" = 2-methyl-but-3-en-1-ol, "K" = 1.5, "L" = 4.0, "M" = 2, "N" = 1.5). The material obtained was then treated according to **method C** ("N" = 100, "Q" = 12). The crude product obtained was fractionated by PHPLC. The first eluted fraction contained a mixture of *(rac)-(4aR,10aS)-*7-hydroxy-10a-methyl-4a,9,10,10a-tetrahydro-*4H*-phenanthren-3-one and *(rac)-(4aS,10aS)*-7-hydroxy-10a-methyl-4a,9,10,10a-tetrahydro-*4H*-phenanthren-3-one, and the second also a mixture but with different proportions.

**Step 4:**
*(rac)-(1S,4aS,10aS)*-7-Hydroxy-10a-methyl-1-(3-methyl-butyl)-1,4,4a,9,10,10a-hexahydro-*2H*-phenanthren-3-one. To rapidly stirred magnesium turnings (6.25 eq.) covered with dry THF (1.0 mL), was added approximately 1/3 of the total amount of 1-bromo-3-methylbutane. The rest of the 1-bromo-3-methylbutane (totally 2.08 eq.) was then added in small portions, after having initiated the reaction with a small crystal of iodine, followed by heating at 60 °C for 30 min. Another portion of dry THF (3.0 mL) was then added before cooling (dry ice / acetone bath). To this cool mixture was added a suspension of CuI (0.5 eq.) in THF (1.0 mL) before allowing it to come to 0°C for 10 min. and again cooling (dry-ice / acetone bath). A solution of a mixture of the enones (*rac*)*-*(*4aR,10aS*)-7-hydroxy-10a-methyl-4a,9,10,10a-tetrahydro-*4H*-phenanthren-3-one and (*rac*)-(*4aS,10aS*)-7-hydroxy-10a-methyl-4a,9,10,10a-tetrahydro-*4H*-phenanthren-3-one (220 mg, 1.0 eq.) in THF (2.0 mL) was then added dropwise. Stirring was continued at -78 °C for 2 h before quenching and workup as in **method E.** The crude product was purified on chromatotron using EtOAc:*n*-heptane (2:8, 3:7,4:6, v:v; stepwise gradient) as eluent to yield *(rac)*-*(1S,4aS,10aS)*-7-hydroxy-10a-methyl-1-(3-methyl-butyl)-1,4,4a,9,10, 10a-hexahydro-*2H*-phenanthren-3-one. ES/MS m/z: 299.2 (neg., M-H); ¹H NMR (270 MHz, CD₃OD) δ 6.90 (d, 1H), 6.5-6.6 (m, 2H), 3.00 (dd, 1H), 2.75-2.90 (m, 4H), 2.40 (t, 2H), 1.90-2.05 (m, 1H), 1.65-1.80 (m, 2H), 1.40-1.60 (m, 2H), 1.25-1.40 (m, 1H), 0.95-1.20 (m, 2H), 1.05 (s, 3H), 0.90 (t, 6H).

### Example 14: Synthesis of (rac)-(1S,4aS,10aS)-3,3-ethanediydimercapto-7-hydroxy-1-(3-methyl-butyl)-10a-methyl-1,2,3,4,4a,9,10,10a-octahydro-phenanthrene (E14).

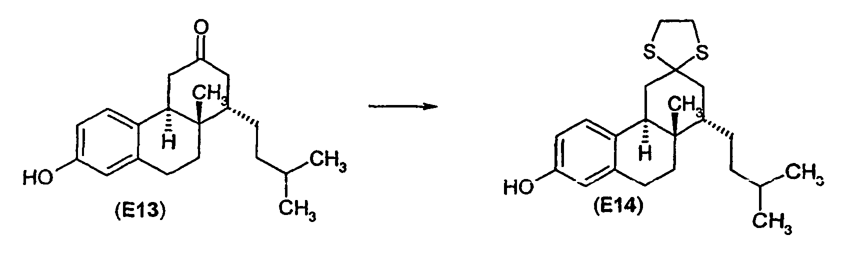

The ketone
(*rac*)-(*1S,4aS,10aS*)-7-hydroxy-10a-methyl-1-(3-methyl-butyl)-1,4,4a,9,10,10a-hexahydro-*2H*-phenanthren-3-one (48.5 mg) was treated according to **method G** ("J" = 3.0, "K" = 1,2-ethanedithiol, "L" = 2.0, "M" = 12). The crude product was purified on chromatotron, using EtOAc:*n*-heptane (2:8, v:v) as eluent to yield *(rac)-(1S,4aS,10aS)*-3,3-ethanediyldimercapto-7-hydroxy-1-(3-methyl-butyl)-10a-methyl-1,2,3,4,4a,9,10,10a-octahy dro-phenanthrene as a glass. ES/MS m/z: 377.2 (pos., M+H), 375.1 (neg., M-H); ¹H NMR (270 MHz, CD₃OD) δ 7.00 (d, 1H), 6.55 (dd, 1H), 6.50 (d, 1H), 3.20-3.40 (m, 4H), 2.55-2.90 (m, 4H), 2.50 (dd, 1H), 2.25 (d, 1H), 1.00-1.95 (m, 9H), 0.90 (dd, 6H), 0.85 (s, 3H).

### Example 15: Synthesis of (rac)-(1S,4aS,10aS)-7-hydroxy-10a-methyl-1-phenethyl-1,4,4a,9,10,10a-hexahydro-2H-phenanthren-3-one (E15).

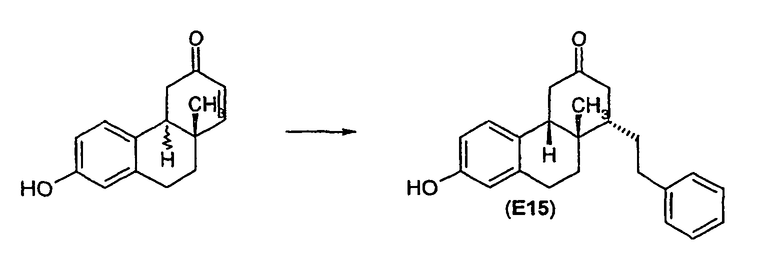

To rapidly stirred magnesium turnings (24 eq.) covered with dry THF (1.0 mL), was added approximately 1/3 of the total amount of 2-bromoethylbenzene. The rest of the 2-bromoethylben zene (totally 2.08 eq. in 2.0 mL THF) was then added in small portions, after having initiated the reaction with a small crystal of iodine, followed by heating at 60 °C for 30 min. Another portion of dry THF (3.0 mL) was then added before cooling (dry ice / acetone bath). To this cool mixture was added a suspension of CuI (0.5 eq.) in THF (0.5 mL) before allowing it to come to 0°C for 10min. and again cooling (dry-ice / acetone bath). A solution of a mixture of the enones (*rac*)-(*4aR,10aS*)-7-hydroxy-10a-methyl-4a, 9,10,10a-tetrahydro-*4H*-phenanthren-3-one and (*rac*)-(*4aS,10aS*)-7-hydroxy-10a-methyl-4a,9,10,10a-tetrahydro-*4H*-phenanthren-3-one (78 mg, 1.0 eq.) in THF (2.0 mL) from **Example 3** was then added dropwise. Stirring was continued at -78 °C for 2 h before quenching and workup as in **method E**. The crude product was purified by silica gel flash chromatography, using EtOAc:*n*-heptane (1:9, 15:85, 2:8, v:v; stepwise gradient) as eluent, followed by PHPLC to yield *(rac)-(1S,4aS,10aS)*-7-hydroxy-10a-methyl-1-phenethyl-1,4,4a,9,10,10a-hexahydro-*2H*-phenanthren-3-one together with unconsumed *(rac)-(4aS,10aS)*-7-hydroxy-10a-methyl-4a,9,10,10a-tetrahydro-*4H*-phenanthren-3-one. ES/MS m/z: 335.2 (pos., M+H), 333.4 (neg., M-H); ¹H NMR (270 MHz, CD₃CN) δ 7.10-7.30 (m, 5H), 6.90 (d, 1H), 6.45-6.75 (m, 3H), 2.95 (dd, 1H), 2.60-2.95 (m, 5H), 2.25-2.50 (m, 3H), 1.65-2.00 (m, 3H), 1.15-1.45 (m, 2H), 0.95 (s, 3H).

### Example 16:Synthesis of (rac)-(1S,2S,4aS,10aS)-7-Hydroxy-2,10a-dimethyl-1-(3-methyl-butyl)-1,4,4a,9,10,10a-hexahydro-2H-phenanthren-3-one (E16a); and (rac)-(1S,2R,4aS,10aS)-7-Hydroxy-2,10a-dimethyl-1-(3-methyl-butyl)-1,4,4a,9, 10,10a-hexahydro-2H-phenanthren-3-one (E16b).

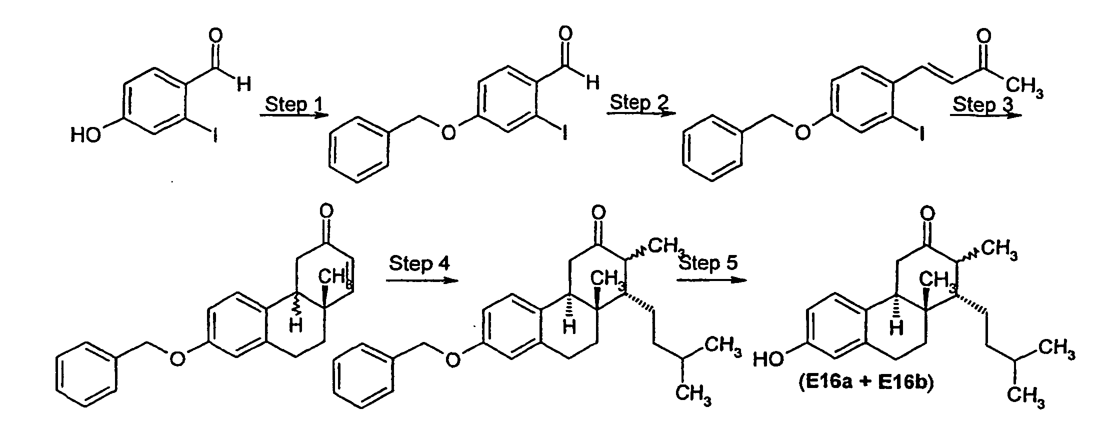

**Step 1:** 4-Benzyloxy-2-iodo-benzaldehyde. A mixture of 4-hydroxy-2-iodo-benzaldehyde (28.73 g, 248 mmol), benzyl bromide (1.3 eq.), ethyl diisopropylamine (1.4 eq.), and sodium iodide (0.1 eq.) in DCM (500 mL) was refluxed for 12 h. The remaining mixture was then washed with 1.0M HCl (aq., 3 x 100 mL), water (200 mL), saturated Sodium bicarbonate (100 mL), and saturated NH₄Cl (100 mL) before drying over anhydrous magnesium sulfate and concentration *in vacuo*. The crude product thus obtained was purified by silica gel flash chromatography using EtOAc:n-heptane (0:100, 1:9, 2:8, stepwise gradient) as eluent, to yield 4-benzyloxy-2-iodo-benzaldehyde as a yellow powder.

**Step 2:** 4-(4-Benzyloxy-2-iodo-phenyl)-but-3-en-2-one. To a stirred suspension of 4-benzyloxy-2-iodo-benzaldehyde (17.73 g, 52.4 mmol) in a water:acetone mixture (150 mL, 1:2), was added aqueous NaOH (16.0M, 1.2 eq.) before heating at reflux for 10 min. The yellowish precipitate formed after having added another portion of water (100 mL) and allowed to cool to room temperature was collected, washed with water, and dried over P₂O₅ to give 4-(4-benzyloxy-2-iodo-phenyl)-but-3-en-2-one as a yellow powder.

**Step 3:**
*(rac)-(4aS,10aR)*-7-Benzyloxy-10a-methyl-4a,9,10,10a-tetrahydro-*4H*-phenanthren-3-one and *(rac)-(4aS,10aS)*-7-Benzyloxy-10a-methyl-4a,9,10,10a-tetrahydro-*4H*-phenanthren-3-one. The aryl iodide 4-(4-benzyloxy-2-iodo-phenyl)-but-3-en-2-one (6.175 g) was treated according to **method A** ("J" = 2-methyl-but-3-en-1-ol, "K" = 1.3, "L" = 4.0, "M" = 0.5, "N" = 1.5). The material obtained was then treated according to **method C** ("N" = 150, "Q" = 2). The crude product obtained was purified by silica gel flash chromatography using EtOAc:n-heptane (5:95, 1:9, 2:8, stepwise gradient) as eluent, to yield *(rac)-(4aS,10aS)*-7-benzyloxy-10a-methyl-4a,9,10,10a-tetrahydro-*4H*-phenanthren-3-one (yellowish powder) as the first eluted material. The second eluted material contained a mixture of *(rac)-(4aS,10aS)*-7-benzyloxy-10a-methyl-4a,9,10,10a-tetrahydro-*4H*-phenanthren-3-one and *(rac)-(4aS,10aR)-7-benzyloxy-*10a-methyl-4a,9,10,10a-tetrahydro-*4H*-phenanthren-3-one.

**Step 4:**
*(rac)-(1S,2S,4aS,10aS)*-7-Benzyloxy-2,10a-dimethyl-1-(3-methyl-butyl)-1,4,4a,9,10,10a-hexahydro-*2H*-phenanthren-3-one and *(rac)-(1S,2R,4aS,10aS)*-7-Benzyloxy-2, 10a-dimethyl-1-(3-methyl-butyl)-1,4,4a,9,10,10a-hexahydro-*2H*-phenanthren-3-one. The enone *(rac)-(4aS,10aS)*-7-benzyloay-10a-methyl-4a,9,10,10a-tetrahydro-*4H-*phenanthren-3-one (70 mg) dissolved in THF (2.0 mL), was treated according to **method E** ("J" = lithium 2-thienylcyanocuprate [Aldrich, no. 32,417-5, 0.25M solution in THF], "K" = 2.0, "L" = 1.0, "M" = THF, "N" = 3-methyl-1-butyl magnesium bromide [0.5M solution in THF], "O" = 2.0, "P" = 0, "Q" = 0.5) with the following exception; Methyl iodide (16 eq. dissolved in 2.0 mL DMPU) was added to the reaction mixture, and stirring was continued for another 96 h at room temperature before quenching. The crude product was purified on PHPLC to yield a mixture of *(rac)-(1S,2S,4aS,10aS)*-7-benzyloxy-2,10a-dimethyl-1-(3-methyl-butyl)-1,4,4a,9,10,10a-hexahydro-*2H*-phenanthren-3-one and *(rac)-(1S,2R,4aS,10aS)*-7-benzyloxy-2, 10a-dimethyl-1-(3-methyl-butyl)-1,4,4a,9,10, 10a-hexahydro-*2H*-phenanthren-3-one (white powder) that was used in the next step (step 5).

**Step 5:**
*(rac)-(1S,2S,4aS,10aS)*-7-Hydroxy-2,10a-dimethyl-1-(3-methyl-butyl)-1,4,4a,9,10,10a-hexahydro-*2H*-phenanthren-3-one and *(rac)-(1S,2R,4aS,10aS)*-7-Hydroxy-2, 10a-dimethyl-1-(3-methyl-butyl)-1,4,4a,9,10,10a-hexahydro-*2H*-phenanthren-3-one. As the substrate, a mixture of (*rac*)*-*(*1S,2S,4aS,10aS*)*-*7-benzyloxy-2,10a-dimethyl-1-(3-methyl-butyl) -1,4,4a,9,10,10a-hexahydro-*2H*-phenanthren-3-one and *(rac)-(1S,*2R,*4aS,10aS*)-7-benzyloxy-2,10a-dimethyl-1-(3-methyl-butyl)-1,4,4a,9,10,10a-hexahydro-*2H*-phenanthren-3-one (29 mg) from the previous step (step 4) was treated according to **method F** ("J" = THF:HOAc [95:5], "K" = 3.0, "L" = 5% Pd/C, "M" =10, "N" = 72). The crude product was purified using PHPLC to yield a mixture of *(rac)-(1S,2S,4aS,10aS)*-7-hydroxy-2, 10a-dimethyl-1-(3-methyl-butyl)-1,4,4a,9,10,10a-hexahydro-*2H*-phenanthren-3-one and *(rac)-(1S,2R,4aS,10aS)*-7-hydroxy-2,10a-dimethyl-1-(3-methyl-butyl)-1,4,4a,9,10, 10a-hexahydro-*2H*-phenanthren-3-one as a colorless glass. ES/MS m/z (mixture): 315.4 (pos., M+H), 313.3 (neg., M-H); ¹H NMR (270 MHz, CD₃CN, selected signals of the mixture) δ 6.95 (d, H-C5, major isomer), 6.90 (d, H-C5, minor isomer), 6.10 (d, CH₃-C2, major isomer), 0.95 (d, CH₃-C2, minor isomer).

### Example 17:Synthesis of (rac)-(4bS,8S,8aS)-6,6-dimethoxy-8a-methyl-8-(3-methyl-butyl)-4b,5,6,7,8,8a, 9.10-octahydro-phenanthren-2-ol (E17a) and(rac)-(1S,4aS,10aS) -7-hydroxy-10a-methyl-1-(3-methyl-butyl)-1,4,4a,9,10,10a-hexahydro-2H-phenanthren-3-one (E17b).

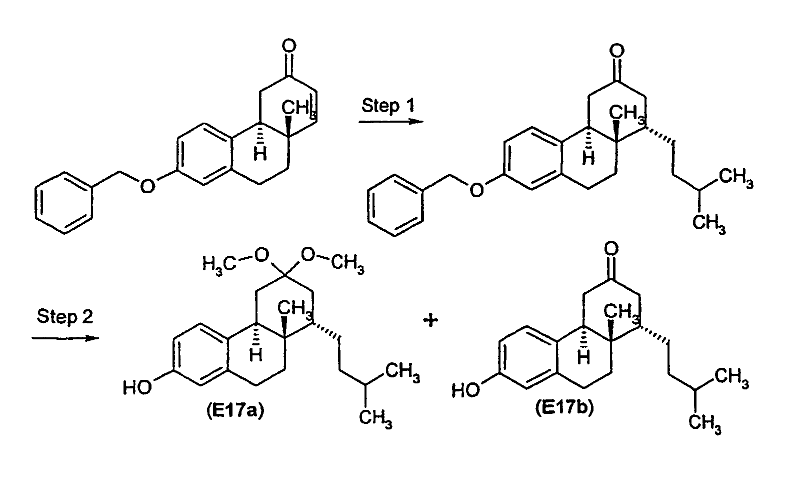

**Step 1:**
*(rac)-(1S,4aS,10aS)*-7-Benzyloxy-10a-methyl-1-(3-methyl-butyl)-1,4,4a,9,10,10a-hexahydro-*2H*-phenanthren-3-one. The enone (*rac*)-(*4aS,10aS*)-7-benzyloxy-10a-methyl-4a,9,10, 10a-tetrahydro-*4H*-phenanthren-3-one (303 mg) from **Example 16** dissolved in THF (4.0 mL), was treated according to **method E** ("J" = lithium 2-thienylcyanocuprate (Aldrich, no. 32,417-5, 0.25M solution in THF), "K" = 2.0, "N" = 3-methyl-1-butyl magnesium bromide (0.5M solution in THF), "O" = 2.0, "P" = 0, "Q" = 0.25). The crude product was purified on chromatotron, using EtOAc:*n*-heptane (1 :9, 2:8, v:v, stepwise gradient) as eluent to yield *(rac)-(1S,4aS,10aS)*-7-benzyloxy-10a-methyl-1-(3-methyl-butyl)-1,4,4a,9,10, 10a-hexahydro-*2H*-phenanthren-3-one as a white powder.

**Step 2:**
*(rac)-(1S, 4aS,10aS)*-7-Hydroxy-10a-methyl-1-(3-methyl-butyl)-1,4,4a,9,10,10a-hexahydro-*2H*-phenanthren-3-one and *(rac)-(4bS,8S,8aS)*-6,6-Dimethoxy-8a-methyl-8-(3-methyl-butyl)-4b,5,6,7,8,8a,9,10-octahydro-phenanthren-2-ol. As the substrate, (*rac*)*-*(*1S,4aS,10aS*)-7-benzyloxy-10a-methyl-1-(3-methyl-butyl)-1,4,4a,9,10, 10a-hexahydro-*2H*-phenanthren-3-one was treated according to **method F** ("J" = methanol:EtOAc [7:3], "K" = 15, "L" = 5% Pd/C, "M" = 40, "N" = 16). The crude product was fractionated on chromatotron, using EtOAc:*n*-heptane (1:9, 2:8, 3:7, v:v, stepwise gradient) as eluent to yield *(rac)-(4bS,8S,8aS)*-6,6-dimethoxy-8a-methyl-8-(3-methyl-butyl)-4b,5,6,7,8,8a,9,10-octahydro-phenanthren-2-ol as a colorless glass in the first eluted fraction, and *(rac)-(1S,4aS,10aS)*-7-hydroxy-10a-methyl-1-(3-methyl-butyl)-1, 4,4a,9,10,10a-hexahydro-*2H*-phenanthren-3-one (for an alternative synthetic method and spectroscopic data see earlier example) as a colorless glass in the second. *(rac)-(4bS,8S,8aS)*-6,6-Dimethoxy-8a-methyl-8-(3-methyl-butyl)-4b,5,6,7,8,8a,9, 10-octahydro-phenanthren-2-ol: ES/MS m/z: 345.1 (neg., M-H); ¹H NMR (270 MHz, CD₃OD) δ 7.05 (d, 1H), 6.55 (dd, 1H), 6.50 (d, 1H), 3.25 (s, 3H), 3.15 (s, 3H), 2.60-2.90 (m, 3H), 2.50 (dt, 1H), 2.10 (dt, 1H), 1.85 (dt, 1H), 1.75 (dd, 1H), 1.25-1.60 (m, 7H), 1.05-1.15 (m, 1H), 0.90 (dd, 6H), 0.85 (s, 3H); ¹³C NMR (CD₃OD) δ 154.60, 137.25, 129.90, 126.00, 114.75, 112.65, 101.35, 46.45, 46.35, 44.60, 38.45, 35.50, 35.10, 33.80, 31.70, 29.65, 28.35, 26.65, 24.85, 22.30, 21.50, 17.55.

### Example 18: Synthesis of

### (rac)-(1S,4aS,10aS)-3,3-ethanediyldioxy-7-hydroxy-1-(3-methyl-butyl)-10a-methyl-1,2,3,4,4a,9,10,10a-octahydro-phenanthrene (E18).

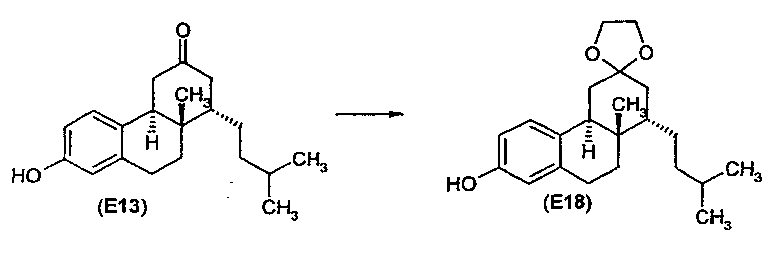

The ketone (*rac*)*-*(*1S,4aS,10aS*)-7-hydroxy-10a-methyl-1-(3-methyl-butyl)-1,4,4a,9, 10,10a-hexahydro-*2H*-phenanthren-3-one (10.4 mg) was treated according to **method H** ("J" = 1,2-dihydroxyethane, "K" = 0.5, "L" = 50, "M" = 1.0, "N" = 22, "O" = 5). The crude product was purified on chromatotron, using EtOAc:*n*-heptane (2:8, 3:7, v:v, stepwise gradient) as eluent to yield *(rac)-(1S,4aS,10aS)*-3,3-ethanediyldioxy-7-hydroxy-1-(3-methyl-butyl)-10a-methyl-1,2,3,4,4a,9,10,10a-octahydro-phenanthrene as a colorless glass. ES/MS m/z: 345.4 (pos., M+H), 343.3 (neg., M-H); ¹H NMR (270 MHz, CDCl₃) δ 7.05 (d, 1H), 6.55-6.65 (m, 2H), 4.60 (s, 1H), 3.90-4.10 (m, 4H), 2.65-2.95 (m, 3H), 2.25 (dt, 1H), 1.75-2.00 (m, 3H), 1.20-1.65 (m, 7H), 1.00-1.15 (m, 1H), 0.90 (dd, 6H), 0.85 (s, 3H); ¹³C NMR (CDCl₃) δ 153.15, 137.85, 131.90, 126.85, 115.15, 112.80, 110.20, 64.80, 63.80, 44.70, 38.35, 36.85, 35.25, 35.00, 33.60, 31.80, 28.40, 27.05, 24.95, 23.35, 22.35, 18.25.

### Example 19: Synthesis of (rac)-(4bS,8R,8aS)-8a-methyl-8-(3-methyl-butyl)-4b,5,6,7,8,8a,9,10-octahydro-phenanthren-2-ol (E19a); and (rac)-(4bS,6R,8S,8aS)-6-ethylsulfanyl-8a-methyl-8-(3-methyl-butyl)-4b,5,6,7,8,8a,9,10-octahydro-phenanthren-2-ol(E19b).

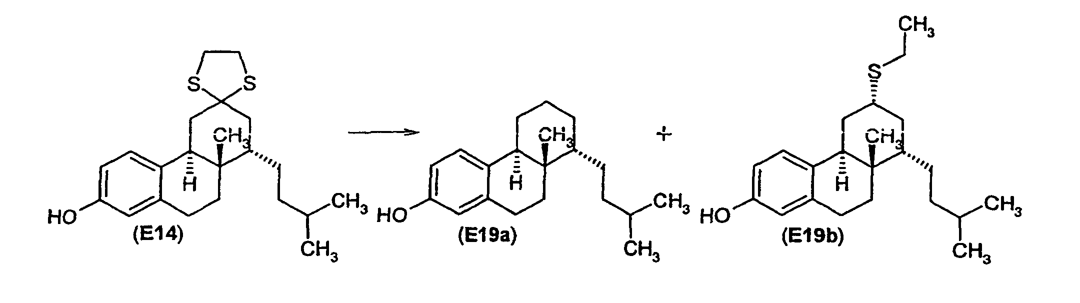

The substrate (*rac*)-(*1S,4aS,10aS*)-3,3-ethanediyldimercapto-7-hydroxy-1-(3-methyl-butyl)-10a-methyl-1,2,3,4,4a,9,10,10a-octahydro-phenanthrene (14.7 mg) was treated according to **method I** ("J" = 1.0, "K" = 240, "L" = 22, "M" = 16). The crude product was first purified on PHPLC, followed by fractionation on chromatotron, using EtOAc:*n*-heptane (2:8, 3:7, v:v, stepwise gradient) as eluent, to yield *(rac)-(4bS,8R,8aS)*-8a-methyl-8-(3-methyl-butyl)-4b,5,6,7,8,8a,9,10-octahydro-phenanthren-2-ol as a colorless glass in the first eluted fraction and *(rac)-(4bS,6R,8S,8aS)*-6-ethylsulfanyl-8a-methyl-8-(3-methyl-butyl)-4b,5,6,7,8,8a,9,10-octahydro-phenanthren-2-ol (1.3 mg) in the second. *(rac)-(4bS,8R,8aS)*-8a-Methyl-8-(3-methyl-butyl)-4b,5,6,7,8,8a,9,10-octahydro-phenanthren-2-ol: ES/MS m/z: 287.2 (pos., M+H), 285.1 (neg., M-H); ¹H NMR (270 MHz, CDCl₃) δ 7.10 (d, 1H), 6.60 (dd, 1H), 6.50 (d, 1H), 4.40 (s, 1H), 2.65-2.95 (m, 2H), 2.60 (dd, 1H), 2.05-2.20 (m, 1H), 1.70-2.00 (m, 2H), 1.00-1.70 (m, 11H), 0.875 (dd, 6H), 0.85 (s, 3H); ¹³C NMR (CDCl₃) δ 152.95, 138.00, 133.00, 126.75, 115.10, 112.60, 44.45, 38.90, 38.40, 35.45, 34.15, 28.45, 27.00, 25.20, 24.0 (two peaks), 23.15, 22.45, 21.10, 18.45. *(rac)-(4bS,6R,8S,8aS)*-6-Ethylsulfanyl-8a-methyl-8-(3-methyl-butyl)-4b,5,6,7,8,8a,9, 10-octahydro-phenanthren-2-ol: ES/MS m/z: 347.2 (pos., M+H), 345.1 (neg., M-H); ¹H NMR (270 MHz, CDCl₃) δ 7.05 (d, 1H), 6.60 (dd, 1H), 6.55 (d, 1H), 4.40 (s, 1H), 3.25-3.35 (m, 1H), 3.05 (dd, 1H), 2.65-2.95 (m, 2H), 2.60 (q, 2H), 2.30 (br. d, 1H), 2.20 (dt, 1H), 1.00-2.00 (m, 10H), 1.25 (t, 3H), 0.90 (dd, 6H), 0.85 (s, 3H).

### Example 20: Synthesis of (rac)-(1S,4aS,10aS)-3,3-(propane-1,3-diyldimercapto)-7-hydroxy-1-(3-methyl-butyl)-10a-methyl-1,2,3,4,4a,9, 10,10a-octahydro-phenanthrene (E20).

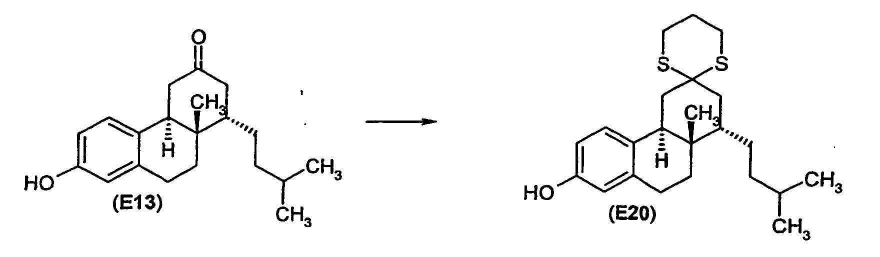

The ketone (*rac*)-(*1S,4aS,10aS*)-7-hydroxy-10a-methyl-1-(3-methyl-butyl)-1, 4,4a,9,10,10a-hexahydro-*2H*-phenanthren-3-one (11 mg) was treated according to **method G** ("J" = 3.0, "K"=1,3-propanedithiol, "L" = 2.0, "M" = 16). The crude product was purified on chromatotron using EtOAc:*n*-heptane (2:8, v:v) as eluent to yield *(rac)-(1S,4aS,10aS)*-3,3-(propane-1,3-diyldimercapto)-7-hydroxy-1-(3-methyl-butyl)-10a-methyl-1,2,3,4,4a,9,10,10a-octahydro-phenanthrene (colorless glass). ES/MS m/z: 391.3 (pos., M+H), 389.2 (neg., M-H); ¹H NMR (270 MHz, CDCl₃) δ 7.10 (d, 1H), 6.65 (dd, 1H), 6.55 (d, 1H), 4.50 (s, 1H), 2.65-3.20 (m, 8H), 1.70-2.35 (m, 7H), 1.30-1.60 (m, 5H), 1.05-1.15 (m, 1H), 0.90 (dd, 6H), 0.85 (s, 3H).

### Example 21: Synthesis of (rac)-(4as,10as)-7-hydroxy-10a-methyl-1,4,4a,9,10, 10a-hexahydro-2H-phenanthren-3-one (E21).

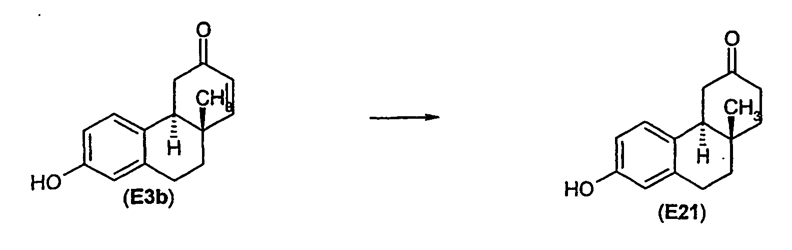

The substrate (*rac*)*-(4aS,10aS*)-7-hydroxy-10a-methyl-4a,9,10,10a-tetrahydro-*4H*-phenanthren-3-one (16 mg) was treated according to **method F** ("J" = methanol, "K" = 1.5, "L" = 5% Pd/C, "M" = 5, "N" = 16) to obtain *(rac)-(4aS,10aS)*-7-hydroxy-10a-methyl-1,4,4a,9,10,10a-hexahydro-*2H*-phenanthren-3-one. ES/MS m/z: 229.0 (neg., M-H); ¹H NMR (270 MHz, CD₃OD) δ 6.90 (d, 1H), 6.45-6.60 (m, 2H), 2.60-3.00 (m, 5H), 2.25-2.40 (m, 2H), 1.50-1.90 (m, 4H), 0.90 (s, 3H).

### Example 22: Synthesis of (rac)-(4aS,10aS)-3,3-ethanediyldimercapto-7-hydroxy-10a-methyl-1,2,3,4,4a,9,10,10a-octahydro-phenanthrene (E22).

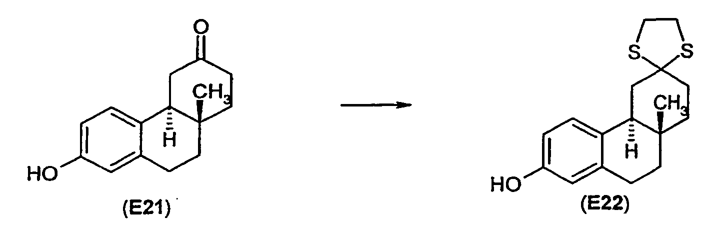

The ketone (*rac*)*-*(*4aS,10aS*)-7-hydroxy-10a-methyl-1,4,4a,9,10,10a-hexahydro-*2H*-phenanthren-3-one (12 mg) was treated according to **method G** ("J" = 3.0, "K" = 1,2-ethanedithiol, "L" = 2.0, "M" =16). The crude product was purified on chromatotron, using EtOAc:*n*-heptane (1:9, 2:8, v:v, stepwise gradient) as eluent to yield *(rac)-(4aS,10aS)*-3,3-ethanediyldimercapto-7-hydroxy-10a-methyl-1,2,3,4,4a,9, 10,10a-octahydro-phenanthrene as a white powder. ES/MS m/z: 307.3 (pos., M+H), 305.2 (neg., M-H); ¹H NMR (270 MHz, CD₃OD) δ 6.90 (d, 1H), 6.45-6.55 (m, 2H), 3.20-3.40 (m, 4H), 2.55-2.90 (m, 4H), 2.15-2.30 (m, 1H), 2.00 (dq, 1H), 1.90 (t, 1H), 1.50-1.60 (m, 4H), 0.70 (s, 3H).

### Example 23: Synthesis of (rac)-(4aS,10aS)-10a-ethyl-7-hydroxy-1,4,4a,9,10, 10a-hexahydro-2H-phenanthren-3-one (E23).

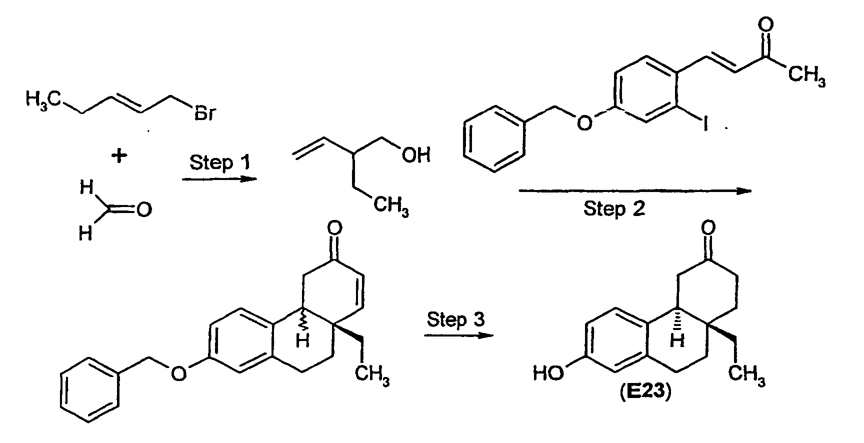

**Step 1:** (rac)-2-Ethyl-but-3-en-1-ol. Using the allylic bromide 1-bromo-2-pentene (10.0 g) and the aldehyde formaldehyde (37% solution in water) as starting materials according to **method D** ("J" = 3.0, "K" = 18), a crude product was obtained. This was purified by distillation (68-70 °C/80 mmHg) to yield (*rac*)-2-ethyl-but-3-en-1-ol as a colorless oil. ¹H NMR (270 MHz, CDCl₃) δ 5.45-5.70 (m, 1H), 5.05-5.20 (m, 2H), 3.55 (dd, 1H), 3.40 (dd, 1H), 2.00-2.20 (m, 1H), 1.15-1.60 (m, 2H), 0.90 (t, 3H).

**Step 2:**
*(rac)-(4aR,10aS)*-7-Benzyloxy-10a-ethyl-4a,9,10,10a-tetrahydro-*4H*-phenanthren-3-one and *(rac)-(4aS,10aS)*-7-Benzyloxy-10a-ethyl-4a,9,10,10a-tetrahydro-*4H*-phenanthren-3-one. The aryl iodide 4-(4-benzyloxy-2-iodo-phenyl)-but-3-en-2-one (1.0 g) was treated according to **method A** ("J" = (*rac*)-2-ethyl-but-3-en-1-ol, "K" = 2.0, "L" = 4.0, "M" = 8.0, "N" = 4.0). The material obtained was then treated according to **method C** ("N" = 70, "Q" = 16). The crude product obtained was purified by silica gel flash chromatography using EtOAc:*n*-heptane (2:8). The first eluted fraction was then further fractionated using PHPLC. *(rac)-(4aR,10aS)*-7-Benzyloxy-10a-ethyl-4a,9,10,10a-tetrahydro-*4H*-phenanthren-3-one (off-white solid) was obtained in the first eluted fraction.
*(rac)-(4aS,10aS)*-7-Benzyloxy-10a-ethyl-4a,9,10,10a-tetrahydro-*4H*-phenanthren-3-one (yellowish solid) was obtained in the second eluted fraction. *(rac)-(4aR,10aS)*-7-Benzyloxy-10a-ethyl-4a,9,10,10a-tetrahydro-*4H*-phenanthren-3-one: ES/MS m/z: 333.1 (pos., M+H); ¹H NMR (270 MHz, CDCl₃) δ 7.30-7.45 (m, 5H), 7.00 (d, 1H), 6.80 (d, 1H), 6.75 (d, 1H), 6.70 (d, 1H), 6.00 (d, 1H), 5.05 (s, 2H), 3.15 (dd, 1H), 2.75-2.85 (m, 2H), 2.45-2.60 (m, 2H), 1.85-1.95 (m, 1H), 1.65-1.75 (m, 1H), 1.50 (q, 2H), 0.90 (s, 3H).
*(rac)-(4aS,10aS)*-7-Benzyloxy-10a-ethyl-4a,9,10,10a-tetrahydro-*4H*-phenanthren-3-one: ES/MS m/z: 333.1 (pos., M+H); ¹H NMR (270 MHz, CDCl₃) δ 7.30-7.50 (m, 5H), 7.00 (d, 2H), 6.82 (d, 1H), 6.78 (d, 1H), 6.05 (d, 1H), 5.05 (s, 2H), 3.30 (dd, 1H), 3.05 (dd, 1H), 2.85-2.95 (m, 2H), 2.60 (dd, 1H), 2.10-2.20(m, 1H), 1.65-1.75 (m, 1H), 1.35-1.50 (m, 2H), 0.90 (s, 3H).

**Step 3:** *(rac)-(4aS,10aS)*-10a-Ethyl-7-hydroxy 1,4,4a,9,10,10a-hexahydro-*2H*-phenanthren-3-one. As substrate, (*rac*)-(*4aS,10aS*)-7-Benzyloxy-10a-ethyl-4a,9,10,10a-tetrahydro -*4H*-phenanthren-3-one (34 mg) was used according to **method F** ("J" = MeOH/EtOAc [4:3], "K" = 4.0, "L" = 5% Pd/C, "M" = 11, N = 24). The crude product was purified on chromatotron, using EtOAc:*n*-heptane (1:9) as eluent. The main fraction contained *(rac)-(4aS,10aS)*-10a-ethyl-7-hydroxy-1,4,4a,9,10,10a-hexahydro-*2H*-phenanthren-3-one. ES/MS m/z: 241.3 (neg., M-H), ¹H NMR (500 MHz, CDCl₃) δ 6.95 (d, 1H), 6.70-6.75 (m, 2H), 5.15 (bs, 1H) 2.90-3.00 (m, 2H), 2.70-2.80 (m, 2H), 2.35-2.45 (m, 3H), 2.05-2.15 (m, 1H), 1.95-2.00 (m, 1H), 1.40-1.55 (m, 3H), 1.20-1.30 (m, 1H), 0.95 (t, 3H).

### Example 24: Synthesis of (rac)-(4aS,10aS)-3,3-ethanediyldimercapto-10a-ethyl-7-hydroxy-1,2,3,4,4a,9,10,10a-octahydro-phenanthrene (E24).

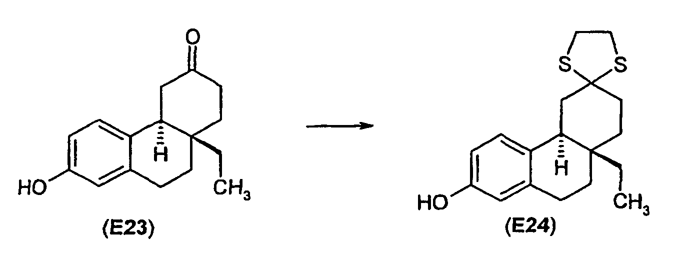

The ketone (*rac*)-(*4aS,10aS*)-10a-ethyl-7-hydroxy-1,4,4a,9,10,10a-hexahydro-*2H*-phenanthren-3-one (8.9 mg) was treated according to **method G** ("J" = 0.8, "K" = 1,2-ethanedithiol, "L" = 2.0, "M" = 4). The crude product was purified on chromatotron, using EtOAc:*n*-heptane (2:8, v:v) as eluent. The first eluted fraction was then further fractionated using PHPLC to yield *(rac)-(4aS,10aS)*-3,3-ethanediyldimereapto-10a-ethyl-7-hydroxy-1,2,3,4,4a,9,10,10a-octahydro-phenanthrene (white solid). ES/MS m/z: 320.9 (pos., M+H), 319.3 (neg., M-H); ¹H NMR (270 MHz, CDCl₃) δ 7.10 (d, 1H), 6.55-6.60 (m, 2H), 4.50 (bs, 1H), 3.25-3.40 (m, 4H), 2.70-2.90 (m, 3H), 2.55-2.65 (m, 1H), 1.95 -2.20 (m, 3H), 1.75-1.90 (m, 2H), 1.25-1.50 (m, 3H), 0.95-1.10 (m, 1H), 0.75 (t, 3H). ¹³C NMR δ 153.29, 138.26, 130.68, 125.83, 115.34, 112.53, 69.22, 46.08, 40.64, 38.99, 38.17, 38.01, 34.66, 34.08, 32.81, 26.1, 17.54, 7.37.

### Example 25: A pharmaceutical formulation comprising (rac)-(1R,2R,4aS,10aS) -1-butyl-3,3-ethanediyldimercapto-7-hydroxy-2-methyl-1,2,3,4,4a,9,10, 10a-octahydro-phenanthrene.

140 mg of *(rac)-(1R,2R,4aS,10aS)*-1-butyl-3,3-ethanediyldimereapto-7-hydroxy -2-methyl-1,2,3,4,4a,9,10,10a-octahydro-phenanthrene, from Example 12, is formulated with sufficient finely divided lactose to provide a total amount of 580 to 590 mg to fill a size 0, hard-gelatine capsule.

### Example 26: Synthesis of (rac)-(1S,4aS,10aS)-1-(3-methyl-butyl)-10a-ethyl-7-hydroxy-1,4,4a,9,10,10a-hexahydro-2H-phenanthren-3-one (E26).

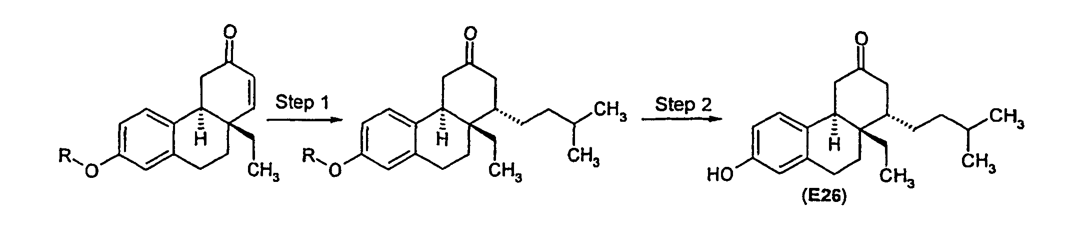

**Step 1:** The enone
*(rac)-(4aS,10aS)*-7-benzyloxy-10a-ethyl-4a,9,10,10a-tetrahydro-*4H*-phenantren-3-one (54.5mg; 0.15mmol), dissolved in 2.0 mL of THF, was treated according to **method E** ("J"= lithium-2-thienylcyanocuprate [Aldrich, no.32,417-5, 0.25M sol. in THF], "K"= 2, "M"= THF, "L"= 2.0, "N"= 3-methyl-1-butyl magnesium bromide [0.5M sol. in THF], "O"= 2eq., "P"= 0, "Q"= 1). The crude product was purified on chromatotron, using EtOAc:*n*-heptane (2:8) as eluent to yield 35mg of *(rac)-(1S,4aS,10aS)*-7-benzyloxy-1-(3-methyl-butyl)-1,4,4a,9,10,10a-hexahydro-*2H*-phenanthren-3-one as a yellowish oil. ES-MS m/z: 405.4 (pos., M+H), 403.0 (neg., M-H; ¹H NMR (CDCl₃) δ 7.30-7.45 (m, 5H), 6.95 (d, 1H), 6.75 (d, 1H), 5.05 (s, 2H), 3.15 (dd, 1H), 2.90 (dd, 1H), 2.70-2.80 (m, 2H), 2.60 (dd, 1H), 2.30-2.45 (m, 2H), 1.40-2.00 (m, 5H), 1.20-1.40 (m, 6H), 0.85 (m, 9H).

**Step 2:** The reaction was carried out on 32.5mg (0.080mmol)
*(rac)-(1S,4aS,10aS)*-7-benzyloxy-1-(3-methyl-butyl)-1,4,4a,9,10,10a-hexahydro-*2H*-phenanthren-3-one according to **method F** ("J"= THF, "K"= 4.0, "L"= 10% Pd/C, "M"= 33, "N"= 20). The crude product was purified by chromatotron using EtOAc: *n*-heptane (2:8) as eluent to yield *(rac)-(1S,4aS,10aS)*-1-(3-methyl-butyl)-10a-ethyl-7-hydroxy-1,4,4a,9,10,10a-hexahydro-*2H*-phenanthren-3-one (18.6 mg) as a white solid. ES-MS m/z: 315.1(pos., M+H), 313.3 (neg., M-H); ¹H NMR (270MHz, CDCl₃) δ 6.90 (d, 1H), 6.65 (d, 1H), 6.60 (s, 1H), 3.15 (dd, 1H), 2.90 (ddd, 1H), 2.70-2.80 (m,2H), 2.60 (dd, 1H), 2.35-2.45 (m, 2H), 1.40-2.00 (m, 5H), 1.20-1.40 (m, 5H), 1.00-1.10 (m,1H), 0.85 (m, 9H).

### Example 27: Synthesis of (1S,4aS,10aS)-7-hydroxy-10a-methyl-1-(3-methyl-butyl)-1,4,4a,9,10,10a-hexahydro-2H-phenanthren-3-one (E27).

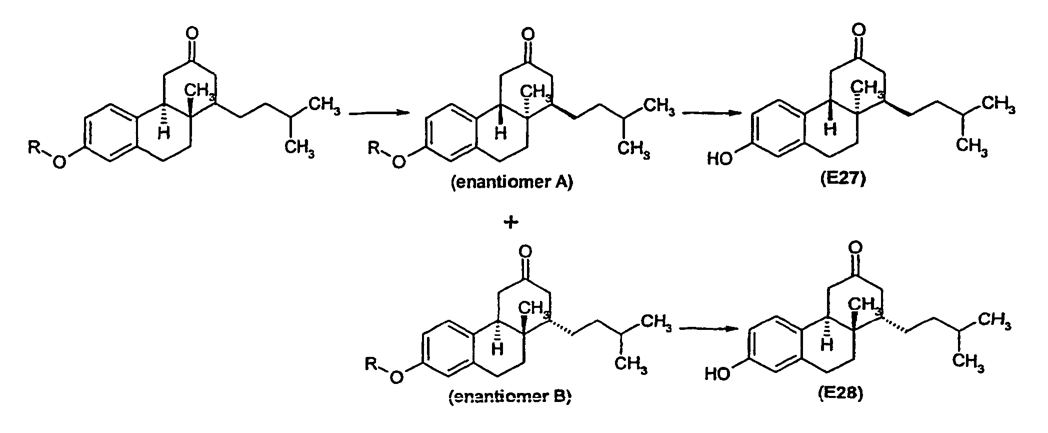

**Step 1:** The racemic mixture of *(1S,4aS,10aS)*-7-benzyloxy-10a-methyl-1-(3-methyl-butyl)-1,4,4a,9,10,10a-hexahydro-*2H*-phenanthren-3-one from **Example 13** was purified by chiral HPLC to afford two pure enantiomers (17 and 21 mg), *(1R,4aR,10aR)*-7-benzyloxy-10a-methyl-1-(3-methyl-butyl)-1,4,4a,9,10,10a-hexahydro-*2H*-phenanthren-3-one resp. *(1S,4aS,10aS)*-7-benzyloxy-10a-methyl-1-(3-methyl-butyl)-1,4,4a,9,10, 10a-hexahydro-*2H*-phenanthren-3-one.

**Step 2:** The reaction was carried out on 17mg *(1R,4aR,10aR)*-7-benzyloxy-10a-methyl-1-(3-methyl-butyl)-1,4,4a,9,10,10a-hexahydro-*2H*-phenanthren-3-one for 20 h according to **method F** using THF (4.0 mL) as solvent, and 10% Pd/C (33.0 mg) as catalyst. The crude product was purified by PHPLC to yield *(1R,4aR,10aR)*-7-hydroxy-10a-methyl-1-(3-methyl-butyl)-1,4,4a,9,10,10a-hexahydro-*2H*-phenanthren-3-one (1.5 mg) as a white solid. ES-MS m/z: 301.3 (pos., M+H); ¹H NMR (270MHz, CDCl₃) δ 6.90 (d, 1H), 6.60-6.65 (m, 2H), 4.50 (s, 1H), 3.05 (dd, 1H), 2.70-3.05 (m, 4H), 2.25-2.45 (m, 2H), 1.90-2.05 (m, 1H), 1.40-1.75 (m, 4H), 1.25-1.40 (m, 1H), 0.95-1.20 (m, 2H), 1.05 (s, 3H), 0.85 (t, 6H).

### Example 28: Synthesis of (1R,4aRS,10aR)-7-benzyloxy-10a-methyl-1-(3-methyl-butyl)-1,4,4a, 9,10,10a-hexahydro-2H-phenanthren-3-one (E28).

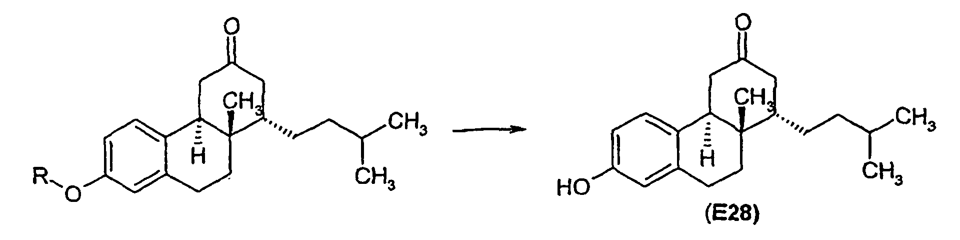

The reaction was carried out on 21 mg *(1S,4aS,10aS)*-7-hydrohy-10a-methyl-1-(3-methyl-butyl)-1,4,4a,9,10,10a-hexahydro-*2H*-phenanthren-3-one for 20 h according to **method F** using THF (4.0 mL) as solvent, and 10% Pd/C (33.0 mg) as catalyst. The crude product was purified by PHPLC to yield *(1S,4aS,10aS)*-7-benzyloxy-10a-methyl-1-(3-methyl-butyl)-1,4,4a,9,10,10a-hexahydro-*2H*-phenanthren-3-one (3.9 mg) as a white solid. ES-MS m/z: 301.3 (pos., M+H); ¹H NMR (270MHz, CDCl₃) δ 6.90 (d, 1H), 6.60-6.65 (m, 2H), 4.65 (s, 1H), 3.05 (dd, 1H), 2.70-3.00 (m, 4H), 2.25-2.45 (m, 2H), 1.90-2.00 (m, 1H), 1.40-1.75 (m, 4H), 1.25-1.40 (m, 1H), 0.95-1.20 (m, 2H), 1.05 (s, 3H), 0.85 (t, 6H).

### Example 29: Synthesis of (rac)-(1S,4aS,10aS)-1-(3-methyl-butyl)-3, 3-ethanediyldimercapto-7-hydroxy-1,4,4a,9,10,10a-octahydrophenanthrene (E29).

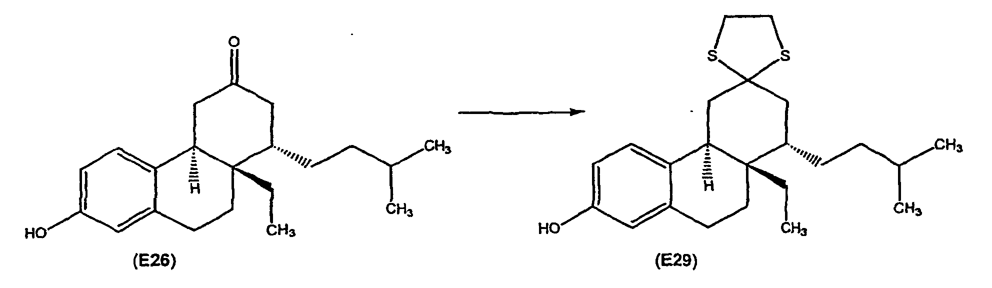

The reaction was carried out on 15 mg (0.048 mmol) of *(rac)-(1S,4aS,10aS)*-1-(3-methyl-butyl)-7-hydroxy-1,4,4a,9,10,10a-hexahydro-*2H*-phenanthren-3-one, according to **method G** ("J"=1.0, "K"=1,2-ethanedithiol, "L"=2.0, "M"=4), using 1 eq of BF₃·OEt₂. The crude product was purified by chromatotron using EtOAc:*n*-heptane (2:8) as eluent and then by PHPLC to yield *(rac)-(1S,4aS,10aS)*-1-(3-methyl-butyl)-3,3-ethanediyldimercapto-7-hydroxy-1,4,4a,9,10,10a-octahydrophenanthrene (7.7 mg) as a white solid. ES-MS m/z: 391.3 (pos., M+H), 389.2 (neg., M-H); ¹H NMR (270MHz, CDCl₃) δ 7.05 (d, 1H), 6.60 (dd, 1H), 6.55 (s, 1H), 4.50 (bs, OH), 3.35 (m, 4H), 3.00 (dd, 1H), 2.55-2.80 (m, 4H), 2.35-2.45 (dd, 1H), 2.15-2.25 (m, 1H9, 2.00-2.10 (m, 1H), 1.40-1.80 (m, 8H), 1.05-1.30 (m, 1H), 0.90 (d, 6H), 0.75 (t, 3H).

### Example 30: Synthesis of (rac)-(4aR,10aR)-7-hydroxy-4a,10a-dimethyl-3,4,4a,9,10,10a-hexahydro-1H-phenanthren-2-one (E30).

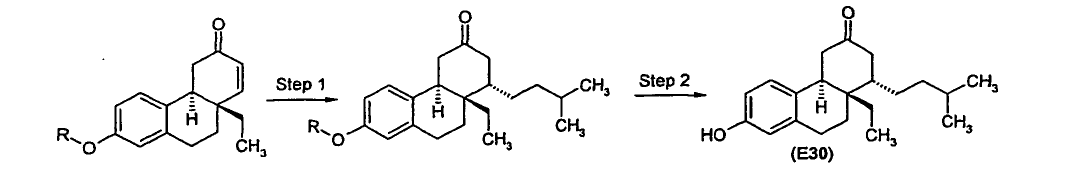

**Step 1:.** 7-Methoxy-4-methyl-1,2-dihydro-naphthalene: A solution of 31.8 g (180 mmol, 1.0 eq.) of 6-methoxy-1-tetralone in dry THF (100 mL) was added dropwise while stirring to a solution of methyl lithium (1.6 M in diethyl ether, 2.3 eq.) during approximately 1 h. Stirring was continued for another 2 h before slow addition of 200 mL of 5.0 M aqueous HCl. The phases were separated after vigorous stirring for 3 h. The aqueous phase was extracted with diethyl ether (4 x 100 mL) and the combined organic phases were dried over anhydrous sodium sulfate. Concentration of the extract in vacuo affored a brown oil which was purified by silica gel flash chromatography using EtOAc:*n*-heptane (0:10, 1:9, stepwise gradient) as eluent to give 7-Methoxy-4-methyl-1,2-dihydro-naphthalene as a colorless oil. ¹H NMR (CDCl₃) δ 7.22 (d, 1H), 6.80-6.85 (m, 2H), 5.75-5.80 (m, 1H), 3.85 (s, 3H), 2.75-2.85 (m, 2H), 2.25-2.35 (m, 2H), 2.15 (s, 3H).

**Step 2:** *(rac)*-6-Methoxy-1-methyl-3,4-dihydro-*1H*-naphthalen-2-one: The reaction was carried out on 24.8 g (142 mmol) of **11**, dissolved in 600 mL DCM according to **method L** to give *(rac)*-6-Methoxy-1-methyl-3,4-dihydro-*1H*-naphthalen-2-one as a yellowish oil after purification of the crude product by silica gel flash chromatography, using EtOAc:n-heptane (1:9, 2:8, stepwise gradient) as eluent ¹H NMR (CDCl₃) δ 7.10 (d, 1H), 6.80 (d, 1H), 6.75 (s, 1H), 3.80 (s, 3H), 3.45 (m, 1H), 3.00-3.10 (m, 2H), 2.45-2.65 (m, 2H), 1.45 (t, 3H); ¹³C NMR (CDCl₃) δ 213.18, 158.77, 138.31, 139.22, 127.36, 113.35, 112.34, 55.15, 46.48, 36.93, 27.95, 14.04.

**Step 3:** *(rac)*-7-Methoxy-4a-methyl-4,4a,9,10-tetrahydro-*3H*-phenanthren-2-one: Coupling of a solution of *(rac)*-6-Methoxy-1-methyl-3,4-dihydro-*1H*-naphthalen-2-one (2.10 g, 11.0 mmol) dissolved in toluene (10 mL) with but-3-en-2-one in accordance with **method M** after purification of the crude product by silica gel flash chromatography using EtOAc:*n*-heptane (3:7) as eluent, afforded *(rac)*-7-Methoxy-4a-methyl-4,4a, 9,10-tetrahydro-*3H*-phenanthren-2-one as a yellowish solid. ES-MS m/z: 243.4 (pos., M+H); ¹H NMR (CDCl₃) δ 7.10 (d, 1H), 6.70 (dd, 1H), 6.50 (d, 1H), 5.80 (s, 1H), 3.70 (s, 3H), 1.80-2.85 (m, 8H), 1.40 (s, 3H); ¹³C NMR (CDCl₃) δ 199.26, 170.35, 157.96, 136.22, 127.38, 124.26, 113.40, 112.99, 54.93, 38.38, 36.80, 34.51, 30.98, 30.80, 27.37.

**Step 4.** 7-Methoxy-4a,1 1 0a-dimethyl-3,4,4a,9,10,10a-hexahydro-*1H*-phenanthren-2-one: The reaction was carried out on 200 mg (0.83 mmol) of *(rac)*-7-Methoxy-4a-methyl-4, 4a,9,10-tetrahydro-*3H*-phenanthren-2-one dissolved in THF (10 mL), according to **method Q** using the following conditions: Titanium (IV) isopropoxide (1.3 eq., suspension in 3 mL THF), methylmagnesium chloride (3M solution in THF, 1.3 eq.), nickel(II)acetylacetonate (0.05 eq.), reaction-time and temperature (1 h from -15°C to 0 °C, quenching at r.t.). The crude product was purified using PHPLC to yield 7-Methoxy-4a, 10a-dimethyl-3,4,4a, 9,10,10a-hexahydro-*1H*-phenanthren-2-one as a yellowish oil. ES-MS m/z: 259.0 (pos., M+H); ¹H NMR (CDCl₃) δ 7.30 (d, 1H), 6.80 (dd, 1H), 6.60 (d, 1H), 3.75 (s, 3H), 2.85 (t, 3H), 1.80-2.50 (m, 7H), 1.25 (s, 3H), 1.00 (t, 3H); ¹³C NMR (CDCl₃) δ 157.85, 136.33, 135.25, 127.04, 113.89, 113.01, 54.97, 50.17, 40.00, 39.29, 38.40, 35.67, 31.38, 25.87, 25.41, 23.06.

**Step 5:**
*(rac)-(4aR,10aR)*-7-Hydroxy-4a, 10a-dimethyl-3,4,4a,9,10,10a-hexahydro-*1H*-phenanthren-2-one: The reaction was carried out on 55 mg of 7-Methoxy-4a,10a-dimethyl-3, 4,4a,9,10, 10a-hexahydro-*1H*-phenanthren-2-one dissolved in 1.0 mL DCM, using 3.5 eq. of BBr₃ for 4 h according to **method O.** The crude product was purified by PHPLC to yield *(rac)-(4aR,10aR)-*7*-* Hydroxy-4a, 10a-dimethyl-3,4,4a,9,10,10a-hexahydro-*1H*-phenanthren-2-one as a yellowish oil. ES-MS m/z: 245.2 (pos., M+H), 243.4 (neg., M-H); ¹H NMR (CDCl₃) δ 7.20 (d, 1H), 6.70 (dd, 1H), 6.55 (d, 1H), 5.30 (b s, 1H), 2.80 (t, 3H), 1.80-2.40 (m, 7H), 1.25 (s, 3H), 1.00 (t, 3H).

### Example 31: Synthesis of (rac)-(1S,4aS,10aS)-7-hydroxy-10a-methyl-4-phenyl-1,4,4a,9,10,10a-hexahydro-2H-phenanthren-3-one (E31).

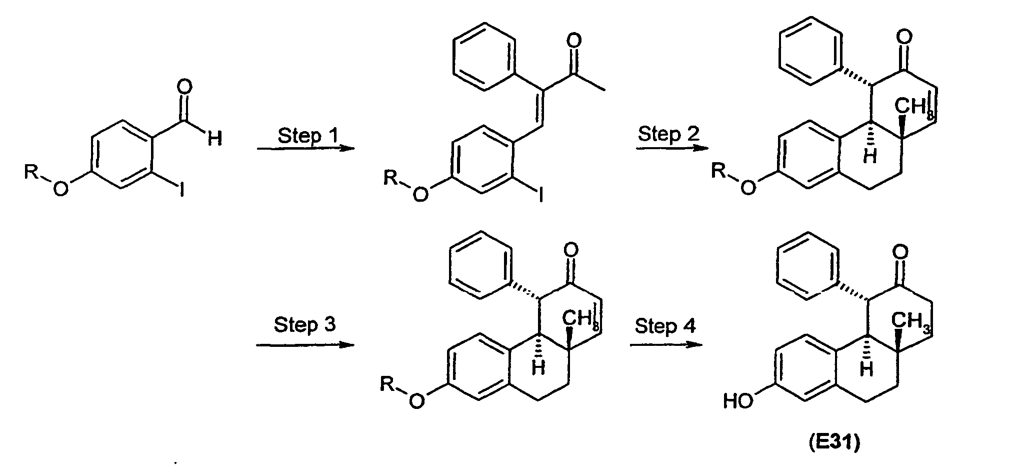

**Step 1:** (*Z*)-5-(4-Benzyloxy-2-iodo-phenyl)-4-phenyl-pent-4-en-2-one. The reaction was carried out on 2.0 g (5.9 mmol) of 4-benzyloxy-2-iodo-benzaldehyde, according to **method L** ("J"= 10, "K"= 1.02, "L"= 0.1, "M"= 20). The crude product was purified by flash chromatography using EtOAc:*n*-heptane (2:8) as eluent to yield (*Z*)-5-(4-Benzyloxy-2-iodo-phenyl)-4-phenyl-pent-4-en-2-one as a yellowish oil. ES-MS m/z: 455.2 (pos., M+H), 452.9 (neg., M-H); ¹H NMR (270 MHz, CDCl₃) δ 7.65 (s, 1H), 7.50 (d, 1H), 7.20-7.40 (m, 7H), 7.05-7.10 (m, 2H), 7.15-7.20 (m, 1H), 6.60 (d, 2H), 5.00 (s, 2H), 2.45 (s, 3H).

**Step 2:**
*(4aS, 10aS)*-7-Benzyloxy10a-methyl-4-phenyl-4a,9,10,10-tetrahydro-*4H*-phenantren-3-one. The reaction was carried out on 630 mg (1.39 mmol) of (Z)-5-(4-benzyloxy-2-iodo-phenyl)-4-phenyl-pent-4-en-2-one, according to **method A** ("J"= 2-methyl-but-3-en-1-ol, "K"= 1.5, "L"= 4.0, "M"= 2.9, "N"= 4). The material obtained was then treated according to **method C** to obtain crude *(4aS*,*10aS)*-7-benzyloxy-10a-methyl-4-phenyl-4a,9,10, 10-tetrahydro-*4H*-phenantren-3-one (90 mg).

**Step 3:**
*(4R,4aS,10aS)*-7-Benzyloxy-10a-methyl-4-phenyl-4a,9,10,10-tetrahydro-*4H*-phenantren-3-one. *(4aS,10aS)*-7-Benzyloxy10a-methyl-4-phenyl-4a,9,10,10-tetrahydro-*4H*-phenantren-3-one was purified with flash chromatography using EtOAc:*n*-heptane (5:1)as eluent to yield two isomers with the ratio 2.5:1. Separation by PHPLC to yield
*(4R,4aS,10aS)*-7-Benzyloxy-10a-methyl-4-phenyl-4a,9,10,10-tetrahydro-*4H*-phenantren-3-one. ES-MS m/z: 395.2(pos., M+H), 393. 1 (neg., M-H); ¹H NMR (270MHz, CDCl₃) δ 7.10-7.20 (m, 10H), 7.00 (d, 1H), 6.95 (d, 1H), 6.90 (d, 1H), 6.50 (dd, 1H), 6.05 (d, 1H), 5.00 (s, 2H), 3.95 (d, 1H), 3.90 (d, 1H); 3.00 (m, 1H), 2.95 (m, 1H); 2.05(m, 1H), 1.95(m, 1H),0.90 (s, 3H).

**Step 4:**
*(4R*,*4aS*,*10aS)*-7-Hydroxy-10a-methyl-4-phenyl-1,4,4a,9,10,10-hexahydro-*2H*-phenantren-3-one. The reaction was carried out on 13 mg of: *(4R*,*4aS*,*10aS)*-7-benzyloxy-10a-methyl-4-phenyl-4a,9,10,10-tetrahydro-*4H*-phenantren-3-one, according to **method F** ("J"= ethanol, "K"= 5, "L"= 10% Pd/C, "M"= 2, "N"=24). The crude product was purified by PHPLC to obtain 9.0 mg of : *(4R*,*4aS*,*10aS)*-7-hydroxy-10a-methyl-4-phenyl-1,4,4a, 9,10,10-hexahydro-*2H*-phenantren-3-one. ES-MS m/z: 307.3(pos., M+H), 305.2(neg., M-H);. ¹H NMR (270 MHz, CDCl₃) δ 7.20-7.40 (m, 5H), 6.70 (d, 1H), 6.60 (d, 1H), 6.35 (dd, 1H), 4.80 (s, b 1H), 3.80 (d, 1H), 3.50 (d, 1H); 2.80 (m, 2H), 2.60 (m, 2H); 2.00(m, 2H), 1.80(m, 1H), 1.65(m, 1H), 0.85(s, 3H).

### Example 32: Synthesis of (rac)-(4R,4aS,10aS)-3,3-ethanediyldimercapto-7-hydroxy-10a-methyl-4-phenyl-1,4,4a,9,10.10a-octahydro-phenanthrene (E32).

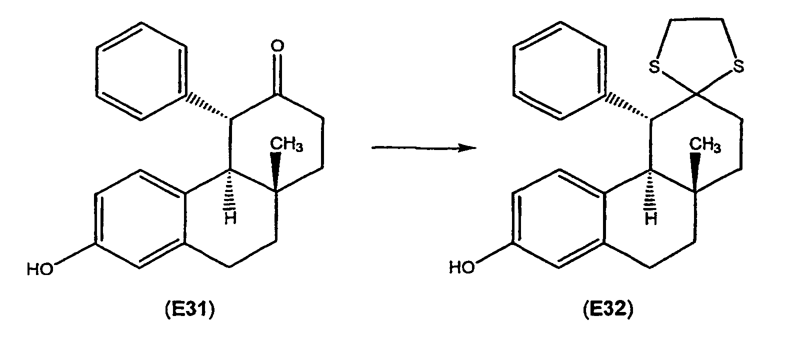

The reaction was carried out on 7.0 mg (0.023 mmol) *(4R,4aS,10aS)*-7-hydroxy-10a-methyl-4-phenyl-1,4,4a,9,10,10-hexahydro-*2H*-phenantren-3 -one, according to **method G** ("J"= 1.0, "K"= 1,2-ethanedithiol, "L"= 9, "M"= 2), using 6 eq of BF₃·OEt₂. The crude product was purified by prep. TLC using EtOAc:*n*-heptane (1:7) as eluent to yield *(rac)-(4R,4aS,10aS)*-3,3-ethanediyldimercapto-7-hydroxy-10a-methyl-4-phenyl-1,4,4a,9,10,10a-octahydro-phenanthrene (5.3 mg) as a white solid. ES-MS m/z: 383.2 (pos., M+H); ¹H NMR (270 MHz, CDCl₃) δ 7.55 (d, 1H), 7.35(d, 1H),7.30(m, 1H), 7.20(m, 1H), 7.10(m, 1H), 6.60 (d, 1H), 6.45 (d, 1H), 6.25 (dd, 1H), 4.40 (bs, 1H), 3.50 (d, 1H), 3.20 (d, 1H); 3.00(m, 1H),2.85 (m, 2H), 2.65 (m, 1H), 2.50(m, 1H), 2.35(m, 2H), 1.85(m, 4H), 1.45(m, 1H), 0.55(s, 3H).

### Example 33: Synthesis of (rac)-(1S,4aS,10aS)-3,3-(ethane-1,2-diyldimercapto)-7-hydroxy-1-(2-phenylethyl)-10a-methyl-1,2,3,4,4a,9,10,10a-octahydro-phenanthrene (E33).

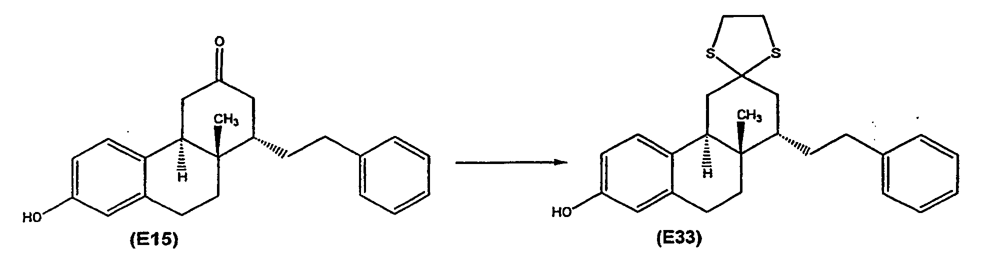

**Step 1:**
*(rac)-(1S,4aS,10aS)*-3,3-(ethane-1,2-diyldimercapto)-7-hydroxy-1-phenylethyl-10a-methyl-1,2,3,4,4a,9,10,10a-octahydro-phenanthrene. The reaction was carried out on 8 mg (24 mmol) of *(rac)-(1S,4aS,10aS)*-7-hydroxy-1-phenylethyl-10a-methyl-1,4,4a,9, 10,10a-hexahydro-*2H*-phenanthren-3-one, according to **method G** ("J"= 2.0, "K"= 1,3-ethanediol, "L"= 2.0, "M"= 6). The crude product was purified on chromatotron using EtOAc:*n*-heptane (2.8, v:v) as eluent to yield *(rac)-(1S,4aS,10aS)*-3,3-(ethane-1,2-diyldimercapto)-7-hydroxy-1-phenylethyl-10a-methyl-10a-methyl-1,2,3,4,4a,9,10, 10a-octahydro-phenanthrene as a colorless glass. ES/MS m/z: 409.3 (neg., M-H): ¹H NMR (270MHz, CDCl₃) δ 7.10-7.30 (m, 5H), 7.05-7.10 (d, 1H), 6.50-6.65 (m, 3H), 4.50 (s, OH); 3.30-3.45 (m, 4H),2.40-3.00 (m, 7H), 1.80-2.10 (m, 3H), 1.10-1.65 (m, 3H), 0.90 (s, 3H). ¹³C NMR (270MHz, CDCl₃) δ 153.3, 143.0, 137.9, 131.1, 128.5, 128.4, 127.0, 126.0, 115.3, 112.8, 68.9, 45.3, 43.4, 40.4, 38.8, 37.5, 37.4, 35.0, 34.7, 33.8, 30.0,26.8,18.5.

### Example 34: Synthesis of (rac)-(1S,4aS,10aS)-3,3-ethanediyldioxy-7-hydroxy-10a-methyl-1,2,3,4,4a,9, 10,10a-octahydro-phenanthrene (E34a) and (rac)-(1S,4aS,10aR)-3,3-ethanediyldioxy-7-hydroxy-10a-methyl-1,2,3,4,4a,9,10,10a-octahydro-phenanthrene (E34b).

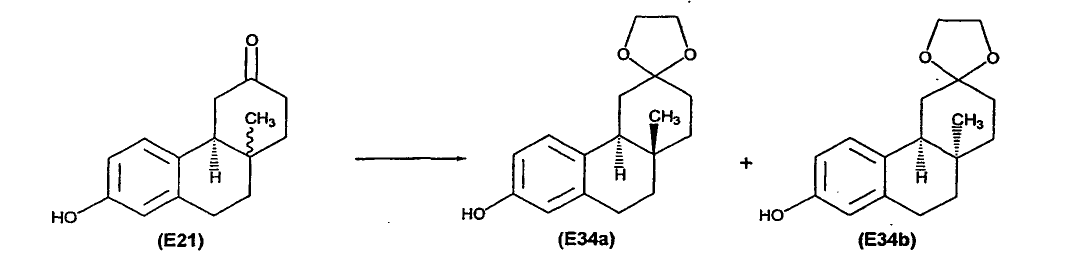

*(rac)-(4aS*,*10aS)*-3,3-ethanediyldioxy-7-hydroxy-10a-methyl-1,2,3,4,4a,9,10,10a-octahydro-phenanthrene. The reaction was carried out on 320mg (1.39 mmol) of a (1:1) mixture of *(rac)-(4aS,10aS)*-7-hydroxy-10a-methyl-1,4,4a,9,10,10a-hexahydro-*2H*-phenanthren-3-one and *(rac)-(1S,4aS,10aR)*-7-hydroxy-10a-methyl-1,4,4a,9,10,10a-hexahydro-*2H*-phenanthren-3-one, according to **method H** ("J"= 1,2-dihydroxyethane, "K"= 10, "L"= 500, "N"= 20, "O"= 16). The crude product was purified by MPLC, using CH₂Cl₂: MTBE (100:0, 90:10, v:v) as eluent to yield colorless glasses of *(rac)-(4aS,10aS)-*3,3-ethanediyldioxy-7-hydroxy-10a-methyl-1,2,3,4,4a,9,10,10a-octahydro-phenanthrene **(E34a),** contaminated with 10% of **E34b** and *(rac)-(4aS,10aR)*-3,3-ethanediyldioxy -7-hydroxy-10a-methyl-1,2,3,4,4a,9,10,10a-octahydro-phenanthrene (E101b), contaminated with 10% of E101a. ES/MS m/z: 275.2 (pos., M+H), 273.1 (neg., M-H); ¹H NMR (270MHz, CDCl₃, isomer A) δ 7.00 (d, 1H), 6.65-6.70 (m, 2H), 4.80 (bs, OH), 3.90-4.10 (m, 4H), 2.75-2.90 (m, 2H), 2.70-2.75 (m, 1H), .20-2.30 (m, 1H), 1.80-1.90 (m, 1H), 1.65-1.70 (m, 1H), 1.50-1.65 (m, 5H), 0.75 (s, 3H).; ¹H NMR (270MHz, CDCl₃, selected signals from isomer B which differ from isomer A) δ 6.90 (d, 1H), 6.55 (m, 2H), 3.90-4.00 (m, 4H), 5.10 (bs, OH), 0.80 (s, 3H).

### Example 35: Synthesis of (rac)-(3S,4aS,10aS)-7-hydroxy-3-pentyl-10a-methyl-1, 2,3,4,4a,9,10,10a-octahydro-phenanthrene (E35a) and (rac)-(3R,4aS,10aS) -7-hydroxy-3-pentyl-10a-methyl-1,2,3,4,4a,9,1,10a-octahydro-phenanthrene (E35b).

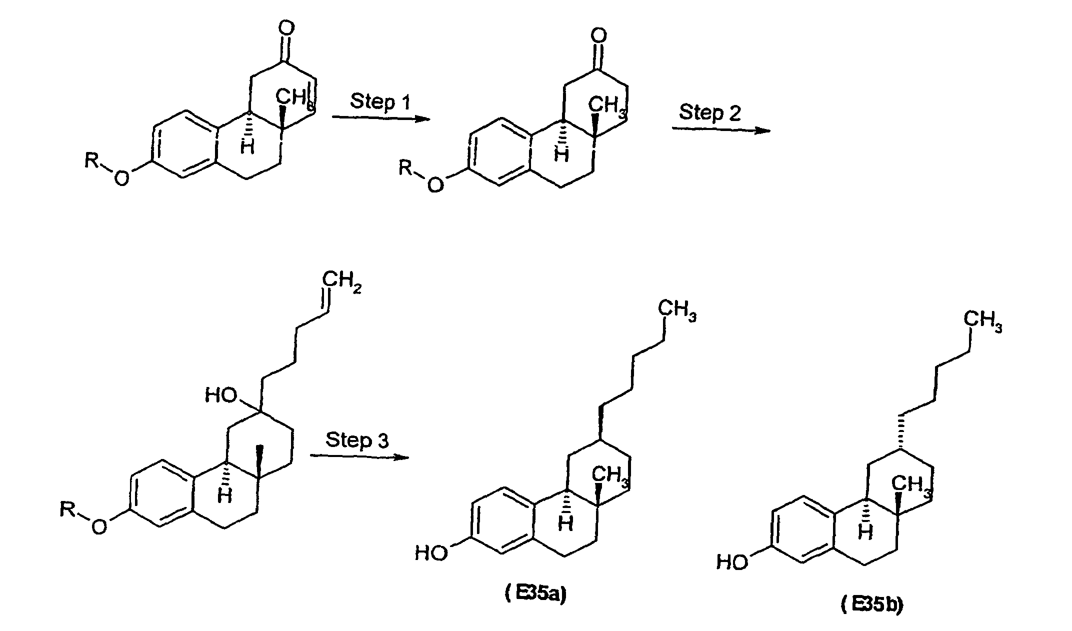

**Step1:**
*(rac)-(4aS,10aS)*-7-benzyloxy-10a-methyl-1,4,4a,9,10,10a-hexahydro-*2H*-phenanmren-3-one and *(rac)-(4aR,10aS)*-7-benzyloxy-10a-methyl-1,4,4a,9,10,10a-hexahydro-*2H*-phenanthren-3-one. The reaction was carried out on 707 mg (2.22 mmol) of a (6:4) mixture of *(rac)-(4aS,10aS)*-7-benzyloxy-10a-methyl-4a,9,10,10a-tetrahydro-*4H*-phenanthren -3-one and *(rac)-(4aR,10aS)*-7-benzyloxy-10a-methyl-4a,9,10,10a-tetrahydro-*4H*-phenanthren-3-one from **Example 3,** according **method F** ("L"= 5% Pd/C, "M"=70, THF/HOAC (95:5, v:v), r.t. 8 days). The crude mixture was purified by MPLC using EtoAC:n-heptane (0:100, 10:90, v:v) as eluent to yield
*(rac)-(4aS,10aS)*-7-benzyloxy-10a-methyl-1,4,4a,9,10,10a-hexahydro-*2H*-phenanthren-3-one and *(rac)-(4aR,10aS)*-7-benzyloxy-10a-methyl-1,4,4a,9,10,10a-hexahydro-*2H*-phenanthren-3-one as a yellowish solid. ES/MS m/z: 321.1 (pos., M+H).

**Step2:** *(rac)-(3S,4aS,10aS)*-7-benzyloxy-3-hydroxy-3-(1-pentyl)-10a-methyl-1,2,3,4, 4a,9,10,10a-octahydro-phenanthrene and *(rac)-(3S,4aR,10aS)*-7-benzyloxy-3-hydroxy-3-(1-pentyl-5-en)-10a-methyl-1,2,3,4,4a,9,1,10a-octahydro-phenanthrene. The reaction was carried out on 40 mg (0.125 mmol) of *(rac)-(4aS,10aS)*-7-benzyloxy-10a-methyl-1,4,4a,9,10,10a-hexahydro-*4H*-phenanthren-3-one and *(rac)-(4aR,10aS)*-7-benzyloxy-10a-methyl-1,4,4a,9,10,10a-hexahydro-*2H*-phenanthren-3-one (85:15 mixture), according **method V,** to give a crude product which was used without purification in the next step.

**Step 3:** *(rac)-(3S,4aS,10aS)*-7-hydroxy-3-(1-pentyl)-10a-methyl-1,2,3,4,4a,9,10,10a-octahydro-phenanthrene. The crude material from step 2 was treated with phosphoric acid (85%) at 140 °C for 1 hour, followed by extraction with EtOAc/ H₂O and dried with anhydrous sodium sulphate. The resulting yellow-brownish oil was reacted according to **method F,** ("L"=10% Pd/C, "J"= MeOH/THF, "M"= 15). The crude product is purified on chromatotron using EtOAc/n-heptane (1:9, v:v) as eluent, followed by PHPLC to yield a mixture of *(rac)-(3S,4aS,10aS)*-7-hydroxy-3-(1-pentyl)-10a-methyl-1,2,3,4,4a,9,10, 10a-octahydro-phenanthrene (**E35a**) and *(rac)-(3R,4aS,10aS)*-7-hydroxy-3-(1-pentyl) -10a-methyl-1,2,3,4,4a,9,10,10a-octahydro-phenanthrene. (**E35b**) ES/MS m/z: 287.2 (pos., M+H), 285.1 (neg., M-H); ¹H NMR (270MHz, CDCl₃, selected signals) δ 7.10 (d, 1H), 6.50-6.60 (m, 3H), 2.60-2.90 (m, 3H), 2.00-2.40 (m, 4H), 0.90 (s, 3H), 0.70 (s, 3H).

### Example 36: Synthesis of (rac)-(1S,2R,4aS,10aS)-2,10a-dimethyl-3,3-(ethane-1, 2-diyldimercapto)-7-hydron-1-(3-methyl-bulyl-1,2,3,4,4a,9,10,10a-octahydro-phenanthrene (E36).

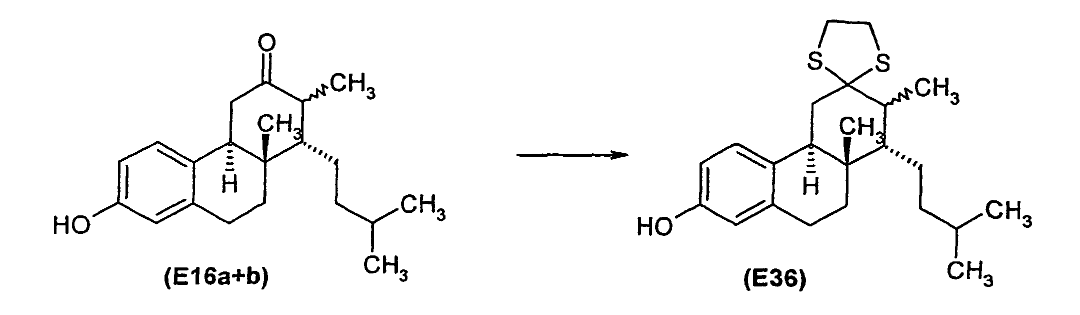

*(rac)-(1S,2R,,4aS,10aS)*-2,10a-dimethyl-3,3-(ethane-1,2-diyldimereapto)-7-hydroxy-1-(3-methyl-butyl-1,2,3,4,4a,9,10,10a-octahydro-phenanthrene. The reaction was carried out on 9 mg (30 µmol) of *(rac)-(1S,2R,4aS,10aS)*-2,10a-dimethyl-7-hydroxy-1-(3-methyl-butyl)-1, 4,4a,9,10,10a-hexahydro-*2H*-phenanthren-3-one, according to **method G** ("J"= 1.0, "K"= 1,2-ethanediol, "L"= 2.0, "M"= 6). The crude product was purified on chromatotron using EtOAc:*n*-heptane (1:9, 2:8, 3:7, v:v) as eluent to yield *(rac)-(1S,2R,4aS,10aS)*-3,3-(ethane-1,2-diyldimercapto)-7-hydroxy-1-phenylethyl-10a-methyl-1,2,3,4,4a,9,10,10a-octahydro-phenanthrene as a colorless glass. ES/MS (mixture) m/z: 391.3 (pos., M+H), 389.2 (neg., M-H): ¹H NMR (270MHz, CDCl₃, selected signals of the mixture) δ 7.10 (d, 1H), 6.50-6.70 (m, 1H), 3.30-3.50 (m, 4H isomer A) 3.10-3.30 (m, 4H isomer B), 0.85-0.90 (m, 9H). ¹³C NMR (270MHz, CDCl₃, mixture) δ 153.2, 137.8, 131.2, 127.0, 115.2, 112.8, 73.9, 52.9, 45.9, 43.4, 40.6, 39.1, 38.2, 36.4, 33.3, 31.9, 29.1, 28.8, 26.7, 22.8, 22.6, 22.4, 18.8, 15.9, 14.2.

### Example 37: Synthesis of (rac)-(1S,4aS,10aS)-7-Hydroxy-1-(3'-methyl-butyl)-1,4, 4a,9,10,10a-hexahydro-2H-phenanthren-3-one (E37).

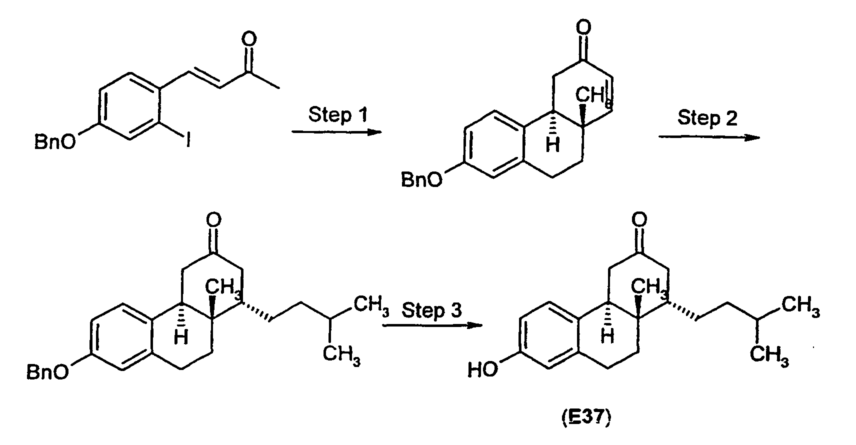

**Step 1:** *(rac)*-*(4aS*,*10aS)*-7-Benzyloxy-4a,9,10,10a-tetrahydro-*4H*-phenanthren-3-one. The aryl iodide 4-(2-iodo-4-Benzylox-y-phenyl)-but-3-en-2-one (6.12 g) was treated according to **method A** ("J" = but-3-en-1-ol, "K" =1.3, "L" = 4.0, "M" = 2, "N" = 2.0). The material obtained was then treated according to **method C** ("N" = 150, "Q" = 14). The crude product obtained was purified by silica gel flash chromatography using EtOAc:*n*-heptane (5:95, 1:8 stepwise gradient) as eluent, followed by fractionation with PHPLC to yield *(rac)-(4aS,10aS)*-7-benzyioxy-4a,9,10,10a-tetrahydro-*4H*-phenanthren-3-one (white solid). ES/MS m/z: 305.2 (pos., M+H); ¹H NMR (500 MHz, CDCl₃) δ 7.30-7.50 (m, 5H), 7.10 (d, 1H), 6.90 (d d, 1H), 6.80 (dd, 1H), 6.75 (d, 1H), 6.10 (d, 1H), 5.05 (s, 2H), 3.20 (dd, 1H), 2.90-3.10 (m, 3H), 2.45 (t, 1H), 2.35 (dd, 1H), 2.10-2.20 (m, 1H), 1.65-1.75 (m, 1H).

**Step 2:** *(rac)-(1S,4aS,10aS)*-7-benzyloxy-1-(3'-methyl-butyl)-1,4,4a,9,10,10a-hexahydro-*2H*-phenanthren-3-one. The enone *(rac)-(4aS,10aS)*-7-benzyloxy-4a,9,10, 10a-tetrahydro-*4H*-phenanthren-3-one (50 mg) dissolved in THF (5.0 mL), was treated according to **method E** ("J" = lithium 2-thienylcyanocuprate (Aldrich, no. 32,417-5, 0.25M solution in THF), "K" = 2.0, "N" = 3-methyl-1-butyl magnesium bromide (0.5M solution in THF), "O" = 2.0, "P" = 0, "Q" = 2). The crude product was by silica gel flash chromatography using EtOAc:*n*-heptane (1:8) as eluent, followed by fractionation with PHPLC to yield 23 mg of *(rac)-(1S,4aS,10aS)*-7-benzyloxy-1-(3'-methyl-butyl)-1, 4,4a,9,10,10a-hexahydro-*2H*-phenanthren-3-one as a white powder that was used in the next step (step 3). ES/MS m/z: 377.2 (pos., M+H).

**Step 3:** *(rac)-(1S,4aS,10aS)*-7-Hydroxy-1-(3'-methyl-butyl)-1,4,4a,9,10,10a-hexahydro-*2H*-phenanthren-3-one. The substrate *(rac)-(1S,4aS,10aS)-*7-benzyloxy-1-(3'-methyl-butyl)-1,4,4a,9,10,10a-hexahydro-*2H*-phenanthren-3-one (14 mg) was treated according to **method F** ("J" = THF:HOAc [95:5], "K" = 4.0, "L" = 10 % Pd/C, "M" = 4, "N" = 2). The crude product was purified using PHPLC to yield *(rac)-(1S,4aS,10aS)*-7-hydroxy-1-(3'-methyl-butyl)-1,4,4a,9,10,10a-hexahydro-*2H*-phenanthren-3-one (white solid). ES/MS m/z: 285.1 (neg., M-H); ¹H NMR (500 MHz, CDCl₃) δ 8.00 (s, 1H), 7.00 (d, 1H), 6.60 (dd, 1H), 6.55 (d, 1H), 2.75-2.95 (m, 4H), 2.55 (dd, 1H), 2.40 (dt, 1H), 2.20 (t, 1H), 1.95-2.05 (m, 1H), 1.65-1.85 (m, 2H), 1.40-1.55 (m, 2H), 1.20-1.35 (m, 2H), 1.05-1.15 (m, 1H), 0.85 (t, 6H).

### Example 38: Synthesis of (rac)-(4aS,10aS)-3,3-(propane-1, 3-diyldimercapto)-7-hydroxy-1,2,3,4,4a,9,10,10a-octahydro-phenanthrene (E38).

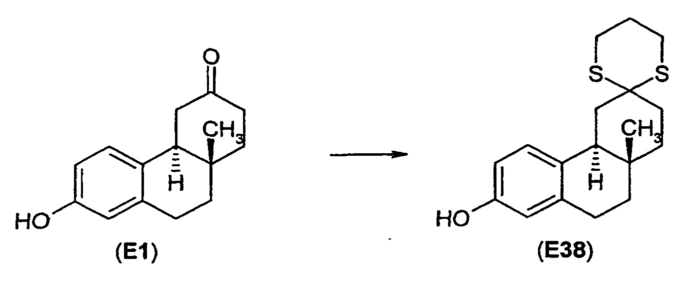

The ketone (*rac*)-(*4aS,10aS*)-7-hydroxy-1,4,4a,9,10,10a-hexahydro-*2H*-phenanthren-3-one (18 mg) was treated according to **method G** ("J" = 4, "K" = 1,3-propanedithiol, "L" = 2.0, "M" = 12). The crude product was purified by column chromatography on silica gel, using EtOAc:*n*-heptane (2:8, v:v) as eluent to yield *(rac)-(4aS,10aS)*-3,3-propanediyldimercapto -7-hydroxy-1,2,3,4,4a,9,10,10a-octahydro-phenanthrene. ES/MS m/z: 307.3 (pos., M+H), 305.2 (neg., M-H); ¹H NMR (270 MHz, CD₃OD) δ 7.05 (d, 1H), 6.55 (dd, 1H), 6.45 (d, 1H), 2.90-3.00 (m, 2H), 2.60-2.85 (m, 6H), 2.40 (m, 1H), 1.95(m, 2H), 1.55-1.85 (m, 4H), 1.35-1.50 (m, 3H), 1.20-1.30 (m, 1H).

### Example 39: (rac)-(1S,4S,4aS,10aS)-7-Hydroxy-1-butyl-4,10a-dimethyl1,4,4a,9,10, 10a-hexahydro-2H-phenanthren-3-one (E39).

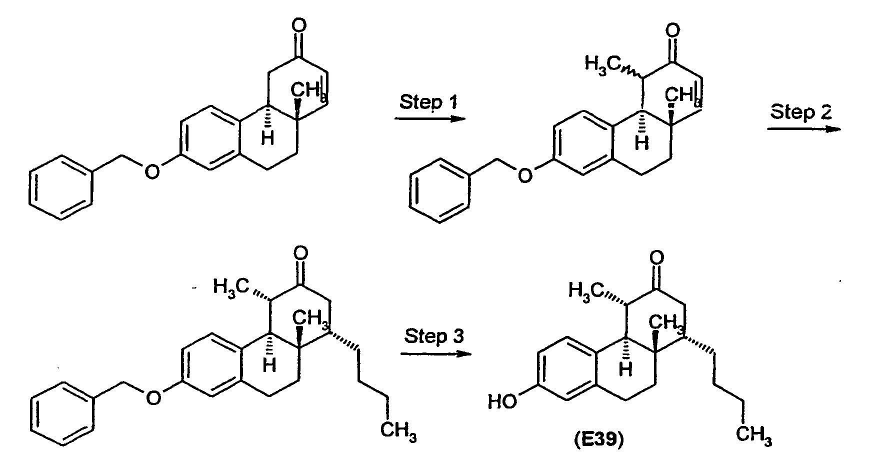

**Step 1**: *(rac)-(4R,4aS,10aS)*-7-Benzyloxy-4,10*a*-dimethyl-4a,9,10,10a-tetrahydro-*4H*-phenanthren-3-one and *(rac)-(4S,4aS,10aS)*-7-benzyloxy-4,10*a*-dimethyl-4a,9,10, 10a-tetrahydro-*4H*-phenanthren-3-one. The enone *(rac)-(4aS,10aS)*-7-benzyloxy-10a-methyl-4a,9,10,10a-tetrahydro-*4H*-phenanthren-3-one. (101 mg) **from Example 15** dissolved in THF (3.0 mL), was treated according to **method E** ("J" = diisopropylamine, "K" = 10, "L" = 2.0, "M" = THF, "N" = *n*-butyl lithium [1.6M solution in hexanes], "O" = 10, "P" = -78, "Q" = 1) with the following exception. Methyl iodide (10 eq.) was added to the reaction mixture, and stirring was continued for another 20 h at room temperature before quenching. The crude product was purified on chromatotron, using EtOAc: *n*-heptane (1:8, v:v) as eluent, to yield 71 mg of *(rac)-(4R, 4aS, 10aS)*-7-benzyloxy-4, 10*a*-dimethyl-4a,9,10,10a-tetrahydro-*4H*-phenanthren-3-one and *(rac)-(4S, 4aS,10aS)*-7-benzyloxy-4,10*a*-dimethyl-4a,9,10,10a-tetrahydro-*4H*-phenanthren-3-one.

**Step 2:** *(rac)-(1S,4S,4aS,10aS)*-7-Benzyloxy-1-butyl-4,10a-dimethyl-1,4,4a,9, 10,10a-hexahydro-*2H*-phenanthren-3-one. The enone *(rac)-(4R, 4aS, 10aS)*-7-benzyloxy-4,10*a*-dimethyl-4a,9,10,10a-tetrahydro-*4H*-phenanthren-3-one and *(rac)-(4S, 4aS, 10aS)*-7-benzyloxy-4,10*a*-dimethyl-4a,9,10,10a-tetrahydro-*4H*-phenanthren-3-one (25 mg) dissolved in THF (2.0 mL), was treated according to **method E** ("J" = CuCN, "K" = 2, "L" = 2.0, "M" = THF, "N" = *n*-butyl lithium [1.6M solution in hexanes], "O" = 4.0, "P" = -78, "Q" = 2) with the following exception; CuCN and *n*-butyl lithium ixed at 0°C and stirred at RT for 1h. The crude product was purified on PHPLC to yield 18mg of *(rac)-(1S,4S,4aS,10aS)*-7-Benzyloxy-1-butyl-4,10a-dimethyl-1,4,4a,9,10,10a-hexahydro-*2H*-phenanthren-3-one (white powder) that was used in the next step (step 3). ES/MS m/z: 379.0 (pos., M+H); ¹H NMR (270 MHz, CD₃Cl) δ 7.30-7.50 (µ, 5H), 7.00 (d,1H), 6.75-6.85 (m, 2H), 5.05 (s, 2H), 2.60-2.75 (m, 5H), 2.45 (dd, 1H), 2.00-2.15 (m, 1H), 1.65-1.75 (m, 2H), 1.0-1.5 (m, 9H), 0.90 (t, 3H), 0.75 (s,3H).

**Step 3:** *(rac)-(1S,4S,4aS,10aS)*-7-Hydroxy-1-butyl-4,10a-dimethyl-1,4,4a,9,10,10a-hexahydro-*2H*-phenanthren-3-one. As the substrate, *(rac)-(1S,4S,4aS,10aS)*-7-Benzyloxy-1-butyl-4,10a-dimethyl-1,4,4a,9,10,10a-hexahydro-*2H*-phenanthren-3-one (12 mg) from the previous step (step 2) was treated according to **method F** ("J" = THF:HOAc [95:5], "K" = 3.0, "L" = 5% Pd/C, "M" = 5, "N" = 24). The crude product was purified using PHPLC to yield *(rac)-(1S,4S,4aS,10aS)*-7-Hydroxy-1-butyl4,10a-dimethyl-1,4,4a, 9,10,10a-hexahydro-*2H*-phenanthren-3-one. ES/MS m/z (mixture): 301.3 (pos., M+H), 299.2 (neg., M-H); ¹H NMR (270 MHz, CD₃Cl) δ 6.95 (d,1H), 6.65-6.75 (m, 2H), 4.77 (s, 1H), 2.60-2.75 (m, 5H), 2.45 (dd, 1H), 2.00-2.15 (m, 1H), 1.65-1.75 (m, 2H), 1.0-1.5 (m, 9H), 0.90 (t, 3H), 0.70 (s,3H).

### Example 40: Synthesis of (rac)-(4S,4aS,10aS)-3,3-ethanediyldimercapto-7-hydroxy-4, 10a-dimethyl-1,2,3,4,4a,9,10,10a-octahydro-phenanthrene (E40).

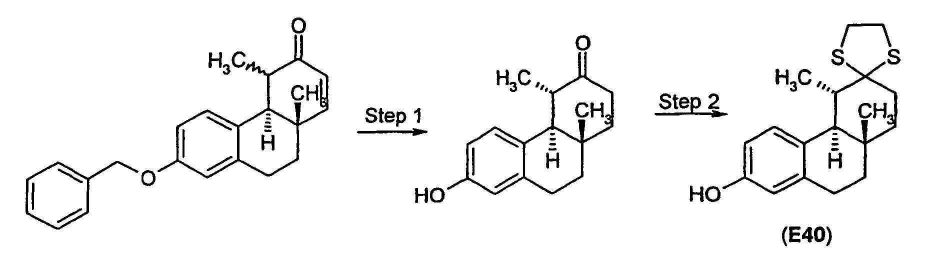

**Step 1:** *(rac)-(4S,4aS,10aS)*-7-Hydroxy-4,10a-dimethyl-1,4,4a,9,10,10a-hexahydro-*2H*-phenanthren-3-one. As the substrate, a mixture of *(rac)-(4R, 4aS, 10aS)*-7-benzyloxy-4,10*a*-dimethyl-4a,9,10,10a-tetrahydro-*4H*-phenanthren-3-one and *(rac)-(4S, 4aS,10aS)*-7-benzyloxy-4,10*a*-dimethyl-4a,9,10,10a-tetrahydro-*4H*-phenanthren-3-one (20 mg) from **Example 39** was treated according to **method F** ("J" = THF:HOAc [95:5], "K" = 4.0, "L" =10% Pd/C, "M" = 7, "N" =12). The crude product was purified using PHPLC to yield *(rac)-(4S,4aS,10aS)*-7-Hydroxy-4,10a-dimethyl-1,4,4a,9,10,10a-hexahydro-*2H*-phenanthren-3-one. ES/MS m/z (mixture): 245.2 (pos., M+H), 243.1 (neg., M-H); ¹H NMR (270 MHz, CD₃Cl) δ 6.95 (d,1H), 6.65-6.75 (m, 2H), 5.05 (s, 1H), 2.65-2.08 (m, 3H), 2.40-2.55 (m, 3H), 1.55-1.90 (m, 4H), 1.30 (t, 3H), 0.60 (s,3H).

**Step 2.** *(rac)-(4S,4aS,10aS)*-3,3-ethanediyldimercapto-7-hydroxy-4,10a-dimethyl-1, 2,3,4,4a,9,10,10a-octahydro-phenanthrene. The ketone *(rac)-(4S,4aS,10aS)*-7-Hydroxy-4, 10a-dimethyl-1,4,4a,9,10,10a-hexahydro-*2H*-phenanthren-3-one (9.4 mg) was treated according to **method G** ("J" = 5.0, "K" = 1,2-ethanedithiol, "L" = 2.0, "M" = 12). The crude product was purified using PHPLC to yield *(rac)-(4S,4aS,10aS)-3,* 3-ethanediyldimercapto-7-hydroxy-4,10a-dimethyl-1,2,3,4,4a,9,10,10a-octahydro-phenanthrene as a glass. ES/MS m/z: 321.1 (pos., M+H), 319.0 (neg., M-H); ¹H NMR (270 MHz, CD₃OD) δ 7.00 (d, 1H), 6.60-6.70 (m, 2H), 4.60 (s, 1H), 3.20-3.35 (m, 4H), 2.70 (dt, 1H), 2.35-2.55 (m, 2H), 2.10- 2.25 (m, 3H), 1.45-1.85 (m, 4H), 1.35 (d, 3H), 0.35 (s, 3H).

### Example 41: (rac)-(4S,4aS,10aS)-7-Hydroxy-4-benzyl-10a-methyl-1,4,4a,9,10, 10a-hexahydro-2H-phenanthren-3-one (E41a) and (rac)-(4aS,10aS)-7-hydroxy-4,4-dibenzyl-10a-methyl-1,4,4a,9,10,10a-hexahydro-2H -phenanthren-3-one (E41b).

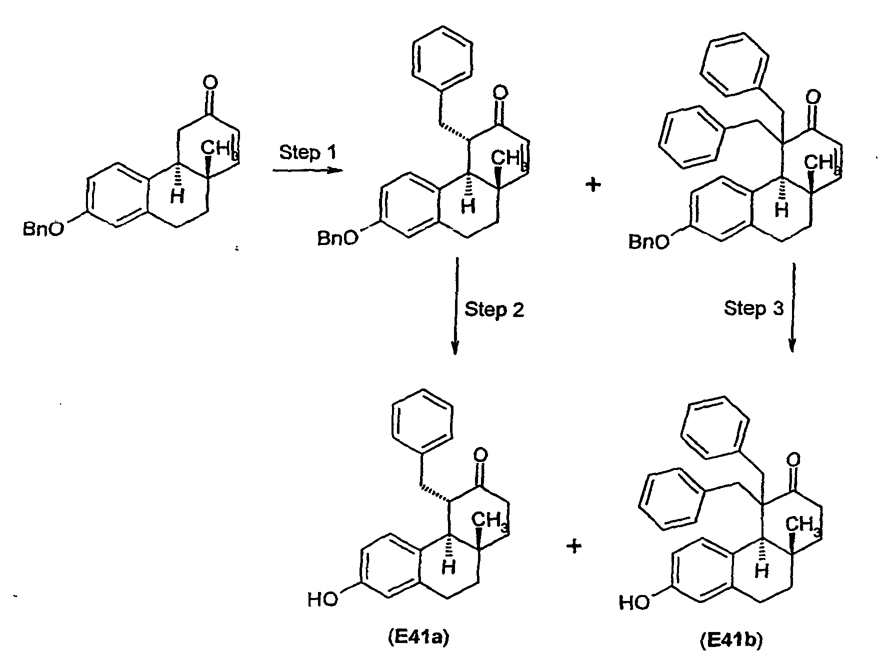

**Step 1:** *(rac)-(4S,4aS,10aS)*-7-Benzyloxy-4-benzyl-10a-methyl-1,4,4a,9,10,10a-hexahydro-*2H*-phenanthren-3-one and *(rac)-(4aS,10aS)*-7-benzyloxy-4, 4-dibenzyl-10a-methyl-1,4,4a,9,10,10a-hexahydro-*2H*-phenanthren-3-one. The enone *(rac)-(4aS,10aS)*-7-benzyloxy-10a-methyl-4a,9,10,10a-tetrahydro-*4H*-phenanthren-3-one. (50 mg) dissolved in THF (3.0 mL), was treated according to **method E** ("J" = diisopropylamine, "K" = 10, "L" = 2.0, "M" = THF, "N" = *n*-butyl lithium [1.6M solution in hexanes], "O" = 10, "P" = -78, "Q" = 0.1) with the following exception. Benzyl bromide (1 eq.) was added to the reaction mixture, and stirring was continued for another 14 h at room temperature before quenching. The crude product was purified using PHPLC to yield 12 mg of *(rac)-(4S,4aS,10aS)*-7-benzyloxy-4-benzyl-10a-methyl-1,4,4a,9,10, 10a-hexahydro-*2H*-phenanthren-3-one. ES/MS m/z: 409.3 (pos., M+H); and 23 mg of *(rac)-(4aS,10aS)*-7-benzyloxy-4,4-dibenzyl-10a-methyl-1,4,4a,9,10,10a-hexahydro-2*H*-phenanthren-3-one. ES/MS m/z: 499.6 (pos., M+H);

**Step 2** *(rac)-(4S,4aS,10aS)*-7-hydroxy-4-benzyl-10a-methyl-1,4,4a,9,10,10a-hexahydro-*2H*-phenanthren-3-one. As the substrate, *(rac)-(4S,4aS,10aS)*-7-benzyloxy-4-benzyl-10a-methyl-1,4,4a,9,10,10a-hexahydro-*2H*-phenanthren-3-one (8 mg) was treated according to **method F** ("J" = THF:HOAc [95:5], "K" = 3.0, "L" = 10% Pd/C, "M" = 4, "N" = 12). The crude product was purified by chromatography on silica gel column, using EtOAc:*n*-heptane (1:8 2:8, v:v, stepwise gradient) as eluent to yield *(rac)-(4S,4aS,10aS)* -7-hydroxy-4-benzyl-10a-methyl-1,4,4a,9,10,10a-hexahydro-*2H*-phenanthren-3-one. ES/MS m/z (mixture): 321.1 (pos., M+H), 319.0 (neg., M-H); ¹H NMR (270 MHz, CD₃Cl) δ 7.15-7.25 (m, 5H), 7.00 (d,1H), 6.60-6.70 (m, 2H), 2.95-3.30 (m, 3H), 2.70 (d, 2H), 2.45-2.55 (m, 1H), 2.25-2.35 (m, 1H), 1.60-1.85 (m, 3H), 1.30-1.50 (m, 2H), 0.60 (s,3H).

**Step 3** *(rac)-(4aS,10aS)*-7-Hydroxy-4,4-dibenzyl-10a-methyl-1,4,4a,9,10,10a-hexahydro-*2H*-phenanthren-3-one. As the substrate, *(rac)-(4aS,10aS)*-7-benzyloxy-4,4-dibenzyl -10a-methyl-1,4,4a,9,10,10a-hexahydro-*2H*-phenanthren-3-one (17 mg) was treatedaccording to **method F** ("J" = THF:HOAc [95:5], "K" = 3.0, "L" = 10% Pd/C, "M" = 5, "N" = 24). The crude product was purified by chromatography on silica gel column, using EtOAc:*n*-heptane (1:8, 2:8, v:v, stepwise gradient) as eluent to yield 13 mg of *(rac)-(4aS,10aS)*-7-hydroay-4,4-dibenzyl-10a-methyl-1,4,4a,9,10,10a-hexahydro-*2H*-phenanthren-3-one. ¹H NMR (270 MHz, CD₃Cl) δ 7.85 (δ, 1H), 7.15-7.30 (µ, 5H), 7.00-7.05 (m, 3H), 6.80-6.90 (m, 3H), 6.65 (d,1H), 3.45 (dd, 2H), 2.95-3.10 (m, 4H), 2.75-2.90 (m, 2H), 2.45-2.60 (m, 2H), 2.20-2.35 (m, 2H), 1.60-1.70 (m, 1H), 1.00 (s,3H).

### Example 42: Synthesis of (rac)-(4aS,10aS)-7-hydroxy-10a-methyl-3, 3-methylene-1,2,3,4,4a,9,10,10a-octahydrophenanthrene (E42).

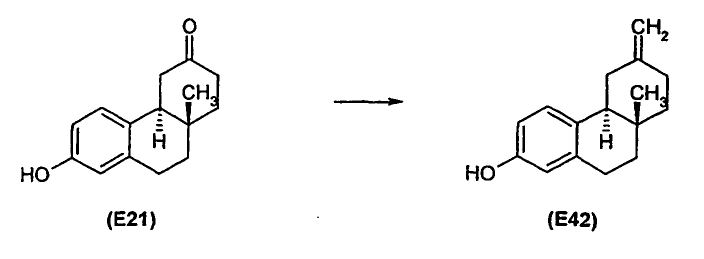

*(rac)-(4aS,10aS)*-7-hydroxy-10a-methyl-3,3-methylene-1,2,3,4,4a,9,10,10a-octahydrophen anthrene. *n*-BuLi (2.0 mL of 1.6 *M* solution in hexanes, 3.15 mmol) was added to a well-stirred suspension of methyltriphenylphosphonium bromide (1.13 g, 3.15 mmol) in THF (80 mL) at -78°C. The cooling bath was removed and the resulting mixture was allowed to warm to r.t. The resulting yellow solution was cooled to 0°C and a solution of (*rac*)*-*(*4aS,10aS*)-7-hydroxy-10a-methyl-1,4,4a,9,10,10a-hexahydro-*2H*-phenanthren-3-one (145 mg, 0.63 mmol) in THF (5 mL) was added. The resulting mixture was stirred at r.t. for 3 days and then quenched with sat. aq. NH₄Cl (10 mL) together with water (10 mL). The organic phase was separated and the aqueous phase was additionally extracted with EtOAc (3 x 15 mL). The combined organic solutions were washed with brine, dried with anhydrous Na₂SO₄ and evaporated. The crude product was purified by silica gel flash chromatography using Et₂O : *iso*-hexane (1:4) as eluent to yield *(rac)-(4aS,10aS)*-7-hydroxy-10a-methyl-3,3-methylene-1,2,3,4,4a,9,10,10a-octahydrophenanthrene (132 mg) as a white oil. ES-MS m/z: 229.3 (pos., M+H), 227.2 (neg., M-H); ¹H NMR (270 MHz, CDCl₃) δ 7.05 (d, 1H), 6.60 (dd, 1H), 6.55 (d, 1H), 4.75 (d, 1H), 4.70 (d, 1H), 4.50 (bs, 1H), 3.00-2.70 (m, 3H), 2.50-1.95 (m, 4H); 1.70-1.45 (m, 3H), 1.40-1.15 (m, 1H), 0.80 (s, 3H).

### Example 43: Synthesis of (rac)-(4aS,10aS)-3,3-ethanediyl-7-hydroxy-10a-methyl-1, 2,3,4,4a,9,10,10a-octahydrophenanthrene (E43).

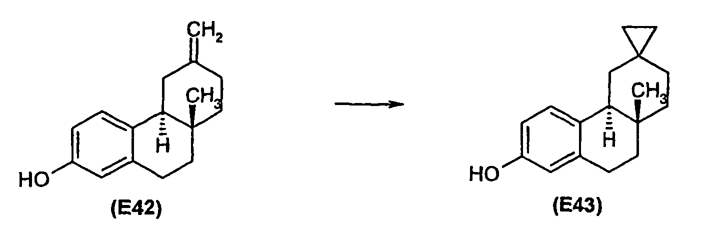

*(rac)-(4aS,10aS)*-3,3-ethanediyl-7-hydroxy-10a-methyl-1,2,3,4,4a,9,10,10a-octahydrophen anthrene. Me₃Al (0.24 mL of 2.0 M solution in hexanes) was added to a well-stirred solution of *(rac)-(4aS,10aS)*-7-hydroxy-10a-methyl-3,3-mehylene-1,2,3,4,4a,9,10, 10a-octahydrophenanthrene (50 mg, 0.219 mmol) and CH₂I₂ (63 mg, 0.263 mmol) in *iso*-hexane (10 mL) at r.t. The resulting mixture was stirred at r.t. for 3h and then quenched with 1 *M* aq. HCI (20 mL). The product was extracted with Et₂O (3×15 mL), the combined extracts were washed with brine, dried with anhydrous sodium sulfate and evaporated. The crude product was purified by PHPLC to yield *(rac)-(4aS,10aS)*-3,3-ethanediyl-7-hydroxy-10a-methyl-1,2,3,4,4a,9,10,10a-octahydrophenanthrene (20 mg) as a white oil. ES-MS m/z: 259.9 (pos., M+NH₄ ⁺), 241.0 (neg., M-H);¹H NMR (270 MHz, CDCl₃) δ 6.92 (d, 1H), 6.65-6.50 (m, 2H), 4.60 (bs, 1H), 2.95-2.70 (m, 2H), 2.65 (dd, 1H); 2.10 (dddd, 1H), 1.80 (ddd, 1H), 1.70-1.35 (m, 4H), 1.25 (ddd, 1H), 0.75 (s, 3H), 0.75-0.60 (m, 1H), 0.45-0.29 (m, 2H), 0.29-0.15 (m, 2H).

### Example 44: Synthesis of (rac)-(3R,4aS,10aS)-1',2',3',4'-tetrachloro-7-hydroxy-10a-methyl-1,2,3,4,4a,9,10,10a-octahydrospiro[phenanthrene-3,6'-cyclohexane]-1',3'-diene (E44).

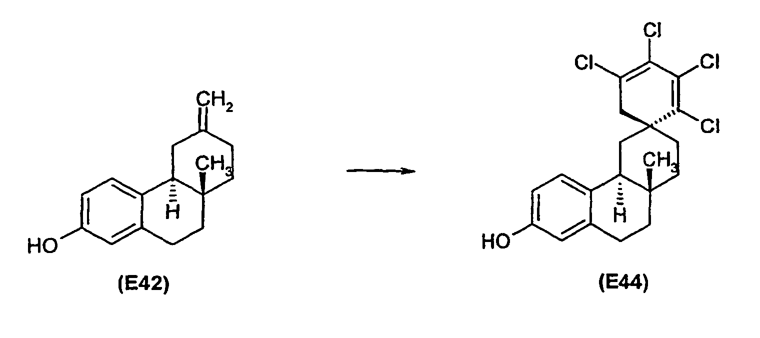

*(rac)-(3R,4aS,10aS)*-1',2',3',4'-tetrachloro-7-hydroxy-10a-methyl-1,2,3,4, 4a,9,10, 10a-octahydrospiro[phenanthrene-3,6'-cyclohexane]-1',3'-diene. A solution of *(rac)-(4aS,10aS)-7-hydroxy-10a-methyl-3,3-methylene*-1,2,3,4,4a,9,10,10a octahydrophenanthrene (75 mg, 0.33 mmol) and tetrachlorothiophen-1,1-dioxide (167 mg, 0.66 mmol) in dichloroethane (20 mL) was refluxed at 100°C for 2 days. An additional amount of tetrachlorothiophen-1,1-dioxide (419 mg, 1.65 mmol) was added and the reaction mixture was refluxed for additional 2 days. The solvent was evaporated and the residue was purified by silica gel flash chromatography using Et₂O : *iso*-hexane (1:3) as eluent to give 90 mg of crude (~70% purity) product. The following PHPLC separation afforded 54 mg of higher purity (~80%) product. For further purification the hydroxy group was converted into *t*-butyldimethylsilyloxy moiety according the following procedure. A solution of crude *(rac)-(3R,4aS,10aS)*-1',2',3',4'-tetrachloro-7-hydroxy-10a-methyl-1, 2,3,4,4a,9,10,10a-octahydrospiro[phenanthrene-3,6'-cyclohexane]-1',3'-diene (54 mg, ~0.13 mmol), *t*-butyldimethylsilylchloride (TBDMS) (196 mg, 1.30 mmol) and Et₃N (132 mg, 1.30 mmol) in CH₂Cl₂ (10 mL) was stirred at 40°C overnight, then the solvent was evaporated and the residue was flashed through a short silica gel column using Et₂O : iso-hexane (1:1) as eluent. Crude TBDMS-protected compound was purified using PHPLC to give 31 mg of the pure product. 9 mg of that compound was deprotected using a solution of tetrabutylammonium fluoride (TBAF) (52 mg, 0.20 mmol) in THF (10 mL). The mixture was stirred at r.t. for 1 h, then the solvent was evaporated, water (5 mL) was added to the residue and the product was extracted with CH₂Cl₂(4×5 mL). The solvent was evaporated to give pure *(rac)-(3R,4aS,10aS)*-1',2',3',4'-tetrachloro-7-hydroxy-10a-methyl-1,2,3,4,4a,9,10,10a-octahydrospiro[phenanthrene-3,6'-cyclohexane]-1',3'-diene (7 mg) as a white oil. ES-MS m/z: 417.2 (neg., M-H); ¹H NMR (270 MHz, CDCl₃) δ 7.00 (d, 1H), 6.64-6.55 (m, 2H), 4.62 (bs, 1H), 2.98-2.73 (m, 3H), 2.90 (s, 2H), 2.52 (dd, 1H); 2.20-2.03 (m, 2H), 1.84 (dd, 1H), 1.70-1.35 (m, 5H), 0.73 (s, 3H).

### Example 45: Synthesis of (rac)-(3S,4aS,10aS)-3.7-dihydroxy-10a-methyl-3-[1-(phenylthio)cyclopropyl]-1,2,3,4,4a,9,10,10a-octahydrophenanthrene (E45).

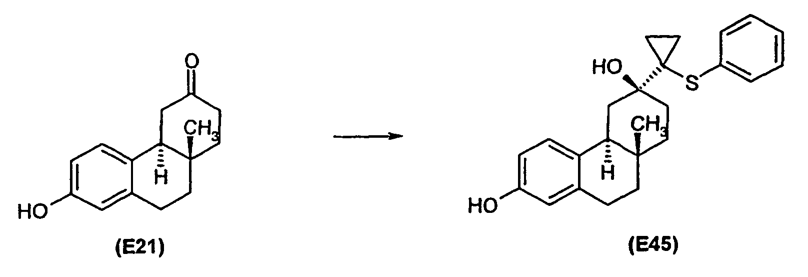

*(rac)-(3S,4aS,10aS)*-3,7-dihydroxy-10a-methyl-3-[1-(phenylthio)cyclopropyl]-1,2,3,4,4a,9, 10,10a-octahydrophenanthrene. *n*-BuLi (2.36 mL of 1.6 M solution in hexanes, 3.78 mmol) was added to a solution of cyclopropylphenylsulphide (379 mg, 2.52 mmol) in THF (15 mL) under cooling to 0°C. The reaction mixture was allowed to warm to r.t. and then was stirred at r.t. for 2 h. The resulting solution was cooled to 0°C and a solution of the ketone *(rac)-(4aS,10aS)*-7-hydroxy-10a-methyl-1,4,4a,9,10,10a-hexahydro-*2H*-phenanthren-3-one (145 mg, 0.63 mmol) in THF (5 mL) was added. The resulting mixture was stirred at r.t. overnight and then was quenched with water (5 mL) together with sat. aq. NH₄Cl (1 mL). The organic phase was separated and the aqueous phase was additionally extracted with EtOAc (3x 10 mL). The combined organic solutions were washed with brine, dried with anhydrous Na₂SO₄ and evaporated. The crude product was purified by PHPLC to yield *(rac)-(3S,4aS,10aS)*-3,7-dihydroxy-10a-methyl-3-[1-(phenylthio)cyclopropyl]-1,2,3, 4,4a,9,10,10a-octahydrophenanthrene (100 mg) as a pale yellow oil. ¹H NMR (270 MHz, CDCl₃) δ 7.50-7.43 (m, 2H), 7.31-7.22 (m, 2H), 7.14 (tt, 1H), 6.90 (d, 1H), 6.59-6.52 (m, 2H), 4.70 (bs, 1H), 2.90-2.67 (m, 3H), 2.20 (ddd, 1H), 1.88-1.74 (m, 1H); 1.68-1.48 (m, 5H), 1.42-1.24 (m,3H), 1.14-0.95 (m, 2H), 0.64 (s, 3H); ¹³C NMR (CDCl₃) δ 153.17, 137.96, 137.49, 131.53, 128.62 (2C), 128.41 (2C), 125.60 (2C), 115.24, 112.43, 74.44, 39.57, 37.62, 36.17, 35.71, 33.97, 32.08, 29.55, 26.21, 14.59, 13.58, 13.32.

### Example 46: Synthesis of (rac)-(3S,4aS,10aS)-7-hydroxy-10a-methyl-1,2,3,4,4a,9, 10,10a-octahydrospiro[phenanthrene-3,2'-cyclobutane]-1'-one (E46a) and (rac)-(3R,4aS,10aS)-7-hydroxy-10a-methyl-1,2,3,4,4a,9,10,10a-octahydrospiro[phenanthrene-3,2'-cyclobutane]-1'-one (E46b).

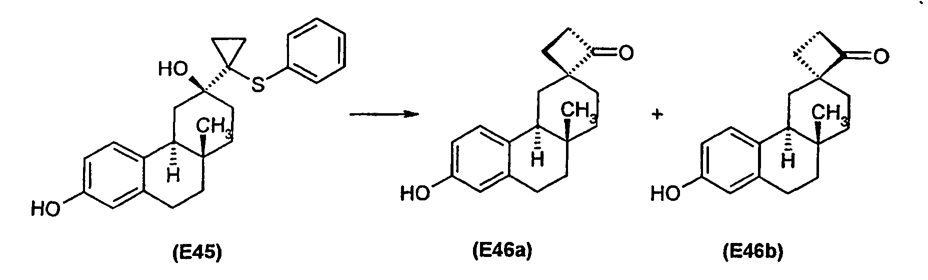

*(rac)-(3S, 4aS, 10aS)*-7-hydroxy-10a-methyl-1,2,3,4,4a,9,10,10a-octahydrospiro[phenanthrene-3,2'-cyclobutane]-1'-one and *(rac)-(3R,4aS,10aS)*-7-hydroxy-10a-methyl-1,2,3,4, 4a,9,10,10a-octahydrospiro[phenanthrene-3,2'-cyclobutane]-1'-one. A mixture *(rac)-(3S,4aS,10aS)*-3,7-dihydroxy-10a-methyl-3-[1-(phenylthio)cyclopropyl]-1,2,3,4, 4a,9,10,10a-octahydrophenanthrene (80 mg, 0.21 mmol) and 4-toluenesulfonic acid monohydrate (200 mg, 1.05 mmol) in toluene (20 mL) was stirred at 100°C overnight. The resulting mixture was quenched with sat. aq. NaHCO₃ (10 mL), the organic layer was separated and the solvent was evaporated. The residue was separated using PHPLC to give two diastereomeric products, *(rac)-(3S,4aS,10aS)*-7-hydroxy-10a-methyl-1,2,3,4,4a,9, 10,10a-octahydrospiro[phenanthrene-3,2'-cyclobutane]-1'-one (32 mg) and *(rac)-(3R,4aS,10aS)*-7-hydroxy-10a-methyl-1,2,3,4,4a,9,10,10a-octahydrospiro[phenanthrene-3,2'-cyclobutane]-1'-one (10 mg) as white oils. *(rac)-(3S,4aS,10aS)*-7-hydroxy-10a-methyl-1,2,3,4,4a,9,10,10a-octahydrospiro[phenanthrene-3,2'-cyclobutane]-'-one: ES-MS m/z: 288.2 (pos., M+NH₄ ⁺), 268.8 (neg., M-H); ¹H NMR (270 MHz, CDCl₃) δ 6.95 (d, 1H), 6.65-6.50 (m, 2H), 5.26 (bs, 1H), 3.07 (t, 2H), 2.92-2.68 (m, 2H), 2.44 (d, 1H), 2.25 (ddd, 1H), 2.00-1.80 (m, 3H), 1.66-1.45 (m, 5H), 1.34 (ddd, 1H), 0.68 (s, 3H). *(rac)-(3R,4aS,10aS)*-7-hydroxy-10a-methyl-1,2,3,4,4a,9,10,10a-octahydrospiro[phenanthrene-3,2'-cyclobutane]-1'-one: ES-MS m/z: 288.2 (pos., M+NH₄ ⁺), 270.2 (neg., M-H); ¹H NMR (270 MHz, CDCl₃) δ 6.97 (d, 1H), 6.64-6.46 (m, 2H), 4.84 (bs, 1H), 3.00 (t, 2H), 2.90-2.68 (m, 2H), 2.43 (dd, 1H), 1.97-1.35 (m, 10H), 0.68 (s, 3H).

### Example 47: Synthesis of (rac)-(3S,4aS,10aS)-1',1'-ethanediyldimercapto-7-hydroxy-10a-methyl-1,2,3,4,4a,9,10,10a-octahydrospiro[phenanthrene-3,2'-cyclobutane] (E47).

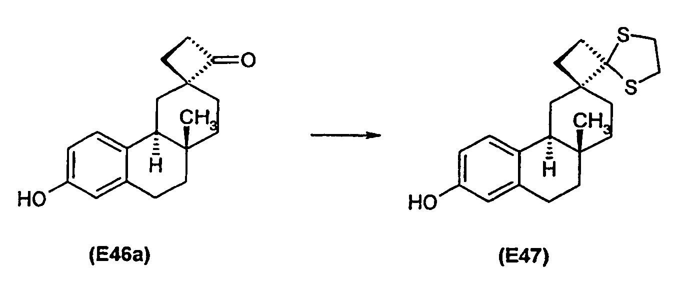

*(rac)-(3S,4aS,10aS)*-1',1'-ethanediyldimercapto-7-hydroxy-10a-methyl-1,2,3,4,4a,9,10, 10a-octahydrospiro[phenanthrene-3,2'-cyclobutane]. The reaction was carried out on 28.0 mg (0.10 mmol) *(rac)-(3S,4aS,10aS)*-7-hydroay-10a-methyl-1,2,3,4,4a,9,10, 10a-octahydrospiro[phenanthrene-3,2'-cyclobutane]-1'-one, according to **method G** ("J"= 10, "K"= 1,2-ethanedithiol, "L"= 2, "M"= 18), using 3 eq. of BF₃·OEt₂ complex. The crude product was purified by silica gel flash chromatography using Et₂O : *iso*-hexane (1:1) as eluent to yield *(rac)-(3S,4aS,10aS)*-1',1'-ethanediyldimercapto-7-hydroxy-10a-methyl-1, 2,3,4,4a,9,10,10a-octahydrospiro[phenanthrene-3,2'-cyclobutane] (31 mg) as a white oil. ES-MS m/z: 347.2 (pos., M+H), 391.0 (neg., M+HCOOH-H); ¹H NMR (270 MHz, CDCl₃) δ 7.12 (d, 1H), 6.62 (dd, 1H), 6.55 (d, 1H), 4.60 (bs, 1H), 3.30-3.10 (m, 4H), 2.90-2.70 (m, 2H), 2.60-2.46 (m, 2H), 2.45-2.34 (m, 2H), 1.98-1.76 (m, 4H), 1.62-1.24 (m, 5H), 0.69 (s, 3H).

### Example 48: Synthesis of (rac)-(4aS,10aS)-7-hydroxy-10a-methyl-1,2,3,4,4a,9,10, 10a-octahydrospiro[phenanthrene-3,1'-cyclobutane] (E48).

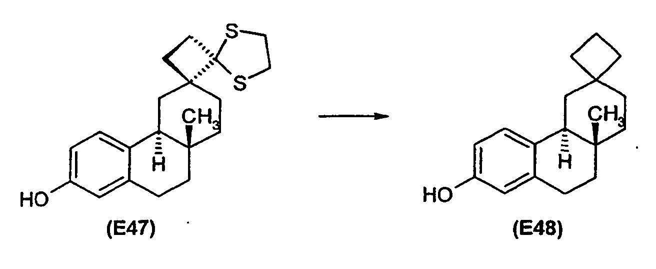

*(rac)-(4aS,10aS)*-7-hydroxy-10a-methyl-1,2,3,4,4a,9,10,10a-octahydrospiro[phenanthrene-3,1'-cyclobutane]. The reaction was carried out on 24.0 mg (0.070 mmol) *(rac)-(3S,4aS,10aS)*-1',1'-ethanediyldimercapto-7-hydroxy-10a-methyl-1,2,3,4,4a,9,10, 10a-octahydrospiro[phenanthrene-3,2'-cyclobutane], according to **method J** ("J" = 100, "K" = 15, "L" = 20, "M" = 0, "N" = 24) at 80°C to give
*(rac)-(4aS,10aS)*-7-hydroay-10a-methyl-1,2,3,4,4a,9,10,10a-octahydrospiro[phenanthrene-3,1'-cyclobutane] (18 mg) as a white oil. ES-MS m/z: 257.2 (pos., M+H), 255.1 (neg., M-H); ¹H NMR (270 MHz, CDCl₃) δ 7.08 (d, 1H), 6.61 (dd, 1H), 6.55 (d, 1H), 4.40 (bs, 1H), 2.94-2.67 (m, 2H), 2.33 (dd, 1H), 2.20 (ddd, 1H), 1.98-1.18 (m, 13H), 0.67 (s, 3H).

### Example 49: Synthesis of (rac)-(4aS,10aS)-3-(1-cyclopenten-1-yl)-7-hydroxy-10a-methyl-1,4,4a,9,10,10a-hexahydrophenanthrene (E49).

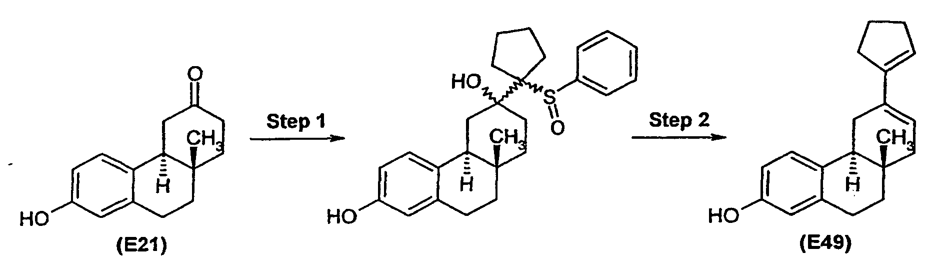

**Step 1.** *(rac)-(4aS,10aS)*-3,7-dihydroxy-10a-methyl-3-[1-(phenylsulphinyl)cyclopentyl]-1,2,3,4,4a,9,10,10a-octahydrophenanthrene (mixture of diastereomers). *n*-BuLi (1.60 mL of 1.6 M solution in hexanes, 2.40 mmol) was added to a solution of cyclopenthylphenylsulphoxide (466 mg, 2.40 mmol) in THF (15 mL) under cooling to -70°C. The reaction mixture was allowed to warm to r.t. and then was cooled to 0°C. A solution of *(rac)-(4aS,10aS)*-7-hydroxy-10a-methyl-1,4,4a,9,10,10a-hexahydro-2H-phenanthren-3-one (110 mg, 0.478 mmol) in THF (5 mL) was added and the resulting mixture was stirred at r.t. overnight. The resulting mixture was quenched with sat. aq. NH₄Cl (10 mL) together with water (10 mL). The organic phase was separated and the aqueous phase was additionally extracted with EtOAc (3x15 mL). The combined organic solutions were washed with brine, dried with anhydrous Na₂SO₄ and evaporated. The product, a mixture of diastereomers of *(rac)-(4aS,10aS)*-3,7-dihydroxy-10a-methyl-3-[1-(phenylsulphinyl)cyclopentyl]-1,2,3,4,4a,9,10,10a-octahydrophenanthrene, was used in the next step without purification or separation.

**Step 2.** *(rac)-(4aS,10aS)*-3-(1-cyclopenten-1-yl)-7-hydroxy-10a-methyl-1,4,4a,9,10, 10a-hexahydrophenanthrene. TiCl₄ (1.43 mmol, 271 mg) was added to a well-stirred mixture of Zn powder (2.87 mmol, 188 mg) and *(rac)-(4aS,10aS)*-3,7-dihydroxy-10a-methyl-3-[1-(phenylsulphinyl)cyclopentyl]-1,2,3,4,4a,9,10, 10a-octahydrophenanthrene (crude mixture of diastereomers from the previous step, about 0.48 mmol) in THF (25 mL) under cooling to 0°C. The resulting mixture was stirred at r.t. for 2 h and then quenched with sat. aq. NaHCO₃ (10 mL). The organic phase was separated and the aqueous phase was additionally extracted with Et₂O (3×15 mL). The combined organic solutions were washed with brine, dried with anhydrous Na₂SO₄ and evaporated. The residue was separated using PHPLC to give *(rac)-(4aS,10aS)*-3-(1-cyclopenten-1-yl)-7-hydroxy-10a-methyl-1,4,4a,9,10,10a-hexahydrophenanthrene (20 mg) as a pale yellow oil. ES-MS m/z: 281.2 (pos., M+H); ¹H NMR (270 MHz, CDCl₃) δ 7.19 (d, 1H), 6.66 (dd, 1H), 6.57 (d, 1H), 5.79 (s, 1H), 5.68 (dd, 1H), 4.55 (bs, 1H), 3.33-2.81 (m, 2H), 2.86-2.60 (m, 2H), 2.55-2.38 (m, 4H), 2.14-1.84 (m, 5H), 1.72-1.50 (2H), 0.75 (s, 3H); ¹³C NMR (CDCl₃) δ 153.15, 144.35, 137.87, 132.85, 131.85, 127.26, 123.92, 123.05, 115.08, 112.93, 41.65, 40.78, 37.11, 33.06, 32.22, 30.97, 28.43, 26.31, 23.08, 16.19.

### Example 50: Synthesis of (rac)-(3S,4aS,10aS)-7-hydroxy-10a-methyl-1,2,3, 4,4a,9,10,10a,2',3',4',5'-dodecahydrospiro[phenanthrene-3,2'-furan] (E50A) and (rac)-(3R,4aS,10aS)-7-hydroxy-10a-methyl-1,2,3,4,4a,9,10,10a,2',3', 4',5'-dodecahydrospiro[phenanthrene-3,2'-furan] (E50b).

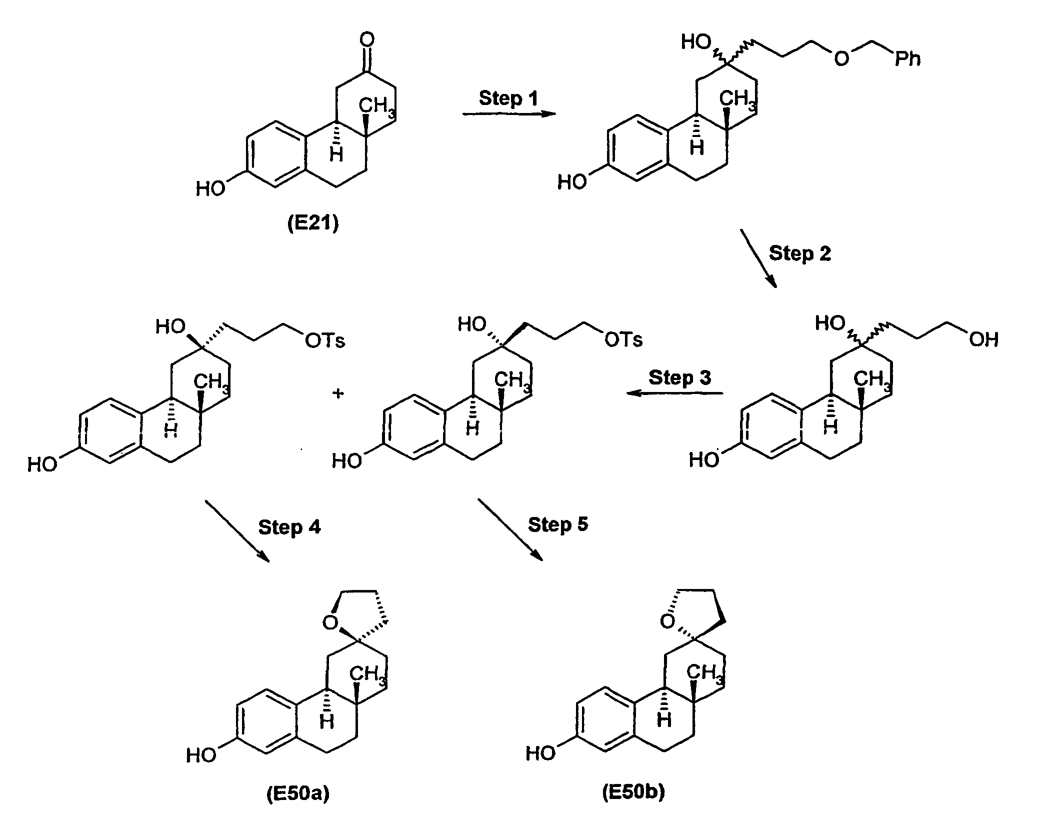

**Step 1.**
*(rac)-(4aS,10aS)*-3,7-dihydroxy-10a-methyl-3-[3-(phenylmethoxy)propyl]-1,2,3,4,4a,9,10,10a-octahydrophenanthrene (mixture of diastereomers). A mixture of Mg pulver (114 mg, 4.70 mmol) and benzyl 3-bromopropyl ether (538 mg, 2.35 mmol) in THF (20 mL) was stirred at 60°C for 2 h. A solution of (*rac*)-(*4aS,10aS*)-7-hydroxy-10a-methyl-1,4,4a,9, 10,10a-hexahydro-*2H*-phenanthren-3-one (108 mg, 0.47 mmol) in THF (5 mL) was added to the resulting mixture at r.t. The resulting mixture was stirred at r.t. overnight and then quenched with sat. aq. NH₄Cl (10 mL) together with water (10 mL). The organic phase was separated and the aqueous phase was additionally extracted with EtOAc (3x15 mL). The combined organic solutions were washed with brine, dried with anhydrous Na₂SO₄ and evaporated. The product, a mixture of diastereomers of *(rac)-(4aS,10aS)*-3,7-dihydroxy-10a-methyl-3-[3-(phenylmethoxy)propyl]-1,2,3,4,4a,9,10,10a-octahydrophenanthrene, was used in the next step without purification or separation.

**Step 2.** *(rac)-(4aS,10aS)*-3,7-dihydroxy-3-(3-hydroxypropyl)-10a-methyl-1,2,3,4,4a,9,10, 10a-octahydrophenanthrene (mixture of diastereomers). The crude mixture of diastereomers of *(rac)*-*(4aS,10aS)*-3,7-dihydroxy-10a-methyl-3-[3-(phenylmethoxy) propyl]-1,2,3,4,4a,9,10,10a-octahydrophenanthrene from the previous step (about 0.47 mmol) was treated according to **method F** ("J" = THF, "K" = 20, "L" = 10% Pd/C (wet, 50% of water), "M" =140, "N" = 72) to obtain a mixture of diastereomers of *(rac)*-*(4aS*,*10aS)*-3,7-dihydroxy-3-(3-hydroxypropyl)-10a-methyl-1,2,3,4,4a,9,10, 10a-octahydrophenanthrene, which was used in the next step without purification or separation.

**Step 3.** *(rac)-(3S,4aS,10aS)*-3,7-dihydroxy-3-[3-[(4-methylphenyl)sulphonyloxy]propyl]-10a-methyl-1,2,3,4,4a,9,10,10a-octahydrophenanthrene and *(rac)-(3R,4aS,10aS)*-3, 7-dihydroxy-3-[3-[(4-methylphenyl)sulphonyloxy]propyl]-10a-methyl-1,2,3,4,4a,9,10, 1 0a-octahydrophenanthrene. A mixture of the diastereomers of *(rac)-(4aS,10aS)*-3,7-dihydroxy-3-(3-hydroxypropyl)-10a-methyl-1,2,3,4,4a,9,10, 10a-octahydrophenanthrene from the previous step (about 0.47 mmol), 4-methylphenylsulphonyl chloride (448 mg, 2.35 mmol), 4-dimethylaminopyridine (57 mg, 0.47 mmol) and pyridine (372 mg, 4.70 mmol) in CH₂Cl₂ (25 mL) was stirred at 40°C overnight. The resulting mixture was diluted with CHCl₃ (50 mL) and washed subsequently with sat. aq. NaHCO₃ (2x 15 mL), 1*M* aq. HCl (2×15 mL), water (10 mL) and then again with sat. aq. NaHCO₃ (15 mL). The resulting solution was dried with anhydrous Na₂SO₄ and the solvent was evaporated. The residue was separated using PHPLC to give pure *(rac)- (3S,4aS,10aS)*-3,7-dihydroxy-3-[3-[(4-methylphenyl)sulphonyloxy]propyl]-10a-methyl-1,2,3,4,4a,9,10,10a-octahydrophenanthrene (53 mg) and *(rac)-(3R,4aS,10aS)*-3,7-dihydroxy-3-[3-[(4-methylphenyl) sulphonyloxy]propyl]-10a-methyl-1,2,3,4,4a,9,10,10a-octahydrophenanthrene (65 mg) as white oils. *(rac)-(3S,4aS,10aS)*-3,7-dihydroxy -3-[3-[(4-methylphenyl)sulphonyloxy]propyl] -10a-methyl-1,2,3,4,4a,9,10,10a octahydrophenanthrene: ES-MS m/z: 444.4 (pos., M+H), 485.2 (neg., M+HCOOH-H); ¹H NMR (270 MHz, CDCl₃) δ 7.70 (d, 2H), 7.29 (d, 2H), 7.04 (d, 1H), 6.75 (d, 1H), 6.65 (dd, 1H), 3.87 (t, 2H), 2.90-2.66 (m, 2H), 2.43 (s, 3H), 2.38 (dd, 1H), 2.07 (ddd, 1H), 2.02-1.88 (m, 2H), 1.88-1.42 (m, 7H), 1.35-1.17 (m, 2H), 0.70 (s, 3H). *(rac)-(3R,4aS,10aS)*-3,7-dihydroxy-3-[3-[(4-methylphenyl)sulphonyloxy] propyl]-10a-methyl-1,2,3,4,4a,9,10,10a-octahydrophenanthrene: ES-MS m/z: 444.4 (pos., M+H), 485.2 (neg., M+HCOOH-H); ¹H NMR (270 MHz, CDCl₃) δ 7.67 (d, 2H), 7.27 (d, 2H), 7.02 (d, 1H), 6.71 (d, 1H), 6.62 (dd, 1H), 3.81 (t, 2H), 2.86-2.64 (m, 3H), 2.42 (s, 3H), 2.11 (dd, 1H), 2.00-1.86 (m, 2H), 1.78-1.46 (m, 9H), 1.44-1.29 (m, 2H), 0.64 (s, 3H).

**Step 4.** *(rac)-(3S,4aS,10aS)*-7-hydroxy-10a-methyl-1,2,3,4,4a,9,10,10a,2',3',4',5' -dodecahydrospiro[phenanthrene-3,2'-furan]. Potassium *tert*-butoxide (*t*-BuOK) (67 mg, 0.595 mmol) was added to a solution of *(rac)-(3S,4aS,10aS)*-3,7-dihydroxy-3-[3-(4-methylphenyl-sulphonyloxy)propyl]-10a-methyl-1,2,3,4,4a,9,10, 10a-octahydrophenanthrene (53 mg, 0.119 mmol) in THF (20 mL). The reaction mixture was stirred at r.t. for 30 min, and then quenched with sat. aq. NH₄Cl (10 mL) together with water (10 mL). The organic phase was separated and the aqueous phase was additionally extracted with EtOAc (3x15 mL). The combined organic solutions were washed with brine, dried with anhydrous Na₂SO₄ and evaporated. The crude product was purified by silica gel flash chromatography using Et₂O : *iso*-hexane (1:1) as eluent to yield *(rac)-(3S,4aS,10aS)* -7-hydroxy-10a-methyl-1,2,3,4,4a,9,10,10a,2',3',4',5'-dodecahydrospiro [phenanthrene-3, 2'-furan] (26 mg) as a white solid. ES-MS m/z: 273.2 (pos., M+H); ¹H NMR (270 MHz, CDCl₃) δ 7.00 (d, 1H), 6.68-6.50 (m, 2H), 5.76 (bs, 1H), 3.92 (t, 2H), 2.94-2.68 (m, 2H), 2.37 (dd, 1H), 2.23 (ddd, 1H), 2.06-1.42 (m, 9H), 1.35-1.17 (m, 2H), 0.72 (s, 3H); ¹³C NMR (CDCl₃) δ 153.65, 137.61, 131.03, 125.66, 115.31, 112.58, 84.63, 66.47, 43.06, 38.53, 37.88, 36.15, 34.89, 32.80, 32.36, 26.34, 26.00, 15.14.

**Step 5**. *(rac)-(3R,4aS,10aS)*-7-hydroxy-10a-methyl-1,2,3, 4,4a,9,10, 10a,2',3',4',5'-dodecahydrospiro[phenanthrene-3,2'-furan]. *(rac)-(3R,4aS,10aS)*-3, 7-dihydroxy-3-[3-[(4-methylphenyl)sulphonyloxy]propyl]-10a-methyl-1,2,3,4,4a,9,10, 10a-octahydrophenanthrene (65 mg, 0.146 mmol) was treated according to procedure, described above in **Step 4** to give
*(rac)-(3R,4aS,10aS)* -7-hydroxy-10a-methyl-1,2,3,4,4a,9,10,10a,2',3',4',5'-dodecahydrospiro[phenanthrene-3,2'-furan] (37 mg) as a white solid. ES-MS m/z: 273.4 (pos., M+H), 271.4 (neg., M-H); ¹H NMR (270 MHz, CDCl₃) δ 6.98 (d, 1H), 6.65-6.47 (m, 2H), 5.71 (s, 1H), 3.87 (td, 2H), 2.90-2.64 (m, 2H), 2.19 (dd, 1H), 2.06-1.24 (m, 11H), 0.68 (s, 3H); ¹³C NMR (CDCl₃) δ 153.27, 137.94, 131.75, 125.57, 115.27, 112.37, 84.41, 66.73, 40.52, 38.81, 37.81, 36.71, 35.22, 32.00, 31.94, 26.25, 25.27, 14.89.

### Example 51: Synthesis of (rac)-(3S,4aS,10aS)-7-hydroxy-10a-methyl-1,2,3,4, 4a,9,10,10a,3',4',5',6'-dodecahydrospiro[phenanthrene-3,2'-2H-pyran] (E51a) and (rac)-(3R,4aS,10aS)-7-hydroxy-10a-methyl-1,2,3,4,4a,9,10,10a,3',4',5',6'-dodecahydrospiro[phenanthrene-3,2'-2H-pyran] (E51b).

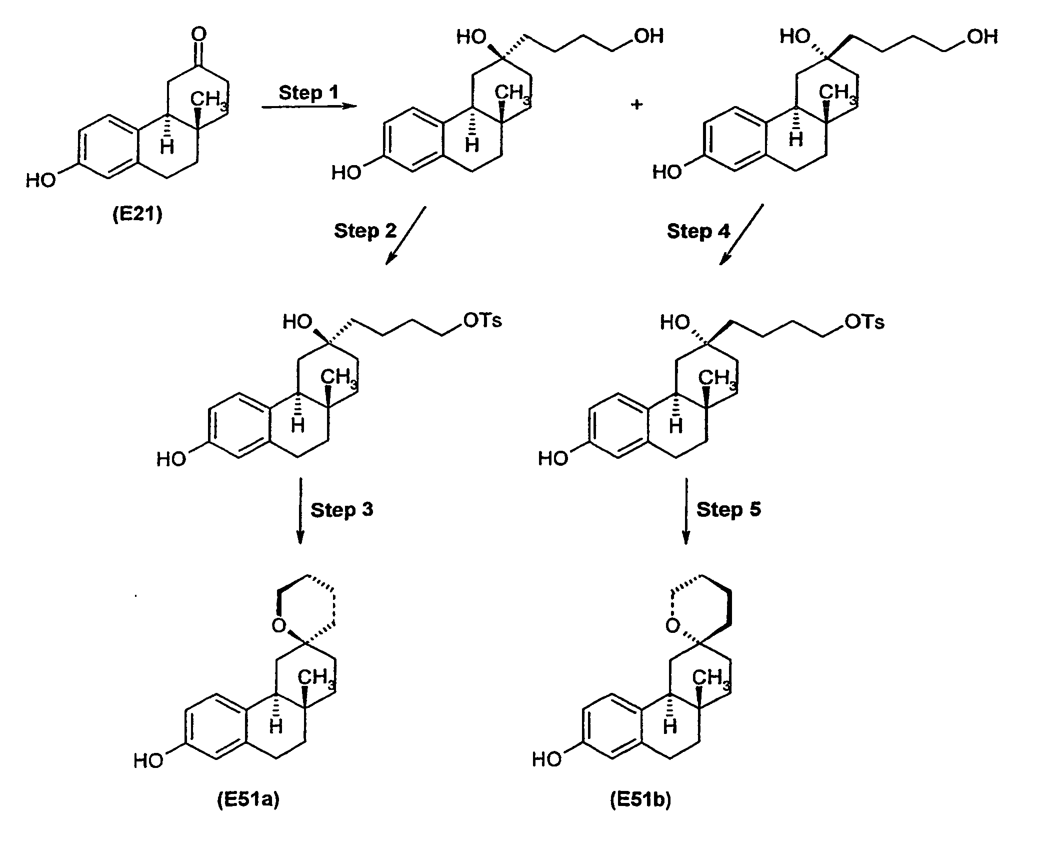

**Step 1.** *(rac)-(3S,4aS,10aS)*-3,7-dihydroxy-3-(4-hydroxybutyl)-10a-methyl-1,2,3,4,4a, 9,10,10a-octahydrophenanthrene and *(rac)-(3R,4aS,10aS)*-3,7-dihydroxy-3-(4-hydroxybutyl)-10a-methyl-1,2,3,4,4a,9,10,10a-octahydrophenanthrene. Benzyl 4-bromobutyl ether (571 mg, 2.35 mmol) was added to a well-stirred suspension of Rieke® Mg (114 mg, 4.70 mmol) in THF (20 mL) at r.t. and the resulting mixture was stirred at 50°C for 2 h. A solution of (*rac*)-(*4aS,10aS*)-7-hydroxy-10a-methyl-1,4,4a,9,10, 10a-hexahydro-*2H*-phenanthren-3-one (108 mg, 0.47 mmol) in THF (5 mL) was added to the mixture at r.t. and the resulting mixture was stirred at r.t. for 3 days. The mixture was quenched with sat. aq. NH₄Cl (10 mL) together with water (10 mL). The organic phase was separated and the aqueous phase was additionally extracted with EtOAc (3x15 mL). The combined organic solutions were washed with brine, dried with anhydrous Na₂SO₄ and evaporated. The residue was separated by silica gel flash chromatography using Et₂O : MeOH (97:3) as eluent to yield *(rac)-(3S,4aS,10aS)*-3,7-dihydroxy-3-(4-hydroxybutyl)-10a-methyl-1,2,3,4,4a,9,10,10a-octahydrophenanthrene (49 mg) and *(rac)-(3R,4aS,10aS)*-3,7-dihydroxy-3-(4-hydroxybutyl)-10a-methyl-1,2,3,4,4a,9,10, 10a-octahydrophenanthrene (76 mg) as white solids.

**Step 2.** *(rac)-(3S,4aS,10aS)*-3,7-dihydroxy-10a-methyl-3-[4-[(4-methylphenyl)-sulphonyloxy]butyl]-1,2,3,4,4a,9,10,10a-octahydrophenanthrene. *(rac)-(3S,4aS,10aS)-*3,7-dihydroxy-3-(4-hydroxybutyl)-10a-methyl-1,2,3,4,4a,9,10,10a-octahydrophenanthrene (49 mg, 0.161 mmol) was treated according to procedure, described above in **Example 50, Step 3** to give *(rac)-(3S,4aS,10aS)-*3,7-dihydroxy-3-[4-[(4-methylphenyl)sulphonyloxy]-1-butyl]-10a-methyl-1,2,3,4,4a,9,10,10a-octahydrophenanthrene, which was used for the next step without purification.

**Step 3.** *(rac)-(3S,4aS,10aS)*-7-hydroay-10a- methyl-1,2,3,4,4a,9, 10,10a,3',4',5',6'-dodecahydrospiro[phenanthrene-3,2'-*2H*-pyran]. Crude *(rac)-(3S,4aS,10aS)*-3,7-dihydroxy-10a-methyl-3-[4-[(4-methylphenyl) sulphonyloxy]butyl]-1,2,3,4,4a,9,10,10a-octahydrophenanthrene from the previous step (about 0.16 mmol) was treated according to procedure, described above in **Example 50, Step 4** to give *(rac)-(3S,4aS,10aS)*-7-hydroxy-10a-methyl-1,2,3,4,4a,9,10,10a,3',4',5',6'-dodecahydrospiro[phenanthrene-3,2'-2*H*-pyran] (31 mg) as a white oil. ES-MS m/z: 287.2 (pos., M+H), 285.0 (neg., M-H); ¹H NMR (270 MHz, CDCl₃) δ 7.04 (d, 1H), 6.67-6.55 (m, 2H), 5.71 (s, 1H), 3.90-3.71 (m, 2H), 2.95-2.69 (m, 2H), 2.50-2.37 (m, 2H), 1.98 (dd, 1H), 1.82-1.20 (m, 12H), 0.72 (s, 3H); ¹³C NMR (CDCl₃) δ 153.59, 137.76, 131.20, 125.57, 115.32, 112.57, 74.62, 61.25, 41.02, 37.89, 37.06, 34.88, 33.02, 31.70, 30.17, 26.33, 26.25, 19.02, 15.31.

**Step 4.** *(rac)-(3R,4aS,10aS)*-3,7-dihydroxy-10a-methyl-3-[4-[(4-methylphenyl)sulphonyloxy]butyl]-1,2,3,4,4a,9,10,10a-octahydrophenanthrene. *(rac)-(3R,4aS,10aS)*-3,7-dihydroxy-3-(4-hydroxybutyl)-10a-methyl-1,2,3,4,4a,9,10,10a-octahydrophenanthrene (76 mg, 0.25 mmol) was treated according to procedure, described above in **Example 50, Step 3** to give *(rac)-(3R,4aS,10aS)*-3,7-dihydroxy-3-[4-[(4-methylphenyl)sulphonyloxy]-1-butyl]-10a-methyl-1,2,3,4,4a,9,10, 10a-octahydrophenanthrene, which was used for the next step without purification.

**Step 5.** *(rac)-(3R,4aS,10aS)*-7-hydroxy-10a-methyl-1,2,3,4,4a,9,10,10a,3',4',5',6'-dodecahydrospiro[phenanthrene-3,2'-*2H*-pyran]. Crude *(rac)-(3R,4aS,10aS)*-3,7-dihydroxy-10a-methyl-3-[4-[(4-methylphenyl) sulphonyloxy]butyl]-1,2,3,4,4a,9,10, 10a-octahydrophenanthrene from the previous step (about 0.25 mmol) was treated according to procedure, described above in **Example 50, Step 4** to give *(rac)-(3R,4aS,10aS)*-7-hydroxy-10a-methyl-1,2,3,4,4a,9,10,10a,3',4',5',6'-dodecahydro spiro[phenanthrene-3,2'-2*H*-pyran] (56 mg) as a white oil. ES-MS m/z: 287.2 (pos., M+H), 285.2 (neg., M-H); ¹H NMR (270 MHz, CDCl₃) δ 7.02 (d, 1H), 6.63-6.52 (m, 2H), 5.78 (s, 1H), 3.66 (dd, 2H), 2.91-2.64 (m, 3H), 2.50 (ddd, 1H), 1.93 (ddd, 1H), 1.78-1.34 (m, 10H), 1.26 (ddd, 1H), 1.14 (dd, 1H), 0.67 (s, 3H); ¹³C NMR (CDCl₃) δ 153.24, 138.07, 131.85, 125.52, 115.29, 112.39, 72.58, 60.56, 38.61, 37.89, 37.59, 35.08, 32.63, 32.52, 29.65, 26.30, 26.14, 19.00, 15.08.

### Example 52: Synthesis of (rac)-(1S,3R,4aS,10aS)-7-hydroxy-10a-methyl-1-(3-methylbutyl)-1,2,3,4,4a,9,10,10a,2',3',4',5'-dodecahydrospiro [phenanthrene-3,2'-furan] (E52).

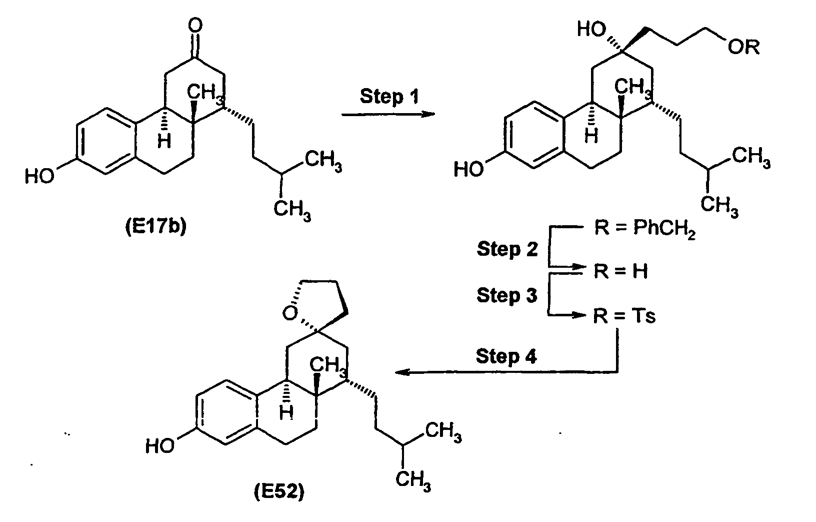

**Step 1.** *(rac)-(1S,3R,4aS,10aS)*-3,7-dihydroxy-10a-methyl-1-(3-methylbutyl)-3-[3-(phenylmethoxy)propyl)-1,2,3,4,4a,9,10,10a-octahydrophenanthrene. *(rac)-(1S,4aS,10aS)*-7-hydroxy-10a-methyl-1-(3-methylbutyl)-1,2,3,4,4a,9,10, 10a-octahydrophenanthrene-3-one (83 mg, 0.277 mmol) was treated according to procedure, described above in **Example 50, Step 1**, but the reaction mixture was stirred overnight at 50°C. The crude product was purified by silica gel flash chromatography using Et₂O : *iso*-hexane (1:1) as eluent to give the only diastereomer, *(rac)-(1S,3R,4aS,10aS)*-3,7-dihydroxy-10a-methyl-1-(3-methylbutyl)-3-[3-(phenylmethoxy)propyl]-1,2,3,4,4a,9,10,10a-octahydrophenthrene (119 mg), as a white oil.

**Step 2.** *(rac)-(1S,3R,4aS,10aS)*-3,7-dihydroxy-3-(3-hydroxypropyl)-10a-methyl-1-(3-methylbutyl)-1,2,3,4,4a,9,10,10a-octahydrophenanthrene. *(rac)-(1S,3R,4aS,10aS)*-3,7-dihydroxy-10a-methyl-1-(3-methylbutyl)-3-[3-(phenylmethoxy)propyl]-1,2,3,4,4a,9,10,10a-octahydrophenanthrene (119 mg, 0.264 mmol) was treated according to **method F** ("J" = THF, "K" = 20, "L" = 10% Pd/C (wet, 50% of water), "M" = 140, "N" = 72), to yield *(rac)-(1S,3R,4aS,10aS)*-3,7-dihydroxy-3-(3-hydroxypropyl)-10a-methyl-1-(3-methylbutyl)-1,2,3,4,4a,9,10,10a-octahydrophenanthrene (95 mg) as a white solid. ¹H NMR (270 MHz, CD₃COCD₃) δ 7.93 (s, 1H), 7.06 (d, 1H), 6.58 (dd, 1H), 6.54 (d, 1H), 3.87 (t, 1H), 3.60 (d, 1H), 3.57 (d, 1H), 3.21 (s, 1H), 3.03 (dd, 1H), 2.83 (ddd, 1H), 2.71 (ddd, 1H), 2.20 (ddd, 1H), 2.00-1.25 (m, 15H), 1.17-1.05 (m, 1H), 0.89 (d, 3H), 0.86 (d, 3H), 0.80 (s, 3H); ¹³C NMR (CDCl₃) δ 155.47, 138.16, 131.85, 127.27, 115.76, 113.58, 79.10, 72.45, 63.34, 45.35, 43.30, 39.27, 38.16, 35.94, 34.92, 34.86, 34.23, 29.09, 27.50, 27.46, 23.51, 22.69, 18.44.

**Step 3.** *(rac)-(1S,3R,4aS,10aS)*-3,7-dihydroxy-10a-methyl-1-(3-methylbutyl)-3-[3-[(4-methylphenyl)sulphonyloxy]propyl]-1,2,3,4,4a,9,10,10a-octahydrophenanthrene. *(rac)-(1S,3R, 4aS,10aS)*-3,7-dihydroxy-3-(3-hydroxypropyl)-10a-methyl-1-(3-methylbutyl)-1,2,3,4,4a,9,10,10a-octahydrophenanthrene (47 mg, 0.13 mmol) was treated according to procedure, described above in **Example 50, Step 3** to give *(rac)-(1S,3R,4aS,10aS)*-3, 7-dihydroxy-10a-methyl-1-(3-methylbutyl)-3-[3-[(4-methylphenyl) sulphonyloxy]propyl]-1,2,3,4,4a,9,10,10a-octahydrophenanthrene, which was used in the next step without further purification.

**Step 4.** *(rac)-(1S,3R,4aS,10aS)*-7-hydroxy-10a-methyl-1-(3-methylbutyl)-1,2,3,4,4a,9,10, 10a,2',3',4',5'-dodecahydrospiro[phenanthrene-3,2'-furan]. *(rac)-(1S,3R,4aS,10aS)*-3,7-dihydroxy-10a-methyl-1-(3-methylbutyl)-3-[3-[(4-methylphenyl)sulphonyloxy]propyl]-1,2, 3,4,4a,9,10,10a-octahydrophenanthrene from the previous step (about 0.13 mmol) was treated according to procedure, described above in **Example 13, Step 4** to give *(rac)-(1S*,*3R*,*4aS*,*10aS)*-7-hydroxy-10a-methyl-1-(3-methylbutyl)-1,2,3,4,4a,9,10, 10a,2',3',4',5'-dodecahydrospiro[phenanthrene-3,2'-furan] (34 mg) as a white oil. ES-MS m/z: 343.4 (pos., M+H); ¹H NMR (270 MHz, CDCl₃) δ 7.07 (d, 1H), 6.57 (dd, 1H), 6.52 (d, 1H), 5.00 (s, 1H), 3.95-3.76 (m, 2H), 2.94 (dd, 1H), 2.92-2.63 (m, 2H), 2.19 (ddd, 1H), 2.00-1.82 (m, 3H), 1.82-1.63 (m, 5H), 1.58-1.21 (m, 6H), 1.26-1.10 (m, 1H), 0.88 (d, 3H), 0.86 (d, 3H), 0.81 (s, 3H); ¹³C NMR (CDCl₃) δ 152.91, 137.97, 132.44, 126.85, 115.04, 112.63, 82.81, 67.13, 44.78, 40.00, 38.60, 36.76, 35.54, 34.89, 33.86, 32.80, 28.38, 26.96, 25.69, 25.18, 23.30, 22.34, 18.15.

### Example 53: Synthesis of(rac)-(1S,4aS,10aS)-7-hydroxy-10a-methyl-1-(3-methylbutyl)-1,2,3,4,4a,9,10,10a-octahydrospiro[phenanthrene-3,1'-cyclobutane](E53).

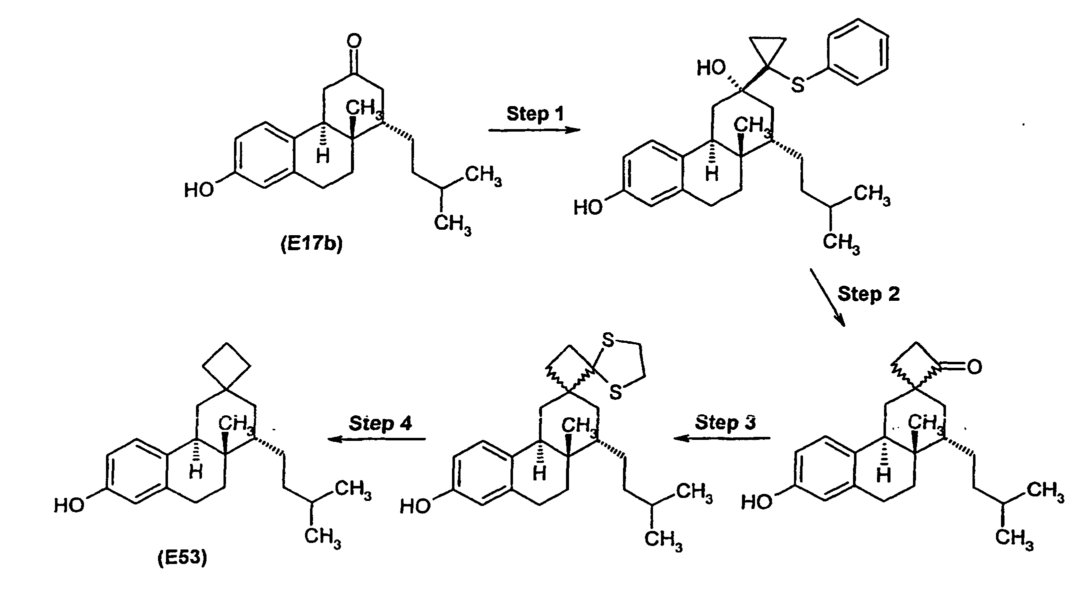

**Step 1.** *(rac)-(1S,3R,4aS,10aS)-*3,7-dihydroxy-10a-methyl-1-(3-methylbutyl)-3-[1-(phenylthio)cyclopropyl]-1,2,3,4,4a,9,10,10a-octahydrophenanthrene. *(rac)-(1S,4aS,10aS)*-7-hydroxy-10a-methyl-1-(3-methylbutyl)-1,2,3,4,4a,9,10, 10a-octahydrophenanthrene-3-one (34 mg, 0.112 mmol) was treated according to procedure, described above in **Example 45** to give the only diastereomer, *(rac)-(1S,3R,4aS,10aS)*-3,7-dihydroxy-10a-methyl-1-(3-methylbutyl)-3-[1-(phenylthio)cyclopropyl]-1,2,3,4,4a,9,10,10a-octahydrophenanthrene (27 mg), as a white oil. ¹H NMR (270 MHz, CDCl₃) δ 7.50-7.42 (m, 2H), 7.31-7.22 (m, 2H), 7.15 (tt, 1H), 7.01 (d, 1H), 6.58 (dd, 1H), 6.53 (d, 1H), 4.90 (s, 1H), 3.56 (d, 1H), 2.99 (dd, 1H), 2.83 (ddd, 1H), 2.70 (ddd, 1H), 2.12 (ddd, 1H), 2.00-1.00 (m, 15H), 0.88 (d, 3H), 0.84 (d, 3H), 0.77 (s, 3H).

**Step 2**. *(rac)-(1S,4aS,10aS)*-7-hydroxy-10a-methyl-1-(3-methylbutyl)-1,2,3,4,4a,9, 10,10a-octahydrospiro[phenanthrene-3,2'-cyclobutane]-1'-one (mixture of diastereomers). *(rac)-(1S,3R,4aS,10aS)*-3,7-dihydroay-10a-methyl-1-(3-methylbutyl)-3-[1-(phenylthio)cyclopropyl]-1,2,3,4,4a,9,10,10a-octahydrophenanthrene was treated according to procedure, described above in **Example 45** to give a mixture of diastereomers of *(rac)-(1S,4aS,10aS)*-7-hydroxy-10a-methyl-1-(3-methylbutyl)-1,2,3,4,4a,9,10, 10a-octahydrospiro[phenanthrene-3,2'-cyclobutane]-1'-one, which was used in the next step without further purification or separation.

**Step 3.** *(rac)-(1S,4aS,10aS)*-1',1'-ethanediyldimercapto-7-hydroxy-10a-methyl-1-(3-methylbutyl)-1,2,3,4,4a,9,10,10a-octahydrospiro[phenanthrene-3,2'-cyclobutane](mixture of diastereomers). *(rac)-(1S,4aS,10aS)*-7-hydroxy-10a-methyl-1-(3-methylbutyl)-1,2,3,4,4a,9,10,10a-octahydrospiro[phenanthrene-3,2'-cyclobutane]-1'-one from the previous step (about 0.05 mmol) was treated according to **method G** ("J"= 10, "K"= 1,2-ethanedithiol, "L"= 2, "M"= 18), using 3 eq. of BF₃·OEt₂ complex. The crude product was purified by silica gel flash chromatography using Et₂O : *iso*-hexane (1:1) as eluent to give a mixture of diastereomers of *(rac)-(1S,4aS,10aS)*-1',1'-ethanediyldimercapto-7-hydroxy-10a-methyl-1-(3-methylbutyl)-1,2,3,4,4a,9,10,10a-octahydrospiro[phenanthrene-3,2'-cyclobutane] (17 mg) as a white oil.

**Step 4.** *(rac)-(1S,4aS,10aS)*-7-hydroxy-10a-methyl-1-(3-methylbutyl)-1,2,3,4,4a,9,10, 10a-octahydrospiro[phenanthrene-3,1'-cyclobutane]. *(rac)-(1S,4aS,10aS)*-1', 1'-ethanediyldimercapto-7-hydroxy-10a-methyl-1-(3-methylbutyl)-1,2,3,4,4a,9,10, 10a-octahydrospiro[phenanthrene-3,2'-cyclobutane] (17 mg, 0.041 mmol) was treated according to procedure, according to **method J** ("J" = 100, "K" = 15, "L" = 20, "M" = 0, "N" = 24) at 80°C to give the crude product, which was purified by PHPLC to yield *(rac)-(1S,4aS,10aS)-*7-hydroxy-10a-methyl-1-(3-methylbutyl)-1,2,3,4,4a,9, 10,10a-octahydrospiro[phenanthrene-3,1'-cyclobutane] (5.4 mg) as a white oil. ES-MS m/z: 327.4 (pos., M+H), 325.3 (neg., M-H); ¹H NMR (270 MHz, CDCl₃) δ 7.20 (d, 1H), 6.63 (dd, 1H), 6.55 (d, 1H), 4.48 (bs, 1H), 2.86 (ddd, 1H), 2.71 (ddd, 1H), 2.64 (dd, 1H), 2.43 (ddd, 1H), 2.10-1.16 (m, 17H), 0.89 (d, 3H), 0.87 (d, 3H), 0.80 (s, 3H).

### Example 54: Synthesis of (rac)-(1S,4aS,10aS)-7-hydroxy-3-(3-hydroxypropyl)-10a-methyl-1-(3-methylbutyl)-1,4,4a,9,10,10a-hexahydrophenanthrene (E54a) and (rac)-(1S,3S,4aS,10aS)-7-hydroxy-10a-methyl-1-(3-methylbutyl)-1,2,3,4,4a,9,10, 10a,2',3',4',5'-dodecahydrospiro[phenanthrene-3,2'-furan] (E54b).

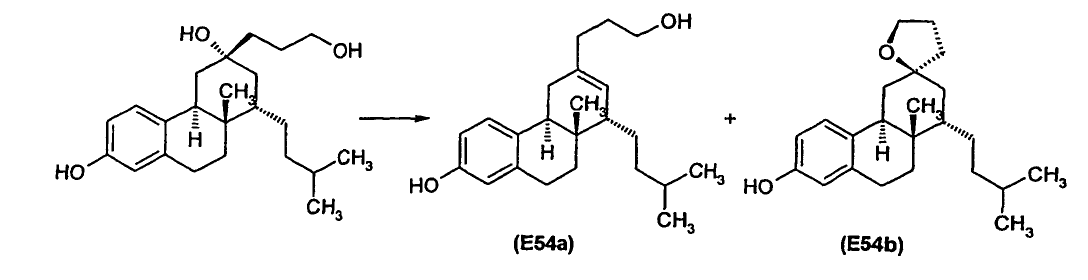

*(rac)-(1S,4aS, 10aS)*-7-hydroxy-3-(3-hydroxypropyl)-10a-methyl-1-(3-methylbutyl)-1,4,4a, 9,10, 10a-hexahydrophenanthrene and *(rac)-(1S,3S,4aS,10aS)*-7-hydroxy-10a-methyl-1-(3-methylbutyl)-1,2,3,4,4a,9,10,10a,2',3',4',5'-dodecahydrospiro[phenanthrene-3,2'-furan]. A mixture of *(rac)-(1S,3R,4aS,10aS)-*3,7-dihydroxy-3-(3-hydroxypropyl)-10a-methyl-1-(3-methylbutyl)-1,2,3,4,4a,9,10,10a-octahydrophenanthrene (47 mg, 0.13 mmol) and 4-toluenesulfonic acid monohydrate (2.5 mg, 0.013 mmol) in toluene (20 mL) was stirred at 100°C overnight. The resulting mixture was quenched with sat. aq. NaHCO₃ (10 mL), the organic layer was separated and the solvent was evaporated. The residue was separated by silica gel flash chromatography using Et₂O : *iso*-hexane (1:1) as eluent to yield pure *(rac)-(1S,4aS,10aS)*-7-hydroxy-3-(3-hydroxypropyl)-10a-methyl-1-(3-methylbutyl)-1, 4,4a,9,10,10a-hexahydrophenanthrene (18 mg) and *(rac)-(1S,3S,4aS,10aS)*-7-hydroxy-10a-methyl-1-(3-methylbutyl)-1,2,3,4,4a,9,10,10a,2',3',4',5'-dodecahydrospiro[phenanthrene-3,2'-furan] (5.3 mg) as white oils. *(rac)-(1S,4aS,10aS)*-7-hydroxy-3-(3-hydroxypropyl)-10a-methyl-1-(3-methylbutyl)-1,4,4a,9,10, 10a-hexahydrophenanthrene: ES-MS m/z: 343.4 (pos., M+H), 387.4 (neg., M+HCOOH-H); ¹H NMR (270 MHz, CDCl₃)δ 7.11 (d, 1H), 6.64 (dd, 1H), 6.55 (d, 1H), 5.56 (d, 1H), 5.10 (bs, 1H), 3.68 (t, 2H), 2.94-2.61 (m, 3H), 2.54 (dd, 1H), 2.13 (t, 2H), 1.88-1.04 (m, 11H), 0.88 (d, 3H), 0.85 (d, 3H), 0.77 (s, 3H). *(rac)-(1S,3S,4aS,10aS)*-7-hydroxy-10a-methyl-1-(3-methylbutyl)-1,2,3,4,4a,9, 10,10a,2',3',4', 5'-dodecahydrospiro[phenanthrene-3,2'-furan]: ES-MS m/z: 343.4 (pos., M+H), 387.4 (neg., M+HCOOH-H); ¹H NMR (270 MHz, CDCl₃) δ 7.09 (d, 1H), 6.60 (dd, 1H), 6.56 (d, 1H), 4.75 (s, 1H), 3.94-3.74 (m, 2H), 2.94-2.66 (m, 2H), 2.60 (dd, 1H), 2.19-1.60 (m, 8H), 1.60-1.06 (m, 8H), 0.89 (d, 3H), 0.87 (d, 3H), 0.85 (s, 3H); ¹³C NMR (CDCl₃) δ 153.27, 137.95, 131.78, 126.83, 115.23, 112.83, 82.62, 65.55, 44.87, 38.46, 37.70, 36.69, 36.49, 35.21, 34.53, 33.87, 28.48, 27.06, 26.63, 25.52, 23.24, 22.33, 18.49.

### Example 55: Synthesis of (rac)-(4aR,10aR)-10a-Butyl-7-hydroxy-4a-methyl-3, 4,4a,9,10,10a-hexahydro-1H-phenanthren-2-one (E55).

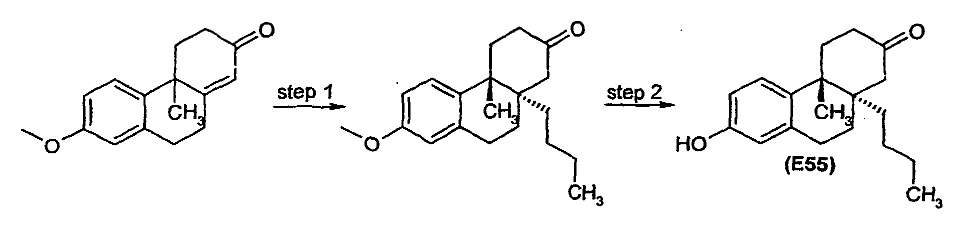

**Step 1:** *(rac)-(4aR,10aR)-*10a-Butyl-7-methoxy-4a-methyl-3,4,4a,9,10, 10a-hexahydro-*1H*-phenanthren-2-one: The reaction was carried out on 200 mg (0.83 mmol) of *(rac)*-7-methoxy-4a-methyl-4,4a,9,10-tetrahydro-*3H*-phenanthren-2-one dissolved in diethylether (3 mL), according to **method P** except that CuI was used instead of CuCN employing the following conditions; Cul (1.2 eq., suspension in 10 mL diethyl ether), alkyl lithium (*n*-BuLi [1.6M solution in hexanes], 2.2 eq.), reaction-time and temperature (30 min. at -78°C, quenching at -40 °C). The crude product was fractionated on a chromatotron using EtOAc:*n*-heptane (3:7, v:v) as eluent. The first collected fraction from the chromatotron was further purified using PHPLC to yield *(rac)-(4aR, 10aR)*-10a-Butyl-7-methoxy-4a-methyl-3,4,4a,9,10,10a-hexahydro-*1H*-phenanthren-2-one as a yellowish oil. ES-MS m/z: 301.3 (pos., M+H); ¹H NMR (CDCl₃) δ 7.25 (d, 1H), 6.75 (dd, 1H), 6.60 (d, 1H), 3.75 (s, 3H), 2.70-2.75 (m, 2H), 2.10-2.30(m, 5H), 1.90-2.05 (m, 1H), 1.65-1.70 (m, 2H), 1.40 (s, 3H), 1.20-1.40 (m, 6H), 0.90 (t, 3H).

**Step 2:** *(rac)-(4aR,10aR)*-10a-Butyl-7-hydroxy-4a-methyl-3,4,4a,9,10, 10a-hexahydro-*1H*-phenanthren-2-one (**E55**): The reaction was carried out on 11.9 mg of *(rac)-(4aR,10aR)*-10a-Butyl-7-methoxy-4a-methyl-3,4,4a,9,10,10a-hexahydro-*1H*-phenanthren-2-one dissolved in 1.0 mL DCM, using 3.5 eq. of BBr₃ for 16 h according to **method O.** The crude product was purified on a chromatotron using EtOAc:*n*-heptane (3:7,v:v) as eluent to yield **E55** as a yellowish oil. ES-MS m/z: 287.2 (pos., M+H), 285.1 (neg., M-H); ¹H NMR (DMSO) δ 7.20 (d, 1H), 6.60 (dd, 1H), 6.45 (s,1H), 3.60 (s, 3H), 2.60 (t, 2H), 1.50-2.20 (m, 10H), 1.20-1.40 (m, 4H), 0.95 (t, 3H).

### Example 56: Synthesis of (rac)-7-Hydroxy-4a-methyl-4,4a,9,10-tetrahydro-3H-phenanthren-2-one (E56).

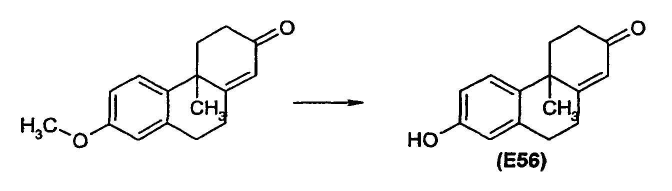

The reaction was carried out on 50 mg of *(rac)*-7-methoxy-4a-methyl-4,4a,9, 10-tetahydro-*3H*-phenanthren-2-one dissolved in 3.0 mL DCM, using 2.5 eq. of BBr₃ for 4 h according to **method O**. The crude product was purified on a chromatotron using EtOAc:*n*-heptane (4:6,v:v) as eluent to yield **E56** as a yellowish solid. GC-MS m/z: 228.5 (M), 213.4 (M-CH₃); ¹H NMR (CD₃OD) δ 7.15 (d, 1H), 6.65 (dd, 1H), 6.50 (d, 1H), 5.85 (s, 1H), 2.35-2.90 (m, 7H), 1.95-2.00 (m, 1H), 1.55 (s, 3H); ¹³C NMR (CD₃OD) δ 201.15, 173.43, 155.44, 135.99, 134.93, 127.17, 123.15, 114.23, 114.07, 38.41, 36.69, 33.99, 30.64, 30.53, 26.32.

### Example 57: Synthesis of (rac)-4b-methyl-4b,5,6,7,9,10-hexahydro-phenanthren-2-ol (E57).

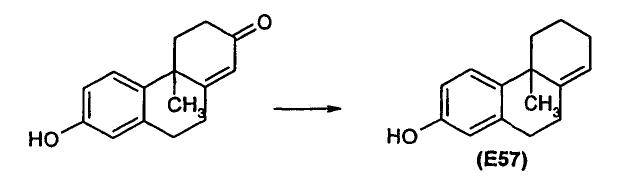

The reaction was carried out on 5.0 mg of *(rac)*-7-hydroxy-4a-methyl-4,4a,9, 10-tetrahydro-*3H*-phenanthren-2-one dissolved in acetonitrile (2.0 mL), according to **method R.** The crude product was purified on a chromatotron using EtOAc:*n*-heptane (3:7) as eluent to give **E57.** ES-MS m/z: 213.0 (neg., M-H); ¹H NMR (CD₃OD) δ 7.10 (d, 1H), 6.55 (dd, 1H), 6.40 (d, 1H), 5.40 (s, 1H), 1.45-2.80 (m, 10H), 1.40 (s, 3H).

### Example 58: (rac)-7-Hydroxy-1,4a-dimethyl-4,4a,9,10-tetrahydro-3H-phenanthren-2-one (E58).

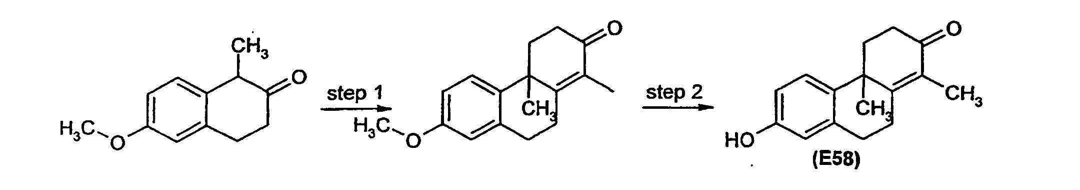

**Step1:** *(rac)*-7-Methoxy-1,4a-dimethyl-4,4a,9,10-tetrahydro-*3H*-phenanthren-2-one: Coupling of 200 mg (1.05 mmol) of 6-methoxy-1-methyl-3,4-dihydro-*1H*-naphthalen-2-one, dissolved in toluene (2.0 mL) with pent-1-en-3-one in accordance to **method M** gave *(rac)*-7-Methoxy-1,4a-dimethyl-4,4a,9,10-tetrahydro-*3H*-phenanthren-2-one which was used without further purification in the next step. ES-MS m/z: 257.2 (pos., M+H).

**Step 2:** *(rac)*-7-Hydroxy-1,4a-dimethyl-4,4a,9,10-teuahydro-*3H*-phenanthren-2-one **(E58):** The reaction was carried out on crude *(rac)*-7-Methoxy-1,4a-dimethyl-4,4a,9, 10-tetrahydro-*3H*-phenanthren-2-one dissolved in 2.0 mL DCM, using 3.5 eq. of BBr₃ for 16 h according to **method O.** A ¼ portion of the crude product was purified using PHPLC to yield **E58** as a yellow solid. ES-MS m/z: 243.4 (pos., M+H), 241.3 (M-H); ¹H NMR (CD₃OD) δ 7.20 (d, 1H), 6.65 (dd, 1H), 6.50 (d, 1H), 2.40-2.90 (m, 7H), 1.80-1.95(m, 1H), 1.89 (s, 3H), 1.45 (s, 3H); ¹³C NMR (CD₃OD) δ 200.05, 165.26, 155.30, 136.88, 135.90, 127.92, 126.64, 114.04, 113.91, 38.86, 35.84, 33.46, 29.30, 26.74, 25.64, 9.10.

### Example 59: Synthesis of (rac)-4a,8-dimethyl-4b,5,6,7,9,10-hexahydro-phenanthren-2-ol (E59).

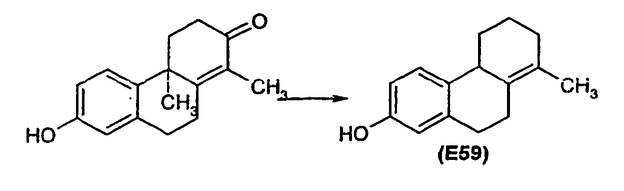

The reaction was carried out on a ¼ portion of the crude *(rac)*-7-hydroxy-1, 4a-dimethyl-4,4a,9,10-tetrahydro-*3H*-phenanthren-2-one (max. 0.262 mmol) dissolved in a mixture of acetonitrile and DCM (2.0+2.0 mL) according to **method R** (0.20 mL of trifluoro acetic acid was used). The crude product was purified on a chromatotron using EtOAc:*n*-heptane (15:85) as eluent followed by PHPLC, to yield **E59.** ES-MS m/z: 229.3 (pos., M+H); ¹H NMR (CDCl₃) δ 7.10 (d, 1H), 6.70 (bs, 1H), 6.65 (dd, 1H), 6.50 (d, 1H), 2.65-2.70 (m, 2H), 1.50-2.15 (m, 8H), 1.70 (s, 3H), 1.35 (s, 3H).

### Example 60: Synthesis of (rac)-4b,8-dimethyl-4b,5,6,7,9,10-hexahydro-phenanthren-2-ol (E60a, E60b, E60c, E60d).

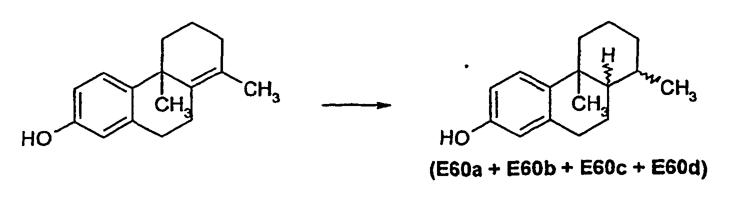

The reaction was carried out on 4.0mg of *(rac)*-4b,8-dimethyl-4b,5,6,7,9, 10-hexahydro-phenanthren-2-ol for 12 h according to **method S** with one exception - the reaction was run under pressure (5.0 bar) - using methanol (3.0 mL) as solvent and 5% Pd/C (5.0 mg) as catalyst. The crude product consisted of a mixture of the four possible diastereomers **E60a + E60b + E60c + E60d**. GC-MS m/z: 215.2 (M-CH₃) 230.2 (M).

### Example 61: Synthesis of (rac)-(3S,4aR)-7-hydroxy-3,4a-dimethyl-4,4a,9, 10-tetrahydro-3H-phenanthren-2-one (E61).

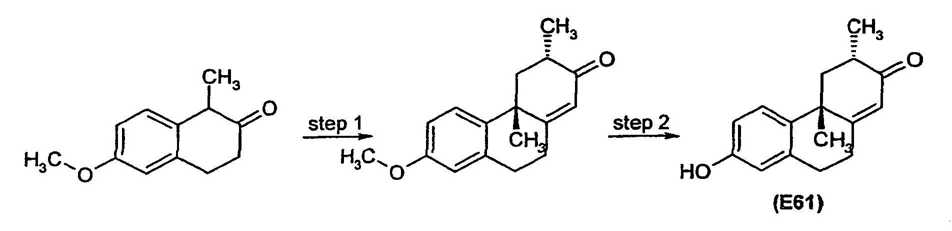

**Step 1:** *(rac)-(3S,4aR)*-7-Methoxy-3,4a-dimethyl-4,4a,9,10-tetrahydro-*3H*-phenanthren-2-one: Coupling of 200 mg (1.05 mmol) of *(rac)*-6-methoxy-1-methyl-3, 4-dihydro-*1H*-naphthalen-2-one dissolved in toluene (2.0 mL) with 3-methyl-but-3-en-2-one in accordance to **method M** gave crude *(rac)-(3S,4aR)*-7-Methoxy-3, 4a-dimethyl-4,4a,9,10-tetrahydro-*3H*-phenanthren-2-one which was used without further purification in the next step. GC-MS m/z: 256.1 (M), 241.1 (M-CH₃).

**Step2:** *(rac)-(3S,4aR)*-7-Hydroxy-3,4a-dimethyl-4,4a,9,10-tetrahydro-*3H*-phenanthren-2-one (**28**): The reaction was carried out on crude *(rac)-(3S, 4aR)*-7-Methoxy-3,4a-dimethyl-4,4a,9,10-tetrahydro-*3H*-phenanthren-2-onc using 3.3 eq. of BBr₃ for 16 h according to **method O**. The crude product was purified using PHPLC to yield **E61**. GC-MS m/z: 242.1(M), 227.1 (M-CH₃); 1H NMR (CDCl₃) δ 7.15 (d, 1H), 6.75 (dd, 1H), 6.55 (d, 1H), 5.86 (s, 1H), 5.15 (bs, 1H), 2.40-3.00 (m, 6H), 2.30 (dd, 1H), 1.55 (s, 3H), 1.15 (d, 3H).

### Example 62: Synthesis of (rac)-(4S, 4aR)-7-Hydroxy-4a-methyl-4-propyl-4,4a,9, 10-tetrahydro-3H-phenanthren-2-one (E62).

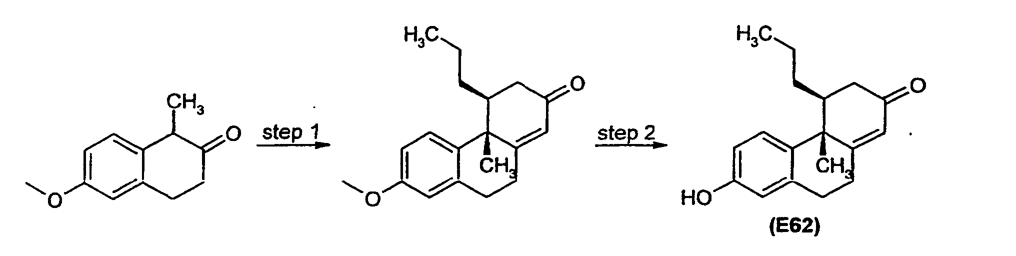

**Step 1:** *(rac)-(4S, 4aR)*-7-Methoxy-4a-methyl-4-propyl-4,4a,9,10-tetrahydro-*3H*-phenanthren-2-one: Coupling of 200 mg (1.05 mmol) of *(rac)*-6-Methoxy-1-methyl-3,4-dihydro-*1H*-naphthalen-2-one dissolved in toluene (2.0 mL) with hept-3-en-2-one in accordance to **method M** gave crude *(rac)-(4S, 4aR)*-7-Methoxy-4a-methyl-4-propyl-4,4a,9,10-tetrahydro-*3H*-phenanthren-2-one which was used without further purification in the next step. ES-MS m/z: 285.1 (pos., M+H).

**Step 2:** *(rac)-(4S, 4aR)*-7-Hydroxy-4a-methyl-4-propyl-4,4a,9, 10-tetrahydro-*3H*-phenanthren-2-one (**E62**): The reaction was carried out on crude *(rac)-(4S, 4aR)*-7-Methoxy-4a-methyl-4-propyl-4,4a, 9,10-tetrahydro-*3H*-phenanthren-2-one using 3.3 eq. of BBr₃ for 16 h according to **method O**. The crude product was purified using PHPLC to yield **E62.** GC-MS m/z: 270.0(M); ¹H NMR (CDCl₃) δ 7.15 (d, 1H), 6.75 (dd, 1H), 6.60 (d, 1H), 6.35 (bs, 1H), 5.90 (s, 1H), 2.80-2.85 (m, 1H), 2.75-2.80 (m, 1H), 2.60 (dd, 1H), 2.55 (m, 2H), 2.30 (dd, 1H), 2.00-2.10 (m, 1H), 1.60-1.70 (m, 1H), 1.45 (s, 3H), 1.30-1.45 (m, 2H), 0.95-1.1 (m, 1H), 0.85 (t, 3H); ¹³C NMR (CDCl₃) δ 139.55, 133.0, 130.72, 123.68, 115.26, 113.0, 43.96, 43.59, 39.73, 33.13, 32.11, 22.0, 19.89, 13.86.

### Example 63: Synthesis of (rac)-(4R,4aR)-7-Hydroxy-4,4a-dimethyl-4,4a,9, 10-tetrahydro-3H-phenanthren-2-one (E63a) and (rac)-(4S,4aR)-7-Hydroxy-4, 4a-dimethyl-4,4a,9,10-tetrahydro-3H-phenanthren-2-one (E63b).

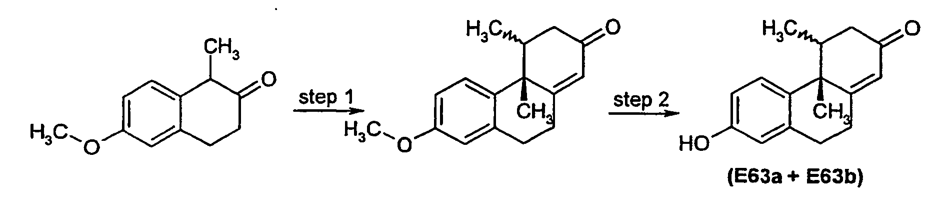

**Step 1:** (*rac*)-(4*R,*4a*R*)-7-Methoxy-4,4a-dimethyl-4,4a,9, 10-tetrahydro-*3H*-phenanthren-2-one and (*rac*)-(*4S,4aR*)-7-Methoxy-4,4a-dimethyl-4,4a,9, 10-tetrahydro-*3H*-phenanthren-2-one: Coupling of 200 mg (1.05 mmol) of *(rac)*-6-methoxy-1-methyl-3,4-dihydro-*1H*-naphthalen-2-one dissolved in toluene (2.0 mL) with pent-3-en-2-one in accordance to **method M** gave a crude mixture of (*rac*)-(4*R*,4a*R*)-7-Methoxy-4,4a-dimethyl-4,4a,9,10-tetrahydro-*3H*-phenanthren-2-one and (*rac*)-(*4S,4aR*)-7-Methoxy-4,4a-dimethyl-4,4a,9,10-tetrahydro-*3H*-phenanthren-2-one which was used without further purification in the next step. GC-MS m/z: 256.0 (M).

**Step 2:** *(rac)-(4R,4aR)*-7-Hydroxy-4,4a-dimethyl-4,4a,9, 10-tetrahydro-*3H*-phenanthren-2-one **(E63a)** and *(rac)-(4S,4aR)*-7-Hydroxy-4,4a-dimethyl-4,4a,9,10-tetrahydro-*3H*-phenanthren-2-one (**E63b**): The reaction was carried out on the crude mixture of (*rac*)-(4*R*,4a*R*)-7-Methoxy-4,4a-dimethyl-4,4a,9,10-tetrahydro-*3H*-phenanthren-2-one and (*rac*)-(*4S*,*4aR*)-7-Methoxy-4,4a-dimethyl-4,4a,9,10-tetrahydro-*3H*-phenanthren-2-one using 3.3 eq. of BBr₃ for 16 h according to **method O.** The crude product was purified using PHPLC to yield a mixture of **E63a** and **E63b.** ¹H NMR (CD₃OD) δ 7.15 (d, 1H), 6.65 (dd, 1H), 6.55 (d, 1H), 5.80 (s, 1H), 2.00-2.80 (m, 7H), 1.45 (s, 3H), 1.10 (d, 3H).

### Example 64: Synthesis of (rac)-1-Ethyl-7-hydroxy-4a-methyl-4,4a,9, 10-tetrahydro-3H-phenanthren-2-one (E64).

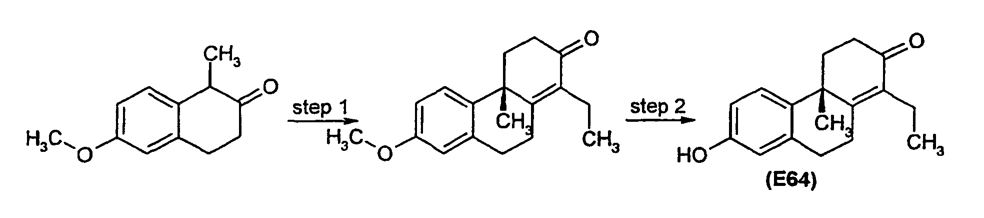

**Step 1:** (*rac)*-1-Ethyl-7-methoxy-4a-methyl-4,4a,9,10-tetrahydro-*3H*-phenanthren-2-one. Coupling of 569 mg (2.99 mmol) of (*rac*)-6-methoxy-1-methyl-3,4-dihydro-*1H*-naphthalen-2-one dissolved in toluene (5.0 mL) with hex-1-en-3-one in accordance to **method M** gave crude (*rac*)-1-Ethyl-7-methoxy-4a-methyl-4,4a,9,10-tetrahydro-*3H*-phenanthren-2-one, which was used without further purification in the next step. ES-MS m/z: 271.3 (pos., M+H).

**Step 2:** *(rac)*-1-Ethyl-7-hydroxy-4a-methyl-4,4a,9,10-tetrahydro-*3H*-phenanthren-2-one. The reaction was carried out on crude (*rac*)-1-Ethyl-7-methoxy-4a-methyl-4,4a, 9,10-tetahydro-*3H*-phenanthren-2-one (max. 2.99 mmol) dissolved in 6.0 mL DCM, using 3.0 eq. of BBr₃ for 5 h according to **method O.** The crude product was purified on a chromatotron using EtOAc:*n*-heptane (3:7, 6:5, 5:5, v:v, stepwise gradient) as eluent to yield **E64.** ES-MS m/z: 257.2 (pos., M+H), 255.4 (neg., M-H); ¹H NMR (CD₃OD) δ 7.1-7.2 (m, 1H), 6.10 (dd, 1H), 6.55 (d, 1H), 2.25-3.00 (m, 10H), 1.55 (s, 3H), 1.25 (t, 3H).

### Example 65: Synthesis of (rac)-8-Ethyl-4b-methyl-4b,5,6,7,9,10-hexahydro-phenanthren-2-ol (E65).

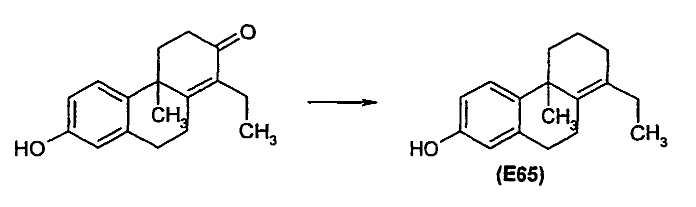

The reaction was carried out on 33 mg (0.13 mmol) of *(rac)*-1-ethyl-7-hydroxy-4a-methyl-4,4a,9,10-tetrahydro-*3H*-phenanthren-2-one dissolved in acetonitrile (5.0 mL) according to **method R.** The crude product was purified using PHPLC to yield **E65.** ES-MS m/z: 243.4 (pos., M+H), 241.3 (neg., M-H); ¹H NMR (CDCl₃, 500MHz) δ 7.15 (d, 1H), 6.65 (dd, 1H), 6.50 (d, 1H), 2.65-2.85 (m, 3H), 2.15-2.25 (m, 1H), 2.10-2.15 (m, 1H), 1.95-2.10 (m,4H), 1.70-1.80 (m,2H), 1.50-1.55 (m, 1H), 1.35 (s, 3H), 0.95 (t, 3H).

### Example 66: Synthesis of (rac)-(4aR,10aR,1S)-1-Ethyl-7-hydroxy-4a-methyl-1,4,4a,9, 10,10a-hexahydro-3H-phenanthren-2-one (E66).

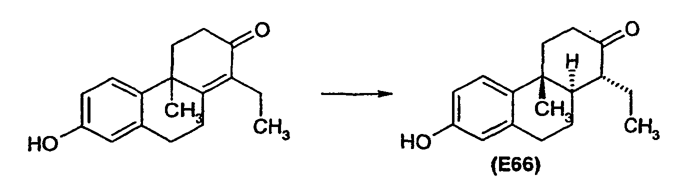

The reaction was carried out on 55 mg of *(rac)*-1-ethyl-7-hydroxy-4a-methyl-4,4a, 9,10-tetrahydro-*3H*-phenanthren-2-one for 16 h according to **method S** using methanol (3.0 mL) as solvent and 5% Pd/C (31 mg) as catalyst. The crude product was purified using PHPLC to yield **E66.** ES-MS m/z: 259.3 (pos., M+H), 257.2 (neg., M-H); ¹H NMR (CD₃OD, 500MHz) δ 7.15 (d, 1H), 6.70 (dd, 1H), 6.45 (d, 1H), 2.80-2.85 (m, 2H), 2.60-2.70 (m, 1H), 2.50-2.60 (ddd, 1H), 2.35-2.44 (m, 2H), 1.90-1.95 (m, 1H), 1.80-1.90 (m, 1H), 1.65-1.80 (m, 3H), 1.55-1.65 (m, 1H), 1.30 (s, 3H), 0.85 (t, 3H).

### Example 67: Synthesis of (rac)-1-Bulyl-7-hydroxy-4a-methyl-4,4a, 9,10-tetrahydro-3H-phenanthren-2-one (E67).

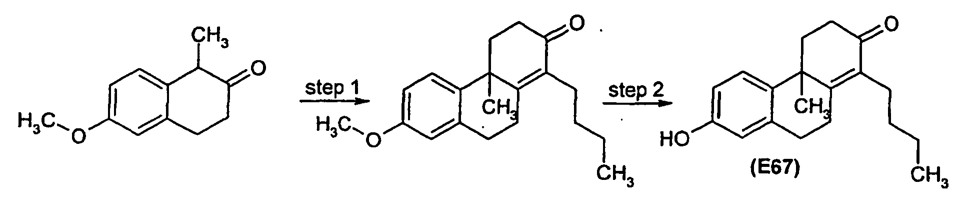

**Step 1:** *(rac)*-1-Butyl-7-methoxy-4a-methyl-4,4a,9,10-tetrahydro-*3H*-phenanthren-2-one: Coupling of 220 mg (1.16 mmol) of *(rac)*-6-methoxy-1-methyl-3, 4-dihydro-*1H*-naphthalen-2-one dissolved in toluene (2.0 mL) with oct-1-en-3-one in accordance to **method M** gave, after purification of the crude product by silica gel flash chromatography using EtOAc:*n*-heptane (3:7) as eluent, *(rac)*-1-Butyl-7-methoxy-4a-methyl-4,4a,9,10-tetrahydro-*3H*-phenanthren-2-one as a yellowish oil. ES-MS m/z: 299.2 (pos., M+H); ¹H NMR (CDCl₃) δ 7.15-7.20 (m, 1H), 6.70-6.85 (m, 1H), 6.55-6.65 (m, 1H), 3.75 (s, 3H), 2.00-3.00 (m, 10H), 1.45-1.50 (m, 1H), 1.25-1.30 (m, 6H), 0.90 (t, 3H).

**Step 2:** *(rac)*-1-Butyl-7-hydroxy-4a-methyl-4,4a,9,10-tetrahydro-*3H*-phenanthren-2-one (**40**). The reaction was carried out on 220 mg of *(rac)*-1-Butyl-7-methoxy-4a-methyl4,4a,9,10-tetrahydro-*3H*-phenanthren-2-one dissolved in 5.0 mL DCM using 3.5 eq. of BBr₃ for 4 h according to **method O**. The crude product was purified on a chromatotron, using EtOAc:*n*-heptane (3:7,v:v) as eluent to yield **E67** as a yellowish oil.
ES-MS m/z: 285.1 (pos., M+H), 283.0 (neg., M-H); ¹H NMR (CDCl₃) δ 7.15 (d, 1H), 6.75 (dd, 1H), 6.60 (d, 1H), 2.20-3.00 (m, 10H), 1.31 (s, 1H), 1.25-1.30 (m, 6H), 0.90 (t, 3H).
¹³C NMR (CDCl₃) δ 199.22, 164.04, 154.11, 136.79, 136.64, 133.43, 127.16, 114.60, 114.40, 39.35, 36.51, 34.51, 31.97, 30.86, 27.67, 26.84, 25.02, 22.96, 14.13.

### Example 68: Synthesis of (rac)-8-Butyl-4b-methy-4b, 5,6,7,9, 10-hexahydro-phenanthren-2-ol (E68).

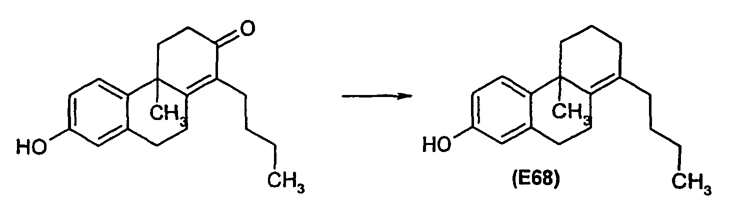

The reaction was carried out on 40 mg (6.14 mmol) of *(rac)*-1-Butyl-7-hydroxy-4a-methyl-4,4a,9,10-tetrahydro-*3H*-phenanthren-2-one dissolved in acetonitrile (10 mL) according to **method P.** The crude **E68** (yellowish oil) was used without further purification. ES-MS m/z: 271.0 (pos., M+H), 269.2 (neg., M-H); ¹H NMR (CDCl₃) δ 7.15 (d, 1H), 6.65 (dd, 1H), 6.50 (d, 1H), 4.50 (b s, 1H), 2.65-2.75 (m, 3H), 1.95-2.25 (m, 6H), 1.70-1.75 (m, 2H), 1.50-1.55 (m, 1H), 1.25-1.35 (m, 7H), 0.90 (t, 3H).

### Example 69: Synthesis of (rac)-(8R,4bR,8aR)-8-Butyl-4b-methyl-4b,5,6.7,8,8a,9, 10-octahydro-phenanthren-2-ol (E69a); and (rac)-(8S,4bR,8aR)-8-Butyl-4b-methyl-4b,5,6,7,8,8a,9,10-octahydro-phenanthren-2-ol (E69b).

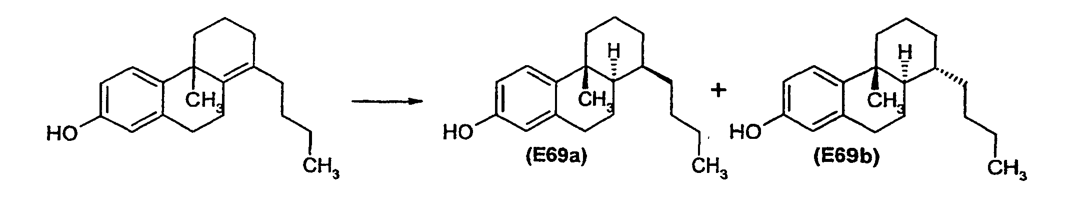

The reaction was carried out on 25 mg of *(rac)*-8-butyl-4b-methyl-4b,5,6,7, 9, 10-hexahydro-phenanthren-2-ol for 12 h according to **method S** except that the reaction was run under pressure (6.0 bar) using methanol (2.0 mL) as solvent, and 5% Pd/C (12 mg) as catalyst. The crude product was fractionated using PHPLC to **E69a** as the first eluted material and **E69b** as the second. **E69a:** ES-MS m/z: 273.1 (pos., M+H) 271.0 (neg., M-H); ¹H NMR (CDCl3) δ 7.10 (d, 1H), 6.60 (dd, 1H), 6.55 (d, 1H), 2.70-2.75 (m, 1H), 2.55-2.65 (m, 1H), 2.30 (d, 1H), 2.00-2.05 (m, 1H), 1.80-1.85 (m, 1H), 1.65-1.70 (m, 1H), 1.45-1.50 (m, 2H), 1.05-1.35 (m, 10H), 1.10 (s, 3H), 0.95-1.00 (m, 1H), 0.90 (t, 3H). **E69b:** ES-MS m/z: 273.1 (pos., M+H), 271.0 (neg., M-H); ¹H NMR (CDCl3) δ 7. 10 (d, 1H), 6.60 (dd, 1H), 6.50 (d, 1H), 2.80 (dd, 2H), 2.25 (d, 1H), 1.90-2.00 (m, 1H), 1.65-1.75 (m, 2H), 1.60-1.75 (m, 1H), 1.60-1.65 (m, 2H), 1.50-1.60 (m, 1H), 1.45-1.50 (m, 1H), 1.25-1.45 (m, 8H), 1.10 (s, 3H), 0.90 (t, 3H).

### Example 70: Synthesis of (rac)-4a-Butyl-7-hydroxy-1-methyl-4,4a,9, 10-tetrahydro-3H-phenanthren-2-one (E70).

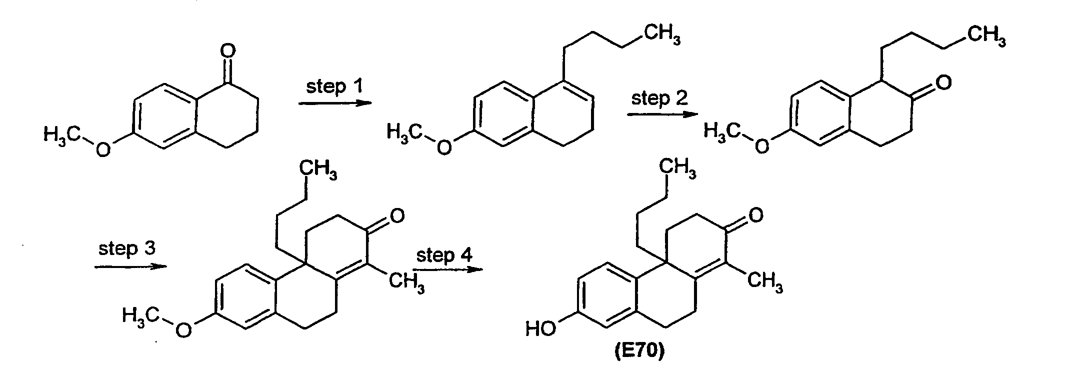

**Step 1:** 4-Butyl-7-methoxy-1,2-dihydro-naphthalene: A solution of 5.29 g (30 mmol, 1.0 eq.) of 6-methoxy-1-tetralone in dry THF (25 mL) was added dropwise while stirring, to a mixture of butyl lithium (1.6 M solution in hexanes, 2.1 eq.) and THF (40 mL) during approximately 30 min. Stirring was continued for another 30 min. before slow addition of 2.0 M aqueous HCl (150 mL). The phases were separated after vigorous stirring for 2 h. The aqueous phase was extracted with diethyl ether (3 x 50 mL) and the combined orgranic phases were dried over anhydrous sodium sulfate. The crude product, obtained upon concentration of the extract *in vacuo,* was purified by silica gel flash chromatography using EtOAc:*n*-heptane (0:10, 1:9, stepwise gradient) as eluent, to give 4-Butyl-7-methoxy-1, 2-dihydro-naphthalene as a yellowish oil. ¹H NMR (CDCl₃) δ 7.15 (d, 1H), 6.70-6.75 (m, 2H), 5.80 (t, 1H), 3.80 (s, 3H), 2.70 (t, 2H), 2.30-2.45 (m, 2H), 2.10-2.25 (m, 2H), 1.20-1.55 (m, 4H), 0.90 (t, 3H).

**Step 2:** *(rac)*-1-Butyl-6-methoxy-3,4-dihydro-*1H*-naphthalen-2-one: The reaction was carried out on 1.28 g (5.92 mmol) of 4-Butyl-7-methoxy-1,2-dihydro-naphthalene dissolved in 50 mL DCM according to **method L** to afford *(rac)*-1-Butyl-6-methoxy-3, 4-dihydro-*1H*-naphthalen-2-one as a yellowish oil after purification of the crude product by silica gel flash chromatography, using EtOAc:n-heptane (5:95, 2:8, stepwise gradient) as eluent. GC-MS m/z: 232.1 ¹H NMR (CDCl₃) δ 7.05 (d, 1H), 6.75-6.80 (m, 2H), 3.80 (s, 3H), 3.30-3.40 (m, 1H), 2.95-3.15 (m, 2H), 2.45-2.60 (m, 2H), 1.75.-1.85 (m, 2H), 1.20-1.30 (m, 4H), 0.85 (t, 3H).

**Step 3:** *(rac)*-4a-Butyl-7-methoy-1-methyl-4,4a,9,10-tetahydro-*3H*-phenanthren-2-one: Coupling of 100 mg (0.43 mmol) of *(rac)*-1-Butyl-6-methoxy-3,4-dihydro-*1H*-naphthalen-2-one dissolved in benzene (1.0 mL) with pent-1-en-3-one according to **method M,** gave after purification of the crude product by silica gel flash chromatography using EtOAc:*n*-heptane (1:99, 1:9, stepwise gradient) as eluent *(rac)*-4a-butyl-7-methoxy-1-methyl-4,4a,9,10-tetrahydro-*3H*-phenanthren-2-one. ES-MS m/z: 299.0 (pos., M+H); ¹H NMR (CDCl₃) δ 7.15 (d, 1H), 6.75 (dd, 1H), 6.65 (d, 1H), 3.80 (s, 3H), 1.70-3.00 (m, 10H), 1.80 (s, 3H), 1.00-1.30 (m, 4H), 0.80 (t, 3H).

**Step 4:** *(rac)*-4a-Butyl-7-hydroxy-1-methyl-4,4a,9,10-tetrahydro-*3H*-phenanthren-2-one (**E70**). The reaction was carried out on 13mg *(rac)*-4a-butyl-7-methoxy-1-methyl-4,4a, 9,10-tetrahydro-*3H*-phenanthren-2-one dissolved in 2.0 mL DCM, using 3.0 eq. of BBr₃ for 4 h according to **method O**. The crude product was purified on a chromatotron, using EtOAc:*n*-heptane (3:7,v:v) as eluent to afford **E70.** ¹H NMR (CD₃OD) δ 7.10 (d, 1H), 6.55-6.60 (m, 2H), 2.30-2.80 (m, 10H), 1.75 (s, 3H), 1.00-1.30 (m, 4H), 0.80 (t, 3H).

### Example 71: Synthesis of (rac)-4a-Butyl-7-hydroxy-4,4a,9,10-tetrahydro-3H-phenanthrea-2-one (E71).

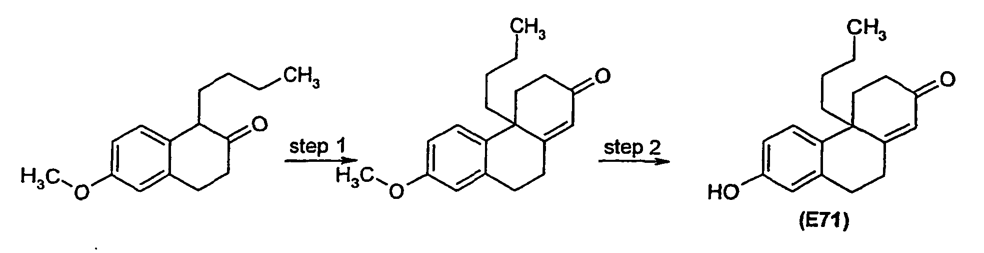

**Step 1:** *(rac)*-4a-Butyl-7-methoxy-4,4a,9,10-tetrahydro-*3H*-phenanthren-2-one: Coupling of 100 mg (0.43 mmol) of *(rac)*-1-butyl-6-methoxy-3,4-dihydro-*1H*-naphthalen-2-one dissolved in benzene (1.0 mL) with but-3-en-2-one in accordance to **method M** gave, after purification of the crude product by silica gel flash chromatography using EtOAc:*n*-heptane (1:99, 2:8, stepwise gradient) as eluent, *(rac)*-4a-Butyl-7-methoxy-4,4a,9,10-tetrahydro-*3H*-phenanthren-2-one. ¹H NMR (CDCl₃) δ 7.15 (d, 1H), 6.80 (dd, 1H), 6.60 (d, 1H), 5.90 (s, 1H), 3.80 (s, 3H), 1.80-3.00 (m, 10H), 1.10-1.30 (m, 4H), 0.80 (t, 3H); ¹³C NMR (CDCl₃) δ 199.72, 170.86, 158.00, 137.39, 134.16, 127.43, 124.93, 113.18, 113.02, 55.02, 41.84, 40.40, 36.06, 34.35, 32.46, 30.49, 27.72, 23.04, 13.53.

**Step2:** *(rac)*-4a-Butyl-7-hydroxy-4,4a,9,10-tetrahydro-*3H*-phenanthren-2-one (**E71**). The reaction was carried out on 33 mg of *(rac)*-4a-Butyl-7-methoxy-4,4a,9, 10-tetrahydro-*3H*-phenanthren-2-one dissolved in 2.0 mL DCM, using 3.0 eq. of BBr₃ for 4 h according to **method O.** The crude product was purified on a chromatotron, using EtOAc:*n*-heptane (3:7,v:v) as eluent to yield **E71** as a yellowish solid. ES-MS m/z: 271.0 (pos., M+H), 269.0 (neg., M-H); ¹H NMR (CD₃OD) δ 7.20 (d, 1H), 6.65 (dd, 1H), 6.55 (d, 1H), 5.90 (s, 1H), 1.80-3.00 (m, 10H), 1.10-1.30 (m, 4H), 0.80 (t, 3H).

### Example 72: Synthesis of (rac)-(4aR,10aR-)4a-Butyl-7-hydroxy-3,4,4a,9,10, 10a-hexahydro-1H-phenanthren-2-one (E72a); and (rac)-(4aR,10aS)-4a-Butyl-7-hydroxy-3,4,4a,9,10,10a-hexahydro-1H-phenanthren-2-one (E72b).

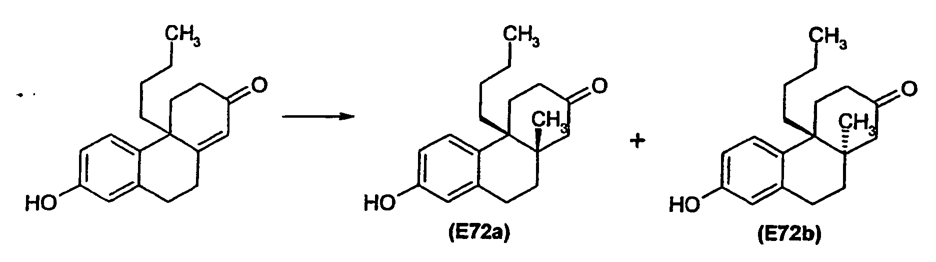

The reaction was carried out on 10 mg of *(rac)*-4a-Butyl-7-hydroxy-4,4a,9, 10-tetrahydro-*3H*-phenanthren-2-one for 2 h according to **method R** using methanol (5.0 mL) as solvent and 5% Pd/C (10 mg) as catalyst. The crude product was fractionated using PHPLC to give **E72a** as the first eluted material and **E72b** as the second. **E72a:** ES-MS m/z: 273.4 (pos., M+H), 271.3 (neg., M-H); ¹H NMR (CD₃OD) δ 7.15 (d, 1H), 6.60 (dd, 1H), 6.50 (d, 1H), 1.60-2.80 (m, 13H), 1.20-1.25 (m, 2H), 0.85 (t, 3H); **E72b:** ES-MS m/z: 273.4 (pos., M+H), 271.3 (neg., M-H); ¹H NMR (CD₃OD) δ 7.10 (d, 1H), 6.55 (dd, 1H), 6.50 (d, 1H), 1.50-2.80 (m, 13H), 1.20-1.25 (m, 2H), 0.85 (t, 3H).

### Example 73: Synthesis of (rac)-(4aR,10aS)-7-hydroxy-4a-methyl-3,4,4a,9-10,10a-hexahydro-1H-phenanthren-2-one (E73).

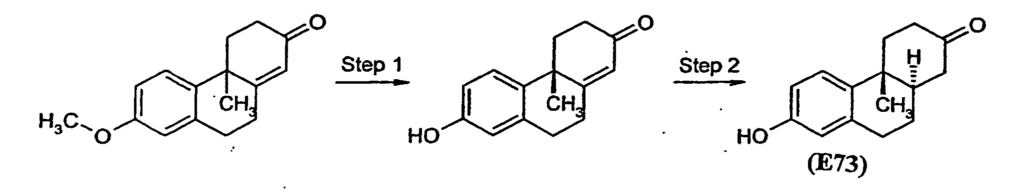

**Step 1.** *(rac)*-7-Hydroxy-4a-methyl-4,4a,9,10-tetrahydro-*3H*-phenanthren-2-one: The reaction was carried out on 570 mg (2.35 mmol) of *(rac)*-7-Methoxy-4a-methyl-4, 4a,9,10-tetrahydro-*3H*-phenanthren-2-one, dissolved in 10.0 mL DCM, using 3.5 eq. of BBr₃ for 20 h according to **method O**. The crude product was purified on a chromatotron using EtOAc:*n*-heptane (3:7) as eluent to yield *(rac)*-7-Hydroxy-4a-methyl-4,4a, 9,10-tetrahydro-*3H*-phenanthren-2-one (190 mg) as a yellow solid. ES-MS m/z: 229.3 (pos., M+H), 227.2 (neg., M-H); ¹H NMR (CDCl₃) δ 7.15 (d, 1H), 6.70 (dd, 1H), 6.55 (d, 1H), 5.85 (s, 1H), 4.80 (s, OH), 1.80-2.85 (m, 8H), 1.50 (s, 3H).

**Step 2:** *(rac)-(4aR,10aS)*-7-Hydroxy-4a-methyl-3,4,4a,9,10,10a-hexahydro-*1H*-phenanthren-2-one (**E73**): The reaction was carried out on 133.0mg (0.58 mmol) *(rac)*-7-hydroxy-4a-methyl-4,4a,9,10-tetrahydro-*3H*-phenanthren-2-one for 2 h according to **method S** using THF (6.0 mL) as solvent, and 10% Pd/C (133.0 mg) as catalyst. The crude product was purified by PHPLC to yield **E73** (81 mg) as a white solid. ES-MS m/z: 231.1(pos., M+H), 229.3(neg., M-H); ¹H NMR (CDCl₃) δ 7.20 (d, 1H), 6.65 (dd, 1H), 6.55 (d, 1H), 5.00 (s, OH), 2.75-2.85 (m, 2H), 2.35-2.50 (m, 2H), 2.20-2.30 (m, 3H), 2:00-2.20 (m, 2H), 1.75-1.90 (m, 1H), 1.55-1.60 (m, 1H), 1.30 (s, 3H).

### Example 74: Synthesis of (rac)-(4aR,10aS)-2,2-ethanediyldimercapto-7-hydroxy-4a-methyl-1.2,3,4,4a,9,10,10a-octahydrophenanthrene E74).

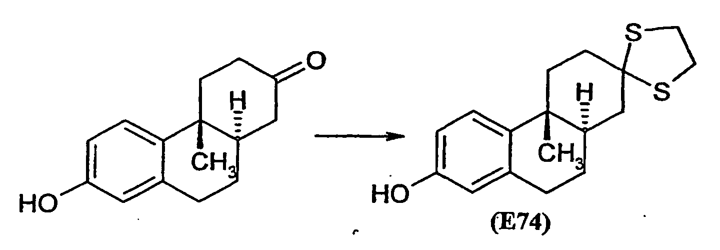

The reaction was carried out on 15 mg (0.065 mmol) of *(rac)-(4aR,10aS)*-7-hydroxy-4a-methyl-3,4,4a,9,10,10a-hexahydro-*1H*-phenanthren-2-one, dissolved in 1.0 mL dichloromethane according to **method T**, using 2eq. of 1,2-ethanedithiol, 1 eq of BF₃·OEt₂, and a reaction time of 5 hours. The crude product was purified by chromatotron using EtOAc:*n*-heptane (3:7) as eluent to yield **E74** (15 mg) as a white solid. ES-MS m/z: 307.3(pos., M+H), 305.2(neg., M-H); ¹H NMR (CDCl₃) δ 7.15 (d, 1H), 6.60 (dd, 1H), 6.50 (d, 1H), 4.45 (s, OH), 3.25 (s, 4H), 2.60-2.80 (m, 2H), 2.10-2.35 (m, 3H), 1.90 (m, 4H), 1.70-1.80 (m, 2H), 1.50 (s, 3H).

### Example 75: Synthesis of (rac)-(4aR,10aS)-7-hydroxy-4a-methyl-3,4,4a,9,10, 10a-hexahydro-1H-phenanthren-2-one oxime (E75).

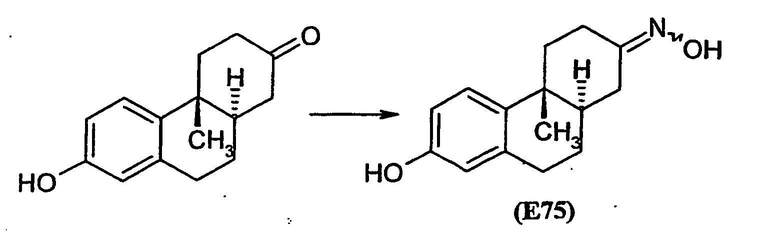

The reaction was carried out on 16 mg (0.065 mmol) of *(rac)-(4aR,10aS)*-7-hydroxy-4a-methyl-3,4,4a,9,10,10a-hexahydro-*1H*-phenanthren-2-one dissolved in 1.5 mL methanol according to **method U,** using 10eq. of NH₂OH·HCl and 10eq. of sodium acetate. The crude product was purified by chromatotron using EtOAc:*n*-heptane (2:8) as eluent to yield two isomers of **E75** in the ratio 2:1 (11 mg) as a white solid. ES-MS m/z: 246.1(pos., M+H); ¹H NMR (CDCl₃) δ 7.15 (d, 1H), 6.65 (dd, 1H), 6.55 (d, 1H), 2.60-2.80 (m, 2H), 1.80-2.40 (m, 7H), 1.55-1.75 (m, 2H), 1.30 (s, 3H).

### Example 76: A pharmaceutical formulation comprising (rac)-4b,8-dimethyl-4b, 5,6,7,9,10-hexahydro-phenanthren-2-ol.

200 mg of *(rac)*-4a,8-dimethyl-4b,5,6,7,9,10-hexahydro-phenanthren-2-ol, from **Example 59,** is formulated with sufficient finely divided lactose to provide a total amount of 580 to 590 mg to fill a size 0, hard-gelatine capsule.

### Description of the Scintistrip ER Binding Assay

### Introduction

The scintistrip assay differs from a traditional hormone binding assay by not requiring the removal of free tracer prior to the measurement of receptor bound tracer. The scintillating agent is in the polystyrene forming the incubation vial and thus a radioactive molecule in the close proximity to the surface will induce scintillation of the plastic. For ³[H]-labeled ligands, the distance between the free tracer and the scintillating polystyrene surface is too far to induce scintillation of the plastic while ³[H]-labeled ligands bound to receptors immobilized on the surface are close enough to induce scintillation' thus enabling a convenient way to measure the competition between a non-radioactive estrogen receptor interacting agent (the compound to be tested) and a fixed concentration of tracer (³[H]-Estradiol).

### Materials and Methods

³[H]-β-Estradiol (NET 317) hereafter referred to as ³[H]-E2 was purchased from New England Nuclear, Boston, MA. The scintistrip wells (1450-419) and the scintillation counters (Microbeta™ 1450-Plus and 1450-Trilux) were all from Wallac, Turku, Finland. Human estrogen receptors (hER) alpha and beta were extracted from the nuclei from SF9-cells infected with a recombinant baculovirus transfer vector containing the cloned hER genes. Recombinant baculovirus was generated utilizing the BAC-TO-BAC expression system (Life Technonlogies) in accordance to instruction from the supplier. The hER coding sequences were cloned into a baculovirus transfer vector by standard techniques. The recombinant baculoviruses expressing hER were amplified and used to infect SF9 cells. Infected cells were harvested 48 hr post infection. A nuclear fraction was obtained as described in² and the nuclei were extracted with a high-salt buffer (17 mM K₂HPO₄, 3 mM KH₂PO₄, 1 mM MgCl₂, 0.5 mM EDTA, 6 mM MTG, 400 mM KCl, 8.7% Glycerol). The concentration of hER's in the extract was measured as specific [³H]-E2 binding with the G25-assay³ and was determined to contain 400 pmols specific bound [3H]-E2/mL nuclear extract in the case of hER-alpha and 1000 pmols/mL nuclear for hER-beta. The total concentration of proteins (as determined with Bradford Reagent, Bio-Rad according to instructions from manufacturer) in the nuclear extracts were ^{~}2 mg/mL. The equilibrium binding constant (K_{d}) for [³H]-E2 to hER in solution was determined to 0.05 nM for hER-alpha and to 0.07 nM for hER-beta with the G25-assay for highly diluted extracts (hER ^{~} 0.1 nM). The extracts were aliquoted and stored at -80°C.

*The scintistrip assay*¹ In brief; the nuclear extracts were diluted (50 fold for hER-alpha and 110 fold for hER-beta) in coating buffer (17 mM K₂HPO₄, 3 mM KH₂PO₄, 40 mM KCl, 6 mM MTG). The diluted extracts were added to Scintistrip wells (200 µL/well) and incubated 18-20 hr. at ambient room temperature (22-25 °C). The estimated final concentration of immobilized hER in all experiments was ^{~} nM. All incubations were performed in 17 mM K₂HPO₄, 3 mM KH₂PO₄, 140 mM KCl, 6 mM MTG (buffer A). The wells were washed twice after hER coating with 250 µL buffer prior to addition of the incubation solution. All steps were carried out at ambient room temperature (22-25 °C.).

*Determination of Equilibrium binding constants to immobilized hER:s:* Dilutions of ³[H]-E2 in buffer ± Triton X100 were added to the wells (200 µL/well), the wells were incubated for 3 hr and then measured in the Microbeta. After the measurement an aliquot of the buffer was taken out and counted by regular liquid scintillation counting for determination of the "free" fraction of ³[H]-E2. In order to correct for non-specific binding parallel incubations were done in presence of a 200-fold excess of unlabeled 17-β-E2. The equilibrium dissociation constants (K_{d}) were calculated as free concentration of ³[H]-E2 at half maximum binding by fitting data to the Hill equation; b = (bₘₐₓ x Lⁿ)/(Lⁿ+K_{d} ⁿ) where b is specific bound ³[H]-E2, bₘₐₓ is the maximum binding level, L is the free concentration of [³H]E2, n is the Hill coefficient (the Hill equation equals the Michaelis-Menten equation when n = 1). The equilibrium binding constants were determined to 0.15 - 0.2 nM for both hER subtypes.

*Regular competition binding:* Samples containing 3 nM [3H]-E2 plus a range of dilutions of the compounds to be tested were added to wells with immobilized hER and incubated for 18-20 hr at ambient room temperature. The compounds to be tested were diluted in 100% DMSO to a concentration 50 fold higher than the desired final concentration, the final concentration of DMSO was thus 2% in all samples. For compounds able to displace ³[H]-E2 from the receptor an IC₅₀-value (the concentration required to inhibit 50% of the binding of ³[H]-E2) was determined by a non-linear four parameter logistic model; b = ((bₘₐₓ-_{bmin})/(1+(I/IC₅₀)^{s}))+bₘᵢₙ I is added concentration of binding inhibitor, IC₅₀ is the concentration of inhibitor at half maximal binding and S is a slope factor.¹ For determinations of the concentration of ³[H]-E2 in the solutions regular scintillation counting in a Wallac Rackbeta 1214 was performed using the scintillation cocktail Supermix™ (Wallac).

The Microbeta-instrument generates the mean cpm (counts per minute) value / minute and corrects for individual variations between the detectors thus generating corrected cpm values. It was found that the counting efficiency between detectors differed with less than five percent.
1) Haggblad, J., Carlsson, B., Kivelä, P., Siitari, H., (1995) *Biotechniques* **18,** 146-151
2) Barkhem, T., Carlsson, B., Simons, J., Moller, B., Berkenstam, A., Gustafsson J.A.G., Nilsson, S. (1991) *J. Steroid Biochem. Molec. Biol.* **38**, 667-75
3) Salomonsson, M., Carlsson, B., Haggblad, J., (1994) *J. Steroid Biochem. Molec. Biol.* **50**,313-318
4) Schultz, J.R., Ruppel, P.I., Johnson, M.A., (1988) in Biopharmaceutical Statistics for Drug Development (Peace, K.E., Ed.) pp. 21-82, Dekker, New York

The compounds of Examples 1-48 exhibit binding affinities to the estrogen receptor α-subtype in the range of IC₅₀ 3 to 10,000 nM and to the estrogen receptor β-subtype in the range of IC₅₀ 3 to 10,000 nM.

## Claims

1. A compound having the general formula **I, II** or **III**: 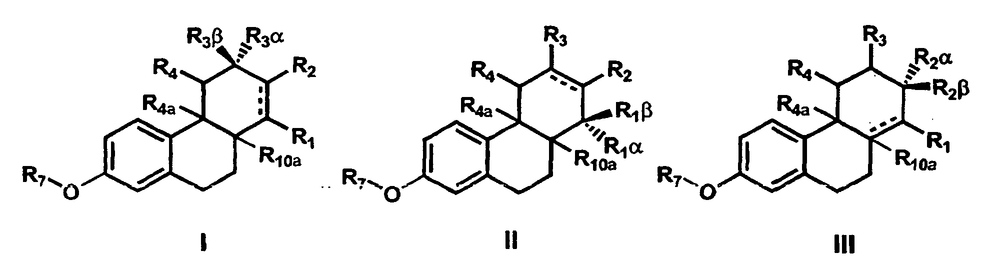 wherein the bond between the C1 and C2 carbon atoms (of compounds of general formula **I**) or the bond between C2 and C3 (of compounds of general formula **II**) or the bond between C1 and C10 (of compounds of general formula **III**) is either a single or double bond;
R₁ (of compounds of general formula **I** or **III**) is an R^{A} group other than a phenyl group;
R^{A} is selected from the group hydrogen, alkyl, alkenyl, alkynyl, cycloalkyl, cycloalkenyl, cycloalkylalkyl, aryl or arylalkyl;
R₁α and R₁β (of compounds of general formula **II**) are the same or are different and each is an R^{A} group;
R₂ (of compounds of general formula **I** or **II**) is a hydroxyl or R^{A} group or is a hydroxyalkyl or aminoalkyl group of 1 to 2 carbon atoms;
R₃α and R₃β (of compounds of general formula **I**) may together be a single oxygen or sulfur atom or R₃α and R₃β may together be a single nitrogen atom which in turn is bonded to a group selected from R^{A} or OR^{A}; or R₃α and R₃β may together be a single carbon atom which in turn is bonded to two R^{A} groups which may be the same or are different; or R₃α and R₃β may be the same or are different and each may be selected from R^{A}, OR^{A}, SR^{A}, or N(R^{A})₂ wherein the individual R^{A} groups may be the same or are different and may optionally be taken together with any attached and intervening atoms to form a 3-8 membered ring;
R₂α and R₂β (of compounds of general formula **III**) may together be a single oxygen or sulfur atom or R₂α and R₂β may together be a single nitrogen atom (*i.e*., an imine or oxime nitrogen atom) which in turn is bonded to a group selected from R^{A} or OR^{A}; or R₂α and R₂β may together be a single carbon atom which in turn is bonded to two R^{A} groups which may be the same or are different; or R₂α and R₂β may be the same or are different and each may be selected from hydroxyalkyl, aminoalkyl, R^{A}, OR^{A}, SR^{A}, or N(R^{A})₂ wherein the individual R^{A} groups may be the same or are different and may optionally be taken together with any attached and intervening atoms to form a 3-8 membered ring;
R₃ (of compounds of general formula **II** or **III**) is an R^{A} group;
R₄ is an R^{A} group;
R₄ₐ is a hydrogen atom or a methyl or ethyl group in compounds of formula **I** or **II**, or a methyl or ethyl group in compounds of formula **III**.;
R₇ is a hydrogen atom or a linear or branched alkyl or cycloalkyl group or acyl group of 1 to 4 carbon atoms;
R₁₀ₐ, which is absent when the bond between C₁ and C₁₀ of formula **III** is a double bond is an R^{A} group;
with the proviso that not all of R₁, R₂, R₃ and R₄ are hydrogen in compounds of formula **I** or **II**, and that R₄ is not hydrogen in compounds of formula **III**.
and pharmaceutically acceptable salts and stereoisomers thereof.

2. A compound according to claim 1 wherein the R₄ₐ and R₁₀ₐ substituents have a trans relative stereochemistry.

3. A compound with the general formula **I** or **III** according to claim 2 wherein R₁ is R^{B} and R^{B} is selected from hydrogen, *n*-propyl, 2-propenyl, 2-propynyl, *n*-butyl, 2-butenyl, 3-butenyl, 2-butynyl, 3-butynyl, *n*-pentyl, 3-methylbutyl, 3-methyl-1-butenyl, 3-methyl-2-butenyl, 3-methylpentyl, 3-ethylpentyl, cyclopropylethyl, cyclopentylethyl, cyclohexylethyl, cycloheptylethyl, cyclopropylpropyl, cyclopentylpropyl, benzyl, or phenethyl.

4. A compound with the general formula **II** according to claim 2 wherein R₁α is R^{B} (where R^{B} is defined in claim 3) and R₁β is a hydrogen atom or methyl group.

5. A compound according to any one of claims 2 to 4 wherein R₁₀ₐ is R^{B}.

6. A compound with the general formula **I** or **II** according to any one of claims 2 to 5 wherein R₂ is a hydrogen atom or a methyl, ethyl, or hydroxymethyl group.

7. A compound with the general formula **III**, according to any one of claims 2 to 5 wherein R₂α and R₂β may be the same or are different and each may be selected from hydroxyalkyl, R^{A}, OR^{A}, or SR^{A} where the individual R^{A} groups (where R^{A} is defined as above) may be the same or are different and may optionally be taken together with any attached and intervening atoms to form a 3-8 membered ring.

8. A compound with the general formula I according to any one of claims 2 to 6 wherein R₃α and R₃β may be the same or are different and each may be selected from R^{A}, OR^{A}, or SR^{A} where the individual R^{A} groups (where R^{A} is defined in claim 1) may be the same or are different and may optionally be taken together with any attached and intervening atoms to form a 3-8 membered ring.

9. A compound according to any one of claims 2 to 8 wherein R₄ is a hydrogen atom or methyl or ethyl group.

10. A compound according to any one of claims 2 to 9 wherein R₇ is hydrogen atom or an acyl group of 1 to 4 carbon atoms.

11. A compound with the general formula **I** or **III** according to any one of claims 2 to 10 wherein R₁ is selected from hydrogen, methyl, or ethyl and R₁₀ₐ is R^{B}.

12. A compound with the general formula **I** or **III** according to any one of claims 2 to 10 wherein R₁ is R^{B} and R₁₀ₐ is selected from hydrogen, methyl, or ethyl.

13. A compound of formula **III** according to any one of claims 2, 9, 10, 11 or 12 wherein R₂α and R₂β may be the same or are different and each may be selected from hydroxyalkyl, R^{A}, OR^{A}, or SR^{A} where the individual R^{A} groups (where R^{A} is defined in claim 1) may be the same or are different and may optionally be taken together with any attached and intervening atoms to form a 3-8 membered ring.

14. A compound with the general formula **II** according to any one of claims 2 to 10 wherein R₁α is R^{B} and R₁₀ₐ is selected from hydrogen, methyl, or ethyl.

15. A compound with the general formula **II** according to any one of claims 2 to 10 wherein R₁α is selected from hydrogen, methyl, or ethyl and R₁₀ₐ is R^{B}.

16. A compound according to claim 1 which is selected from:
*(rac)-(4aS,10aS)*-7-hydroxy-1,4,4a,9,10,10a-hexahydro-*2H*-phenanthren-3-one (**E1**);
*(rac)-(4aS,10aS)*-3,3-ethanediyldimercapto-7-hydroxy-1,2,3,4,4a,9,10,10a-octahydro-phenanthrene (**E2**);
*(rac)-(4aR,10aS)*-7-hydroxy-10a-methyl-4a,9,10,10a-tetrahydro-*4H*-phenanthren-3-one (**E3a**);
*(rac)-(4aS,10aS)*-7-hydroxy-10a-methyl-4a,9,10,10a-tetrahydro-*4H*-phenanthren-3-one (**E3b**);
*(rac)-(4aS,10aS)*-10a-butyl-7-hydroxy-4a,9,10,10a-tetrahydro-*4H*-phenanthren-3-one (**E4**);
*(rac)-(1S,4aS,10aS)*-1-butyl-7-hydroxy-1,4,4a,9,10,10a-hexahydro-*2H*-phenanthren -3-one (**E5a**);
*(rac)-(1R,4aS,10aS)*-1-butyl-7-hydroxy-1,4,4a,9,10,10a-hexahydro-*2H*-phenanthren -3-one (**E5b**);
*(rac)-(1S,4aS,10aS)*-1-butyl-3,3-ethanediyldimercapto-7-hydroxy-1,2,3,4,4a,9,10,10a-octahydro-phenanthrene (**E6a**);
*(rac)-(1R,4aS,10aS)*-1-butyl-3,3-ethanediyldimercapto-7-hydroxy-1,2,3,4,4a,9,10,10a-octahydro-phenanthrene (**E6b**);
*(rac)-(1S,4aS,10aS)*-1-butyl-7-hydroxy-10a-methyl-1,4,4a,9,10,10a-hexahydro-*2H*-phenanthren-3-one (**E7**);
*(rac)-(1S,4aS,10aS)*-1-butyl-3,3-ethanediyldimercapto-7-hydroxy-10a-methyl-1,2,3, 4,4a,9,10,10a-octahydro-phenanthrene (**E8**);
*(rac)-(1S,4aS,10aS)*-1-butyl-7-hydroxy-10a-methyl-1,2,3,4,4a,9,10,10a-octahydro-phenanthrene (**E9**);
*(rac)-(4aS,10aR)*-10a-ethyl-7-hydroxy-3-methyl-4a,9,10,10a-tetrahydro-*4H*-phenanthren-1-one (**E10a**);
*(rac)-(4aR,10aR)*-10a-ethyl-7-hydroxy-3-methyl-4a,9,10,10a-tetrahydro-*4H*-phenanthren-1-one (**E10b**);
*(rac)-(1R,2S,4aS,10aS)*-1-butyl-7-hydroxy-2-methyl-1,4,4a,9,10,10a-hexahydro-*2H*-phenanthren-3-one (**E11a**);
*(rac)-(1R,2R,4aS,10aS)*-1-butyl-7-hydroxy-2-methyl-1,4,4a,9,10,10a-hexahydro-*2H*-phenanthren-3-one (**E11b**);
*(rac)-(1s,4R,4aR,10aS)*-1-butyl-7-hydroxy-4-methyl-1,4,4a,9,10,10a-hexahydro-2*H*-phenanthren-3-one (**E11c**);
*(rac)-(1R,2R,4aS,10aS)*-1-butyl-3,3-ethanediyldimercapto-7-hydroxy-2-methyl-1,2, 3,4,4a,9,10,10a-octahydro-phenanthrene (**E12a**);
*(rac)-(1R,2S,4aS,10aS)*-1-butyl-3,3-ethanediyldimercapto-7-hydroxy-2-methyl-1,2, 3,4,4a,9,10,10a-octahydro-phenanthrene (**E12b**);
*(rac)-(1S,4aS,10aS)*-7-hydroxy-10a-methyl-1-(3-methyl-butyl)-1,4,4a,9,10,10a-hexahydro-*2H*-phenanthren-3-one (**E13**);
*(rac)-(1S,4aS,10aS)*-3,3-ethanediyldimercapto-7-hydroxy-1-(3-methyl-butyl)-10a-m
*(rac)-(1S,4aS,10aS)*-7-hydroxy-10a-methyl-1-phenethyl-1,4,4a,9,10,10a-hexahydro-*2H*-phenanthren-3-one (**E15**);
*(rac)-(1S,2S,4aS,10aS)*-7-hydroxy-2,10a-dimethyl-1-(3-methyl-butyl)-1,4,4a,9,10, 10a-hexahydro-*2H*-phenanthren-3-one (**E16a**);
*(rac)-(1S,2R,4aS,10aS)*-7-hydroxy-2,10a-dimethyl-1-(3-methyl-butyl)-1,4,4a,9,10, 10a-hexahydro-*2H*-phenanthren-3-one (**E16b**);
*(rac)-(4bS,8S,8aS)*-6,6-dimethoxy-8a-methyl-8-(3-methyl-butyl)-4b,5,6,7,8,8a,9,10-octahydro-phenanthren-2-ol (**E17a**);
*(rac)-(1S,4aS,10aS)*-7-hydroxy-10a-methyl-1-(3-methyl-butyl)-1,4,4a,9,10,10a-hexahydro-*2H*-phenanthren-3-one (**E17b**);
*(rac)-(1S,4aS,10aS)*-3,3-ethanediyldioxy-7-hydroxy-1-(3-methyl-butyl)-10a-methyl-1,2,3,4,4a,9,10,10a-octahydro-phenanthrene (**E18**);
*(rac)-(4bS,8R,8aS)*-8a-methyl-8-(3-methyl-butyl)-4b,5,6,7,8,8a,9,10-octahydro-phenanthren-2-ol (**E19a**);
*(rac)-(4bS,6R,8S,8aS)*-6-ethylsulfanyl-8a-methyl-8-(3-methyl-butyl)-4b,5,6,7,8,8a,9, 10-octahydro-phenanthren-2-ol (**E19b**);
*(rac)-(1S,4aS,10aS)*-3,3-(propane-1,3-diyldimercapto)-7-hydroxy-1-(3-methyl-butyl)-10a-methyl-1,2,3,4,4a,9,10,10a-octahydro-phenanthrene (**E20**);
*(rac)-(4aS,10aS)*-7-hydroxy-10a-methyl-1,4,4a,9,10,10a-hexahydro-2H-phenanthren-3-one (**E21**);
*(rac)-(4aS,10aS)*-3,3-ethanediyldimercapto-7-hydroxy-10a-methyl-1,2,3,4,4a,9,10, 10a-octahydro-phenanthrene (**E22**);
*(rac)-(4aS,10aS)*-10a-ethyl-7-hydroxy-1,4,4a,9,10,10a-hexahydro-*2H*-phenanthren-3-one (**E23**);
*(rac)-(4aS,10aS)*-3,3-ethanediyldimercapto-10a-ethyl-7-hydroxy-1,2,3,4,4a,9,10,10a-octahydro-phenanthrene (**E24**);
*(rac)-(1S,4aS,10aS)*-1-(3-methyl-butyl)-10a-ethyl-7-hydroxy-1,4,4a,9,10,10a-hexahydro-*2H*-phenanthren-3-one (**E26**);
*(1S,4aS,10aS)*-7-hydroxy-10a-methyl-1-(3-methyl-butyl)-1,4,4a,9,10, 10a-hexahydro-*2H*-phenanthren-3-one (**E27**);
*(1R,4aRS,10aR)*-7-benzyloxy-10a-methyl-1-(3-methyl-butyl)-1,4,4a,9,10, 10a-hexahydro-*2H*-phenanthren-3-one (**E28**);
*(rac)-(1S,4aS,10aS)*-1-(3-methyl-butyl)-3,3-ethanediyldimercapto-7-hydroxy-1,4,4a, 9,10, 10a-octahydrophenanthrene (**E29**);
*(rac)-(4aR,10aR)*-7-hydroxy-4a,10a-dimethyl-3,4,4a,9,10,10a-hexahydro-*1H*-phenanthren-2-one (**E30**);
*(rac)-(1S,4aS,10aS)*-7-hydroxy-10a-methyl-4-phenyl-1,4,4a,9,10, 10a-hexahydro-*2H*-phenanthren-3-one (**E31**);
*(rac)-(4R,4aS,10aS)*-3,3-ethanediyldimercapto-7-hydroxy-10a-methyl-4-phenyl-1,4, 4a,9,10,10a-octahydro-phenanthrene (**E32**);
*(rac)-(1S,4aS,10aS)*-3,3-(ethane-1,2-diyldimercapto)-7-hydroxy-1-(2-phenylethyl)-10a-methyl-1,2,3,4,4a,9,10,10a-octahydro-phenanthrene (**E33**);
*(rac)-(1S,4aS,10aS)*-3,3-ethanediyldioxy-7-hydroxy-10a-methyl-1, 2,3,4,4a,9,10,10a-octahydro-phenanthrene (**E34a**);
*(rac)-(1S,4aS,10aR)*-3,3-ethanediyldioxy-7-hydroxy-10a-methyl-1, 2,3,4,4a,9,10,10a-octahydro-phenanthrene (**E34b**);
*(rac)-(3S,4aS,10aS)*-7-hydroxy-3-pentyl-10a-methyl-1, 2,3,4,4a,9,10,10a-octahydro-phenanthrene (**E35a**);
*(rac)-(3R,4aS,10aS)*-7-hydroxy-3-pentyl-10a-methyl-1, 2,3,4,4a,9,10,10a-octahydro-phenanthrene (**E35b**);
*(rac)-(1S,2R,4aS,10aS)*-2,10a-dimethyl-3,3-(ethane-1,2-diyldimercapto)-7-hydroxy-1-(3-methyl-butyl-1,2,3,4,4a,9,10,10a-octahydro-phenanthrene (**E36**);
*(rac)-(1S,4aS,10aS)*-7-Hydroxy-1-(3'-methyl-butyl)-1,4,4a,9,10,10a-hexahydro-*2H*-phenanthren-3-one (**E37**);
*(rac)-(4aS,10aS)*-3,3-(propane-1,3-diyldimercapto)-7-hydroxy-1,2,3,4,4a,9,10,10a-octahydro-phenanthrene (**E38**);
*(rac)-(1S,4S,4aS,10aS)*-7-Hydroxy-1-butyl-4,10a-dimethyl-1,4,4a,9,10,10a-hexahydro-*2H*-phenanthren-3-one (**E39**);
*(rac)-(4S,4aS,10aS)*-3,3-ethanediyldimercapto-7-hydroxy-4, 10a-dimethyl-1,2,3,4,4a,9,10,10a-octahydro-phenanthrene (**E40**);
*(rac)-(4S,4aS,10aS)*-7-Hydroxy-4-benzyl-10a-methyl-1,4,4a,9,10,10a-hexahydro-*2H*-phenanthren-3-one (**E41a**);
*(rac)-(4aS,10aS)*-7-hydroxy-4,4-dibenzyl-10a-methyl-1,4,4a,9,10,10a-hexahydro-*2H*-phenanthren-3-one (**E41b**);
*(rac)-(4aS,10aS)*-7-hydroxy-10a-methyl-3,3-methylene-1,2,3,4,4a,9,10,10a-octahydrophenanthrene (**E42**);
*(rac)-(4aS,10aS)*-3,3-ethanediyl-7-hydroxy-10a-methyl-1,2,3,4,4a,9,10,10a-octahydrophenanthrene (**E43**);
*(rac)-(3R,4aS,10aS)*-1',2',3',4'-tetrachloro-7-hydroxy-10a-methyl-1,2,3,4,4a,9,10, 10a-octahydrospiro[phenanthrene-3,6'-cyclohexane]-1',3'-diene (**E44**);
*(rac)-(3S,4aS,10aS)*-3,7-dihydroxy-10a-methyl-3-[1-(phenylthio)cyclopropyl]-1,2,3, 4,4a,9,10,10a-octahydrophenanthrene (**E45**);
*(rac)-(3S,4aS,10aS)*-7-hydroxy-10a-methyl-1,2,3,4,4a,9,10,10a-octahydrospiro[phenanthrene-3,2'-cyclobutane]-1'-one (**E46a**);
*(rac)-(3R,4aS,10aS)*-7-hydroxy-10a-methyl-1,2,3,4,4a,9,10,10a-octahydrospiro[phenanthrene-3,2'-cyclobutane]-1'-one (**E46b**);
*(rac)-(3S,4aS,10aS)*-1',1'-ethanediyldimercapto-7-hydroxy-10a-methyl-1,2,3,4,4a,9,10, 10a-octahydrospiro[phenanthrene-3,2'-cyclobutane] (**E47**);
*(rac)-(4aS,10aS)*-7-hydroxy-10a-methyl-1,2,3,4,4a,9,10,10a-octahydrospiro[phenanthrene-3,1'-cyclobutane] (**E48**);
*(rac)-(4aS,10aS)*-3-(1-cyclopenten-1-yl)-7-hydroxy-10a-methyl-1,4,4a,9,10,10a-hexahydrophenanthrene (**E49**);
*(rac)-(3S,4aS,10aS)*-7-hydroxy-10a-methyl-1,2,3,4,4a,9,10, 10a,2',3',4',5'-dodecahydrospiro[phenanthrene-3,2'-furan] (**E50a**);
*(rac)-(3R,4aS,10aS)*-7-hydroxy-10a-methyl-1,2,3,4,4a,9,10, 10a,2',3',4',5'-dodecahydrospiro[phenanthrene-3,2'-furan] (**E50b**);
*(rac)-(3S,4aS,10aS)*-7-hydroxy-10a-methyl-1,2,3,4,4a,9,10, 10a,3',4',5',6'-dodecahydrospiro[phenanthrene-3,2'-2*H*-pyran] (**E51a**);
*(rac)-(3R,4aS,10aS)*-7-hydroxy-10a-methyl-1,2,3,4,4a,9,10, 10a,3',4',5',6'-dodecahydrospiro[phenanthrene-3,2'-2*H*-pyran] (**E51b**);
*(rac)-(1S,3R,4aS,10aS)*-7-hydroxy-10a-methyl-1-(3-methylbutyl)-1,2,3,4,4a,9,10,10a, 2',3',4',5'-dodecahydrospiro[phenanthrene-3,2'-furan] (**E52**);
*(rac)-(1S,4aS,10aS)*-7-hydroxy-10a-methyl-1-(3-methylbutyl)-1,2,3,4,4a,9,10,10a-octahydrospiro[phenanthrene-3,1'-cyclobutane] (**E53**);
*(rac)-(1S,4aS,10aS)*-7-hydroxy-3-(3-hydroxypropyl)-10a-methyl-1-(3-methylbutyl)-1,4,4a,9,10,10a-hexahydrophenanthrene (**E54a**);
*(rac)-(1S,3S,4aS,10aS)*-7-hydroxy-10a-methyl-1-(3-methylbutyl)-1,2,3,4,4a,9,10,10a, 2',3',4',5'-dodecahydrospiro[phenanthrene-3,2'-furan] (**E54b**);
*(rac)-(4aR,10aR)*-10a-Butyl-7-hydroxy-4a-methyl-3,4,4a,9,10,10a-hexahydro-*1H*-phenanthren-2-one (**E55**);
*(rac)*-7-Hydroxy-4a-methyl-4,4a,9,10-tetrahydro-*3H*-phenanthren-2-one (**E56**);
*(rac)*-4b-methyl-4b,5,6,7,9,10-hexahydro-phenanthren-2-ol (**E57**);
*(rac)*-7-Hydroxy-1,4a-dimethyl-4,4a,9,10-tetrahydro-*3H*-phenanthren-2-one (**E58**);
*(rac)*-4a,8-dimethyl-4b,5,6,7,9,10-hexahydro-phenanthren-2-ol (**E59**);
(*rac*)-4b,8-dimethyl-4b,5,6,7,9,10-hexahydro-phenanthren-2-ol (**E60a, E60b, E60c, E60d);**
*(rac)-(3S,4aR)*-7-hydroxy-3,4a-dimethyl-4,4a,9,10-tetrahydro-*3H-*phenanthren-2-one (**E61**)**;**
*(rac)-(4S, 4aR)*-7-Hydroxy-4a-methyl-4-propyl-4,4a,9, 10-tetrahydro-*3H*-phenanthren-2-one **(E62);**
*(rac)-(4R,4aR)*-7-Hydroxy-4,4a-dimethyl-4,4a,9,10-tetrahydro-*3H*-phenanthren-2-one (**E63a**);
*(rac)-(4S,4aR)*-7-Hydroxy-4,4a-dimethyl-4,4a,9,10-tetrahydro-*3H*-phenanthren-2-one (**E63b**);
*(rac)*-1-Ethyl-7-hydroxy-4a-methyl-4,4a,9,10-tetrahydro-*3H*-phenanthren-2-one (**E64**);
*(rac)*-8-Ethyl-4b-methyl-4b,5,6,7,9,10-hexahydro-phenanthren-2-ol (**E65**);
*(rac)-(4aR,10aR,1S)*-1-Ethyl-7-hydroxy-4a-methyl-1,4,4a,9,10,10a-hexahydro-*3H*-phenanthren-2-one (**E66**);
*(rac)*-1-Butyl-7-hydroxy-4a-methyl-4,4a,9,10-tetrahydro-*3H*-phenanthren-2-one (**E67**);
*(rac)*-8-Butyl-4b-methyl-4b,5,6,7,9,10-hexahydro-phenanthren-2-ol (**E68**);
*(rac)-(8R,4bR,8aR)*-8-Butyl-4b-methyl-4b,5,6,7,8,8a,9,10-octahydro-phenanthren-2-ol (**E69a**);
*(rac)-(8S,4bR,8aR)*-8-Butyl-4b-methyl-4b,5,6,7,8,8a,9,10-octahydro-phenanthren-2-ol (**E69b**);
*(rac)*-4a-Butyl-7-hydroxy-1-methyl-4,4a,9,10-tetrahydro-*3H*-phenanthren-2-one (**E70**);
*(rac)*-4a-Butyl-7-hydroxy-4,4a,9,10-tetrahydro-*3H*-phenanthren-2-one (**E71**);
*(rac)-(4aR,10aR-)*4a-Butyl-7-hydroxy-3,4,4a,9,10,10a-hexahydro-*1H*-phenanthren-2-one (**E72a**);
*(rac)-(4aR,10aS)*-4a-Butyl-7-hydroxy-3,4,4a,9,10,10a-hexahydro-*1H*-phenanthren-2-one (**E72b**);
*(rac)-(4aR,10aS)*-7-hydroxy-4a-methyl-3,4,4a,9,10,10a-hexahydro-*1H*-phenanthren-2-one (**E73**);
*(rac)-(4aR,10aS)*-2,2-ethanediyldimercapto-7-hydroxy-4a-methyl-1,2,3,4,4a,9,10, 10a-octahydrophenanthrene (**E74**);
*(rac)-(4aR,10aS)*-7-hydroxy-4a-methyl-3,4,4a,9,10,10a-hexahydro-*1H*-phenanthren-2-one oxime (**E75**);
and pharmaceutically acceptable salts and stereoisomers thereof.

17. A compound according to any one of claims 1 to 16 for use in medical therapy.

18. A pharmaceutical composition comprising a compound according to any one of claims 1 to 16 and a pharmaceutically acceptable carrier.

19. A process for making a pharmaceutical composition comprising combining a compound according to any one of claims 1 to 16 and a pharmaceutically acceptable carrier.

20. The use of a compound according to any one of claims 1 to 16 in the manufacture of a medicament for the therapeutic treatment or prevention of bone loss, bone fractures, osteoporosis, cartilage degeneration, endometriosis, uterine fibroid disease, hot flashes, increased levels in LDL cholesterol, cardiovascular disease, impairment of cognitive functioning, cerebral degenerative disorders, restinosis, gynecomastia, vascular smooth muscle cell proliferation, obesity, incontinence, autoimmune disease, and lung, colon, breast, uterus and prostate cancer.

## Patentansprüche

1. Verbindung der allgemeinen Formel I, II oder III: 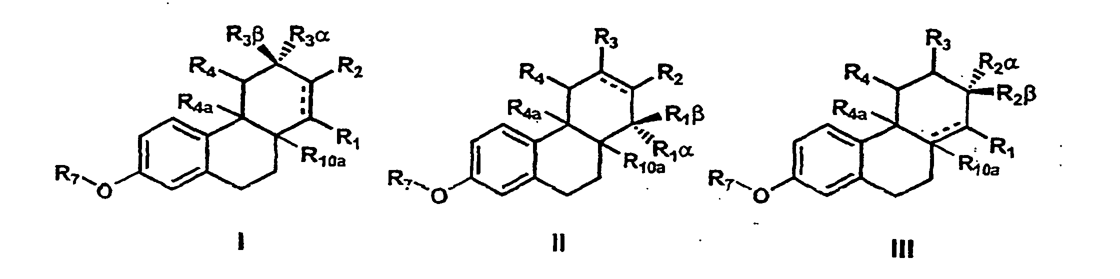 wobei die Bindung zwischen den Kohlenstoffatomen C1 und C2 (der Verbindungen der allgemeinen Formel **I**) oder die Bindung zwischen C2 und C3 (der Verbindungen der allgemeinen Formel **II**) oder die Bindung zwischen C1 und C10 (der Verbindungen der allgemeinen Formel **III**) entweder eine Einfach- oder eine Doppelbindung ist;
R₁ (der Verbindungen der allgemeinen Formel **I** oder **III**) eine R^{A}-Gruppe mit Ausnahme einer Phenylgruppe ist;
R^{A} ausgewählt ist aus der Gruppe Wasserstoff, Alkyl, Alkenyl, Alkinyl, Cycloalkyl, Cycloalkenyl, Cycloalkylalkyl, Aryl oder Arylalkyl;
R₁α und R₁β (der Verbindungen der allgemeinen Formel **II**) gleich oder verschieden sind und jeweils eine R^{A}-Gruppe darstellen;
R₂ (der Verbindungen der allgemeinen Formel **I** oder **II**) eine Hydroxy- oder R^{A}-Gruppe ist oder eine Hydroxyalkyl- oder Aminoalkylgruppe mit 1 bis 2 Kohlenstoffatomen ist;
R₃α und R₃β (der Verbindungen der allgemeinen Formel **I**) gemeinsam ein einzelnes Sauerstoff- oder Schwefelatom darstellen können oder R₃α und R₃β gemeinsam ein einzelnes Stickstoffatom darstellen können, das wiederum an eine aus R^{A} oder OR^{A} ausgewählte Gruppe gebunden ist; oder R₃α und R₃β gemeinsam ein einzelnes Kohlenstoffatom darstellen können, das wiederum an zwei R^{A}-Gruppen gebunden ist, die gleich oder verschieden sein können; oder R₃α und R₃β gleich oder verschieden sein können und jeweils aus R^{A}, OR^{A}, SR^{A} oder N(R^{A})₂ ausgewählt sein können, wobei die einzelnen R^{A}-Gruppen gleich oder verschieden sein können und gegebenenfalls zusammen mit den daran gebundenen oder dazwischenliegenden Atomen einen 3-8-gliedrigen Ring bilden können;
R₂α und R₂β (der Verbindungen der allgemeinen Formel **III**) gemeinsam ein einzelnes Sauerstoff- oder Schwefelatom darstellen können oder R₂α und R₂β gemeinsam ein einzelnes Stickstoffatom (d.h. ein Imin- oder Oximstickstoffatom) darstellen können, das wiederum an eine aus R^{A} oder OR^{A} ausgewählte Gruppe gebunden ist; oder R₂α und R₂β gemeinsam ein einzelnes Kohlenstoffatom darstellen können, das wiederum an zwei R^{A}-Gruppen gebunden ist, die gleich oder verschieden sein können; oder R₂α und R₂β gleich oder verschieden sein können und jeweils aus Hydroxyalkyl, Aminoalkyl, R^{A}, OR^{A}, SR^{A} oder N(R^{A})₂ ausgewählt sein können, wobei die einzelnen R^{A}-Gruppen gleich oder verschieden sein können und gegebenenfalls zusammen mit den daran gebundenen und dazwischenliegenden Atomen einen 3-8-gliedrigen Ring bilden können;
R₃ (der Verbindungen der allgemeinen Formel **II** oder **III**) eine R^{A}-Gruppe ist;
R₄ eine R^{A}-Gruppe ist;
R₄ₐ in Verbindungen der Formel **I** oder **II** ein Wasserstoffatom oder eine Methyl- oder Ethylgruppe oder in Verbindungen der Formel **III** eine Methyl- oder Ethylgruppe ist;
R₇ ein Wasserstoffatom oder eine geradkettige oder verzweigte Alkyl- oder Cycloalkylgruppe oder eine Acylgruppe mit 1 bis 4 Kohlenstoffatomen ist;
R₁₀ₐ, welches fehlt, wenn die Bindung zwischen C₁ und C₁₀ der Formel **III** eine Doppelbindung ist, eine R^{A}-Gruppe ist;
mit der Einschränkung, daß R₁, R₂, R₃ und R₄ in Verbindungen der Formel **I** oder **II** nicht alle Wasserstoff sind und daß R₄ in Verbindungen der Formel **III** nicht Wasserstoff ist;
und pharmazeutisch verträgliche Salze und Stereoisomere davon.

2. Verbindung nach Anspruch 1, wobei die R₄ₐ- und R₁₀ₐ-Substituenten eine zueinander trans-ständige Stereochemie haben.

3. Verbindung der allgemeinen Formel **I** oder **III** nach Anspruch 2, wobei R₁ R^{B} ist und R^{B} ausgewählt ist aus Wasserstoff, *n*-Propyl, 2-Propenyl, 2-Propinyl, *n*-Butyl, 2-Butenyl, 3-Butenyl, 2-Butinyl, 3-Butinyl, *n*-Pentyl, 3-Methylbutyl, 3-Methyl-1-butenyl, 3-Methyl-2-butenyl, 3-Methylpentyl, 3-Ethylpentyl, Cyclopropylethyl, Cyclopentylethyl, Cyclohexylethyl, Cycloheptylethyl, Cyclopropylpropyl, Cyclopentylpropyl, Benzyl oder Phenethyl.

4. Verbindung der allgemeinen Formel **II** nach Anspruch 2, wobei R₁α R^{B} ist (wobei R^{B} die in Anspruch 3 angegebene Bedeutung hat) und R₁β ein Wasserstoffatom oder eine Methylgruppe ist.

5. Verbindung nach irgendeinem der Ansprüche 2 bis 4, wobei R₁₀ₐ R^{B} ist.

6. Verbindung der allgemeinen Formel **I** oder **II** nach irgendeinem der Ansprüche 2 bis 5, wobei R₂ ein Wasserstoffatom oder eine Methyl-, Ethyl- oder Hydroxymethylgruppe ist.

7. Verbindung der allgemeinen Formel **III** nach irgendeinem der Ansprüche 2 bis 5, wobei R₂α und R₂β gleich oder verschieden sein können und jeweils ausgewählt sein können aus Hydroxyalkyl, R^{A}, OR^{A} oder SR^{A}, wobei die einzelnen R^{A}-Gruppen (wobei R^{A} die oben angegebene Bedeutung hat) gleich oder verschieden sein können und gegebenenfalls zusammen mit den daran gebundenen und dazwischenliegenden Atomen einen 3-8-gliedrigen Ring bilden können.

8. Verbindung der allgemeinen Formel **I** nach irgendeinem der Ansprüche 2 bis 6, wobei R₃α und R₃β gleich oder verschieden sein können und jeweils ausgewählt sein können aus R^{A}, OR^{A} oder SR^{A}, wobei die einzelnen R^{A}-Gruppen (wobei R^{A} die in Anspruch 1 angegebene Bedeutung hat) gleich oder verschieden sein können und gegebenenfalls zusammen mit den daran gebundenen und dazwischenliegenden Atomen einen 3-8-gliedrigen Ring bilden können.

9. Verbindung nach irgendeinem der Ansprüche 2 bis 8, wobei R₄ ein Wasserstoffatom oder eine Methyl- oder Ethylgruppe ist.

10. Verbindung nach irgendeinem der Ansprüche 2 bis 9, wobei R₇ ein Wasserstoffatom oder eine Acylgruppe mit 1 bis 4 Kohlenstoffatomen ist.

11. Verbindung der allgemeinen Formel **I** oder **III** nach irgendeinem der Ansprüche 2 bis 10, wobei R₁ ausgewählt ist aus Wasserstoff, Methyl oder Ethyl und R₁₀ₐ R^{B} ist.

12. Verbindung der allgemeinen Formel **I** oder **III** nach irgendeinem der Ansprüche 2 bis 10, wobei R₁ R^{B} ist und R₁₀ₐ ausgewählt ist aus Wasserstoff, Methyl oder Ethyl.

13. Verbindung der Formel **III** nach irgendeinem der Ansprüche 2, 9, 10, 11 oder 12, wobei R₂α und R₂β gleich oder verschieden sein können und jeweils ausgewählt sein können aus Hydroxyalkyl, R^{A}, OR^{A} oder SR^{A}, wobei die einzelnen R^{A}-Gruppen (wobei R^{A} die in Anspruch 1 angegebene Bedeutung hat) gleich oder verschieden sein können und gegebenenfalls zusammen mit den daran gebundenen und dazwischenliegenden Atomen einen 3-8-gliedrigen Ring bilden können.

14. Verbindung der allgemeinen Formel **II** nach irgendeinem der Ansprüche 2 bis 10, wobei R₁α R^{B} ist und R₁₀ₐ ausgewählt ist aus Wasserstoff, Methyl oder Ethyl.

15. Verbindung der allgemeinen Formel **II** nach irgendeinem der Ansprüche 2 bis 10, wobei R₃α ausgewählt ist aus Wasserstoff, Methyl oder Ethyl und R₁₀ₐ R^{B} ist.

16. Verbindung nach Anspruch 1, welche ausgewählt ist aus:
*(rac)-(4aS,10aS)*-7-Hydroxy-1,4,4a,9,10,10a-hexahydro-*2H*-phenanthren-3-on (**E1**);
*(rac)-(4aS,10aS)*-3,3-Ethandiyldimercapto-7-hydroxy-1,2,3,4,4a,9,10,10a-octahydrophenanthren (**E2**);
*(rac)-(4aR,10aS)*-7-Hydroxy-10a-methyl-4a,9,10,10a-tetrahydro-*4H*-phenanthren-3-on (**E3a**);
*(rac)-(4aS,10aS)*-7-Hydroxy-10a-methyl-4a,9,10,10a-tetrahydro-*4H*-phenanthren-3-on (**E3b**);
*(rac)-(4as,10aS)*-10a-Butyl-7-hydroxy-4a,9,10,10a-tetrahydro-*4H*-phenanthren-3-on (**E4**);
*(rac)*-*(1S,4aS,10aS)*-1-Butyl-7-hydroxy-1,4,4a,9,10,10a-hexahydro-*2H*-phenanthren-3-on **(E5a);**
*(rac)*-*(1R,4aS,10aS)*-1-Butyl-7-hydroxy-1,4,4a,9,10,10a-hexahydro-2H-phenanthren-3-on **(E5b);**
*(rac)-(1S,4aS,10aS)*-1-Butyl-3,3-ethandiyldimercapto-7-hydroxy-1,2,3,4,4a,9,10,10a-octahydrophenanthren (**E6a**);
*(rac)-(1R,4aS,10aS)*-1-Butyl-3,3-ethandiyldimercapto-7-hydroxy-1,2,3,4,4a,9,10,10a-octahydrophenanthren (**E6b**);
*(rac)*-*(1S,4aS,10aS)*-1-Butyl-7-hydroxy-10a-methyl-1,4,4a,9,10,10a-hexahydro-*2H*-phenanthren-3-on (**E7**);
*(rac)*-*(1S,4aS,10aS)*-1-Butyl-3,3-ethandiyldimercapto-7-hydroxy-10a-methyl-1,2,3,4,4a,9,10,10a-octahydrophenanthren (**E8**);
*(rac)-(1S,4aS,10aS)*-1-Butyl-7-hydroxy-10a-methyl-1,2,3,4,4a,9,10,10a-octahydrophenanthren (**E9**);
*(rac)-(4aS,10aR)*-10a-Ethyl-7-hydroxy-3-methyl-4a,9,10,10a-tetrahydro-*4H*-phenanthren-1-on (**E10a**);
*(rac)*-*(4aR,10aR)*-10a-Ethyl-7-hydroxy-3-methyl-4a,9,10,10a-tetrahydro-4H-phenanthren-1-on (**E10b**);
*(rac)-(1R,2S,4aS,10aS)*-1-Butyl-7-hydroxy-2-methyl-1,4,4a,9,10,10a-hexahydro-*2H*-phenanthren-3-on (**E11a**);
*(rac)-(1R,2R,4aS,10aS)*-1-Butyl-7-hydroxy-2-methyl-1,4,4a,9,10,10a-hexahydro-2H-phenanthren-3-on (**E11b**);
*(rac)*-*(1s,4R,4aR,10aS)*-1-Butyl-7-hydroxy-4-methyl-1,4,4a,9,10,10a-hexahydro-*2H*-phenanthren-3-on (**E11**c);
*(rac)-(1R,2R,4aS,10aS)*-1-Butyl-3,3-ethandiyldimercapto-7-hydroxy-2-methyl-1,2,3,4,4a,9,10,10a-octahydrophenanthren (**E12a**);
*(rac)-(1R,2S,4aS,10aS)*-1-Butyl-3,3-ethandiyldimercapto-7-hydroxy-2-methyl-1,2,3,4,4a,9,10,10a-octahydrophenanthren (**E12b**);
*(rac)-(1S,4aS,10aS)*-7-Hydroxy-10a-methyl-1-(3-methylbutyl)-1,4,4a,9,10,10a-hexahydro-2*H*-phenanthren-3-on (**E13**);
*(rac)-(1S,4aS,10aS)*-3,3-Ethandiyldimercapto-7-hydroxy-1-(3-methylbutyl)-10a-methyl-1,2,3,4,4a,9,10,10a-octahydrophenanthren (**E14**);
*(rac)-(1S,4aS,10aS)*-7-Hydroxy-10a-methyl-1-phenethyl-1,4,4a,9,10,10a-hexahydro-*2H*-phenanthren-3-on (**E15**);
*(rac)-(1S,2S,4aS,10aS)*-7-Hydroxy-2,10a-dimethyl-1-(3-methylbutyl)-1,4,4a,9,10,10a-hexahydro-*2H*-phenanthren-3-on (**E16a**);
*(rac)-(1S,2R,4aS,10aS)*-7-Hydroxy-2,10a-dimethyl-1-(3-methylbutyl)-1,4,4a,9,10,10a-hexahydro-*2H*-phenanthren-3-on (**E16b**);
*(rac)-(4bS,8S,8aS)*-6,6-Dimethoxy-8a-methyl-8-(3-methylbutyl)-4b,5,6,7,8,8a,9,20-octahydrophenanthren-2-ol (**E17a**);
*(rac)-(1S,4aS,10aS)*-7-Hydroxy-10a-methyl-1-(3-methylbutyl)-1,4,4a,9,10,10a-hexahydro-2*H*-phenanthren-3-on (**E17b**);
*(rac)-(1S,4aS,10aS)*-3,3-Ethandiyldioxy-7-hydroxy-1-(3-methylbutyl)-10a-methyl-1,2,3,4,4a,9,10,10a-octahydrophenanthren (**E18**);
*(rac)-(4bS,8R,8aS)*-8a-Methyl-8-(3-methylbutyl)-4b,5,6,7,8,8a,9,10-octahydrophenanthren-2-ol (**E19a**);
*(rac)*-*(4bS*,*6R*,*8S*,*8aS)*-6-Ethylsulfonyl-8a-methyl-8-(3-methylbutyl)-4b,5,6,7,8,8a,9,10-octahydrophenanthren-2-ol (**E19b**);
*(rac)-(1S,4aS,10aS)-3,*3-(Propan-1,3-diyldimercapto)-7-hydroxy-1-(3-methylbutyl)-10a-methyl-1,2,3,4,4a,9,10,10a-octahydrophenanthren **(E20);**
*(rac)*-*(4aS*,*10aS)*-7-Hydroxy-10a-methyl-1,4,4a,9,10,10a-hexahydro-*2H*-phenanthren-3-on (**E21**);
*(rac)-(4aS*,*10aS)-3,*3-Ethandiyldimercapto-7-hydroxy-10a-methyl-1,2,3,4,4a,9,10,10a-octahydrophenanthren (**E22**);
*(rac)-(4aS,10aS)*-10a-Ethyl-7-hydroxy-1,4,4a,9,10,10a-hexahydro-*2H*-phenanthren-3-on (**E23**);
*(rac)-(4aS,10aS)*-3*,*3-Ethandiyldimercapto-10a-ethyl-7-hydroxy-1,2,3,4,4a,9,10,10a-octahydrophenanthren (**E24**);
*(rac)-(1S,4aS,10aS)-1-*(3-Methylbutyl)-10a-ethyl-7-hydroxy-1,4,4a,9,10,10a-hexahydro-*2H*-phenanthren-3-on (**E26**);
*(1S,4aS,10aS)*-7-Hydroxy-10a-methyl-1-(3-methylbutyl)-1,4,4a,9,10,10a-hexahydro-2H-phenanthren-3-on **(E27);**
*(1R,4aRS,10aR)*-7-Benzyloxy-10a-methyl-1-(3-methylbutyl)-1,4,4a,9,10,10a-hexahydro-*2H*-phenanthren-3-on **(E28);**
*(rac)-(1S,4aS,10aS)*-1-(3-Methylbutyl)-3,3-ethandiyldimercapto-7-hydroxy-1,4,4a,9,10,10a-octahydrophenanthren **(E29);**
*(rac)-(4aR,10aR)*-7-Hydroxy-4a,10a-dimethyl-3,4,4a,9,10,10a-hexahydro-*1H*-phenanthren-2-on **(E30);**
*(rac)-(1S,4aS,10aS)*-7-Hydroxy-10a-methyl-4-phenyl-1,4,4a,9,10,10a-hexahydro-*2H*-phenanthren-3-on (**E31**);
*(rac)-(4R,4aS,10aS)*-3,3-Ethandiyldimercapto-7-hydroxy-10a-methyl-4-phenyl-1,4,4a,9,10,10a-octahydrophenanthren (**E32**);
*(rac)-(1S,4aS,10aS)*-3,3-(Ethan-1,2-diyldimercapto)-7-hydroxy-1-(2-phenylethyl)-10a-methyl-1,2,3,4,4a,9,10,10a-octahydrophenanthren (**E33**);
*(rac)-(1S,4aS,10aS)*-3,3-Ethandiyldioxy-7-hydroxy-10a-methyl-1,2,3,4,4a,9,10,10a-octahydrophenanthren (**E34a**);
*(rac)-(1S,4aS,10aR)*-3,3-Ethandiyldioxy-7-hydroxy-10a-methyl-1,2,3,4,4a,9,10,10a-octahydrophenanthren (**E34b**);
*(rac)-(3S,4aS,10aS)*-7-Hydroxy-3-pentyl-10a-methyl-1,2,3,4,4a,9,10,10a-octahydrophenanthren (**E35a**);
*(rac)-(3R,4aS,10aS)*-7-Hydroxy-3-pentyl-10a-methyl-1,2,3,4,4a,9,10,10a-octahydrophenanthren (**E35b**);
*(rac)-(1S,2R,4aS,10aS)*-2,10a-Dimethyl-3,3-(ethan-1,2-diyldimercapto)-7-hydroxy-1-(3-methylbutyl-1,2,3,4,4a,9,10,10a-octahydrophenanthren (**E36**);
*(rac)-(1S,4aS,10aS)*-7-Hydroxy-1-(3'-methylbutyl)-1,4,4a,9,10,10a-hexahydro-2*H*-phenanthren-3-on (**E37**);
*(rac)-(4aS,10aS)*-3*,*3-(Propan-1,3-diyldimercapto)-7-hydroxy-1,2,3,4,4a,9,10,10a-octahydrophenanthren (**E38**);
*(rac)-(1S,4S,4aS,10aS)*-7-Hydroxy-1-butyl-4,10a-dimethyl-1,4,4a,9,10,10a-hexahydro-*2H*-phenanthren-3-on (**E39**);
*(rac)-(4S,4aS,10aS)*-3,3-Ethandiyldimercapto-7-hydroxy-4,10a-dimethyl-1,2,3,4,4a,9,10,10a-octahydrophenanthren (**E40**);
*(rac)-(4S,4aS,10aS)*-7-Hydroxy-4-benzyl-10a-methyl-1,4,4a,9,10,10a-hexahydro-*2H*-phenanthren-3-on (**E41a**);
*(rac)-(4aS,10aS)*-7-Hydroxy-4,4-dibenzyl-10a-methyl-1,4,4a,9,10,10a-hexahydro-*2H*-phenanthren-3-on (**E41b**);
*(rac)-(4aS,10aS)*-7-Hydroxy-10a-methyl-3, 3-methylen-1,2,3,4,4a,9,10,10a-octahydrophenanthren (**E42**);
*(rac)-(4aS,10aS)*-3,3-Ethandiyl-7-hydroxy-10a-methyl-1,2,3,4,4a,9,10,10a-octahydrophenanthren (**E43**);
*(rac)-(3R,4aS,10aS)*-1',2',3',4'-Tetrachlor-7-hydroxy-10a-methyl-1,2,3,4,4a,9,10,10a-octahydrospiro[phenanthren-3,6'-cyclohexan]-1',3'-dien (**E44**);
*(rac)-(3S,4aS,10aS)*-3,7-Dihydroxy-10a-methyl-3-[1-(phenylthio)cyclopropyl]-1,2,3,4,4a,9,10,10a-octahydrophenanthren (**E45**);
*(rac)-(3S,4aS,10aS)*-7-Hydroxy-10a-methyl-1,2,3,4,4a,9,10,10a-octahydrospiro[phenanthren-3,2'-cyclobutan]-1'-on (**E46a**);
*(rac)-(3R,4aS,10aS)*-7-Hydroxy-10a-methyl-1,2,3,4,4a,9,10,10a-octahydrospiro[phenanthren-3,2'-cyclobutan]-1'-on (**E46b**);
*(rac)-(3S,4aS,10aS)*-1',1'-Ethandiyldimercapto-7-hydroxy-10a-methyl-1,2,3,4,4a,9,10,10a-octahydrospiro-[phenanthren-3,2'-cyclobutan] (**E47**);
*(rac)-(4aS,10aS)*-7-Hydroxy-10a-methyl-1,2,3,4,4a,9,10,10a-octahydrospiro[phenanthren-3,1'-cyclobutan] (**E48**);
*(rac)-(4aS,10aS)*-3-(1-Cyclopenten-1-yl)-7-hydroxy-10a-methyl-1,4,4a,9,10,10a-hexahydrophenanthren (**E49**);
*(rac)-(3S,4aS,10aS)*-7-Hydroxy-10a-methyl-1,2,3,4,4a,9,10,10a,2',3',4',5'-dodecahydrospiro[phenanthren-3,2'-furan] (**E50a**);
*(rac)-(3R,4aS,10aS)*-7-Hydroxy-10a-methyl-1,2,3,4,4a,9,10,10a,2',3',4',5'-dodecahydrospiro-[phenanthren-3,2'-furan] (**E50b**);
*(rac)-(3S,9aS,10aS)*-7-Hydroxy-10a-methyl-1,2,3,4,4a,9,10,10a,3',4',5',6'-dodecahydrospiro-[phenanthren-3,2'-2*H*-pyran] (**E51a**);
*(rac)-(3R,4aS,10aS)*-7-Hydroxy-10a-methyl-1,2,3,4,4a,9,10,10a,3',4',5',6'-dodecahydrospiro-[phenanthren-3,2'-2*H*-pyran] (**E51b**);
*(rac)-(1S,3R,4aS,10aS)*-7-Hydroxy-10a-methyl-1-(3-methylbutyl)-1,2,3,4,4a,9,10,10a,2',3',4',5'-dodecahydrospiro[phenanthren-3,2'-furan] (**E52**);
*(rac)-(1S,4aS,10aS)*-7-Hydroxy-10a-methyl-1-(3-methylbutyl)-1,2,3,4,4a,9,10,10a-octahydrospiro-[phenanthren-3,1'-cyclobutan] (**E53**);
*(rac)-(1S,4aS,10aS)*-7-Hydroxy-3-(3-hydroxypropyl)-10a-methyl-1-(3-methylbutyl)-1,4,4a,9,10,10a-hexahydrophenanthren (**E54a**);
*(rac)-(1S,3S,4aS,10aS)*-7-Hydroxy-10a-methyl-1-(3-methylbutyl)-1,2,3,4,4a,9,10,10a,2',3',4',5'-dodecahydrospiro[phenanthren-3,2'-furan] (**E54b**);
*(rac)-(4aR,10aR)*-10a-Butyl-7-hydroxy-4a-methyl-3,4,4a,9,10,10a-hexahydro-*1H*-phenanthren-2-on **(E55);**
*(rac)*-7-Hydroxy-4a-methyl-4,4a,9,10-tetrahydro-*3H*-phenanthren-2-on **(E56);**
*(rac)*-4b-Methyl-4b,5,6,7,9,10-hexahydrophenanthren-2-ol **(E57);**
*(rac)*-7-Hydroxy-1,4a-dimethyl-4,4a,9,10-tetrahydro-*3H*-phenanthren-2-on **(E58);**
*(rac)*-4a,8-Dimethyl-4b,5,6,7,9,10-hexahydrophenanthren-2-ol (E59);
*(rac)*-4b,8-Dimethyl-4b,5,6,7,9,10-hexahydrophenanthren-2-ol (**E60a, E60b, E60c, E60d**);
*(rac)-(3S,4aR)*-7-Hydroxy-3,4a-dimethyl-4,4a,9,10-tetrahydro-*3H*-phenanthren-2-on (**E61**);
*(rac)-(4S,4aR)*-7-Hydroxy-4a-methyl-4-propyl-4,4a,9,10-tetrahydro-*3H*-phenanthren-2-on (**E62**);
*(rac)-(4R,4aR)*-7-Hydroxy-4,4a-dimethyl-4,4a,9,10-tetrahydro-*3H*-phenanthren-2-on (**E63a**);
*(rac)-(4S,4aR)*-7-Hydroxy-4,4a-dimethyl-4,4a,9,10-tetrahydro-*3H*-phenanthren-2-on (**E63b**);
*(rac)*-1-Ethyl-7-hydroxy-4a-methyl-4,4a,9,10-tetrahydro-*3H*-phenanthren-2-on (**E64**);
*(rac)*-8-Ethyl-4b-methyl-4b,5,6,7,9,10-hexahydrophenanthren-2-ol (**E65**);
*(rac)-(4aR,10aR,1S)*-1-Ethyl-7-hydroxy-4a-methyl-1,4,4a,9,10,10a-hexahydro-*3H*-phenanthren-2-on (**E66**);
*(rac)*-1-Butyl-7-hydroxy-4a-methyl-4,4a,9,10-tetrahydro-*3H*-phenanthren-2-on (**E67**);
*(rac)*-8-Butyl-4b-methyl-4b,5,6,7,9,10-hexahydrophenanthren-2-ol (**E68**);
*(rac)-(8R,4bR,8aR)*-8-Butyl-4b-methyl-4b,5,6,7,8,8a,9,10-octahydrophenanthren-2-ol (**E69a**);
*(rac)-(8S,9bR,8aR)*-8-Butyl-4b-methyl-4b,5,6,7,8,8a,9,10-octahydrophenanthren-2-ol (**E69b**);
*(rac)*-4a-Butyl-7-hydroxy-1-methyl-4,4a,9,10-tetrahydro-*3H*-phenanthren-2-on (**E70**);
*(rac)*-4a-Butyl-7-hydroxy-4,4a,9,10-tetrahydro-*3H*-phenanthren-2-on (**E71**);
*(rac)-(4aR,10aR)*-4a-Butyl-7-hydroxy-3,4,4a,9,10,10a-hexahydro-*1H*-phenanthren-2-on (**E72a**);
*(rac)-(4aR,10aS)*-4a-Butyl-7-hydroxy-3,4,4a,9,10,10a-hexahydro-*1H*-phenanthren-2-on (**E72b**);
*(rac)-(4aR,10aS)*-7-Hydroxy-4a-methyl-3,4,4a,9,10,10a-hexahydro-*1H*-phenanthren-2-on (**E73**);
*(rac)-(4aR,10aS)*-2,2-Ethandiyldimercapto-7-hydroxy-4a-methyl-1,2,3,4,4a,9,10,10a-octahydrophenanthren (**E74**);
*(rac)-(4aR,10aS)*-7-Hydroxy-4a-methyl-3,4,4a,9,10,10a-hexahydro-*1H*-phenanthren-2-on-oxim (**E75**);
und pharmazeutisch verträglichen Salzen und Stereoisomeren davon.

17. Verbindung nach irgendeinem der Ansprüche 1 bis 16 zur Verwendung in der medizinischen Therapie.

18. Pharmazeutische Zusammensetzung, umfassend eine Verbindung nach irgendeinem der Ansprüche 1 bis 16 und einen pharmazeutisch verträglichen Träger.

19. Verfahren zur Herstellung einer pharmazeutischen Zusammensetzung, umfassend das Vereinigen einer Verbindung nach irgendeinem der Ansprüche 1 bis 16 und eines pharmazeutisch verträglichen Trägers.

20. Verwendung einer Verbindung nach irgendeinem der Ansprüche 1 bis 16 bei der Herstellung eines Medikaments zur therapeutischen Behandlung oder zur Prävention von Knochenverlust, Knochenbrüchen, Osteoporose, Knorpeldegeneration, Endometriose, Uterusfibrose, Hitzewallungen, erhöhten LDL-Cholesterinspiegeln, kardiovaskulären Erkrankungen, Beeinträchtigungen der Wahrnehmungsfunktion, zerebralen degenerativen Störungen, Restenosen, Gynäkomastie, Proliferation vaskulärer glatter Muskelzellen, Fettsucht, Inkontinenz, Autoimmunerkrankungen und Lungen-, Kolon-, Brust-, Uterus- und Prostatakrebs.

## Revendications

1. Composé de formule générale I, II ou III : 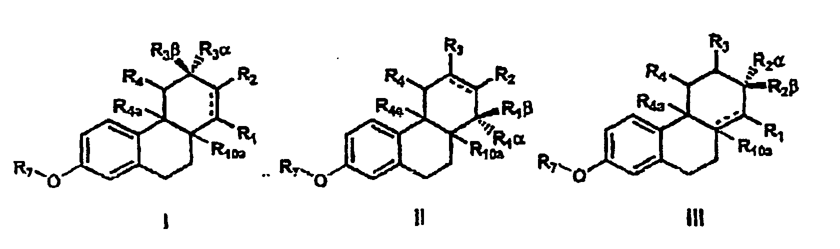 dans lequel
la liaison entre les atomes de carbone C1 et C2 (pour les composés de formule générale I) ou la liaison entre C2 et C3 (pour les composés de formule générale II) ou la liaison entre C1 et C10 (pour les composés de formule générale III) est, soit une simple liaison, soit une double liaison ;
R₁ (pour les composés de formule générale I ou III) est un groupe R^{A} autre qu'un groupe phényle ;
R^{A} est choisi parmi l'hydrogène et les groupes alkyle, alcényle, alcynyle, cycloalkyle, cycloalcényle, cycloalkylalkyle, aryle ou arylalkyle ;
R_{1α} et R_{1β} (pour les composés de formule générale II) sont identiques ou différents et correspondent chacun à un groupe R^{A} ;
R₂ (pour les composés de formule générale I ou II) représente un groupe hydroxyle ou R^{A} ou encore un groupe hydroxyalkyle ou aminoalkyle comportant de 1 à 2 atomes de carbone ;
R_{3α} et R_{3β} (pour les composés de formule générale I) peuvent ensemble correspondre à un unique atome d'oxygène ou de soufre ou R_{3α} et R_{3β} peuvent ensemble correspondre à un unique atome d'azote qui, à son tour, est lié à un groupe choisi parmi R^{A} ou OR^{A}; ou R_{3α} et R_{3β} peuvent ensemble correspondre à un unique atome de carbone qui, à son tour, est lié à deux groupes R^{A} qui peuvent être identiques ou différents ; ou R_{3α} et R_{3β} peuvent être identiques ou différents et chacun peut être choisi parmi R^{A}, OR^{A}, SR^{A} ou N(R^{A})₂ où chaque groupe R^{A} individuel peut être identique ou différent et peut éventuellement être considéré avec tout atome lié et intermédiaire pour former un composé cyclique comportant de 3 à 8 membres ;
R_{2α} et R_{2β} (pour les composés de formule générale III) peuvent ensemble correspondre à un unique atome d'oxygène ou de soufre ou R_{2α} et R_{2β} peuvent ensemble correspondre à un unique atome d'azote (c'est à dire un atome d'azote imine ou oxime) qui, à son tour, est lié à un groupe choisi parmi R^{A} ou OR^{A} ; ou R_{2α} et R_{2β} peuvent ensemble correspondre à un unique atome de carbone qui, à son tour, est lié à deux groupes R^{A} qui peuvent être identiques ou différents ; ou R_{2α} et R_{2β} peuvent être identiques ou différents et chacun peut être choisi parmi les groupes hydroxyalkyle, aminoalkyle, R^{A}, OR^{A}, SR^{A} ou N(R^{A})₂, où chaque groupe R^{A} peut être identique ou différent et peut éventuellement être considéré avec tout atome lié et intermédiaire pour former un composé cyclique comportant de 3 à 8 membres ;
R₃ (pour les composés de formule générale II ou III) correspond à un groupe R^{A} ;
R₄ correspond à un groupe R^{A} ;
R₄ₐ correspond à un atome d'hydrogène ou à un groupe méthyle ou éthyle dans les composés de formule I ou II, ou encore à un groupe méthyle ou éthyle dans les composés de formule III ;
R₇ correspond à un atome d'hydrogène ou à un groupe alkyle ou cycloalkyle linéaire ou ramifié ou encore à un groupe acyle comportant de 1 à 4 atomes de carbone ;
R₁₀ₐ, qui est absent lorsque la liaison entre les atomes de carbone C₁ et C₁₀ de la formule III est une double liaison, correspond à un groupe R^{A} ;
sous réserve que R₁, R₂, R₃ et R₄ ne correspondent pas tous à de l'hydrogène dans les composés de formule I ou II et que R₄ ne corresponde pas à de l'hydrogène dans les composés de formule III,
de même que les sels et stéréoisomères, acceptables sur le plan pharmaceutique, dudit composé.

2. Composé selon la revendication 1, dans lequel les substituants R₄ₐ et R₁₀ₐ présentent une stéréochimie relative trans.

3. Composé de formule générale I ou III selon la revendication 2, dans lequel R₁ correspond à R^{B} et R^{B} est choisi parmi l'hydrogène, le n-propyle, le 2-propényle, le 2-propynyle, le n-butyle, le 2-butényle, le 3-butényle, le 2-butynyle, le 3-butynyle, le n-pentyle, le 3-méthylbutyle, le 3-méthyl-1-butényle, le 3-méthyl-2-butényle, le 3-méthylpentyle, le 3-éthylpentyle, le cyclopropyléthyle, le cyclopentyléthyle, le cyclohexyléthyle, le cycloheptyléthyle, le cyclopropylpropyle, le cyclopentylpropyle, le benzyle ou le phénétyle.

4. Composé de formule générale II selon la revendication 2, dans lequel R_{1α} correspond à R^{B} (R^{B} étant comme défini dans la revendication 3) et R_{1β} correspond à un atome d'hydrogène ou à un groupe méthyle.

5. Composé selon l'une quelconque des revendications 2 à 4, dans lequel R₁₀ₐ correspond à R^{B}.

6. Composé de formule générale I ou II selon l'une quelconque des revendications 2 à 5, dans lequel R₂ correspond à un atome d'hydrogène ou à un groupe méthyle, éthyle ou hydroxyméthyle.

7. Composé de formule générale III selon l'une quelconque des revendications 2 à 5, dans lequel R_{2α} et R_{2β} peuvent être identiques ou différents et peuvent chacun être choisis parmi l'hydroxyalkyle, R^{A}, OR^{A} ou SR^{A} où chaque groupe R^{A} (R^{A} étant comme défini plus haut) peut être identique ou différent et peut éventuellement être considéré en même temps que tout atome lié et intermédiaire pour former un composé cyclique comportant de 3 à 8 membres.

8. Composé de formule générale I selon l'une quelconque des revendications 2 à 6, dans lequel R_{3α} et R_{3β} peuvent être identiques ou différents et peuvent chacun être choisis parmi R^{A}, OR^{A} ou SR^{A} où chaque groupe R^{A} (R^{A} étant comme défini dans la revendication 1) peut être identique ou différent et peut éventuellement être considéré en même temps que tout atome lié et intermédiaire pour former un composé cyclique comportant de 3 à 8 membres.

9. Composé selon l'une quelconque des revendications 2 à 8, dans lequel R₄ correspond à un atome d'hydrogène ou à un groupe méthyle ou éthyle.

10. Composé selon l'une quelconque des revendications 2 à 9, dans lequel R₇ correspond à un atome d'hydrogène ou à un groupe acyle comportant de 1 à 4 atomes de carbone.

11. Composé de formule générale I ou III selon l'une quelconque des revendications 2 à 10, dans lequel R₁ est choisi parmi l'hydrogène, le méthyle ou l'éthyle et R₁₀ₐ correspond à R^{B}.

12. Composé de formule générale I ou III selon l'une quelconque des revendications 2 à 10, dans lequel R₁ correspond à R^{B} et R₁₀ₐ est choisi parmi l'hydrogène, le méthyle ou l'éthyle.

13. Composé de formule III selon l'une quelconque des revendications 2, 9, 10, 11 ou 12, dans lequel R_{2α} et R_{2β} peuvent être identiques ou différents et peuvent chacun être choisis parmi l'hydroxyalkyle, R^{A}, OR^{A} ou SR^{A} où chaque groupe R^{A} (R^{A} étant comme défini dans la revendication 1) peut être identique ou différent et peut éventuellement être considéré en même temps que tout atome lié et intermédiaire pour former un composé cyclique comportant de 3 à 8 membres.

14. Composé de formule générale II selon l'une quelconque des revendications 2 à 10, dans lequel R_{1α} correspond à R^{B} et R₁₀ₐ est choisi parmi l'hydrogène, le méthyle ou l'éthyle.

15. Composé de formule générale II selon l'une quelconque des revendications 2 à 10, dans lequel R_{1α} est choisi parmi l'hydrogène, le méthyle ou l'éthyle et R₁₀ₐ correspond à R^{B}.

16. Composé selon la revendication 1 choisi parmi les composés suivants :
(*rac*)-(*4aS,10aS*)-7-hydroxy-1,4,4a,9,10,10a-hexahydro-*2H*-phénanthren-3-one (E1) ;
(*rac*)-(*4aS,10aS*)-3,3-éthanediyldimercapto-7-hydroxy-1,2,3,4,4a,9,10,10a-octahydrophénanthrène (E2) ;
(*rac*)-(*4aR,10aS*)-7-hydroxy-10a-méthyl-4a,9,10,10a-tétrahydro-*4H*-phénanthren-3-one (E3a) ;
(*rac*)-(*4aS,10aS*)-7-hydroxy-10a-méthyl-4a,9,10,10a-tétrahydro-*4H*-phénanthren-3-one (E3b) ;
(*rac*)-(*4aS,10aS*)-10a-butyl-7-hydroxy-4a,9,10,10a-tétrahydro-*4H*-phénanthren-3-one (E4) ;
(*rac*)-(*1S,4aS,10aS*)-1-butyl-7-hydroxy-1,4,4a,9,10,10a-hexahydro-*2H*-phénanthren-3-one (E5a) ;
(*rac*)-(*1R,4aS,10aS*)-1-butyl-7-hydroxy-1,4,4a,9,10,10a-hexahydro-*2H*-phénanthren-3-one (E5b) ;
(*rac*)-(*1S,4aS,10aS*)-1-butyl-3,3-éthanediyldimercapto-7-hydroxy-1,2,3,4,4a,9,10, 10a-octahydrophénanthrène (E6a) ;
(*rac*)-(*1R,4aS,10aS*)-1-butyl-3,3-éthanediyldimercapto-7-hydroxy-1,2,3,4,4a,9,10, 10a-octahydrophénanthrène (E6b) ;
(*rac*)-(*1S,4aS,10aS*)-1-butyl-7-hydroxy-10a-méthyl-1,4,4a,9,10,10a-hexahydro-*2H*-phénanthren-3-one (E7) ;
(*rac*)-(*1S,4aS,10aS*)-1-butyl-3,3-éthanediyldimercapto-7-hydroxy-10a-méthyl-1,2,3, 4,4a,9,1 0, 10a-octahydrophénanthrène (E8) ;
(*rac*)*-*(*1S,4aS,10aS*)-1-butyl-7-hydroxy-10a-méthyl-1,2,3,4,4a,9,10,10a-octahydrophénanthrène (E9) ;
(*rac*)-(*4aS,10aR*)-10a-éthyl-7-hydroxy-3-méthyl-4a,9,10,10a-tétrahydro-*4H*-phénanthren-1-one (E10a) ;
(*rac*)-(*4aR,10aR*)-10a-éthyl-7-hydroxy-3-méthyl-4a,9,10,10a-tétrahydro-*4H*-phénanthren-1-one (E10b) ;
(*rac*)-(*1R,2S,4aS,10aS*)-1-butyl-7-hydroxy-2-méthyl-1,4,4a,9,10,10a-hexahydro-*2H*-phénanthren-3-one (E11a) ;
(*rac*)-(*1R,2R,4aS,10aS*)-1-butyl-7-hydroxy-2-méthyl-1,4,4a,9,10,10a-hexahydro-*2H*-phénanthren-3-one (E11b) ;
(*rac*)-(*1S,4R,4aR,10aS*)-1-butyl-7-hydroxy-4-méthyl-1,4,4a,9,10,10a-hexahydro-*2H*-phénanthren-3-one (E11c) ;
(*rac*)-(*1R,2R,4aS,10aS*)-1-butyl-3,3-éthanediyldimercapto-7-hydroxy-2-méthyl-1,2,3, 4,4a,9,10,10a-octahydrophénanthrène (E12a) ;
(*rac*)-(*1R,2S,4aS,10aS*)-1-butyl-3,3-éthanediyldimercapto-7-hydroxy-2-méthyl-1,2,3, 4,4a,9,10,10a-octahydrophénanthrène (E 12b) ;
(*rac*)-(*1S,4aS,10aS*)-7-hydroxy-10a-méthyl-1-(3-méthyl-butyl)-1,4,4a,9,10,10a-hexahydro-*2H*-phénanthren-3-one (E13) ;
(*rac*)-(*1S,4aS,10aS*)-3,3-éthanediyldimercapto-7-hydroxy-1-(3-méthyl-butyl)10a-méthyl-1,2,3,4,4a,9,10,10a-octahydrophénanthrène (E 14) ;
(*rac*)-(*1S,4aS,10aS*)-7-hydroxy-10a-méthyl-1-phénéthyl-1,4,4a,9,10,10a-hexahydro-*2H*-phénanthren-3-one (E15) ;
(*rac*)-(*1S,2S,4aS,10aS*)-7-hydroxy-2,10a-diméthyl-1-(3-méthyl-butyl)-1,4,4a,9,10, 10a-hexahydro-*2H*-phénanthren-3-one (E16a) ;
(*rac*)-(*1S,2R,4aS,10aS*)-7-hydroxy-2,10a-diméthyl-1-(3-méthyl-butyl)-1,4,4a,9,10, 10a-hexahydro-*2H*-phénanthren-3-one (E16b) ;
(*rac*)-(*4bS,8S,8aS*)-6,6-diméthoxy-8a-méthyl-8-(3-méthyl-butyl)-4b,5,6,7,8,8a,9,10-octahydrophénanthren-2-ol (E17a) ;
(*rac*)-(*1S,4aS,10aS*)-7-hydroxy-10a-méthyl-1-(3-méthyl-butyl)-1,4,4a,9,10,10a-hexahydro-2H-phénanthren-3-one (E17b) ;
(*rac*)-(*1S,4aS,10aS*)-3,3-éthanediyldioxy-7-hydroxy-1-(3-méthyl-butyl)-10a-méthyl-1,2,3,4,4a,9,10,10a-octahydrophénanthrène (E18) ;
(*rac*)-(*4bS,8R, 8aS*)-8a-méthyl-8-(3-méthyl-butyl)-4b,5,6,7,8,8a,9,10-octahydrophénanthren-2-ol (E19a) ;
(*rac*)-(*4bS,6R, 8S,8aS*)-6-éthylsulfanyl-8a-méthyl-8-(3-méthyl-butyl)-4b,5,6,7,8,8a,9, 10-octahydrophénanthren-2-ol (E 19b) ;
(*rac*)-(*1S,4aS,10aS*)-3,3-(propane-1,3-diyldimercapto)-7-hydroxy-1-(3-méthyl-butyl)-10a-méthyl-1,2,3,4,4a,9,10,10a-octahydrophénanthrène (E20) ;
(*rac*)-(*4aS,10aS*)-7-hydroxy-10a-méthyl-1,4,4a,9,10,10a-hexahydro-*2H*-phénanthren-3-one (E21) ;
(*rac*)-(*4aS,10aS*)-3,3-éthanediyldimercapto-7-hydroxy-10a-méthyl-1,2,3,4,4a,9,10, 10a-octahydrophénanthrène (E22) ;
*(rac)-(4aS,10aS)*-10a-éthyl-7-hydroxy-1,4,4a,9,10,10a-hexahydro-*2H*-phénanthren-3-one (E23) ;
*(rac)-(4aS,10aS)*-3,3-éthanediyldimercapto-10a-éthyl-7-hydroxy-1,2,3,4,4a,9,10,10a-octahydrophénanthrène (E24) ;
(*rac*)-(*1S*,*4aS*,*10aS*)-1-(3-méthyl-butyl)-10a-éthyl-7-hydroxy-1,4,4a,9,10,10a-hexahydro-*2H*-phénanthren-3-one (E26) ;
(*1S,4aS,10aS*)-7-hydroxy-10a-méthyl-1-(3-méthyt-butyl)-1,4,4a,9,10,10a-hexahydro-*2H*-phénanthren-3-one (E27) ;
(*1R,4aRS,10aR*)-7-benzyloxy-10a-méthyl-1-(3-méthyl-butyl)-1,4,4a,9,10,10a-hexahydro-*2H*-phénanthren-3-one (E28) ;
(*rac*)*-*(*1S,4aS,10aS*)-1-(3-méthyl-butyl)-3,3-éthanediyldimercapto-7-hydroxy-1,4,4a, 9,10,10a-octahydrophénanthrène (E29);
(*rac)-*(*4aR,10aR*)-7-hydroxy-4a,10a-diméthyl-3,4,4a,9,10,10a-hexahydro-*1H*-phénanthren-2-one (E30) ;
(*rac)*-(*1S*,*4aS*,*10aS*)-7-hydroxy-10a-méthyl-4-phényl-1,4,4a,9,10,10a-hexahydro-*2H*-phénanthren-3-one (E31) ;
(*rac*)*-(4R,4aS,10aS*)-3,3-éthanediyldimercapto-7-hydroxy-10a-méthyl-4-phényl-1,4, 4a,9,10,10a-octahydrophénanthrène (E32) ;
(*rac*)*-*(*1S,4aS,10aS*)-3,3-(éthane-1,2-diyldimercapto)-7-hydroxy-1-(2-phényléthyl)-10a-méthyl-1,2,3,4,4a,9,10,10a-octahydrophénanthrène (E33);
(*rac*)*-*(*1S,4aS, 10aS*)-3,3-éthanediyldioxy-7-hydroxy-10a-méthyl-1,2,3,4,4a,9,10,10a-octahydrophénanthrène (E34a) ;
(*rac*)*-*(*1S,4aS, 10aR*)-3,3-éthanediyldioxy-7-hydroxy-10a-méthyl-1,2,3,4,4a,9,10,10a-octahydrophénanthrène (E34b) ;
(*rac*)-(*3S*,*4aS*,*10aS*)-7-hydroxy-3-pentyl-10a-méthyl-1,2,3,4,4a,9,10,10a-octahydrophénanthrène (E35a) ;
(*rac*)*-(3R,4aS,10aS*)-7-hydroxy-3-pentyl-10a-méthyl-1,2,3,4,4a,9,10,10a-octahydrophénanthrène (E35b) ;
(r*ac*)*-*(*1S,2R, 4aS, 10aS*)-2,10a-diméthyl-3,3-(éthane-1,2-diyldimercapto)-7-hydroxy-1-(3-méthyl-butyl)-1,2,3,4,4a,9,10,10a-octahydrophénanthrène (E36) ;
(*rac*)*-*(*1S,4aS,10aS*)-7-hydroxy-1-(3'-méthyl-butyl)-1,4,4a,9,10,10a-hexahydro-*2H*-phénanthren-3-one (E37) ;
(*rac*)-(*4aS,10aS*)-3,3-(propane-1,3-diyldimercapto)-7-hydroxy-1,2,3,4,4a,9,10,10a-octahydrophénanthrène (E38) ;
(*rac*)-(*1S,4S,4aS,1 0aS*)-7-hydroxy-1-butyl-4,10a-diméthyl-1,4,4a,9,10,10a-hexahydro-*2H*-phénanthren-3-one (E39) ;
(*rac*)-(*4S,4aS,10aS*)-3,3-éthanediyldimercapto-7-hydroxy-4,10a-diméthyl-1,2,3,4,4a,9,10,10a-octahydrophénanthrène (E40) ;
(*rac*)-(*4S,4aS,10aS*)-7-hydroxy-4-benzyl-10a-méthyl-1,4,4a,9,10,10a-hexahydro-*2H*-phénanthren-3-one (E41a) ;
(*rac*)-(*4aS,10aS*)-7-hydroxy-4,4-dibenzyl-10a-méthyl-1,4,4a,9,10,10a-hexahydro-*2H*-phénanthren-3-one (E41b) ;
(*rac*)-(*4aS,10aS*)-7-hydroxy-10a-méthyl-3,3-méthylène-1,2,3,4,4a,9,10,10a-octahydrophénanthrène (E42) ;
(*rac*)-(*4aS,10aS*)-3,3-éthanediyl-7-hydroxy-10a-méthyl-1,2,3,4,4a,9,10,10a-octahydrophénanthrène (E43) ;
(*rac*)-(*3R,4aS,10aS*)-1',2',3',4'-tétrachloro-7-hydroxy-10a-méthyl-1,2,3,4,4a,9,10, 10a-octahydrospiro[phénanthrène-3,6'-cyclohexane]1',3'-diène (E44) ;
(*rac*)-(*3S,4aS,10aS*)-3,7-dihydroxy-10a-méthyl-3[1-(phénylthio)cyclopropyl]-1,2,3,4, 4a,9,10,10a-octahydrophénanthrène (E45) ;
(*rac*)-(*3S,4aS,10aS*)-7-hydroxy-10a-méthyl-1,2,3,4,4a,9,10,10a-octabydrospiro[phénanthrène-3,2'-cyclobutane]1'-one (E46a) ;
(*rac*)-(*3R,4aS,10aS*)-7-hydroxy-10a-méthyl-1,2,3,4,4a,9,10,10a-octahydrospiro[phénanthrène-3,2'-cyclobutane]1'-one (E46b) ;
(*rac*)-(*3S,4aS,10aS*)-1',1'-éthanediyldimercapto-7-hydroxy-1Oa-méthyl-1,2,3,4,4a,9, 10,10a-octahydrospiro[phénanthrène-3,2'-cyclobutane] (E47) ;
(*rac*)-(*4aS,10aS*)-7-hydroxy-10a-méthyl-1,2,3,4,4a,9,10, 10a-octahydrospiro[phénanthrène-3,1'-cyclobutane] (E48) ;
(*rac*)-(*4aS,10aS*)-3-(1-cyclopenten-1-yl)-7-hydroxy-10a-méthyl-1,4,4a,9,10,10a-hexahydrophénanthrène (E49) ;
(*rac*)-(*3S,4aS,10aS*)-7-hydroxy-10a-méthyl-1,2,3,4,4a,9,10,10a,2',3',4',5'-dodécahydrospiro[phénanthrène-3,2'-furane] (E50a) ;
(*rac*)-(*3R,4aS,10aS*)-7-hydroxy-10a-méthyl-1,2,3,4,4a,9,10,10a,2',3',4',5'-dodécahydrospiro[phénanthrène-3,2'-furane] (E50b) ;
(*rac*)-(*3S,4aS, 10aS*)-7-hydroxy-10a-méthyl-1,2,3,4,4a,9,10,10a,3',4',5',6'-dodécahydrospiro[phénanthrène-3,2'-*2H*-pyrane] (E51a) ;
(*rac*)-(*3R,4aS,10aS*)-7-hydroxy-10a-méthyl-1,2,3,4,4a,9,10,10a,3',4',5',6'-dodécahydrospiro[phénanthrène-3,2'-*2H*-pyrane] (E51b) ;
(*rac*)-(*1S,3R,4aS,10aS*)-7-hydroxy-10a-méthyl-1-(3-méthylbutyl)-1,2,3,4,4a,9,10, 10a,2',3',4',5'-dodécahydrospiro[phénanthrène-3,2'-furane] (E52) ;
(*rac*)-(*1S,4aS,10aS*)-7-hydroxy-10a-méthyl-1-(3-méthylbutyl)-1,2,3,4,4a,9,10,10a-octahydrospiro[phénanthrène-3,1'-cyclobutane] (E53) ;
(*rac*)-(*1S,4aS,10aS*)-7-hydroxy-3-(3-hydroxypropyl)-10a-méthyl-1-(3-méthylbutyl)-1,4,4a,9,10,10a-hexahydrophénanthrène (E54a) ;
(*rac*)-(*1S,3S,4aS,10aS*)-7-hydroxy-10a-méthyl-1-(3-méthylbutyl)-1,2,3,4,4a,9,10,10a, 2',3',4',5'-dodécahydrospiro[phénanthrène-3,2'-furane] (E54b) ;
(*rac*)-(*4aR,10aR*)-10a-butyl-7-hydroxy-4a-méthyl-3,4,4a,9,10,10a-hexahydro-*1H*-phénanthren-2-one (E55) ;
*(rac)*-7-hydroxy-4a-méthyl-4,4a,9,10,10a-tétrahydro-*3H*-phénanthren-2-one (E56) ;
*(rac)*-4b-méthyl-4b,5,6,7,9,10-hexahydrophénanthren-2-ol (E57) ;
*(rac)*-7-hydroxy-1,4a-diméthyl-4,4a,9,10-tétrahydro-*3H*-phénanthren-2-one (E58) ;
*(rac)*-4a,8-diméthyl-4b,5,6,7,9,10-hexahydrophénanthren-2-ol (E59) ;
*(rac)*-4b,8-diméthyl-4b,5,6,7,9,10-hexahydrophénanthren-2-ol (E60a, E60b, E60c, E60d);
*(rac)*-*(3S*,*4aR)*-7-hydroxy-3,4a-diméthyl-4,4a,9,10-tétrahydro-*3H*-phénanthren-2-one (E61) ;
*(rac)-(4S,4aR)*-7-hydroxy-4a-méthyl-4-propyl-4,4a,9,10-tétrahydro-*3H*-phénanthren-2-one (E62) ;
*(rac)*-(4R,*4aR)*-7-hydroxy-4,4a-diméthyl-4-propyl-4,4a,9,10-tétrahydro-*3H*-phénanthren-2-one (E63a) ;
*(rac)-(4S,4aR)*-7-hydroxy-4,4a-diméthyl-4,4a,9,10-tétrahydro-*3H*-phénanthren-2-one (E63b) ;
*(rac)-*1-éthyl-7-hydroxy-4a-méthyl-4,4a,9,10-tétrahydro-*3H*-phénanthren-2-one (E64) ;
(*rac*)-8-éthyl-4b-méthyl-4b,5,6,7,9,10-hexahydrophénanthren-2-ol (E65) ;
(*rac)-*(*4aR,10aR,1S*)*-*1-éthyl-7-hydroxy-4a-méthyl-1,4,4a,9,10,10a-hexahydro-*3H*-phénanthren-2-one (E66) ;
(*rac)-*1-butyl-7-hydroxy-4a-méthyl-4,4a,9,10-tetrahydro-*3H*-phénanthren-2-one (E67) ;
(*rac)*-8-butyl-4b-méthyl-4b,5,6,7,9,10-hexahydrophénanthren-2-ol (E68) ;
(*rac)-*(*8R,4bR, 8aR*)-8-butyl-4b-méthyl-4b,5,6,7,8,8a,9,10-octahydrophénanthren-2-ol (E69a) ;
(*rac)-*(*8S,4bR,8aR*)-8-butyl-4b-méthyl-4b,5,6,7,8,8a,9,10-octahydrophénanthren-2-ol (E69b) ;
(*rac)*-4a-butyl-7-hydroxy-1-méthyl-4,4a,9,10-tetrahydro-*3H*-phénanthren-2-one (E70) ;
(*rac)*-4a-butyl-7-hydroxy-4,4a,9,10-tetrahydro-*3H*-phénanthren-2-one (E71) ;
(*rac)*-(*4aR,10aR*)-4a-butyl-7-hydroxy-3,4,4a,9,10,10a-hexahydro-*1H*-phénanthren-2-one (E72a) ;
(*rac)*-(*4aR,10aS*)-4a-butyl-7-hydroxy-3,4,4a,9,10,10a-hexahydro-*1H*-phénanthren-2-one (E72b) ;
(*rac)-*(*4aR,10aS*)-7-hydroxy-4a-méthyl-3,4,4a,9,10,10a-hexahydro-*1H*-phénanthren-2-one (E73) ;
(*rac)*-(*4aR,10aS*)-2,2-éthanediyldimercapto-7-hydroxy-4a-méthyl-1,2,3,4,4a,9,10, 10a-octahydrophénanthrène (E74) ;
(*rac)*-(*4aR,10aS*)-7-hydroxy-4a-méthyl-3,4,4a,9,10,10a-hexahydro-*1H*-phénanthren-2-one-oxime (E75) ;
et les sels et stéréoisomères, acceptables sur le plan pharmaceutique, de ceux-ci.

17. Composé selon l'une quelconque des revendications 1 à 16, destiné à être utilisé dans le cadre d'une thérapie médicale.

18. Composition pharmaceutique comprenant un composé selon l'une quelconque des revendications 1 à 16, et un excipient acceptable sur le plan pharmaceutique.

19. Procédé de fabrication d'une composition pharmaceutique comprenant l'association d'un composé, selon l'une quelconque des revendications 1 à 16, avec un excipient acceptable sur le plan pharmaceutique.

20. Utilisation d'un composé selon l'une quelconque des revendications 1 à 16, dans la fabrication d'un médicament destiné au traitement thérapeutique, ou à la prévention, de la perte osseuse, des fractures osseuses, de l'ostéoporose, de la dégénérescence cartilagineuse, de l'endométriose, du fibrome utérin, des bouffées de chaleur, de l'augmentation du taux de cholestérol LDL, des maladies cardiovasculaires, de l'altération de la fonction cognitive, des troubles associés à la dégénérescence cérébrale, de la resténose, de la gynécomastie, de la prolifération des cellules musculaires lisses vasculaires, de l'obésité, de l'incontinence, des maladies auto-immunes et des cancers du poumon, du colon, du sein, de l'utérus et de la prostate.
